(19) Europäisches Patentamt
European Patent Office
Office européen des brevets

(11) EP 1 043 325 B1

(12) EUROPEAN PATENT SPECIFICATION

(45) Date of publication and mention of the grant of the patent:
**16.06.2004 Bulletin 2004/25**

(51) Int Cl.[7]: **C07D 491/04**, A61K 31/436

(21) Application number: **00113829.6**

(22) Date of filing: **13.12.1995**

# (54) Steroid receptor modulator compounds and methods

Steroidrezeptor-Modulator Verbindungen und Methoden

Composés modulateurs des récepteurs des stéroides et procédés d'utilisations

(84) Designated Contracting States:
**AT BE CH DE DK ES FR GB GR IE IT LI LU MC NL PT SE**

(30) Priority: **22.12.1994 US 363529**
**05.06.1995 US 464541**
**05.06.1995 US 463231**
**05.06.1995 US 464546**
**05.06.1995 US 465429**
**05.06.1995 US 464360**
**05.06.1995 US 462643**
**05.06.1995 US 465556**

(43) Date of publication of application:
**11.10.2000 Bulletin 2000/41**

(62) Document number(s) of the earlier application(s) in accordance with Art. 76 EPC:
**95944089.2 / 0 800 519**

(73) Proprietor: **LIGAND PHARMACEUTICALS INCORPORATED**
**San Diego, California 92121 (US)**

(72) Inventors:
- **Jones, Todd K.**
  **Solana Beach, CA 92075 (US)**
- **Edwards, James E.**
  **San Diego, CA 92129 (US)**
- **West, Sarah J.**
  **San Diego, CA 92126 (US)**
- **Tegley, Christopher M.**
  **Thousand Oaks, CA 91360 (US)**
- **Zhi, Lin**
  **San Diego, CA 92129 (US)**

(74) Representative:
**Winter, Brandl, Fürniss, Hübner, Röss, Kaiser, Polte Partnerschaft**
**Patent- und Rechtsanwaltskanzlei**
**Alois-Steinecker-Strasse 22**
**85354 Freising (DE)**

(56) References cited:
**EP-A- 0 260 744**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### Related Patent Applications

This application is a Continuation-In-Part of United States Patent Application Serial No. 08/363,529, filed December 22, 1994, the entire disclosure of which is herein incorporated by reference.

### Field of the Invention

This invention relates to non-steroidal compounds that are modulators (i.e. agonists and antagonists) of steroid receptors (e.g., progesterone receptor, androgen receptor, estrogen receptor, glucocorticoid receptor and mineralocorticoid receptor), and to methods for the making and use of such compounds.

### Background of the Invention

Intracellular receptors (IRs) form a class of structurally-related genetic regulators scientists have named "ligand dependent transcription factors." R.M. Evans, 240 *Science,* 889 (1988). Steroid receptors are a recognized subset of the IRs, including the progesterone receptor (PR), androgen receptor (AR), estrogen receptor (ER), glucocorticoid receptor (GR) and mineralocorticoid receptor (MR). Regulation of a gene by such factors requires both the IR itself and a corresponding ligand which has the ability to selectively bind to the IR in a way that affects gene transcription.

Ligands to the IRs can include low molecular weight native molecules, such as the hormones progesterone, estrogen and testosterone, as well as synthetic derivative compounds such as medroxyprogesterone acetate, diethylstilbesterol and 19-nortestosterone. These ligands, when present in the fluid surrounding a cell, pass through the outer cell membrane by passive diffusion and bind to specific IR proteins to create a ligand/receptor complex. This complex then translocates to the cell's nucleus, where it binds to a specific gene or genes present in the cell's DNA. Once bound to DNA, the complex modulates the production of the protein encoded by that gene. In this regard, a compound which binds an IR and mimics the effect of the native ligand is referred to as an "agonist", while a compound that inhibits the effect of the native ligand is called an "antagonist."

Ligands to the steroid receptors are known to play an important role in health of both women and men. For example, the native female ligand, progesterone, as well as synthetic analogues, such as norgestrel (18-homonorethisterone) and norethisterone (17$\alpha$-ethinyl-19-nortestosterone), are used in birth control formulations, typically in combination with the female hormone estrogen or synthetic estrogen analogues, as effective modulators of both PR and ER. On the other hand, antagonists to PR are potentially useful in treating chronic disorders, such as certain hormone dependent cancers of the breast, ovaries, and uterus, and in treating non-malignant conditions such as uterine fibroids and endometriosis, a leading cause of infertility in women. Similarly, AR antagonists, such as cyproterone acetate and flutamide have proved useful in the treatment of hyperplasia and cancer of the prostate.

The effectiveness of known modulators of steroid receptors is often tempered by their undesired side-effect profile, particularly during long-term administration. For example, the effectiveness of progesterone and estrogen agonists, such as norgestrel and diethylstilbesterol respectively, as female birth control agents must be weighed against the increased risk of breast cancer and heart disease to women taking such agents. Similarly, the progesterone antagonist, mifepristone (RU486), if administered for chronic indications, such as uterine fibroids, endometriosis and certain hormone-dependent cancers, could lead to homeostatic imbalances in a patient due to its inherent cross-reactivity as a GR antagonist. Accordingly, identification of compounds which have good specificity for one or more steroid receptors, but which have reduced or no cross-reactivity for other steroid or intracellular receptors, would be of significant value in the treatment of male and female hormone responsive diseases.

A group of quinoline analogs having an adjacent polynucleic ring system of the indene or fluorene series or an adjacent polynucleic heterocyclic ring system with substituents having a nonionic character have been described as photoconductive reducing agents, stabilizers, laser dyes and antioxidants. See e.g., U.S. Patent Nos. 3,798,031; 3,830,647; 3,832,171; 3,928,686; 3,979,394; 4,943,502 and 5,147,844 as well as Soviet Patent No. 555,119; R.L. Atkins and D.E. Bliss, "Substituted Coumarins and Azacoumarins: Synthesis and Fluorescent Properties", 43 *J. Org. Chem*., 1975 (1978), E.R. Bissell et al., "Synthesis and Chemistry of 7-Amino-4-(trifluoromethyl)coumarin and Its Amino Acid and Peptide Derivatives", 45 *J. Org. Chem*., 2283 (1980) and G.N. Gromova and K.B. Piotrovskii, "Relative Volatility of Stabilizers for Polymer Materials," 43 *Khim. Prom-st*., 97 (Moscow, 1967). However, no biological activity of any kind has been ascribed to these compounds.

### Summary of the Invention

The present invention is directed to compounds, pharmaceutical compositions, and methods for modulating

processes mediated by steroid receptors. More particularly, the invention relates to non-steroidal compounds and compositions which are high affinity, high specificity agonists, partial agonists and antagonists for the PR, AR, ER, GR and MR steroid receptors, as well as to compounds with combined activity on one or more of these receptors. Also provided are methods of making such compounds and pharmaceutical compositions, as well as critical intermediates used in their synthesis.

These and various other advantages and features of novelty which characterize the invention are pointed out with particularity in the claims annexed hereto and forming a part hereof. However, for a better understanding of the invention, its advantages, and objects obtained by its use, reference should be had to the accompanying drawings and descriptive matter, in which there is illustrated and described preferred embodiments of the invention.

## Definitions and Nomenclature

As used herein, the following terms are defined with the following meanings, unless explicitly stated otherwise. Furthermore, in an effort to maintain consistency in the naming of compounds of similar structure but differing substituents, the compounds described herein are named according to the following general guidelines. The numbering system for the location of substituents on such compounds is also provided.

The term alkyl, alkenyl, alkynyl and allyl includes straight-chain, branched-chain, cyclic, saturated and/or unsaturated structures, and combinations thereof.

The term aryl refers to an optionally substituted six-membered aromatic ring, including polyaromatic rings.

The term heteroaryl refers to an optionally substituted five-membered heterocyclic ring containing one or more heteroatoms selected from the group consisting of carbon, oxygen, nitrogen and sulfur, including polycyclic rings, or a six-membered heterocyclic ring containing one or more heteroatoms selected from the group consisting of carbon and nitrogen, including polycyclic rings.

A **quinoline** is defined by the following structure, and may be recognized as a benzannulated pyridine. Compounds of structures **4, 5, 13, 79, 83** and **86** herein are named as quinolines.

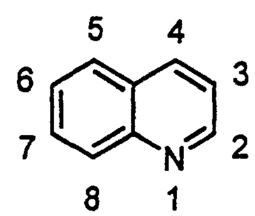

An **indeno[1,2-*g*]quinoline** is defined by the following structure. Compounds of structures **16** (X=C) and **20** herein are named as indeno[1,2-*g*]quinolines.

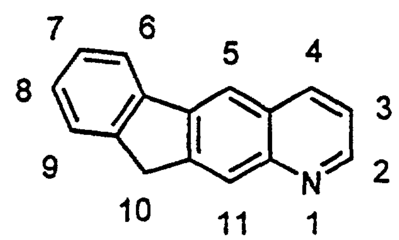

An **indeno[2,1-*f*]quinoline** is defined by the following structure. Compounds of structure **17** (X=C) herein are named as indeno[2,1-*f*]quinolines.

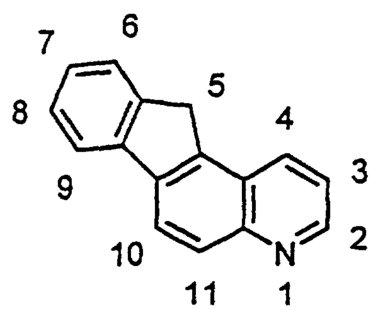

A **benzo[b]furano[3,2-*g*]quinoline** is defined by the following structure. Compounds of structure **16** (X=O) herein are named as benzo[b]furano[3,2-*g*]quinolines.

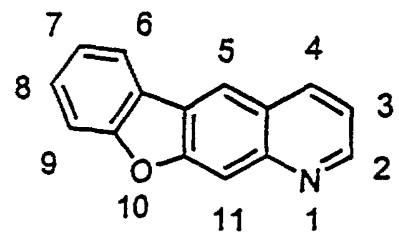

A **benzo[b]furano[2,3-*f*]quinoline** is defined by the following structure. Compounds of structure **17** (X=O) herein are named as benzo[b]furano[2,3-*f*]quinolines.

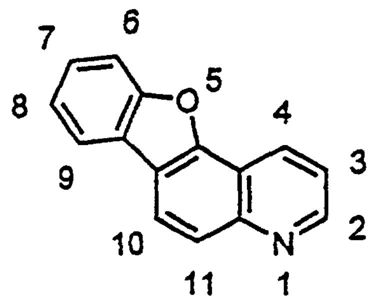

An **indolo[3,2-*g*]quinoline** is defined by the following structure. Compounds of structure **16** (X=N) herein are named as indolo[3,2-*g*]quinolines.

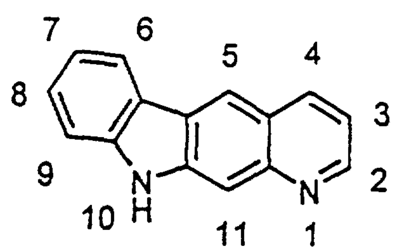

An **indolo[2,3-*f*]quinoline** is defined by the following structure. Compounds of structures **17** (X=N) and **29** herein are named as indolo[2,3-*f*]quinolines.

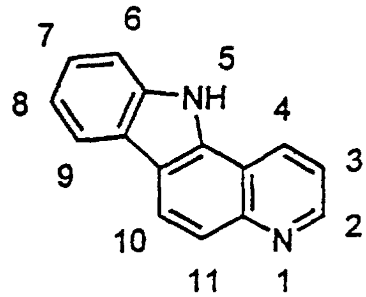

A **coumarino[3,4-*f*]quinoline** is defined by the following structure. Compound **159** and compounds of structures **41** and **88** herein are named as coumarino[3,4-*f*]quinolines.

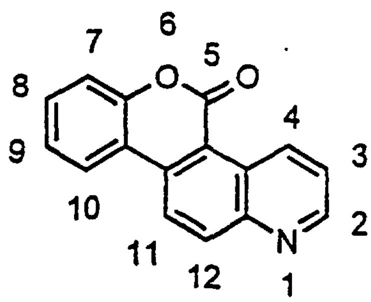

A ***5H*-chromeno[3,4-*f*]quinoline** is defined by the following structure. Compounds of structures **34, 35, 42, 45 to 54, 93, 95, 97 to 99, 1A, 4A, 7A to 11A, 17A to 19A and 25A to 27A** herein are named as *5H*-chromeno[3,4-*f*] quinolines.

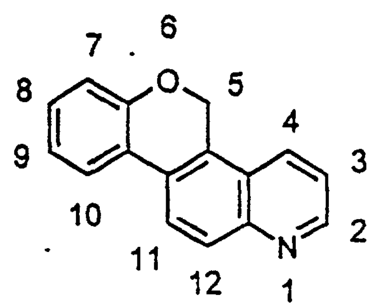

An **8-pyranono[5,6-*g*]quinoline** is defined by the following structure. Compounds of structures **57** (Z=O), **60** (Z=O), **63** (Z=O), **69** (Z=O), **73** (Z=O), **28A** (Z=O) **33A, 34A, 37A** (X=O), **38A** (X=O), **40A** (X=O), **41A** (X=O), **45A, 65A** (X=O) and **67A** (X=O) herein are named as 8-pyranono[5,6-*g*]quinolines.

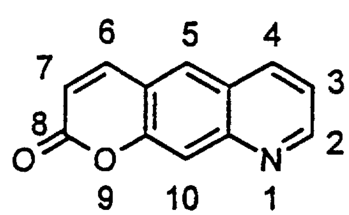

A **10-isocoumarino[4,3-*g*]quinoline** is defined by the following structure. Compounds of structures **57**

($R^2$=$R^3$=benzo, Z=O), **60** ($R^2$=$R^3$=benzo, Z=O), and **63** ($R^2$=$R^3$=benzo, Z=O),herein are named as 10-isocoumarino [4,3-*g*]quinolines.

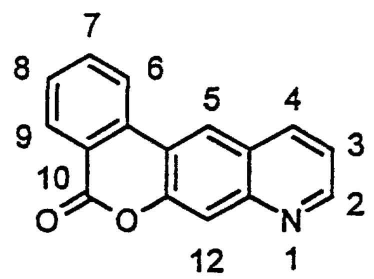

A **10-isoquinolino[4,3-*g*]quinoline** is defined by the following structure. Compounds of structures**57** ($R^2$=$R^3$=benzo, Z=NH), **60** ($R^2$=$R^3$=benzo, Z=NH), and **63** ($R^2$=$R^3$=benzo, Z=NH), herein are named as 10-isoquinolino [4,3-*g*]quinolines.

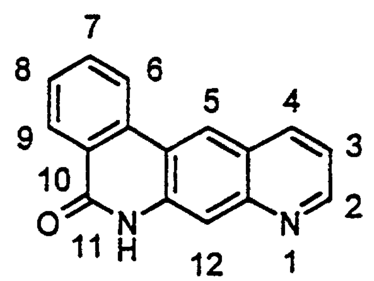

An **8-pyridono[5,6-*g*]quinoline** is defined by the following structure. Compounds of structures **57** (Z=N), **60** (Z=N), **63** (Z=N), **69** (Z=N), **73** (Z=N), **28A** (Z=N), **37A** (X=N), **38A** (X=N), **40A** (X=N), **41A** (X=N), **47A, 53A, 62A, 63A, 65A** (X=N), **67A.** (X=N), **70A, 72A, 74A, 79A, 80A, 81A and 84A** herein are named as 8-pyridono[5,6-*g*]quinolines.

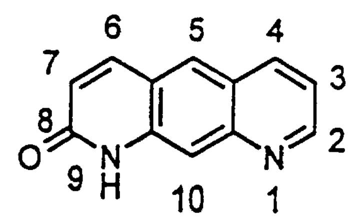

A **10*H*-isochromeno[4,3-*g*]quinoline** is defined by the following structure. Compounds of structures **61** ($R^2$=$R^3$=benzo, Z=O),and **62** ($R^2$=$R^3$=benzo, Z=O)herein are named as 10*H*-isochromeno[4,3-*g*]quinolines.

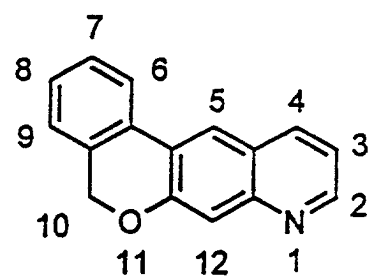

An **8*H*-pyrano[3,2-*g*]quinoline** is defined by the following structure. Compounds of structures **61** (Z=O) and

**62** (Z=O) herein are named as 8*H*-pyrano[3,2-*g*]quinolines.

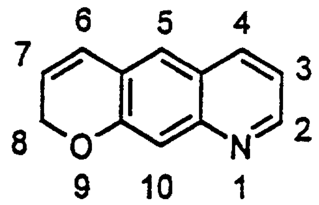

A **10-thioisoquinolino[4,3-*g*]quinoline** is defined by the following structure. Compounds of structures **58** ($R^2$=$R^3$=benzo, Z=NH)and **76** ($R^2$=$R^3$=benzo, Z=NH)herein are named as 10-thioisoquinolino[4,3-*g*]quinolines.

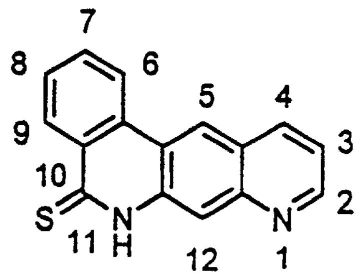

A 9-**pyrido[3,2-*g*]quinoline** is defined by the following structure. Compounds of structures **71** (Z=N) and **75** (Z=N) herein are named as 9-pyrido[3,2-*g*]quinolines.

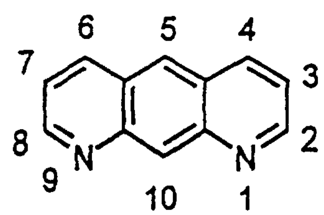

An **8-thiopyranono[5,6-*g*]quinoline** is defined by the following structure. Compounds of structures **58** (Z=O), **76** (Z=O) and **29A** (Z=O) herein are named as 8-thiopyranono[5,6-*g*]quinolines.

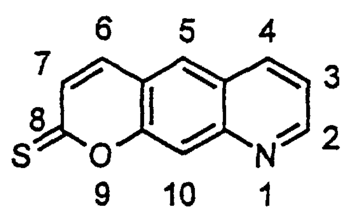

An **6-pyridono[5,6-*g*]quinoline** is defined by the following structure. Compounds of structures **70** (Z=N) and **74** (Z=N) herein are named as 6-pyridono[5,6-*g*]quinolines.

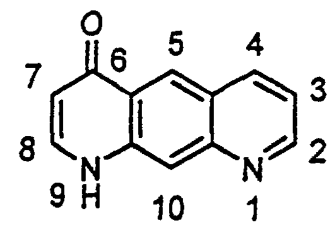

A **9-thiopyran-8-ono[5,6-*g*]quinoline** is defined by the following structure. Compounds of structure **57** (Z=S), **28A** (Z=S), **37A** (X=S), **38A** (X=S), **40A** (X=S), **41A** (X=S), **65A** (X=S) and **67A** (X=S) herein are named as 9-thiopyran-8-ono[5,6-*g*]quinolines.

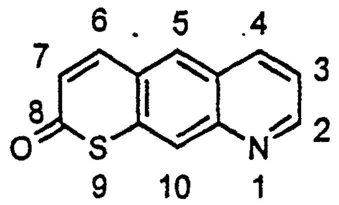

An **7-pyridono[5,6-*f*]indoline** is defined by the following structure. Compounds of structures **49A, 50A, 57A,** and **83A** are named as 7-pyridono[5,6-*f*]indolines.

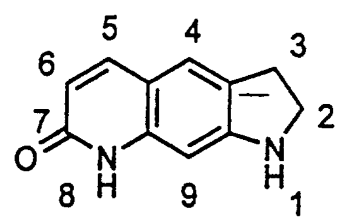

An **5H-isochromeno[3,4-*f*]quinoline** is defined by the following structure. Compounds of structures **22A, 23A and 24A** are named as 5H-isochromeno[3,4-*f*]quinolines.

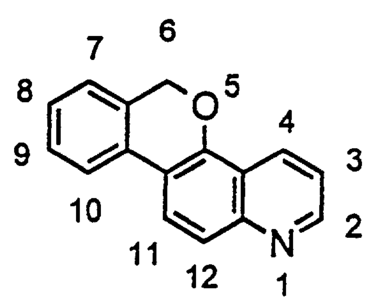

## Detailed Description of Embodiments of the Invention

Compounds of the present invention are defined as those having the formulae:

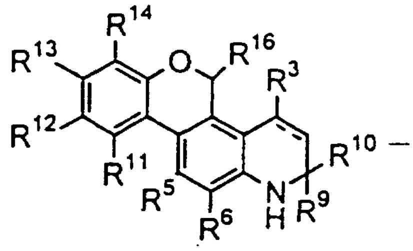

(V)

OR

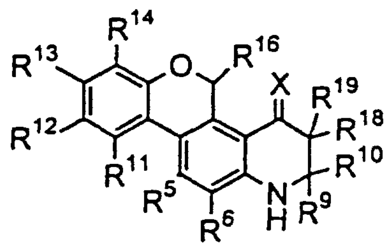

(VI)

OR

wherein:

$R^3$ is hydrogen or methyl
$R^5$ and $R^6$ each are hydrogen
$R^9$ and $R^{10}$ each are methyl
$R^{11}$ through $R^{14}$ each independently are hydrogen, F, Cl, Br, I, $COR^2$, $CF_3$, a $C_1$-$C_4$ alkyl or $OR^2$, where $R^2$, is hydrogen, $C_1$-$C_4$ alkyl or $CF_3$;
X is $CH_2$ or O;
$R^{16}$ is hydrogen, OH, $OR^{17}$, $SR^{17}$, $C_1$ - $C_6$ alkyl, $C_2$-$C_6$ alkenyl, $C_2$-$C_6$ alkynyl, phenylmethyl, phenyl optionally substituted with $C_1$-$C_4$ alkyl, F, Cl, Br, I, $CF_3$, $C_1$-$C_4$ alkoxyl, or dimethyl amino; pyridyl optionally substituted with methyl, F, Cl, Br, $CF_3$, or $C_1$-$C_4$ alkoxy; furanyl optionally substituted with $C_1$-$C_4$ alkyl or $C_1$-$C_4$ alkoxy; thienyl optionally substituted with $C_1$-$C_4$ alkyl or $C_1$-$C_4$ alkoxy; pyrrolyl optionally substituted with $C_1$-$C_4$ alkyl; 1-triazolyl; 2-benzofuranyl; or 2-benzothienyl; where $R^{17}$ is a $C_1$-$C_4$ alkyl or haloalkyl group;

and

the dotted lines in the structures depict optional double bonds.

Preferably, the compounds of formulae V and VI comprise PR modulators (i.e. both PR agonists and antagonists)

In a preferred aspect, the present invention provides a pharmaceutical composition comprising an effective amount of a steroid receptor modulating compound of the formulae:

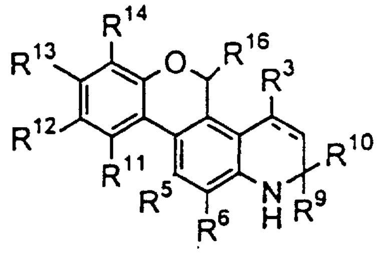

(V)

OR

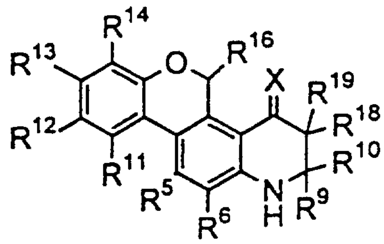

(VI)

wherein:

$R^3$ is hydrogen, or methyl;
$R^5$ through $R^6$ are hydrogen
$R^9$ and $R^{10}$ methyl
$R^{11}$ through $R^{14}$ each independently are hydrogen, F, Cl, Br, I, $COR^2$, $CF_3$, a $C_1$-$C_4$ alkyl, $OR^2$, where $R^2$ is hydrogen, $C_1$-$C_4$ alkyl or $CF_3$,
X is $CH_2$ or O;
$R^{16}$ is hydrogen, OH, $OR^{17}$, $SR^{17}$, $C_1$ - $C_6$ alkyl, $C_2$-$C_6$ alkenyl, $C_2$-$C_6$ alkynyl, phenylmethyl, phenyl optionally substituted with $C_1$-$C_4$ alkyl, F, Cl, Br, I, $CF_3$, $C_1$-$C_4$ alkoxyl, or dimethyl amino; pyridyl optionally substituted with methyl, F, Cl, Br, $CF_3$, or $C_1$-$C_4$ alkoxy; furanyl optionally substituted with $C_1$-$C_4$ alkyl or $C_1$-$C_4$ alkoxy; thienyl optionally substituted with $C_1$-$C_4$ alkyl or $C_1$-$C_4$ alkoxy; pyrrolyl optionally substituted with $C_1$-$C_4$ alkyl; 1-triazolyl; 2-benzofuranyl; or 2-benzothienyl; where $R^{17}$ is a $C_1$-$C_4$ alkyl or haloalkyl group;
$R^{18}$ and $R^{19}$ are both hydrogen or both methyl;

the dotted lines in the structures depict optional double bonds; and
a pharmaceutically acceptable carrier.

Preferably, the compounds of formulae V and VI comprise PR modulators (i.e. both PR agonists and antagonists)

In a further preferred aspect, the present invention comprises a method of modulating processes mediated by steroid receptors comprising administering to a patient an effective amount of a compound of the formulae V through VI shown above, wherein $R^2$ through $R^{19}$ and X, all have the same definitions as those given above for the preferred pharmaceutical composition of the present invention.

Any of the compounds of the present invention can be synthesized as pharmaceutically acceptable salts for incorporation into various pharmaceutical compositions. As used herein, pharmaceutically acceptable salts include, but are not limited to, hydrochloric, hydrobromic, hydroiodic, hydrofluoric, sulfuric, citric, maleic, acetic, lactic, nicotinic, succinic, oxalic, phosphoric, malonic, salicylic, phenylacetic, stearic, pyridine, ammonium, piperazine, diethylamine, nicotinamide, formic, urea, sodium, potassium, calcium, magnesium, zinc, lithium, cinnamic, methylamino, methanesulfonic, picric, tartaric, triethylamino, dimethylamino, and tris(hydoxymethyl)aminomethane. Additional pharmaceutically acceptable salts are known to those skilled in the art.

The PR agonist, partial agonist and antagonist compounds of the present invention are particularly useful for female hormone replacement therapy and as modulators of fertility (e.g., as contraceptives, contragestational agents or abortifacients), either alone or in conjunction with ER modulators. The PR active compounds are also useful in the treatment of dysfunctional uterine bleeding, dysmenorrhea, endometriosis, leiomyomas (uterine fibroids), hot flashes, mood disorders, meningiomas as well as in various hormone-dependent cancers, including, without limitation, cancers of the ovaries, breast, endometrium and prostate.

It will be understood by those skilled in the art that while the compounds of the present invention will typically be employed as a selective agonists, partial agonists or antagonists, that there may be instances where a compound with a mixed steroid receptor profile is preferred. For example, use of a PR agonist (i.e., progestin) in female contraception often leads to the undesired effects of increased water retention and acne flare ups. In this instance, a compound that is primarily a PR agonist, but also displays some AR and MR modulating activity, may prove useful. Specifically, the mixed MR effects would be useful to control water balance in the body, while the AR effects would help to control any acne flare ups that occur.

Furthermore, it will be understood by those skilled in the art that the compounds of the present invention, including pharmaceutical compositions and formulations containing these compounds, can be used in a wide variety of combination therapies to treat the conditions and diseases described above. Thus, the compounds of the present invention can be used in combination with other hormones and other therapies, including, without limitation, chemotherapeutic agents such as cytostatic and cytotoxic agents, immunological modifiers such as interferons, interleukins, growth hormones and other cytokines, hormone therapies, surgery and radiation therapy.

Representative PR antagonist compounds include: 1,2,3,4-Tetrahydro-2,2,4-trimethyl-6-phenylquinoline (Compound 100); 1,2-Dihydro-2,2,4-trimethyl-6-(1,2,3-thiadiazol-5-yl)quinoline (Compound 101); 1,2-Dihydro-2,2,4-trimethyl-6-(1,3-oxazol-5-yl)quinoline (Compound 102); 6-(4,5-Dichloroimidazol-1-yl)-1,2-dihydro-2,2,4-trimethylquinoline (Compound 103); 6-(4-Bromo-1-methylpyrazol-3-yl)-1,2-dihydro-2,2,4-trimethylquinoline (Compound 104); 1,2-Dihydro-2,2,4-trimethyl-6-(3-pyridyl)quinoline (Compound 105); 6-(4-Fluorophenyl)-1,2,-dihydro-2,2,4-trimethylquinoline (Compound 106); 1,2-Dihydro-6-(3-trifluoromethylphenyl)-2,2,4-trimethylquinoline (Compound 107); 1,2-Dihydro-2,2,4-trimethyl-6-(4-nitrophenyl)quinoline (Compound 108); 6-(2,3-Dichlorophenyl)-1,2-dihydro-2,2,4-trimethylquinoline (Compound 109); 1,2-Dihydro-6-(2-hydroxycarbonyl-4-nitrophenyl)-2,2,4-trimethylquinoline (Compound 110); 6-(3,4-Dichlorophenyl)-1,2-dihydro-2,2,4-trimethylquinoline (Compound 111); 4-Ethyl-1,2-dihydro-2,2-dimethyl-6-phenylquinoline (Compound 112); 1,2-Dihydro-2,2-dimethyl-6-phenyl-4-propylquinoline (Compound 113); 6-(2-Chlorophenyl)-1,2-dihydro-2,2,4-trimethylquinoline (Compound 114); 1,2-Dihydro-2,2,4-trimethylindeno[1,2-*g*]quinoline (Compound 115); 1,2-Dihydro-2,2,4-trimethylindeno[2, 1-*f*]quinoline (Compound 116); 8-Bromo-1,2-dihydro-2,2,4-trimethylindeno[1,2-*g*]quinoline (Compound 117); 1,2-Dihydro-2,2,4-trimethylbenzo[b]furano[3,2,*g*]quinoline (Compound 118); 1,2-Dihydro-2,2,4-trimethylbenzo[b]furano[2,3-*f*]quinoline (Compound 119); 6-Fluoro-1,2-dihydro-2,2,4-trimethylindeno[2,1-*f*]quinoline (Compound 120); 9-Fluoro-1,2-dihydro-2,2,4-trimethylindeno[1,2-*g*]quinoline (Compound 121); 1,2-Dihydro-9-hydroxylmethyl-2,2,4-trimethylindeno[1,2-*g*]quinoline (Compound 122); 8-Chloro-1,2-dihydro-2,2,4-trimethylindeno[1,2-*g*]quinoline (Compound 123); 8-Fluoro-1,2-dihydro-2,2,4-trimethylindeno[1,2-*g*]quinoline (Compound 124); 8-Acetyl-1,2-dihydro-2,2,4-trimethylindeno[1,2-g]quinoline (Compound 125); 6-Fluoro-1,2-dihydro-2,2,4-trimethylindeno[1,2-*g*]quinoline (Compound 126); 7-Bromo-1,2-dihydro-2,2,4-trimethylindeno[2,1-*f*]quinoline (Compound 127); 1,2-Dihydro-2,2,4-trimethyl-7-nitroindeno[2,1-*f*]quinoline (Compound 128); 1,2-Dihydro-2,2,4-trimethyl-8-nitroindeno[1,2-*g*]quinoline (Compound 129); 6,9-Difluoro-1,2-dihydro-2,2,4-trimethylindeno[1,2-*g*]quinoline (Compound 130); 7-Fluoro-1,2-dihydro-2,2,4-trimethyl-11-(thiomethyl)indeno[2,1-*f*]quinoline (Compound 131); 5,8-Difluoro-1,2-dihydro-10-hydroxy-2,2,4-trimethylindeno[1,2-*g*]quinoline (Compound 132); 7,9-Difluoro-1,2-dihydro-10-hydroxy-2,2,4-trimethylindeno[1,2-*g*]quinoline (Compound 133); 7,10-Difluoro-1,2-dihydro-2,2,4-trimethyl-5-oxoindeno[3,2-*f*]quinoline (Compound 134); 7,9-Difluoro-1,2-dihydro-2,2,4-trimethyl-10-oxoindeno[1,2-*g*]quinoline (Compound 135); 8-Fluoro-1,2-dihydro-10-hydroxy-2,2,4-trimethylindeno[1,2-*g*]quinoline (Compound 136); 8-Fluoro-1,2-dihydro-2,2,4-trimethyl-10-oxoindeno[1,2-*g*]quinoline (Compound 137); 7-Fluoro-1,2-dihydro-2,2,4-trimethyl-8-nitroindeno[1,2-*g*]quinoline (Compound 138); 5-Chloro-1,2-dihydro-10-hydroxy-2,2,4-trimethylindeno[1,2-*g*]quinoline (Compound 139); 6-Fluoro-1,2-dihydro-2,2,4-trimethyl-10-oxoindeno[1,2-*g*]quinoline (Compound 140); 6-Fluoro-1,2-dihydro-10-hydroxy-2,2,4-trimethylindeno[1,2-*g*]quinoline (Compound 141); 5,8-Difluoro-1,2-dihydro-2,2,4-trimethyl-10-(trifluoroacetoxy)indeno[1,2-*g*]quinoline (Compound 142); 6-(3,5-Difluorophenyl)-1,2,3,4-tetrahydro-2,2,4-trimethylquinoline (Compound 143); 1,2-Dihydro-2,2,4-trimethylindolo[3,2-*g*]quinoline (Compound 144); 5-Ethyl-1,2-dihydro-2,2,4-trimethylindolo[2,3-*f*]quinoline (Compound 145); 6-(3-Chlorophenyl)-1,2-dihydro-2,2,4-trimethylquinoline (Compound 146); 6-(3,5-Difluorophenyl)-1,2-dihydro-2,2,4-trimethylquinoline (Compound 147); 6-(3-Fluorophenyl)-1,2-dihydro-2,2,4-trimethylquinoline (Compound 148); 1,2-Dihydro-2,2,4-trimethyl-6-(4-pyridyl)quinoline (Compound 149); 6-(3-Cyanophenyl)-1,2-dihydro-2,2,4-trimethylquinoline (Compound 150); 6-(3,5-Dichlorophenyl)-1,2-dihydro-2,2,4-trimethylquinoline (Compound 151); 6-(2,3-Difluorophenyl)-1,2-dihydro-2,2,4-trimethylquinoline (Compound 152);1,2-Dihydro-2,2,4-trimethyl-6-(pentafluorophenyl)quinoline(Compound153);1,2-Dihydro-2,2,4-trimethyl-6-[4-(trifluoroacetyl)phenyl]quinoline (Compound 154); 1,2-Dihydro-2,2,4-trimethyl-6-(1,3-pyrimid-5-yl)quinoline (Compound 155); 6-(3-Cyanophenyl)-1,2,3,4-tetrahydro-2,2,4-trimethylquinoline (Compound 156); 5,8-Difluoro-1,2-dihydro-2,2,4-trimethylindeno[1,2-*g*]quinoline (Compound 157); 7,10-Difluoro-1,2-dihydro-2,2,4-trimethylindeno[2,1-*f*]quinoline (Compound 158); 8-Cyano-1,2-dihydro-2,2,4-trimethylindeno[3,2-*e*]quinoline (Compound 270); 6-(3-Cyano-5-fluorophenyl)-1,2-dihydro-2,2,4-trimethylquinoline (Compound 271); 6-(3-Cyano-4-fluorophenyl)-1,2-dihydro-2,2,4-trimethylquinoline (Compound 272); 6-(3-Cyano-6-fluorophenyl)-1,2-dihydro-2,2,4-trimethylquinoline (Compound 273); 6-[5-fluoro-3-(trifluoromethyl)phenyl]-1,2-dihydro-2,2,4-trimethylquinoline (Compound 274); 6-(3-chloro-2-methylphenyl)-1,2-dihydro-2,2,4-trimethylquinoline (Compound 275); 1,2-Dihydro-2,2,4-trimethyl-6-(3-nitrophenyl)quinoline (Compound 276); 6-(3-Acetylphenyl)-1,2-dihydro-2,2,4-trimethylquinoline (Compound 277); 6-(3-cyano-2-methylphenyl)-1,2-dihydro-2,2,4-trimethylquinoline (Compound 278); 1,2-Dihydro-2,2,4-trimethyl-6-(3-methylphenyl)quinoline (Compound 279); 6-(5-Fluoro-3-nitrophenyl)-1,2-dihydro-2,2,4-trimethylquinoline (Compound 280); 1,2-Dihydro-6-(3-methoxyphenyl)-2,2,4-trimethylquinoline (Compound 281); 6-(5-Cyano-3-pyridyl)-1,2-dihydro-2,2,4-trimethylquinoline (Compound 282); 1,2-Dihydro-2,2,4-trimethyl-6-(2-methyl-3-nitrophenyl)quinoline (Compound 283); 6-(2-Amino-3,5-difluorophenyl)-1,2-dihydro-2,2,4-trimethylquinoline (Compound 284); 6-(3-Bromo-2-chloro-5-fluorophenyl)-1,2-dihydro-2,2,4-trimethylquinoline (Compound 285); 6-(3-Cyano-5-fluorophenyl)-1,2-dihydro-2,2,4-trimethyl-3-quinolone (Compound 286); 6-(3-Fluoro-2-methylphenyl)-1,2-dihydro-2,2,4-trimethylquinoline (Compound 287); 1,2-Dihydro-2,2,4-trimethyl-6-(3-methylthiophenyl)quinoline (Compound 288); 6-(5-Chloro-2-thienyl)-1,2-dihydro-2,2,4-trimethylquinoline (Compound 289); 1,2-Dihydro-2,2,4-trimethyl-6-(3-methyl-2-thienyl)quinoline (Compound 290); 8-Fluoro-1,2-dihydro-2,2,4-trimethyl-6-(3-nitrophenyl)quinoline (Compound 291); 1,2-Dihydro-6-(3-nitrophenyl)-2,2,4,8-tetramethylquinoline (Compound 292); 6-(5-Bromo-3-pyridyl)-1,2-dihydro-2,2,4-trimethylquinoline (Compound 293); 6-(3-Bromo-2-pyridyl)-1,2-dihydro-2,2,4-trimethylquinoline (Compound 294); 6-(3-Bromo-2-thienyl)-1,2-dihydro-2,2,4-trimethylquinoline (Compound 295); 1,2-Dihydro-6-(2,3,5,6-tetrafluoro-4-pyridyl)-2,2,4-trimethylquinoline (Compound 296); 5,8-Difluoro-1,2-dihydro-6-(3-nitrophenyl)-2,2,4-trimethylquinoline (Compound 297); 2,4-Diethyl-8-fluoro-1,2-dihydro-2-methyl-6-(3-nitrophenyl)quinoline (Compound 298); 6-(3-Bromophenyl)-1,2-dihydro-2,2,4-trimethylquinoline (Compound 299); 1,2-Dihydro-2,2,4-trimethyl-6-(5-nitro-2-thienyl)quinoline (Compound 300); 1,2-Dihydro-6-(2,4,5-trifluorophenyl)-2,2,4-trimethylquinoline (Compound 301); 6-(3-Bromo-5-fluorophenyl)-1,2-dihydro-2,2,4-trimethylquinoline (Compound 302); 6-(5-Carboxaldehyde-3-thienyl)-1,2-dihydro-2,2,4-trimethylquinoline (Compound 303); 1,2-Dihydro-2,2,4,7-tetramethyl-6-(3-nitrophenyl)quinoline (Compound 304); 6-(5-Fluoro-2-methoxy-3-nitrophenyl)-1,2-dihydro-2,2,4-trimethylquinoline (Compound 305); 6-(3-Chloro-2-methoxyphenyl)-1,2-dihydro-2,2,4-trimethylquinoline (Compound 306); 1,2-Dihydro-2,2,4-trimethyl-6-(2,3,4-trifluorophenyl)quinoline (Compound 307); 6-(3-Bromo-2-methylphenyl)-1,2-dihydro-2,2,4-trimethylquinoline (Compound 308); 7-Chloro-1,2-dihydro-2,2,4-trimethyl-6-(3-nitrophenyl)quinoline (Compound 309); 5-Chloro-1,2-dihydro-2,2,4-trimethyl-6-(3-nitrophenyl)quinoline (Compound 310); 8-Chloro-1,2-dihydro-2,2,4-trimethyl-6-(3-nitrophenyl)quinoline (Compound 311); 8-Ethyl-1,2-dihydro-2,2,4-trimethyl-6-(3-nitrophenyl)quinoline (Compound 312); 9-Chloro-1,2-dihydro-2,2-dimethyl-5-coumarino[3,4-*f*]quinoline (Compound 313); 1,2-Dihydro-9-methoxy-2,2,4-trimethyl-5-coumarino[3,4-*f*]quinoline (Compound 314); 9-Fluoro-1,2-dihydro-2,2,4,11-tetramethyl-5-coumarino[3,4-*f*]quinoline (Compound 315); 1,2-Dihydro-2,2,4,9-tetramethyl-5-coumarino[3,4-*f*]quinoline (Compound 316); 7-Chloro-1,2-dihydro-2,2,4-trimethyl-5-coumarino[3,4-*f*]quinoline (Compound 317); (*R*/*S*)-9-Chloro-1,2-dihydro-5-methoxy-2,2,4-trimethyl-5*H*-chromeno[3,4-*f*]quinoline (Compound 319); (*R*/*S*)-9-Fluoro-1,2-dihydro-2,2,4-trimethyl-5*H*-chromeno[3,4-*f*]quinoline (Compound 328); 6-(5-Cyano-2-thienyl)-1,2-dihydro-2,2,4-trimethylquinoline (Compound 451); 6-(5-Cyano-3-thienyl)-1,2-dihydro-2,2,4-trimethylquinoline (Compound 452); 6-(3-Formylphenyl)-1,2-dihydro-2,2,4-trimethylquinoline (Compound 453); 1,2-Dihydro-2,2,4-trimethyl-6-[3-(methylsulfonyl)phenyl]quinoline (Compound 454); (*R*/*S*)-6-(3-Cyano-5-fluorophenyl)-1,2,3,4-Tetrahydro-2,2,4-trimethylquinoline (Compound 455); and (*R*/*S*)-9-Chloro-1,2-dihydro-2,2,4-trimethyl-5-phenyl-5*H*-chromeno[3,4-*f*]quinoline (Compound 456).

Representative PR modulator compounds (i.e., agonists and antagonists) according to the present invention include: (*R*/*S*)-5-Butyl-1,2-dihydro-2,2,4-trimethyl-*5H*-chromeno[3,4-*f*]quinoline (Compound 160); (*R*/*S*)-1,2-Dihydro-2,2,4-trimethyl-5-phenyl-*5H*-chromeno[3,4-*f*]quinoline (Compound 161); (*R*/*S*)-1,2,3,4-Tetrahydro-2,2-dimethyl-4-methylidene-5-phenyl-*5H*-chromeno[3,4-*f*]quinoline (Compound 162); (*R*/*S*)-5-(4-Chlorophenyl)-1,2-dihydro-2,2,4-trimethyl-*5H*-chromeno[3,4-*f*]quinoline (Compound 163); (*R*/*S*)-5-(4-Chlorophenyl)-1,2,3,4-tetrahydro-2,2-dimethyl-4-methylidene-*5H*-chromeno[3,4-*f*]quinoline (Compound 164); (*R*/*S*)-5-(4-Fluorophenyl)-1,2-dihydro-2,2,4-trimethyl-*5H*-chromeno[3,4-*f*]quinoline (Compound 165); (*R*/*S*)-5-(4-Acetylphenyl)-1,2-dihydro-2,2,4-trimethyl-*5H*-chromeno[3,4-*f*]quinoline (Compound 166); (*R*/*S*)-1,2-Dihydro-2,2,4-trimethyl-5-(4-methylphenyl)-*5H*-chromeno[3,4-*f*]quinoline (Compound 167); (*R*/*S*)-1,2-Dihydro-5-(4-methoxyphenyl)-2,2,4-trimethyl-*5H*-chromeno[3,4-*f*]quinoline (Compound 168); (*R*/*S*)-1,2-Dihydro-2,2,4-trimethyl-5-[4-(trifluoromethyl)phenyl]-*5H*-chromeno[3,4-*f*]quinoline (Compound 169); (*R*/*S*)-1,2-Dihydro-2,2,4-trimethyl-5-(thiophen-3-yl)-*5H*-chromeno[3,4-*f*]quinoline (Compound 170); (-)-1,2-Dihydro-2,2,4-trimethyl-5-(4-methylphenyl)-*5H*-chromeno[3,4-*f*]quinoline (Compound 171); (-)-5-(4-Chlorophenyl)-1,2-dihydro-2,2,4-trimethyl-*5H*-chromeno[3,4-*f*]quinoline (Compound 172); (*R*/*S*)-1,2-Dihydro-2,2,4-trimethyl-5-(3-methylphenyl)-*5H*-chromeno[3,4-*f*]quinoline (Compound 173); (+)-(4*l*,5*l*)-5-(4-Chlorophenyl)-1,2,3,4-tetrahydro-2,2,4-trimethyl-*5H*-chromeno[3,4-*f*]quinoline (Compound 174); (-)-(4*l*,5*l*)-5-(4-Chlorophenyl)-1,2,3,4-tetrahydro-2,2,4-trimethyl-*5H*-chromeno[3,4-*f*]quinoline (Compound 175); (*R*/*S*-4*l*,5*u*)-5-(4-Chlorophenyl)-1,2,3,4-tetrahydro-2,2,4-trimethyl-*5H*-chromeno[3,4-*f*]quinoline (Compound 176); (*R*/*S*)-5-(3-Chlorophenyl)-1,2-dihydro-2,2,4-trimethyl-*5H*-chromeno[3,4-*f*]quinoline (Compound 177); (*R*/*S*)-5-(3-Chlorophenyl)-1,2,3,4-tetrahydro-2,2-dimethyl-4-methylidene-*5H*-chromeno[3,4-*f*]quinoline (Compound 178); (*R*/*S*)-5-(4-Bromophenyl)-1,2-dihydro-2,2,4-trimethyl-*5H*-chromeno[3,4-*f*]quinoline (Compound 179); (*R*/*S*)-5-(4-Bromophenyl)-1,2,3,4-tetrahydro-2,2-dimethyl-4-methylidene-*5H*-chromeno[3,4-*f*]quinoline (Compound 180); (*R*/*S*)-5-(3-Bromophenyl)-1, 2-dihydro-2,2,4-trimethyl-*5H*-chromeno [3,4-*f*]quinoline (Compound 181); (*R*/*S*)-5-(3-Bromophenyl)-1,2,3,4-tetrahydro-2,2-dimethyl-4-methylidene-*5H*-chromeno[3,4-*f*]quinoline (Compound 182); (*R*/*S*)-5-(3,4-Dichlorophenyl)-1, 2-dihydro-2,2,4-trimethyl-*5H*-chromeno [3,4-*f*]quinoline (Compound 183); (*R*/*S*)-5-(3-Bromo-2-pyridyl)-1,2-dihydro-2,2,4-trimethyl-*5H*-chromeno[3,4-*f*]quinoline (Compound 184); (*R*/*S*)-1,2-Dihydro-5-hydroxy-2,2,4-trimethyl-*5H*-chromeno[3,4-*f*]quinoline (Compound 185); (*R*/*S*)-1,2-Dihydro-2,2,4-trimethyl-5-methoxy-*5H*-chromeno[3,4-*f*]quinoline (Compound 186); (*R*/*S*)-1,2-Dihydro-2,2,4-trimethyl-5-propoxy-*5H*-chromeno[3,4-*f*]quinoline (Compound 187); (*R*/*S*)-5-Allyl-1,2-dihydro-2,2,4-trimethyl-*5H*-chromeno[3,4-*f*]quinoline (Compound 188); (*R*/*S*)-1,2-Dihydro-2,2,4-trimethyl-5-propyl-*5H*-chromeno[3,4-*f*]quinoline (Compound 189); (*R*/*S*)-1,2-Dihydro-2,2,4-trimethyl-5-(2-pyridyl)-*5H*-chromeno[3,4-*f*]quinoline (Compound 190); (*R*/*S*)-5-(3-Fluorophenyl)-1,2-dihydro-2,2,4-trimethyl-*5H*-chromeno[3,4-*f*]quinoline (Compound 191); (*R*/*S*)-5-(3-Fluorophenyl)-1,2,3,4-tetrahydro-2,2-dimethyl-4-methylidene-5H-chromeno[3,4-*f*]quinoline (Compound 192); (*R*/*S*)-1,2-Dihydro-2,2,4-trimethyl-5-propylthio-*5H*-chromeno[3,4-*f*]quinoline (Compound 193); (*R*/*S*)-1,2-Dihydro-5-(3-methoxyphenyl)-2,2,4-trimethyl-*5H*-chromeno[3,4-*f*]quinoline (Compound 194); (*R*/*S*) 1,2-Dihydro-2,2,4-trimethyl-5-[3-(trifluoromethyl)phenyl]-*5H*-chromeno[3,4-*f*]quinoline (Compound 195); (*R*/*S*)-5-(3-Fluoro-4-methylphenyl)-1,2-dihydro-2,2,4-trimethyl-*5H*-chromeno[3,4-*f*]quinoline (Compound 196); (*R*/*S*)-5-(4-Bromo-3-pyridyl)-1,2-dihydro-2,2,4-trimethyl-*5H*-chromeno[3,4-*f*]quinoline (Compound 197); (*R*/*S*)-1,2-Dihydro-2,2,4-trimethyl-5-(3-pyridyl)-*5H*-chromeno[3,4-*f*]quinoline (Compound 198); (*R*/*S*)-5-(4-Chloro-3-fluorophenyl)-1,2-dihydro-2,2,4-trimethyl-*5H*-chromeno[3,4-*f*]quinoline (Compound 199); (*R*/*S*)-1,2-Dihydro-2,2,4,5-tetramethyl-*5H*-chromeno[3,4-*f*]quinoline (Compound 200); (*R*/*S*)-1,2-Dihydro-5-hexyl-2,2,4-trimethyl-5*H*-chromeno[3,4-*f*]quinoline (Compound 201); 1,2-Dihydro-2,2,4-trimethyl-5*H*-chromeno[3,4-*f*]quinoline (Compound 202); (*R*/*S*)-1,2-Dihydro-5-(3-methylbutyl)-2,2,4-trimethyl-5*H*-chromeno[3,4-*f*]quinoline (Compound 203); (*R*/*S*)-5-(4-Chlorobutyl)-1,2-dihydro-2,2,4-trimethyl-5*H*-chromeno[3,4-*f*]quinoline (Compound 204); (*R*/*S*)-5-Benzyl-1,2-dihydro-2,2,4-trimethyl-5*H*-chromeno[3,4-*f*]quinoline (Compound 205); (*R*/*S*)-5-(4-Bromobutyl)-1,2-dihydro-2,2,4-trimethyl-5*H*-chromeno[3,4-*f*]quinoline (Compound 206); (*R*/*S*)-5-Butyl-9-fluoro-1,2-dihydro-2,2,4-trimethyl-*5H*-chromeno[3,4-*f*]quinoline (Compound 210); (*R*/*S*)-5-Butyl-8-fluoro-1,2-dihydro-2,2,4-trimethyl-*5H*-chromeno[3,4-*f*]quinoline (Compound 211); (*R*/*S*)-5-(3-Chlorophenyl)-9-fluoro-1,2-dihydro-2,2,4-trimethyl-*5H*-chromeno[3,4-*f*]quinoline (Compound 212); (*R*/*S*)-5-(4-Chloro-3-methylphenyl)-9-fluoro-1,2-dihydro-2,2,4-trimethyl-*5H*-chromeno[3,4-*f*]quinoline (Compound 213); (*R*/*S*)-5-(4-Chlorophenyl)-9-fluoro-1,2-dihydro-2,2,4-trimethyl-*5H*-chromeno[3,4-*f*]quinoline (Compound 214); (*R*/*S*)-9-Fluoro-1,2-dihydro-5-(4-methoxyphenyl)-2,2,4-trimethyl-*5H*-chromeno[3,4-*f*]quinoline (Compound 215); (*R*/*S*)-8-Fluoro- 1,2-dihydro-5-methoxyl-2,2,4-trimethyl-*5H*-chromeno [3,4-*f*]quinoline (Compound 216); (*R*/*S*)-5-(4-Chlorophenyl)-8-fluoro-1,2-dihydro-2,2,4-trimethyl-*5H*-chromeno[3,4-*f*]quinoline (Compound 217); and (*R*/*S*)-9-Chloro-5-(4-chlorophenyl)-1,2-dihydro-2,2,4-trimethyl-*5H*-chromeno[3,4-*f*]quinoline (Compound 218); 9-Chloro-1,2-dihydro-2,2,4-trimethyl-5*H*-chromeno[3,4-*f*]quinoline (Compound 320); (*R*/*S*)-9-Fluoro-1,2-dihydro-5-methoxy-2,2,4-trimethyl-5*H*-chromeno[3,4-*f*]quinoline (Compound 322); (*R*/*S*)-9-Fluoro-1,2-dihydro-2,2,4-trimethyl-5-thiopropoxy-5*H*-chromeno[3,4-*f*]quinoline (Compound 323); (*R*/*S*)-9-Fluoro-1,2-dihydro-2,2,4-trimethyl-5-propoxy-5*H*-chromeno[3,4-*f*]quinoline (Compound 324); (*R*/*S*)-1,2-Dihydro-9-methoxy-2,2,4-trimethyl-5*H*-chromeno[3,4-*f*]quinoline (Compound 329); (*R*/*S*)-1,2-Dihydro-2,2,4,9-tetramethyl-5*H*-chromeno[3,4-*f*]quinoline (Compound 330); (*R*/*S*)-7-Chloro-1,2-dihydro-2,2,4-trimethyl-5*H*-chromeno[3,4-*f*]quinoline (Compound 331); (*R*/*S*)-5-(4-Bromo-3-pyridyl)-1,2,3,4-tetrahydro-2,2-dimethyl-4-methylidene-5*H*-chromeno[3,4-*f*]quinoline (Compound 347); (*R*/*S*)-5-(3,5-Difluorophenyl)-1,2-dihydro-2,2,4-trimethyl-5*H*-chromeno[3,4-*f*]quinoline (Compound 348); (*R*/*S*)-5-(3-Bromo-5-fluorophenyl)-1,2,3,4-tetrahydro-2,2-dimethyl-4-methylidene-5*H*-chromeno[3,4-*f*]quinoline (Compound 352); (Z)-1,2,-Dihydro-5-(2,4,6-trimethylbenzylidene)-2,2,4-trimethyl-5*H*-chromeno[3,4-*f*]quinoline (Compound 364); (Z)-5-Benzylidene-9-fluoro-1,2-dihydro-2,2,4,11-tetramethyl-5*H*-chromeno[3,4-*f*]quinoline (Compound 377); (*R*/*S*)-5-(4-Chlorophenyl)-1,2,3,4-tetrahydro-2,2-dimethyl-5*H*-chromeno[3,4-*f*]-4-quinolinone (Compound 378); (*R*/*S*)-5-(4-Chlorophenyl)-1,2,3,4-tetrahydro-2,2,3,3-tetramethyl-5*H*-chromeno[3,4-*f*]-4-quinolinone (Compound 379); (*R*/*S*)-5-(4-Chlorophenyl)-1,2-dihydro-2,2-dimethyl-5*H*-chromeno[3,4-*f*]quinoline (Compound 380); (+)-(*R**-*4l*,*5l*)-5-(4-Chlorophenyl)-1,2,3,4-tetrahydro-2,2,4-trimethyl-5*H*-chromeno[3,4-*f*]-3-quinolinone (Compound 381); (-)-(*R**-*4l*,*5l*)-5-(4-Chlorophenyl)-1,2,3,4-tetrahydro-2,2,4-trimethyl-5*H*-chromeno[3,4-*f*]-3-quinolinone (Compound 382); (*R*/*S*)-5-(4-Chlorophenyl)-1,2,3,4-tetrahydro-2,2-dimethyl-5*H*-chromeno[3,4-*f*]-3-quinolinone (Compound 383); (*R*/*S*)-3-(3-Fluorobenzyl)-5-(3-fluorobenzylidene)-1,2,3,4-tetrahydro-3-hydroxy-2,2,4-trimethyl-5*H*-chromeno[3,4-*f*]quinoline (Compound 384); (*R*/*S*)-3,5-Dibutyl-1,2,3,4-tetrahydro-3-hydroxy-2,2,4-trimethyl-5*H*-chromeno[3,4-*f*]quinoline (Compound 385); (*R*/*S*)-5-Butyl-1,2,3,4-tetrahydro-2,2,4-trimethyl-5*H*-chromeno[3,4-*f*]-3-quinolinone (Compound 386); (*R*/*S*-*4l*,*5l*)-1,2,3,4-Tetrahydro-2,2,4-trimethyl-5-phenyl-5*H*-chromeno[3,4-*f*]-3-quinolinone (Compound 387); (*R*/*S*-*4l*,*5u*)-1,2,3,4-Tetrahydro-2,2,4-trimethyl-5-phenyl-5*H*-chromeno[3,4-*f*]-3-quinolinone (Compound 388); (*R*/*S*-*4l*,*6u*)-1,2,3,4-Tetrahydro-2,2,4-trimethyl-6-phenyl-5*H*-isochromeno[3,4-*f*]-3-quinolinone (Compound 390); (*R*/*S*-*4l*,*6l*)-1,2,3,4-Tetrahydro-2,2,4-trimethyl-6-phenyl-5H-isochromeno[3,4-*f*]-3-quinolinone (Compound 391); (*R*/*S*-*3l*,*4u*,*5u*)-5-(4-Chlorophenyl)-1,2,3,4-tetrahydro-3-methoxy-2,2,4-trimethyl-5*H*-chromeno[3,4-*f*]quinoline (Compound 397); (*R*/*S*-*3l*,*4u*,*5l*)-5-(4-Chlorophenyl)-1,2,3,4-tetrahydro-3-methoxy-2,2,4-trimethyl-5*H*-chromeno[3,4-*f*]quinoline (Compound 398); (*R*/*S*-*3l*,*4u*,*5l*)-5-(4-Chlorophenyl)-1,2,3,4-tetrahydro-3-propyloxy-2,2,4-trimethyl-5*H*-chromeno[3,4-*f*]quinoline (Compound 399); (*R*/*S*-*3l*,*4u*,*5u*)-5-(4-Chlorophenyl)-1,2,3,4-tetrahydro-3-propyloxy-2,2,4-trimethyl-5*H*-chromeno[3,4-*f*]quinoline (Compound 400); and (*R*/*S*-*4l*,*5l*)-3-Benzylidene- 5-(4-chlorophenyl)-1,2,3,4-tetrahydro-2,2,4-trimethyl-5*H*-chromeno[3,4-*f*]quinoline (Compound 401).

Representative PR agonists include: (Z)-5-Butylidene-1,2-dihydro-2,2,4-trimethyl-5*H*-chromeno[3,4-*f*]quinoline (Compound 219); (Z)-5-Benzylidene-1,2-dihydro-2,2,4-trimethyl-*5H*-chromeno[3,4-*f*]quinoline (Compound 220); (Z)-5-(4-Fluorobenzylidene)-1,2-dihydro-2,2,4-trimethyl-*5H*-chromeno[3,4-*f*]quinoline (Compound 221); (Z)-5-(4-Bromobenzylidene)-1,2-dihydro-2,2,4-trimethyl-*5H*-chromeno[3,4-*f*]quinoline (Compound 222); (Z)-5-(3-Bromobenzylidene)-1,2-dihydro-2,2,4-trimethyl-*5H*-chromeno[3,4-*f*]quinoline (Compound 223); (Z)-5-(3-Chlorobenzylidene)-1,2-dihydro-2,2,4-trimethyl-*5H*-chromeno[3,4-*f*]quinoline (Compound 224); (Z)-5-(3-Fluorobenzylidene)-1,2-dihydro-2,2,4-trimethyl-*5H*-chromeno[3,4-*f*]quinoline (Compound 225); (Z)-5-(2-Chlorobenzylidene)-1,2-dihydro-2,2,4-trimethyl-*5H*-chromeno[3,4-*f*]quinoline (Compound 226); (Z)-5-(2-Bromobenzylidene)-1,2-dihydro-2,2,4-trimethyl-*5H*-chromeno[3,4-*f*]quinoline (Compound 227); (Z)-5-(2-Fluorobenzylidene)-1,2-dihydro-2,2,4-trimethyl-*5H*-chromeno[3,4-*f*]quinoline (Compound 228); (Z)-5-(2,3-Difluorobenzylidene)-1,2-dihydro-2,2,4-trimethyl-*5H*-chromeno[3,4-*f*]quinoline (Compound 229); (Z)-5-(2,5-Difluorobenzylidene)-1,2-dihydro-2,2,4-trimethyl-*5H*-chromeno[3,4-*f*]quinoline (Compound 230); (Z)-9-Fluoro-5-(3-fluorobenzylidene)-1,2-dihydro-2,2,4-trimethyl-*5H*-chromeno[3,4-*f*]quinoline (Compound 231); (Z)-9-Fluoro-5-(3-methoxybenzylidene)-1,2-dihydro-2,2,4-trimethyl-*5H*-chromeno[3,4-*f*]quinoline (Compound 232); (Z)-8-Fluoro-5-(3-fluororbenzylidene)-1,2-dihydro-2,2,4-trimethyl-*5H*-chromeno[3,4-*f*]quinoline (Compound 233); (*R*/*S*-4*l*, 5*u*)-5-(4-Chlorophenyl)-1,2,3,4-tetrahydro-2,2,4-trimethyl-*5H*-chromeno[3,4-*f*]-3-quinolinone (Compound 234); (*R*/*S*-4*l*, 5*l*)-5-(4-Chlorophenyl)-1,2,3,4-tetrahydro-2,2,4-trimethyl-*5H*-chromeno[3,4-*f*]-3-quinolinone (Compound 235); and (*R*/*S*)-5-(4-Chlorophenyl)-1,2,3,4-tetrahydro-2,2,4,4-tetramethyl-*5H*-chromeno[3,4-*f*]-3-quinolinone (Compound 236); 5-(3-Fluorobenzyl)-1,2-dihydro-2,2,4-trimethyl-5*H*-chromeno[3,4-*f*]quinoline (Compound 318); (*R*/*S*)-9-Chloro-1,2-dihydro-2,2,4-trimethyl-5-propyloxy-5*H*-chromeno[3,4-*f*]quinoline (Compound 321); (*R*/*S*)-5-Butyl-9-chloro-1,2-dihydro-2,2,4-trimethyl-5*H*-chromeno[3,4-*f*]quinoline (Compound 325); (*R*/*S*)-5-Butyl-1,2-dihydro-9-methoxy-2,2,4-trimethyl-5*H*-chromeno[3,4-*f*]quinoline (Compound 326); (*R*/*S*)-9-Fluoro-1,2-dihydro-2,2,4,5-tetramethyl-5*H*-chromeno[3,4-*f*]quinoline (Compound 327); (*R*/*S*)-9-Chloro-1,2-dihydro-2,2,4,5-tetramethyl-5*H*-chromeno[3,4-*f*]quinoline (Compound 332); (*R*/*S*)-5-(4-Bromophenyl)-9-chloro-1,2-dihydro-2,2,4-trimethyl-5*H*-chromeno[3,4-*f*]quinoline (Compound 333); (*R*/*S*)-9-Chloro-5-(3-chlorophenyl)- 1,2-dihydro-2,2,4-trimethyl-5*H*-chromeno[3,4-*f*]quinoline (Compound 334); (*R*/*S*)-9-Chloro-1,2-dihydro-2,2,4-trimethyl-5-(3-methylphenyl)-5*H*-chromeno[3,4-*f*]quinoline (Compound 335); (*R*/*S*)-9-Chloro-5-(4-chloro-3-methylphenyl)-1,2-dihydro-2,2,4-trimethyl-5*H*-chromeno[3,4-*f*]quinoline (Compound 336); (*R*/*S*)-9-Chloro- 1,2-dihydro-5-[3-(trifluoromethyl)phenyl]-2,2,4-trimethyl-5*H*-chromeno[3,4-*f*]quinoline (Compound 337); (*R*/*S*)-9-Chloro-5-(3,5-dichlorophenyl)-1,2-dihydro-2,2,4-trimethyl-5*H*-chromeno[3,4-*f*]quinoline (Compound 338); (*R*/*S*)-9-Chloro-1,2-dihydro-5-(4-methoxyphenyl)-2,2,4-trimethyl-5*H*-chromeno[3,4-*f*]quinoline (Compound 339); (*R*/*S*)-9-Chloro-5-(3-fluoro-4-methoxyphenyl)-1,2-dihydro-2,2,4-trimethyl-5*H*-chromeno[3,4-*f*]quinoline (Compound 340); (*R*/*S*)-9-Chloro-5-(4-fluorophenyl)-1,2-dihydro-2,2,4-trimethyl-5*H*-chromeno[3,4-*f*]quinoline(Compound 341); (*R*/*S*)-9-Chloro-5-(3-chloro-4-methoxy-5-methylphenyl)-1,2-dihydro-2,2,4-trimethyl-5*H*-chromeno[3,4-*f*]quinoline (Compound 342); (*R*/*S*)-9-Chloro-5-(4-fluoro-3-methylphenyl)-1,2-dihydro-2,2,4-trimethyl-5*H*-chromeno[3,4-*f*]quinoline (Compound 343); (*R*/*S*)-9-Chloro-5-(3-fluorophenyl)-1,2-dihydro-2,2,4-trimethyl-5*H*-chromeno[3,4-*f*]quinoline (Compound 344); (*R*/*S*)-1,2-Dihydro-2,2,4-trimethyl-5-[(3,4-methylenedioxy)phenyl]-5*H*-chromeno[3,4-*f*]quinoline (Compound 345); (*R*/*S*)-5-(4-Chloro-3-methylphenyl)-1,2-dihydro-2,2,4-trimethyl-5*H*-chromeno[3,4-*f*]-quinoline (Compound 346); (*R*/*S*)-5-(3,5-Dichlorophenyl)-1,2-dihydro-2,2,4-trimethyl-5*H*-chromeno[3,4-*f*]quinoline (Compound 349); (*R*/*S*)-5-(3-Bromo-5-methylphenyl)-1,2-dihydro-2,2,4-trimethyl-5*H*-chromeno[3,4-*f*]quinoline (Compound 350); (*R*/*S*)-5-(3-Bromo-5-fluorophenyl)-1,2-dihydro-2,2,4-trimethyl-5*H*-chromeno[3,4-*f*]quinoline (Compound 351); (*R*/*S*)-5-[4-Fluoro-3-(trifluoromethyl)phenyl]-1,2-dihydro-2,2,4-trimethyl-5*H*-chromeno[3,4-*f*]quinoline (Compound 353); (*R*/*S*)-9-Fluoro-1,2-dihydro-2,2,4-trimethyl-5-(3-methylphenyl)-5*H*-chromeno[3,4-*f*]quinoline (Compound 354); (*R*/*S*)-1,2-Dihydro-9-methoxy-2,2,4-trimethyl-5-(3-methylphenyl)-5*H*-chromeno[3,4-*f*]quinoline (Compound 355); (*R*/*S*)-9-Fluoro-5-(3-fluoro-4-methoxyphenyl)-1,2-dihydro-2,2,4-trimethyl-5*H*-chromeno[3,4-*f*]quinoline (Compound 356); (*R*/*S*)-9-Fluoro-1,2-dihydro-2,2,4-trimethyl-5-[3-(trifluoromethyl)phenyl]-5*H*-chromeno[3,4-*f*]quinoline (Compound 357); (*R*/*S*)-9-Fluoro-5-(4-fluoro-3-methylphenyl)-1,2-dihydro-2,2,4-trimethyl-5*H*-chromeno[3,4-*f*]quinoline (Compound 358); (Z)-5-(2,4-Difluorobenzylidene)-1,2-dihydro-2,2,4-trimethyl-5*H*-chromeno[3,4-*f*]quinoline (Compound 359); (Z)-5-(3,4-Difluorobenzylidene)-1,2-dihydro-2,2,4-trimethyl-5*H*-chromeno[3,4-*f*quinoline (Compound 360); (Z)-5-(3-Fluorobenzylidene)-1,2,3,4-tetrahydro-2,2,4-trimethyl-*5H*-chromeno[3,4-*f*]quinoline (Compound 361); (Z)-5-(2,6-Difluorobenzylidene)-1,2-dihydro-2,2,4-trimethyl-*5H*-chromeno [3,4-*f*]quinoline (Compound 362); (Z)-1,2,-Dihydro-5-(2-methylbenzylidene)-2,2,4-trimethyl-*5H*-chromeno[3,4-*f*]quinoline (Compound 363); (Z)-9-Chloro-5-(2,5-difluorobenzylidene)-1,2-dihydro-2,2,4-trimethyl-*5H*-chromeno[3,4-*f*]quinoline (Compound 365); (Z)-5-Benzylidene-9-chloro-1,2-dihydro-2,2,4-trimethyl-*5H*-chromeno[3,4-*f*]quinoline (Compound 366); (Z)-9-Chloro-1,2-dihydro-2,2,4-trimethyl-5-(2-methylbenzylidene)-*5H*-chromeno[3,4-*f*]quinoline (Compound 367); (Z)-5-Benzylidene-9-chloro-1,2-dihydro-2,2-dimethyl-5*H*-chromeno[3,4-*f*]quinoline (Compound 368); (Z)-9-Chloro-5-(2-fluorobenzylidene)-1,2-dihydro-2,2,4-trimethyl-5*H*-chromeno[3,4-*f*]quinoline (Compound 369); (Z)-9-Chloro-5-(3-fluorobenzylidene)-1,2-dihydro-2,2,4-trimethyl-5*H*-chromeno[3,4-*f*]quinoline (Compound 370); (*E*/Z)-5-Benzylidene-9-fluoro-1,2-dihydro-2,2,4-trimethyl-5*H*-chromeno[3,4-*f*]quinoline (Compound 371); (Z)-5-Benzylidene-8-fluoro-1,2-dihydro-2,2,4-trimethyl-5*H*-chromeno[3,4-*f*]quinoline (Compound 372); (Z)-5-Benzylidene-1,2-dihydro-9-methoxy-2,2,4-trimethyl-5*H*-chromeno[3,4-*f*]quinoline (Compound 373); (Z)-9-Fluoro-1,2-dihydro-2,2,4-trimethyl-5-(2-methylbenzylidene)-5*H*-chromeno[3,4-*f*]quinoline (Compound 374); (Z)-8-Fluoro-1,2-dihydro-2,2,4-trimethyl-5-(2-methylbenzylidene)-5*H*-chromeno[3,4-*f*]quinoline (Compound 375); (Z)-1,2-Dihydro-9-methoxy-2,2,4-trimethyl-5-(2-methylbenzylidene)-5*H*-chromeno[3,4-*f*]quinoline (Compound 376); (Z)-(*R*/*S*)-5-(3-Fluorobenzylidene)-1,2,3,4-tetrahydro-2,2,4-trimethyl-5*H*-chromeno[3,4-*f*]-3-quinolinone (Compound 389); (Z)-(*R*/*S*)-5-(Benzylidene)-1,2,3,4-tetrahydro-2,2,4-trimethyl-5*H*-chromeno[3,4-*f*]-3-quinolinone (Compound 392); (*R*/*S-4l,5u*)-5-(3-Fluorophenyl)-1,2,3,4-tetrahydro-2,2,4-trimethyl-5*H*-chromeno[3,4-*f*]-3-quinolinone (Compound 393); (*R*/*S-4l,5l*)-5-(3-Fluorophenyl)-1,2,3,4-tetrahydro-2,2,4-trimethyl-5*H*-chromeno[3,4-*f*]-3-quinolinone (Compound 394); (*R*/*S-4l,5l*)-1,2,3,4-Tetrahydro-2,2,4-trimethyl-5-[3-(trifluoromethyl)phenyl]-5*H*-chromeno[3,4-*f*]-3-quinolinone (Compound 395); (*R*/*S-4l,5u*)-1,2,3,4-Tetrahydro-2,2,4-trimethyl-5-[3-(trifluoromethyl)phenyl]-5*H*-chromeno[3,4-*f*]-3-quinolinone (Compound 396); (*R*/*S*-*4l,5u*)-5-(4-Chlorophenyl)-1,2,3,4-tetrahydro-2,2,4-trimethyl-5*H*-chromeno[3,4-*f*]-3-quinolinone (Compound 402); (*R*/*S*-*4l,5l*)-5-(4-Chlorophenyl)-1,2,3,4-tetrahydro-2,2,4-trimethyl-5*H*-chromeno[3,4-*f*]-3-quinolinone (Compound 403); and (*R*/*S*)-5-Butyl-1,2-dihydro-2,2,4,9-tetramethyl-*5H*-chromeno[3,4-*f*]quinoline (Compound 457).

Compounds of the present invention, comprising classes of quinoline compounds and their derivatives, that can be obtained by routine chemical synthesis by those skilled in the art, e.g., by modification of the quinoline compounds disclosed or by a total synthesis approach.

The sequence of steps for several general schemes to synthesize the compounds of the present invention are shown below. In each of the Schemes the R groups (e.g., $R^1$, $R^2$, etc...) correspond to the specific substitution patterns noted in the Examples. However, it will be understood by those skilled in the art that other functionalities disclosed herein at the indicated positions of compounds of formulas V through VI also comprise potential substituents for the analogous positions on the structures within the Schemes.

**Scheme VIII**

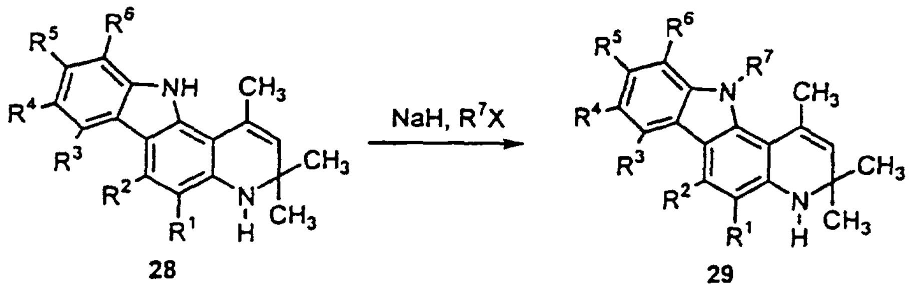


The process of **Scheme VIII** involves the alkylation of N(5) of an indolo[2,3-*f*]quinoline (structure **28**) by deprotonation with a strong base, for example, sodium hydride, followed by alkylation with an alkylating agent such as iodomethane.

## Scheme IX

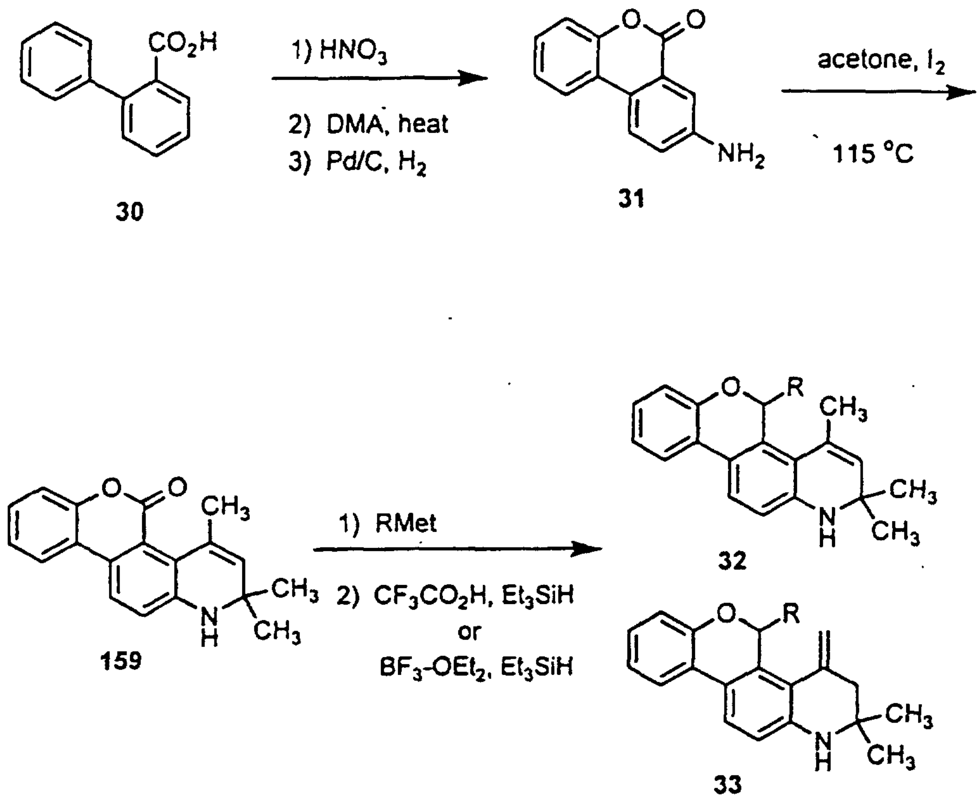


30 31 159 32 33

The process of **Scheme IX** begins with the nitration of 2-biphenylcarboxylic acid with, for example, concentrated nitric acid, to afford a mixture of nitro compounds, including 4,2'-dinitro-2-biphenylcarboxylic acid. The crude material is heated to 150-170°C in a high-boiling solvent such as dimethylacetamide to effect cyclization of 4,2'-dinitro-2-biphenylcarboxylic acid to the corresponding benzocoumarin. See G.I. Migachev, "Investigations in the Series of Ortho-Substituted Bi-phenyls. I. Nitration of 2-Biphenylcarboxylic Acid and the Chemical Properties of its Nitro Derivatives", *Zh. Organich. Khim.* **1979,** *15*, 567, the disclosure of which is herein incorporated by reference. Reduction of the nitro group with, for example, hydrogen over a metal catalyst, affords Compound **31**. Treatment of Compound **31** with acetone in the presence of a catalyst, for example, iodine, affords Compound **159**. The addition of an organometallic reagent, such as an organolithium or organomagnesium reagent, to Compound **159**, affords an intermediate which may be reduced by a trialkylsilane, such as triethylsilane, in the presence of a strong protic acid such as trifluoroacetic acid or a Lewis acid such as boron trifluoride. One or both of two regioisomeric products, structures **32** and **33**, are thus obtained.

## Scheme X

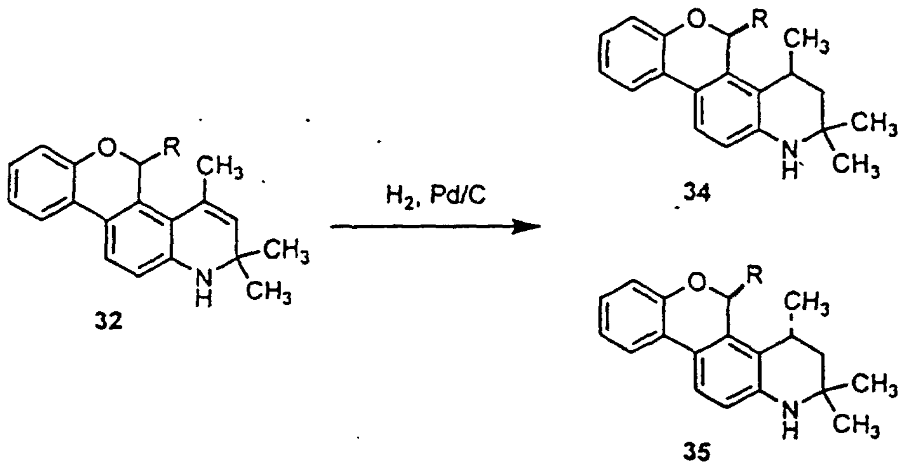


32 34 35

The process of **Scheme X** involves the reduction of a dihydroquinoline (structure **32**) to a mixture of two diastereomeric 1,2,3,4-tetrahydroquinolines (structures **34** and **35**) with, for example, hydrogen over a metal catalyst such as palladium on carbon.

## Scheme XI

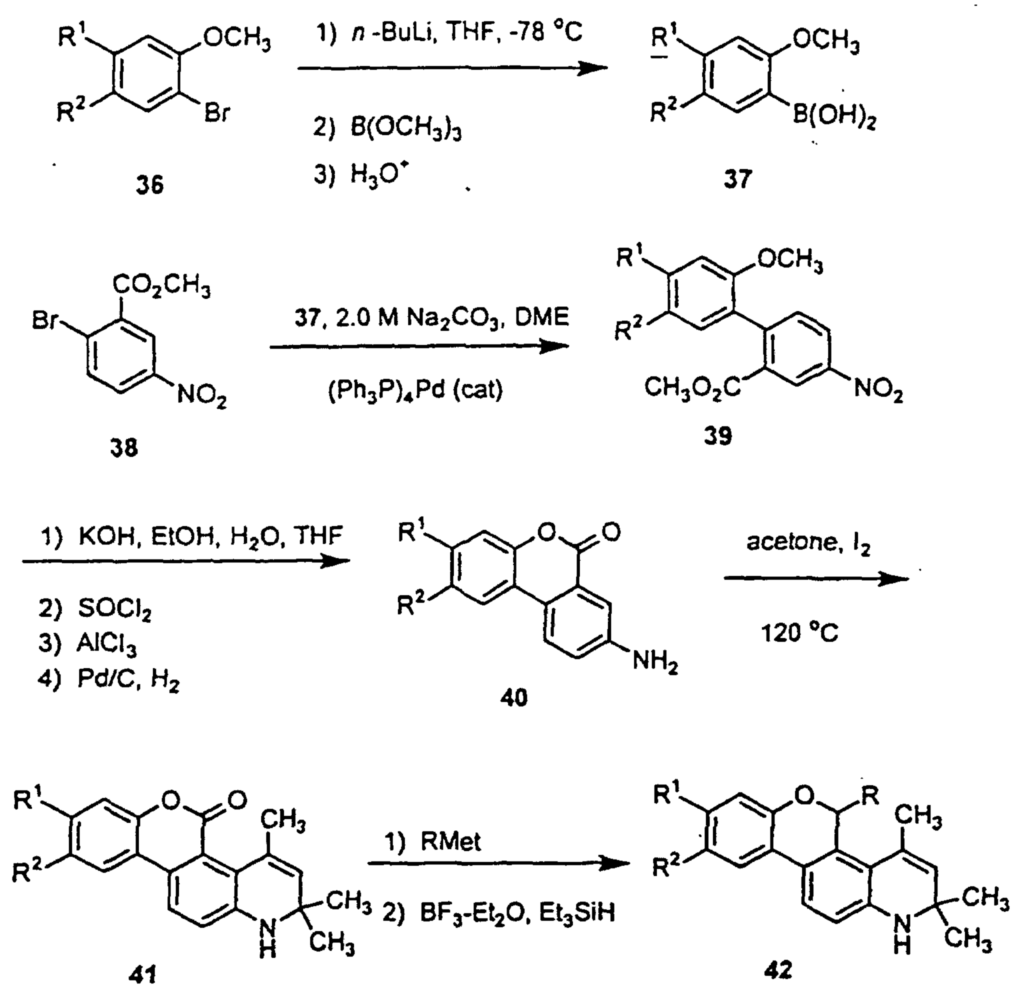


36 37 38 39 40 41 42

The process of **Scheme XI** involves the preparation of benzocoumarins from acyclic precursors. Thus, an ortho-bromoanisole (structure **36**) is lithiated with an alkyllithium, for example, *n*-butyllithium, and allowed to react with a trialkylborate such as trimethylborate. Hydrolysis of the intermediate with acid, for example, dilute hydrochloric acid, affords the corresponding boronic acid (structure **37**). Palladium-catalyzed coupling of a 2-methoxyphenylboronic acid (structure **37**) with methyl 2-bromo-5-nitrobenzoate (Compound **38)** with a palladium catalyst such as tetrakis(triphenylphosphine)palladium and an aqueous base such as aqueous potassium carbonate, affords the biphenyl carboxylate (structure **39**). Hydrolysis of the ester with base, for example, potassium hydroxide, is followed by conversion of the acid to the acid chloride with, for example, thionyl chloride. Intramolecular acylation is then effected by a Lewis acid such as aluminum trichloride.

Reduction of the nitro group with, for example, hydrogen over a metal catalyst, affords the desired aniline (structure **40).** Treatment of compounds of structure **40** with acetone and a catalyst such as iodine affords the dihydroquinoline (structure **41).** The addition of an organometallic reagent, for example an organolithium or organomagnesium reagent, to a compound of structure **41**, followed by treatment of the intermediate with a strong protic or Lewis acid and a trialkylsilane, for example, boron trifluoride and triethylsilane, affords a compound of structure **42**.

## Scheme XIV

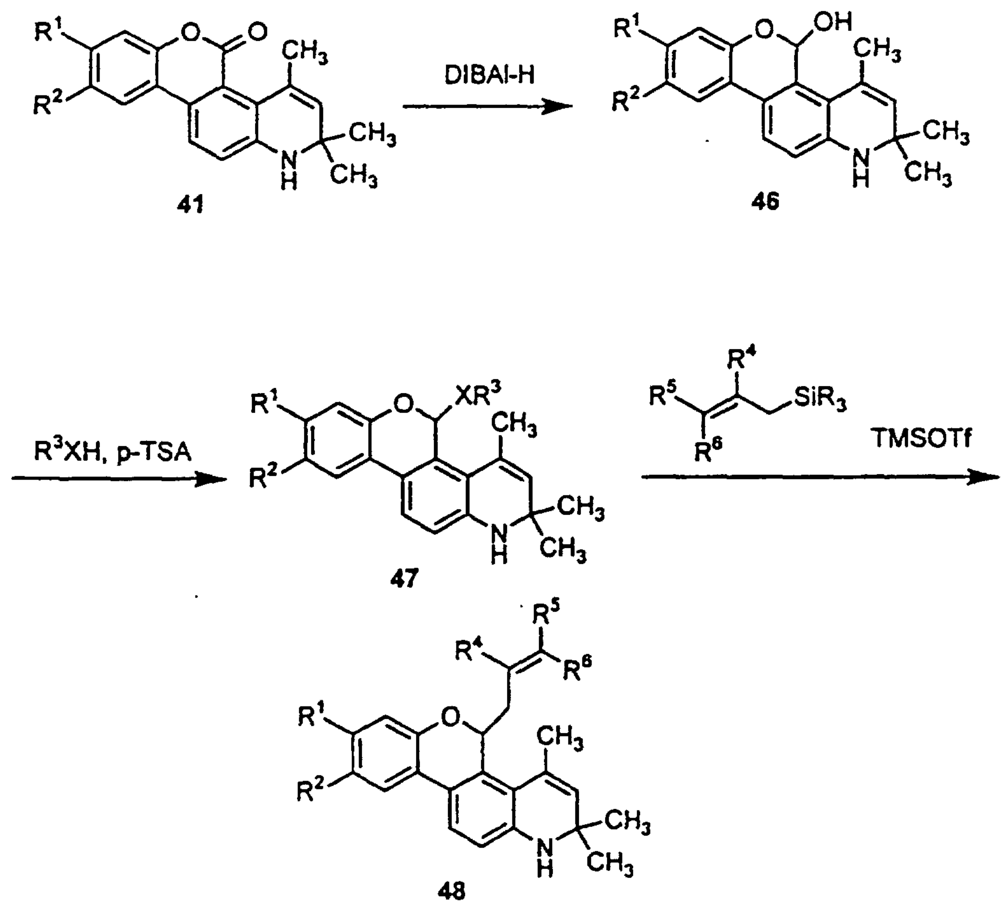


41 46 47 48

The process of **Scheme XIV** involves the reduction of a compound of structure **41** with a metal hydride, for example, diisobutylaluminum hydride, to afford a compound of structure **46.** Treatment of a compound of structure **46** with an alcohol such as methanol or a thiol such as propanethiol in the presence of an acid such as *para*-toluenesulphonic acid affords a compound of structure **47** (X= O or S). Treatment of a ketal of structure **47** (X=O) with an allyl silane and a Lewis acid such as trimethylsilyl trifluoromethanesulfonate affords a compound of structure **48**.

Scheme XXIII

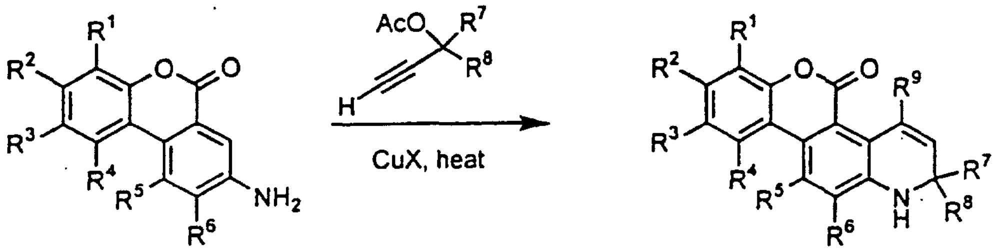


87

88 ($R^9$ = H)

The process of **Scheme XXIII** involves the reaction of an aminobenzocoumarin (a compound of structure **87**) with a propargyl acetate in the presence of a copper salt, such as copper(I) chloride, to afford a compound of structure **88.** See N. R. Easton and D. R. Cassady, "A Novel Synthesis of Quinolines and Dihydroquinolines.", *J. Org. Chem.* **1962, 27**,4713, and N. R. Easton and G. F. Hennion, "Metal Catalyst Process for Converting α-Amino-Acetylenes to Dihydroquinoline", U. S. Patent 3,331,846 (1967), the disclosure of which is herein incorporated by reference.

## Scheme XXIV

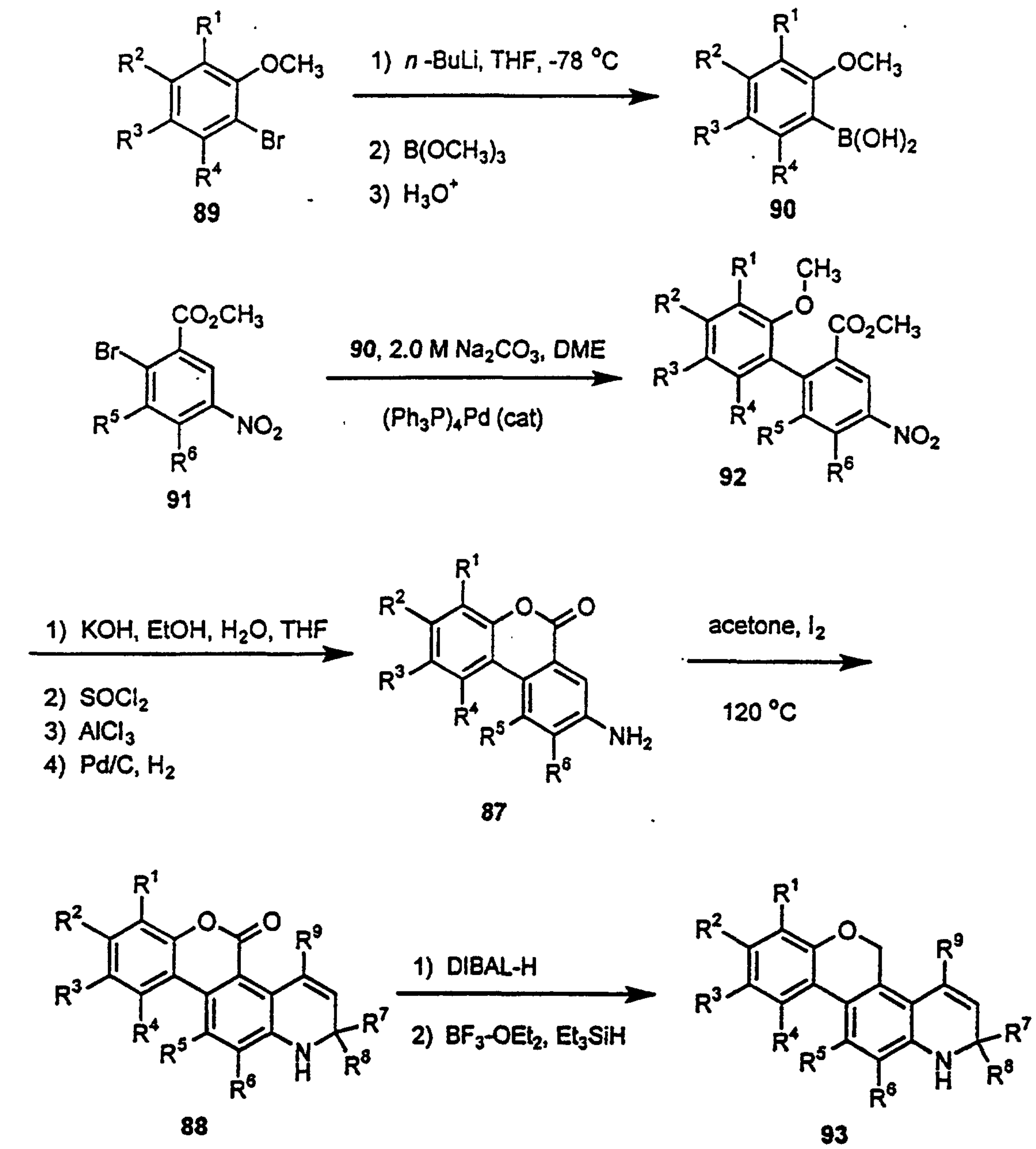


89 90 91 92 87 88 93

The process of **Scheme XXIV** involves the preparation of benzocoumarins from acyclic precursors. Thus, an ortho-bromoanisole (structure **89**) is lithiated with, for example, *n*-butyllithium and allowed to react with a trialkylborate such as trimethylborate. Hydrolysis of the intermediate with, for example, dilute hydrochloric acid affords the corresponding boronic acid (structure **90**). Palladium-catalyzed coupling of a 2-methoxyphenylboronic acid (structure **90**) with a methyl 2-bromo-5-nitrobenzoate (structure **91**) with, for example, tetrakis(triphenylphosphine)palladium and potassium carbonate, affords the biphenyl carboxylate (structure **92**). Hydrolysis of the ester with, for example, potassium hydroxide, is followed by conversion of the acid to the acid chloride with, for example, thionyl chloride. Intramolecular Friedel-Crafts acylation is then effected by a Lewis acid such as aluminum trichloride. Reduction of the nitro group with, for example, hydrogen over palladium on carbon, affords the desired aniline (structure **87**). Treatment of compounds of structure **87** with acetone and iodine affords the dihydroquinoline (structure **88**). The reduction of a compound of structure **88** with, for example, diisobutylaluminum hydride, followed by treatment of the intermediate with, for example, boron trifluoride and triethylsilane, affords a compound of structure **93**.

## Scheme XXV

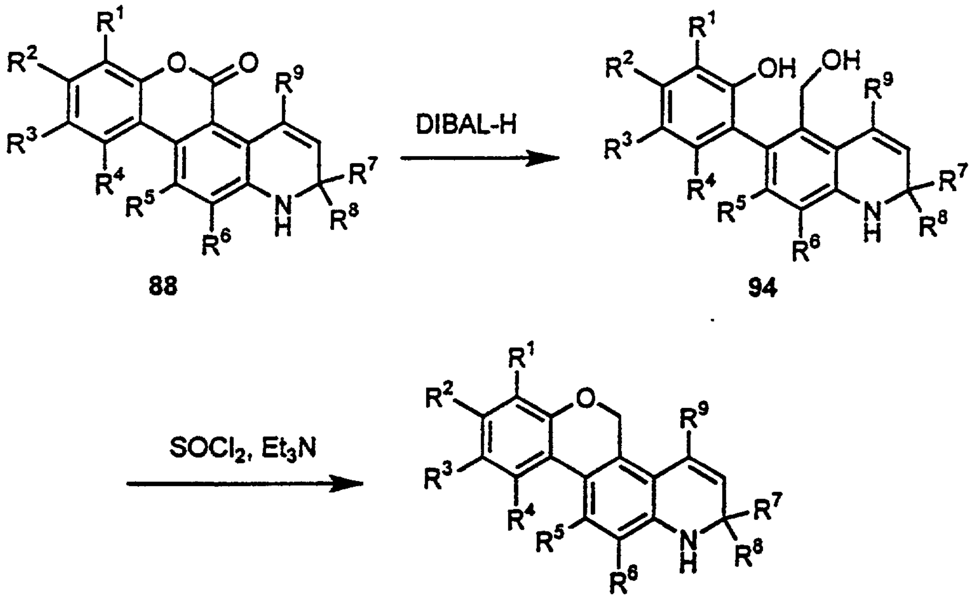


88 94 93

The process of **Scheme XXV** involves the reduction of a compound of structure **88** with a reducing agent, for example, diisobutylaluminum hydride, to a compound of structure **94.** Conversion of the benzyl alcohol to a leaving group by treatment with, for example, thionyl chloride, in the presence of a base such as triethylamine, effects ring closure to a compound of structure **93**.

## Scheme XXVI

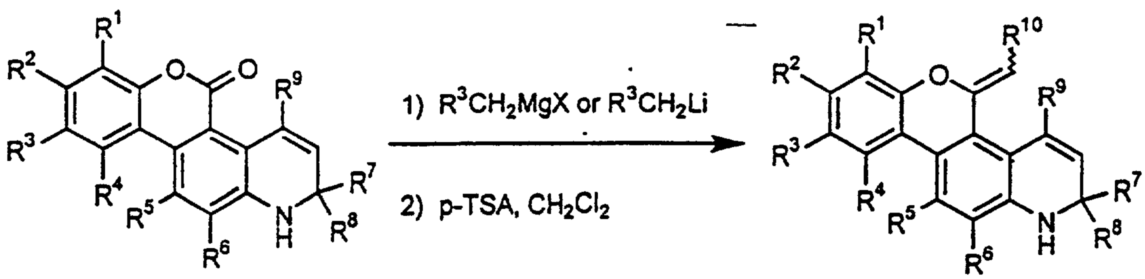


88 95

The process of **Scheme XXVI** begins with the addition of an organolithium or organomagnesium reagent to a compound of structure **88,** followed by treatment of the intermediate thus obtained with an acid such as *para*-toluenesulfonic acid, to afford a compound of structure **95**.

## Scheme XXVII

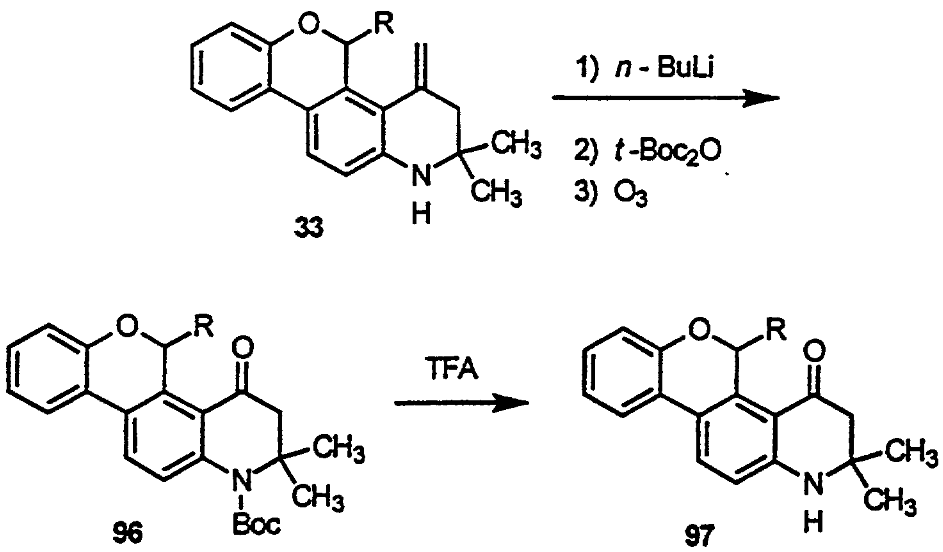


33

96

97

The process of **Scheme XXVII** begins with the protection of the nitrogen atom of a compound of structure **33** by treatment with a base, for example n-butyllithium, followed by the addition of an acylating agent such as di-*tert*-butyldicarbonate. Ozonolysis of the olefin affords a compound of structure **96.** Subsequent removal of the protecting group with, for example, trifluoroacetic acid, affords a compound of structure **97**.

## Scheme XXVIII

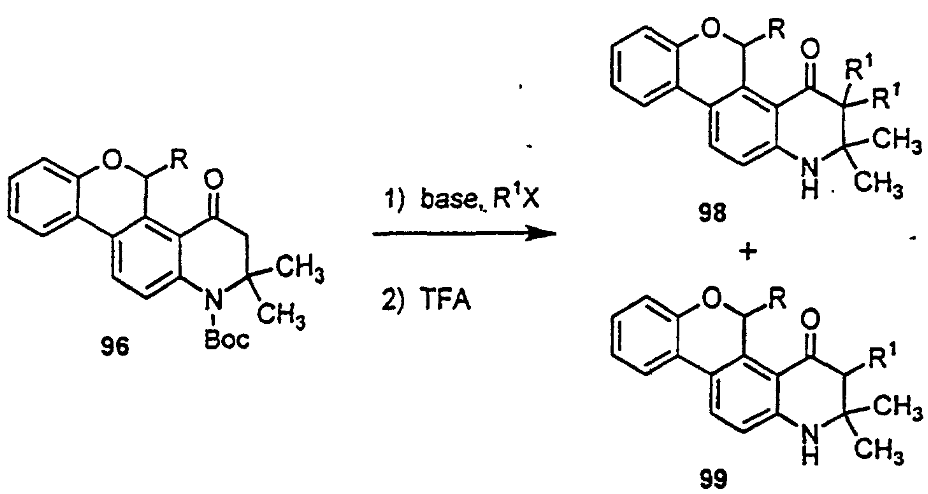


96

98

+

99

The process of **Scheme XXVIII** begins with the deprotonation of a compound of structure **96** with, for example, sodium hydride or lithium diisopropylamide, followed by the addition of an alkylating agent such as iodomethane, to afford a mono-alkylated product, or a mixture of mono- and di-alkylated products. Subsequent removal of the protecting group with, for example, trifluoroacetic acid, affords either one or both compounds of structures **98** and **99**.

**Scheme XXIX**

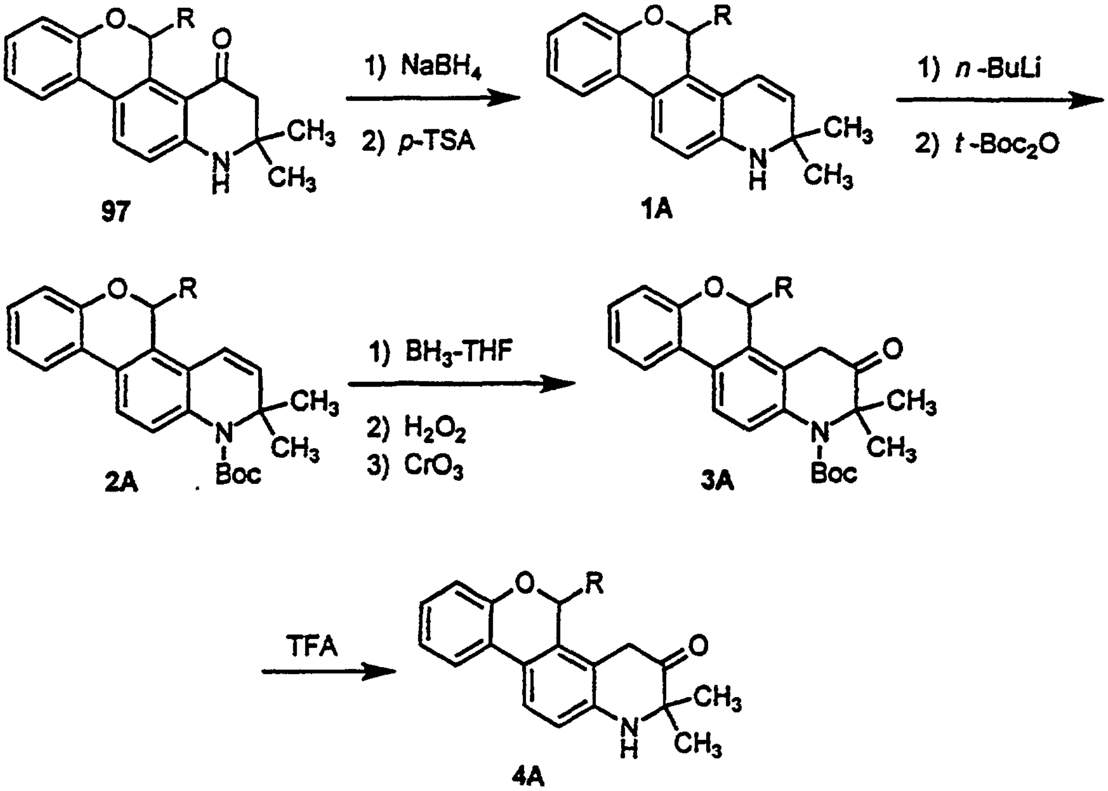


97, 1A, 2A, 3A, 4A

The process of **Scheme XXIX** begins with the reduction of a compound of structure **97** with, for example sodium borohydride, followed by dehydration of the resulting alcohol by treatment with an acid such as *para*-toluenesulfonic acid, to afford a compound of structure **1A** . The nitrogen atom of a compound of structure **1A** is then protected by treatment with a base, for example *n*-butyllithium, followed by the addition of an acylating agent such as di-*tert*-butyldicarbonate, to afford a compound of structure **2A.** Hydroboration of a compound of structure **2A** with a borane species, for example, boranetetrahydrofuran, followed by an oxidative work-up using, for example, basic hydrogen peroxide, affords a 3-hydroxyltetrahydroquinoline. Oxidation of the alcohol with a typical oxidant, for example chromium trioxide, affords a compound of structure **3A,** and deprotection with a strong acid such as trifluoroacetic acid affords a compound of structure **4A.**

**Scheme XXXI**

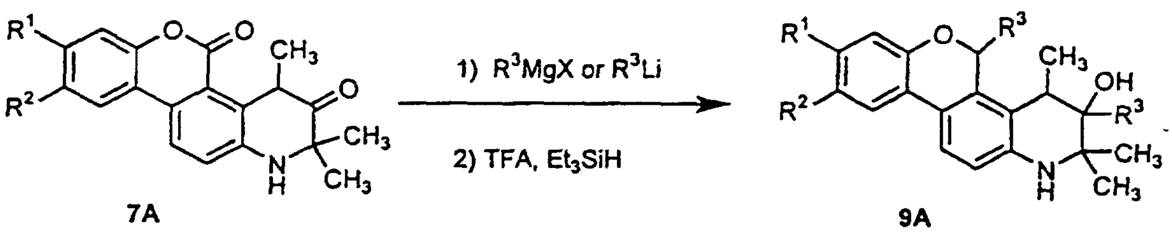


7A, 9A

The process of **Scheme XXXI** begins with the addition of an organolithium or organomagnesium reagent to a compound of structure **7A,** followed by reduction of the intermediate hemiketal with, for example, trifluoroacetic acid and triethylsilane, to afford a compound of structure **9A.**

Scheme XXXVI

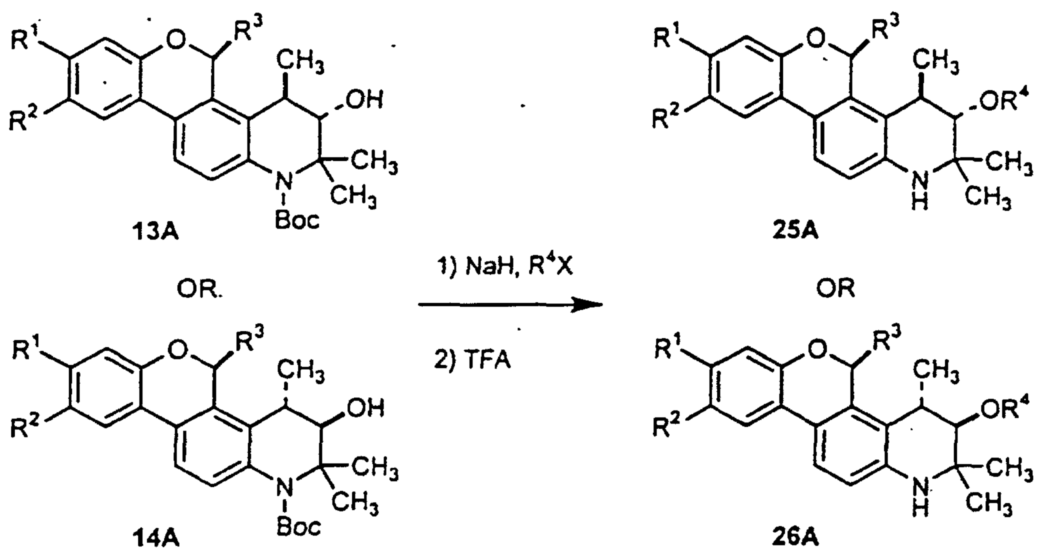


13A OR. 14A

25A OR 26A

The process of **Scheme XXXVI** involves the alkylation of the oxygen atom of a compound of structure **13A** or **14A**. The addition of a base such as sodium hydride and an alkylating agent such as iodomethane, followed by deprotection of the nitrogen atom with, for example, trifluoroacetic acid, affords a compound of structure **25A** (from a compound of structure **13A**) or structure **26A** (from a compound of structure **14A**).

**Process 3** is depicted in **Scheme XI** and involves the preparation of benzocoumarins from acyclic precursors. Thus, an ortho-bromoanisole (structure **36**) is lithiated with an alkyllithium (for example, *n*-butyllithium, *t*-butyllithium) at -100°C to 80°C in an inert solvent (typical solvents include toluene, ether, THF), and allowed to react with a trialkylborate (for example, trimethylborate, triisopropylborate) at -100°C to 100°C. Hydrolysis of the intermediate with acid (for example, dilute hydrochloric acid or sulfuric acid) at -40°C to 100°C, affords the corresponding 2-methoxyphenyl boronic acid (structure **37**). Alternatively, the organolithium or organomagnesium intermediate may be treated with a trialkyltin halide (for example, trimethyltin chloride, tributyltin chloride, etc.) at -100°C to 200°C to afford a trialkyltin aryl compound, a species useful in the coupling processes described above in **Process 1**. The important steps of process 3 begin with the palladium-catalyzed coupling of a 2-methoxyphenyl boronic acid (structure **37**) with a 2-halo-5-nitrobenzoic acid derivative (typical derivatives include the acid; any one of a number of esters, including methyl, ethyl, allyl, *t*-butyl, phenyl; or any one of a number of amides, including dimethyl, methyl, diallyl, allyl, dibenzyl) with a palladium catalyst (for example, tetrakis(triphenylphosphine) palladium, allylpalladium chloride dimer, bis(triphenylphosphine) palladium dichloride), and aqueous base (for example, sodium carbonate, potassium carbonate) at -40°C to 200°C affords the biaryl carboxylate (structure **39**). The product obtained from use of the acid as a coupling partner may be used directly; alternatively, deprotection by hydrolysis of ihe ester or the amide is accomplished with aqueous base (for example, potassium hydroxide or sodium hydroxide) or aqueous acid (for example, trifluoroacetic acid, hydrochloric acid, sulfuric acid) at -60°C to 300°C. The acid is converted to the acid chloride with, for example, thionyl chloride in an inert solvent (typical solvents include methylene dichloride, toluene, or 1,2-dichloroethane) at -80°C to 300°C. Intramolecular cyclization (acylation) is then effected by treatment of a solution of the acid chloride in an inert solvent (typical solvents include methylene dichloride, toluene, or 1,2-dichloroethane) with a Lewis acid (for example, aluminum trichloride, boron trifluoride) at -80°C to 300°C to yield the nitrobenzocoumarin. Reduction of the nitro group of the nitrobenzocoumarin with, for example, 1-200 atmospheres of hydrogen over a metal catalyst (for example, Pd/C, $PtO_2$), affords the desired aminobenzocoumarin (structure **40).** Treatment of compounds of structure **40** with acetone and a catalyst such as iodine affords the coumarino[3,4-*f*]quinoline (structure **41),** as described above in **Process 1**. The addition of an organometallic reagent, for example an organolithium or organomagnesium reagent, to a solution of a compound of structure **41** in an inert solvent at -100°C to 100°C affords an adduct. This adduct may be reduced by treatment of a solution of the adduct in an inert solvent (such as dichloromethane or toluene) with a strong protic or Lewis acid and a trialkylsilane, (for example, boron trifluoride or trifluoroacetic acid and triethylsilane or methyldiphenylsilane) at -80°C to 200°C, to afford a 5*H*-chromeno[3,4-*f*]quinoline (Compound of structure **42**).

The compounds of the present invention also includes racemate, stereoisomers and mixtures of said compounds, including isotopically-labeled and radio-labeled compounds. Such isomers can be isolated by standard resolution techniques, including fractional crystallization and chiral column chromatography.

As noted above, any of the steroid modulator compounds of the present invention can be combined in a mixture with a pharmaceutically acceptable carrier to provide pharmaceutical compositions useful for treating the biological conditions or disorders noted herein in mammalian, and more preferably, in human patients. The particular carrier employed in these pharmaceutical compositions may take a wide variety of forms depending upon the type of administration desired, e.g., intravenous, oral, topical, suppository or parenteral.

In preparing the compositions in oral liquid dosage forms (e.g., suspensions, elixirs and solutions), typical pharmaceutical media, such as water, glycols, oils, alcohols, flavoring agents, preservatives, coloring agents and the like can be employed. Similarly, when preparing oral solid dosage forms (e.g., powders, tablets and capsules), carriers such as starches, sugars, diluents, granulating agents, lubricants, binders, disintegrating agents and the like will be employed. Due to their ease of administration, tablets and capsules represent the most advantageous oral dosage form for the pharmaceutical compositions of the present invention.

For parenteral administration, the carrier will typically comprise sterile water, although other ingredients that aid in solubility or serve as preservatives, may also be included. Furthermore, injectable suspensions may also be prepared, in which case appropriate liquid carriers, suspending agents and the like will be employed.

For topical administration, the compounds of the present invention may be formulated using bland, moisturizing bases, such as ointments or creams. Examples of suitable ointment bases are petrolatum, petrolatum plus volatile silicones, lanolin, and water in oil emulsions such as Eucerin™ (Beiersdorf). Examples of suitable cream bases are Nivea™ Cream (Beiersdorf), cold cream (USP), Purpose Cream™ (Johnson & Johnson) hydrophilic ointment (USP), and Lubriderm™ (Warner-Lambert).

The pharmaceutical compositions and compounds of the present invention will generally be administered in the form of a dosage unit (e.g., tablet, capsule etc.) at from about 1 µg/kg of-body weight to about 500 mg/kg of body weight, more preferably from about 10 µg/kg to about 250 mg/kg, and most preferably from about 20 µg/kg to about 100 mg/kg. As recognized by those skilled in the art, the particular quantity of pharmaceutical composition according to the present invention administered to a patient will depend upon a number of factors, including, without limitation, the biological activity desired, the condition of the patient, and tolerance for the drug.

The compounds of this invention also have utility when radio- or isotopically-labeled as ligands for use in assays to determine the presence of PR, AR, ER, GR or MR in a cell background or extract. They are particularly useful due to their ability to selectively activate progesterone and androgen receptors, and can therefore be used to determine the presence of such receptors in the presence of other steroid receptors or related intracellular receptors.

Due to the selective specificity of the compounds of this invention for steroid receptors, these compounds can be used to purify samples of steroid receptors *in vitro.* Such purification can be carried out by mixing samples containing steroid receptors with one or more of the compounds of the present invention so that the compounds bind to the receptors of choice, and then separating out the bound ligand/receptor combination by separation techniques which are known to those of skill in the art. These techniques include column separation, filtration, centrifugation, tagging and physical separation, and antibody complexing, among others.

The compounds and pharmaceutical compositions of the present invention can advantageously be used in the treatment of the diseases and conditions described herein. In this regard, the compounds and compositions of the present invention will prove particularly useful as modulators of human fertility, and in the treatment of female and male sex steroid-dependent diseases and conditions such as hormone replacement therapy, dysfunctional uterine bleeding, endometriosis, leiomyomas, acne, male-pattern baldness, osteoporosis, prostatic hyperplasia and various hormone-dependent cancers, such as cancers of the breast, ovaries, endometrium and prostate. The GR and MR active compounds and compositions of the present invention will also prove useful as affectors of carbohydrate, protein and lipid metabolism, electrolyte and water balance, as well as modulators of the functions of the cardiovascular, kidney, central nervous, immune, skeletal muscle and other organ and tissue systems.

The compounds and pharmaceutical-compositions of the present invention possess a number of advantages over previously identified steroidal and non-steroidal compounds.

Furthermore, the compounds and pharmaceutical compositions of the present invention possess a number of advantages over previously identified steroid modulator compounds. For example, the compounds are extremely potent activators of PR and AR, preferably displaying 50% maximal activation of PR and/or AR at a concentration of less than 100 nM, more preferably at a concentration of less than 50 nM, more preferably yet at a concentration of less than 20 nM, and most preferably at a concentration of 10 nM or less. Also, the selective compounds of the present invention generally do not display undesired cross-reactivity with other steroid receptors, as is seen with the compound mifepristone (RU486; Roussel Uclaf), a known PR antagonist that displays an undesirable cross reactivity on GR and AR, thereby limiting its use in long-term, chronic administration. In addition, the compounds of the present invention, as small organic molecules, are easier to synthesize, provide greater stability and can be more easily administered in oral dosage forms than other known steroidal compounds.

The invention will be further illustrated by reference to the following non-limiting Examples.

General Method 1: biaryl coupling of an aryl boronic acid with 6-Bromo-1,2-dihydro-2,2,4-trimethylquinoline ( Compound **8**):

A 25 mL recovery flask equipped with a magnetic stir bar was charged with Compound **8** (1.0 equiv) in toluene (0.1 M).
Tetrakis(triphenylphosphine)palladium (0.03 equiv), boronic acid ($R^1B(OH)_2$) (1.3 equiv, 0.1 M in ethanol) and potassium carbonate (2.0 equiv, 2.0 M) were added to the reaction flask sequentially under a nitrogen atmosphere. A reflux condenser was fitted to the flask and the cloudy reddish solution was stirred rapidly and heated to reflux for about 4 h until the starting material had been completely consumed as judged by TLC (15% ethyl acetate/hexane). The product mixture was then cooled to room temperature and quenched with saturated $NH_4Cl$ (4-5 mL). Ethyl acetate (5 mL) was used to partition the mixture. The organic layer was rinsed with saturated $NH_4Cl$ (2 x 5 mL). The aqueous layers were extracted with ethyl acetate (5 mL). The organic layers were combined, dried ($Na_2SO_4$), and purified as indicated.

General Method 2: biaryl coupling of an arylbromide and (1-*tert*-butyloxycarbonyl-1,2-dihydro-2,2,4-trimethyl-6-quinolinyl)boronic acid (Compound **9**):

A 25 mL recovery flask equipped with a magnetic stirring bar, was charged with aryl bromide (1.0 equiv, 0.1 M in toluene). Tetrakis(triphenylphosphine)palladium (0.03-0.1 equiv), Compound **9** (1.0 equiv, 0.1 M in ethanol) and $K_2CO_3$ (2.0 equiv, 2.0 M) were added to the reaction flask sequentially under a nitrogen atmosphere. A reflux condenser was fitted to the flask and the cloudy reddish solution was stirred rapidly and heated to reflux for about 4 h until the starting material had been completely consumed as judged by TLC (15% ethyl acetate/hexane). The product mixture was then cooled to room temperature and quenched with saturated ammonium chloride (4-5 mL). Ethyl acetate (5 mL) was used to partition the mixture. The organic layer was rinsed with saturated ammonium chloride (2 x 5 mL). The aqueous layers were back extracted with ethyl acetate (5 mL). The organic layers were combined, dried ($Na_2SO_4$), and concentrated. A solution of the crude product in dichloromethane (0.1-0.3 M) was cooled to -20°C (ice/acetone) and treated with trifluoroacetic acid (10-40 equiv). Stirring was continued for 5-15 min and the reaction mixture was treated with excess saturated $NaHCO_3$. The product was extracted with ethyl acetate (3 x 5 mL).The extracts were washed with saturated $NaHCO_3$ (3 x 5 mL), combined, dried ($Na_2SO_4$), concentrated, and purified as indicated.

## EXAMPLE 45

5-Ethyl-1,2-dihydro-2,2,4-trimethylindolo[2,3-*f*]quinoline (Compound **145**, structure **29** of **Scheme VIII**, where $R^{1-6}$=H. $R^7$=Et)

To a suspension of sodium hydride (60% in mineral oil, 16 mg, 0.405 mmol) in 1 mL of THF at 0°C was slowly added 1,2-dihydro-2,2,4-trimethylindolo[2,3-*f*] quinoline (structure **28** of **Scheme VIII**, where $R^{1-6}$=H) (30 mg, 0.116 mmol) in 1 mL of THF and the resulting mixture was stirred at 0°C for 30 minutes. Iodoethane (9.3 mL, 0.116 mmol) was added dropwise *via* a microsyringe and the reaction mixture was brought to rt and stirred for 16 h. The reaction was quenched with 1 mL of water and extracted with 10 mL of ethyl acetate. The organic phase was dried ($Na_2SO_4$) and concentrated *in vacuo* to a residue that was purified by flash chromatography (silica gel, hexane/ethyl acetate, 9: 1) which gave 27 mg (81 %) of Compound **145.** Data for Compound **145:** $^1$**H NNIR** (400 MHz, $CDCl_3$) 7.81 (d, *J* = 7.1, 1H), 7.63 (d, *J* = 8.1; 1H); 7.34 (d, *J* = 8.0, 1H); 7.25 (br s, 1H); 7.12 (apparent t, *J* = 7.4, 1H); 6.51 (br s, 1H); 5.38 (br s, 1H); 4.25 (q, *J* = 7.0, 2H); 4.22 (br s, 1H); 2.16 (s, 3H); 1.26 (s, 6H); 0.91 (t, *J* = 7.0, 3H).

## EXAMPLE 59

1,2-Dihydro-2,2,4-trimethyl-5-coumarino[3,4-*f*]quinoline (Compound **159**, **Scheme IX**)

The intermediate 2-nitro-3,4-benzocoumarin was prepared by a modified literature procedure. See *J. Org. Chem*., U.S.S.R., 15 (3), 503 **(1979),** the disclosure of which is herein incorporated by reference. To a flask charged with 2-biphenylcarboxylic acid (5 g, 25 mmol) was added 7 mL of 70 % nitric acid and the resulting yellow slurry was stirred at rt for 30 min. To this slurry 20 mL of fuming nitric acid was introduced dropwise, giving rise to a clear yellow solution. The reaction mixture was stirred at rt for 15 h, and was then poured into ice water (100 mL). The crude mixture was extracted with ethyl acetate (3 x 60 mL) and the combined extracts were washed with water (2 x 20 mL) and brine (2 x 20 mL). Removal of solvent under reduced pressure afforded a crude yellow solid, which was a 2:1 mixture of two regioisomers. The mixture of the dinitrobiphenylcarboxylic acids was dissolved in 80 mL of DMA and the solution was

heated at reflux for 12 hours. The reaction was cooled to rt and diluted with 20 mL of water. The desired product precipitated from the solution upon standing at rt overnight. Filtration of the mixture afforded 2.9 g (50%) of 2-nitro-3,4-benzocoumarin, which was used directly in next reaction without further purification. 2-Nitro-3,4-benzocoumarin (2.9 g, 12 mmol) was dissolved in 600 mL of ethyl acetate and treated with 10% Pd/C (1.0 g, 0.94 mmol) and stirred under a hydrogen balloon for 24 h. Filtration of the catalyst and removal of solvent afforded 2.2 g (86%) of 2-amino-3,4-benzocoumarin as a yellowish solid. An Ace-Thred pressure tube charged with 2-amino-3,4-benzocoumarin (2.2 g, 10.4 mmol), iodine (0.8 g, 3.1 mmol) and acetone (150 mL) was sealed. The tube was heated in an oil bath at 80-120 °C for 24 h and then cooled to rt. The dark reaction mixture was concentrated under reduced pressure and the crude residue was purified by silica gel chromatography (hexane/EtOAc, 4/1) to give 1.5 g (50%) of Compound **159** as a yellow solid. Data for Compound **159**: mp 190-191 °C; **IR** (KBr) 3352, 2966, 2924, 1712, 1626, 1450, 1356, 1251, 1205; **$^1$H NMR** (400 MHz, $CDCl_3$) 7.90 (d, *J* = 7.8, I H), 7.78 (d, *J* = 8.4, 1 H), 7.38-7.22 (m, 3 H), 7.01 (d, *J* = 8.4, 1 H), 5.58 (s, 1 H), 4.31 (br s, 1 H), 2.12 (s, 3 H), 1.33 (s, 6 H); **$^{13}$C NMR** (100 MHz, $CDCl_3$) 160.3, 150.5, 145.7, 132.4, 131.6, 128.4, 124.2, 122.0, 121.4, 121.2, 119.3, 118.4, 117.2, 50.8, 29.9, 28.6; **Anal.** Calcd for $C_{19}H_{17}NO_2$: C, 78.33; H, 5.88; N, 4.81. Found: C, 78.19; H, 6.12; N, 4.52.

## EXAMPLE 60

(*R*/*S*)-5-Butyl-1,2-dihydro-2,2,4-trimethyl-*5H*-chromeno[3,4-*f*]quinoline (Compound **160**, structure **32** of **Scheme IX** where R=*n*-butyl)

GENERAL METHOD 5: Preparation of compounds of structures **32** and **33** from Compound **159**.

This transformation involved a two step sequence: addition of a nucleophile (either a commercial reagent or prepared *in situ* from a metal-halogen exchange reaction), followed by reduction of the resulting cyclic hemiacetal. To a solution of an aryl bromide compound in THF (0.1-0.3 M) at - 78 °C was slowly added 1.1 equiv. of *n*-BuLi (as a hexane solution) and the resulting reaction mixture was allowed to stir at -78°C until the anion was formed. A yellow solution (0.2-0.5 M) of Compound **159** in THF was cannulated into the above solution and the resulting dark red mixture was slowly allowed to warm. As soon as the red color faded (around - 30 °C), the reaction was quenched with water to give a light yellow solution. The reaction mixture was extracted with ethyl acetate and the combined extracts were washed with brine. Removal of solvent under reduced pressure and purification of the crude residue on a silica gel column using a 1:3 mixture of ethyl acetate and hexane as eluents afforded the hemiacetal intermediate as a yellow oil. To a solution of the hemiacetal intermediate in dichloromethane (0.1 M) at - 78°C was added 5-10 equiv of trifluoroacetic acid and triethylsilane (or, alternatively, 2-3 equiv of boron trifluoride etherate and 5-6 equiv of triethylsilane) and the resulting slurry was allowed to warm to rt, giving rise to a dark green solution. The mixture was allowed to stir at rt or reflux in some cases, until the reaction went to completion. The reaction was then quenched with 5% NaOH (aq) and was extracted with ethyl acetate. The combined extracts were washed with brine and concentrated. The crude mixture was purified on a silica gel column using a 1:5 mixture of ethyl acetate and hexane as eluents, affording the desired product in moderate yield. A second silica gel chromatography was needed in several cases to remove the silane oxide and/or separate the isomers of structures 32 and 33 using a 1:2 mixture of dichloromethane and hexane as eluents.

(*R*/*S*)-5-Butyl-1,2-dihydro-2,2,4-trimethyl-*5H*-chromeno[3,4-*f*]quinoline (Compound **160**, structure **32** of **Scheme IX** where R=*n*-butyl)

This compound was prepared by General Method 5 from *n*-BuLi (1.6 M, 0.2 mL) and Compound **159** (50 mg, 0.17 mmol) to afford 40 mg (71 %) of Compound **160** as a colorless oil. Data for Compound **160: IR** (neat) 3388, 2980, 1593, 1468 and 1435 $cm^{-1}$; **$^1$H NMR** (400 MHz, $CDCl_3$) 7.62 (d, *J* = 7.8, 1 H), 7.44 (d, *J* = 8.3, 1 H), 7.14 (t, *J* = 7.8, 1 H), 6.98 (t, *J* = 7.8, 1 H), 6.92 (d, *J* = 7.8, 1 H), 6.59 (d, *J* = 8.3, 1 H), 5.88 (dd, *J* = 9.8, 3.1, 1 H), 5.49 (s, 1 H), 3.88 (br s, 1 H), 2.25 (s, 3 H), 1.90-1.79 (m, 1 H), 1.55-1.25 (m, 5 H), 1.28 (s, 3 H), 1.20 (s, 3 H), 0.84 (t, *J* = 7.3, 3 H).

## EXAMPLE 61

(*R*/*S*)-1,2-Dihydro-2,2,4-trimethyl-5-phenyl-*5H*-chromeno[3,4-*f*]quinoline (Compound **161**, structure **32** of **Scheme IX**, where R=phenyl)

This compound was prepared by General Method 5 (EXAMPLE 60) from bromobenzene (0.15 mL, 1.4 mmol) and Compound **159** (50 mg, 0.17 mmol) to afford 15 mg (25%) of Compound **161** as a colorless oil, along with 6 mg (10%) of Compound **162** (EXAMPLE 62). Data for Compound **161**: **$^1$H NMR** (400 MHz, $CDCl_3$) 7.53 (d, *J* = 7.8, 1 H),

7.50 (d, $J$ = 8.2, 1 H), 7.22-7.12 (m, 5 H), 7.00 (t, $J$ = 7.8, 1 H), 6.92 (s, 1 H), 6.88 (t, $J$ = 7.8, 1 H), 6.83 (d, $J$ = 7.8, 1 H), 6.69 (d, $J$ = 8.2, 1 H), 5.46 (s, 1 H), 3.92 (brs, 1 H), 1.99 (s, 3 H), 1.29 (s, 3 H), 1.26 (s, 3 H).

**EXAMPLE 62**

(*R*/*S*)-1,2,3,4-Tetrahydro-2,2-dimethyl-4-methylidene-5-phenyl-*5H*-chromeno[3,4-*f*]quinoline (Compound **162**, structure **33** of **Scheme IX**, where R=phenyl)

This compound (6 mg, 10%) was obtained along with Compound **161** as described above (EXAMPLE 61). Data for Compound **162:** $^{1}$**H NMR** (400 MHz, $CDCl_3$) 7.53 (d, $J$ = 7.3, 1 H), 7.51 (d, $J$ = 8.4, 1 H), 7.24-7.12 (m, 5 H), 6.97 (t, $J$ = 7.3, 1 H), 6.87 (t, $J$ = 7.3, 1 H), 6.80 (d, $J$ = 7.3, 1 H), 6.64 (s, 1 H), 6.59 (d, $J$ = 8.4, 1 H), 4.93 (s, 1 H), 4.64 (s, 1 H), 4.09 (br s, 1 H), 2.44 (d, $J$ = 12.1, 1 H), 2.18 (d, $J$ = 12.1, 1 H), 1.34 (s, 3 H) and 1.13 (s, 3 H).

**EXAMPLE 63**

(*R*/*S*)-5-(4-Chlorophenyl)-1,2-dihydro-2,2,4-trimethyl-*5H*-chromeno[3,4-*f*]quinoline (Compound **163**, structure **32** of **Scheme IX**, where R=4-chlorophenyl)

This compound was prepared by General Method 5 (EXAMPLE 60) from 4-bromochlorobenzene (1.4 g, 7 mmol) and Compound **159** (0.5 g, 1.7 mmol) to afford 0.27 g (40%) of Compound **163** as a white solid, in addition to 60 mg (9%) of Compound **164** (EXAMPLE 64). Data for Compound **163:** mp 139-140 °C; **IR** (KBr) 3371, 2964, 1593, 1469, 1435 $cm^{-1}$; $^{1}$**H NMR** (400 MHz, acetone-$d_6$) 7.59 (d, $J$ = 7.8, 1 H), 7.56 (d, $J$ = 8.4, 1 H), 7.24 (d, $J$ = 9.1, 2 H), 7.21 (d, $J$ = 9.1, 2 H), 6.98 (t, $J$ = 7.8, 1 H), 6.92 (s, 1 H), 6.86 (t, $J$ = 7.8, 1 H), 6.83 (d, $J$ = 8.4, 1 H), 6.77 (d, $J$ = 7.8, 1 H), 5.54 (br s, 1 H), 5.48 (s, 1 H), 1.99 (s, 3 H), 1.26 (s, 3 H), 1.24 (s, 3 H).

**EXAMPLE 64**

(*R*/*S*)-5-(4-Chlorophenyl)-1,2,3,4-tetrahydro-2,2-dimethyl-4-methylidene-*5H*-chromeno[3,4-*f*]quinoline (Compound **164**, structure **33** of **Scheme IX**, where R=4-chlorophenyl)

This compound (60 mg, 9%) was obtained along with Compound **163** as described above (EXAMPLE 63). Data for Compound **164:** $^{1}$**H NMR** (400 MHz, $CDCl_3$) 7.53 (d, $J$ = 7.7, 1 H), 7.51 (d, $J$ = 8.3, 1 H), 7.18 (d, $J$ = 8.7, 2 H), 7.15 (d, $J$ = 8.7, 2 H), 6.99 (t, $J$ = 7.7, 1 H), 6.90 (t, $J$ = 7.7, 1 H), 6.79 (d, $J$ = 7.7, 1 H), 6.59 (s, 1 H), 6.58 (d, $J$ = 8.3, 1 H), 4.93 (s, 1 H), 4.59 (s, 1 H), 4.09 (br s, 1 H), 2.43 (d, $J$ = 12.3, 1 H), 2.18 (d, $J$ = 12.3, 1 H), 1.34 (s, 3 H) 1.13 (s, 3 H).

**EXAMPLE 65**

(*R*/*S*)-5-(4-Fluorophenyl)-1,2-dihydro-2,2,4-trimethyl-*5H*-chromeno[3,4-*f*]quinoline (Compound **165**, structure **32** of **Scheme IX**, where R=4-fluorophenyl)

This compound was prepared by General Method 5 (EXAMPLE 60) from 4-fluorophenylmagnesium bromide (1.0 M in THF, 1 mL) and Compound **159** (30 mg, 0.1 mmol) to afford 15 mg (38%) of Compound **165** as a colorless oil. Data for Compound **165: IR** (KBr) 3360, 2962, 1707, 1601, 1506, 1469, 1221, 1157 $cm^{-1}$; $^{1}$**H NMR** (400 MHz, acetone-$d_6$) 7.60 (d, $J$ = 7.8, 1 H), 7.56 (d, $J$ = 8.3, 1 H), 7.26 (dd, $J$ = 8.7, 5.7, 2 H), 6.98 (t, $J$ = 8.7, 2 H), 6.97 (t, $J$ = 7.8, 1 H), 6.92 (s, 1 H), 6.87 (t, $J$ = 7.8, 1 H), 6.83 (d, $J$ = 8.3, 1 H), 6.76 (d, $J$ = 7.8, 1 H), 5.54 (br s, 1 H), 5.47 (s, 1 H), 1.99 (s, 3 H), 1.26 (s, 3 H), 1.24 (s, 3 H).

**EXAMPLE 66**

(*R*/*S*)-5-(4-Acetylphenyl)-1,2-dihydro-2,2,4-trimethyl-*5H*-chromeno[3,4-*f*]quinoline (Compound **166**, structure **32** of **Scheme IX**, where R=4-acetylphenyl)

This compound was prepared by General Method 5 (EXAMPLE 60) from 2-(4-bromophenyl)-2-methyl-1,3-dioxane (219 mg, 1.0 mmol) and Compound **159** (30 mg, 0.1 mmol) to afford 4.5 mg (10%) of Compound **166** as a colorless oil. Data for Compound **166:** $^{1}$**H NMR** (400 MHz, acetone-$d_6$) 7.83 (d, $J$ = 8.3, 2 H), 7.60 (d, $J$ = 7.6, 1 H), 7.57 (d, $J$ = 8.4, 1 H), 7.36 (d, $J$ = 8.3, 2 H), 6.99 (s, 1 H), 6.98 (t, $J$ = 7.6, 1 H), 6.89-6.79 (m, 3 H), 5.56 (br s, 1 H), 5.50 (s, 1 H), 2.49 (s, 3 H), 2.00 (s, 3 H), 1.28 (s, 3 H), 1.25 (s, 3 H).

**EXAMPLE 67**

(*R*/*S*)-1,2-Dihydro-2,2,4-trimethyl-5-(4-methylphenyl)-*5H*-chromeno[3,4-*f*]quinoline (Compound **167**, structure **32** of **Scheme IX**, where R=4-methylphenyl)

This compound was prepared by General Method 5 (EXAMPLE 60) from 4-bromotoluene (171 mg, 1.0 mmol) and Compound **159** (20 mg, 0.07 mmol) to afford 15 mg (58%) of Compound **167** as a colorless oil. Data for Compound **167: IR** (KBr) 3362, 2964, 1707, 1593, 1469, 1437, 1259, 1169 $cm^{-1}$; $^1$H NMR (400 MHz, acetone-$d_6$) 7.58 (d, *J* = 7.9, 1 H), 7.54 (d, *J* = 8.5, 1 H), 7.10 (d, *J* = 8.0, 2 H), 7.00 (d, *J* = 8.0, 2 H), 6.97 (t, *J* = 7.9, 1 H), 6.89 (s, 1 H), 6.84 (d, *J* = 7.9, 1 H), 6.81 (d, *J* = 8.5, 1 H), 6.75 (d, *J* = 7.9, 1 H), 5.47 (bs, 1 H), 5.45 (s, 1 H), 2.19 (s, 3 H), 1.99 (s, 3 H ), 1.25 (s, 3 H), 1.23 (s, 3 H).

**EXAMPLE 68**

(*R*/*S*)-1,2-Dihydro-5-(4-methoxyphenyl)-2,2,4-trimethyl-*5H*-chromeno[3,4-*f*]quinoline (Compound **168**, structure **32** of **Scheme IX**, where R=4-methoxyphenyl)

This compound was prepared by General Method 5 (EXAMPLE 60) from 4-bromoanisole (187 mg, 1.0 mmol) and Compound **159** (10 mg, 0.03 mmol) to afford 2.5 mg (10%) of Compound **168** as a colorless oil. Data for Compound **168: $^1$H NMR** (400 MHz, acetone-$d_6$) 7.59 (d, *J* = 7.7, 1 H), 7.54 (d, *J* = 8.4, 1 H), 7.13 (d, *J* = 8.7, 2 H), 6.95 (t, *J* = 7.7, 1 H), 6.87 (s, 1 H), 6.86 (d, *J* = 7.7, 1 H), 6.81 (d, *J* = 8.4, 1 H), 6.75 (d, *J* = 8.7, 2 H), 6.74 (t, *J* = 7.7, 1 H), 5.47 (br s, 1 H), 5.45 (s, 1 H), 3.69 (s, 3 H), 1.99 (s, 3 H ), 1.25 (s, 3 H), 1.23 (s, 3 H).

**EXAMPLE 69**

(*R*/*S*)-1,2-Dihydro-2,2,4-trimethyl-5-[4-(trifluoromethyl)phenyl]-*5H*-chromeno[3,4-*f*]quinoline (Compound **169**, structure **32** of **Scheme IX**, where R=4-(trifluoromethyl)phenyl)

This compound was prepared by General Method 5 (EXAMPLE 60) from 4-bromobenzotrifluoride (130 mg, 1.0 mmol) and Compound **159** (20 mg, 0.07 mmol) to afford 10 mg (35%) of Compound **169** as a colorless oil. Data for Compound **169: $^1$H NMR** (400 MHz, acetone-$d_6$) 7.61-7.56 (m, 4 H), 7.45 (d, *J* = 8.3, 2 H), 7.01 (s, 1 H), 6.97 (d, *J* = 7.7, 1 H), 6.86 (t, *J* = 7.7, 1 H), 6.85 (d, *J* = 8.4, 1 H), 6.81 (d, *J* = 7.7, 1 H), 5.57 (br s, 1 H), 5.49 (s, 1 H), 1.99 (s, 3 H), 1.27 (s, 3 H), 1.25 (s, 3 H).

**EXAMPLE 70**

(*R*/*S*)-1,2-Dihydro-2,2,4-trimethyl-5-(thiophen-3-yl)-*5H*-chromeno[3,4-*f*]quinoline (Compound **170**, structure **32** of **Scheme IX**, where R=thiophen-3-yl)

This compound was prepared by General Method 5 (EXAMPLE 60) from 3-bromothiophene (163 mg, 1.0 mmol) and Compound **159** (8 mg, 0.03 mmol) to afford 1.1 mg (11%) of Compound **170** as a colorless oil. Data for Compound **170: $^1$H NMR** (400 MHz, acetone-$d_6$) 7.60 (d, *J* = 7.3, 1 H), 7.54 (d, *J* = 8.4, 1 H), 7.31 (dd, *J* = 5.0, 3.0, 1 H), 7.08 (d, *J* = 5.0, 1 H), 6.98 (t, *J* = 7.3, 1 H), 6.93 (s, 1 H), 6.89 (t, *J* = 7.3, 1 H), 6.88 (d, *J* = 3.0, 1 H), 6.79 (d, *J* = 8.1, 2 H), 5.48 (br s, 1 H), 2.06 (s, 3 H), 1.25 (s, 3 H), 1.24 (s, 3 H).

**EXAMPLE 71**

(-)-1,2-Dihydro-2,2,4-trimethyl-5-(4-methylphenyl)-*5H*-chromeno[3,4-*f*]quinoline (Compound **171**, structure **32** of **Scheme IX**, where R=4-methylphenyl)

This compound was prepared by optical resolution of Compound **167** via HPLC using a chiral column, Chiracel OD-R, using a 9:1 mixture of methanol and water as the mobile phase. The optical purity of Compound **171** was determined by HPLC to be > 99% *e.e.*; $[\alpha]^{20}_D$ = - 246 (MeOH).

**EXAMPLE 72**

(-)-5-(4-Chlorophenyl)-1,2-dihydro-2,2,4-trimethyl-*5H*-chromeno[3,4-*f*]quinoline (Compound **172**, structure **32** of **Scheme IX**, where R=4-chlorophenyl)

This compound was prepared by optical resolution of Compound **163** via HPLC using a chiral column, Chiracel OD-R, using a 9:1 mixture of methanol and water as mobile phase. The optical purity of Compound **172** was determined by HPLC to be > 99% *e.e.*; $[\alpha]^{20}_D$ = - 254 (MeOH).

**EXAMPLE 73**

(*R*/*S*)-1,2-Dihydro-2,2,4-trimethyl-5-(3-methylphenyl)-*5H*-chromeno[3,4-*f*]quinoline (Compound **173**, structure **32** of **Scheme IX**, where R=3-methylphenyl)

This compound was prepared by General Method 5 (EXAMPLE 60) from 3-bromotoluene (171 mg, 1.0 mmol) and Compound **159** (15 mg, 0.05 mmol) to afford 3.6 mg (19%) of Compound **173** as a colorless oil. Data for Compound **173: $^1$H NMR** (400 MHz, acetone-$d_6$) 7.59 (d, *J* = 7.8, 1 H), 7.54 (d, *J* = 8.4, 1 H), 7.10-6.94 (m, 5 H), 6.89 (s, 1 H), 6.85 (d, *J* = 7.8, 1 H), 6.82 (d, *J* = 8.4, 1 H), 6.77 (d, *J* = 8.0, 1 H), 5.49 (br s, 1 H), 5.46 (s, 1 H), 2.19 (s, 3 H), 2.00 (s, 3 H ), 1.26 (s, 3 H), 1.24 (s, 3 H).

**EXAMPLE 74**

(+)-(4*l*,5*l*)-5-(4-Chlorophenyl)-1,2,3,4-tetrahydro-2,2,4-trimethyl-*5H*-chromeno[3,4-*f*]quinoline (Compound **174**, structure **34** of **Scheme X**, where R=4-chlorophenyl)

Hydrogenation of Compound **163** (15 mg, 0.04 mmol) in the presence of 10% Pd/C (10%) afforded 12 mg (80%) of (*R*/*S*-4*l*,5*l*)-5-(4-chlorophenyl)-1,2,3,4-tetrahydro-2,2,4-trimethyl-*5H*-chromeno[3,4-*f*]quinoline as a white solid in addition to 1.1 mg (7%) of Compound **176** (EXAMPLE 76) as a white solid. The enantiomers of (*R*/*S*-4*l*,5*l*)-5-(4-chlorophenyl)-1,2,3,4-tetrahydro-2,2,4-trimethyl-*5H*-chromeno[3,4-*f*]quinoline were resolved via HPLC using a chiral column, Chiracel OD-R, using a 9:1 mixture of methanol and water as mobile phase (0.55 mL/min). A 10 mg sample of (*R*/*S*-4*l*,5*l*)-5-(4-chlorophenyl)-1,2,3,4-tetrahydro-2,2,4-trimethyl-*5H*-chromeno[3,4-*f*]quinoline afforded 3.1 mg of the first eluting, (+) enantiomer (Compound **174**) (24 min), and 3.0 mg of the second eluting, (-) enantiomer (Compound **175**, EXAMPLE 75) (30 min). The optical purity of Compound **174** was determined by HPLC to be >99% *e.e*. Data for Compound **174**: mp 158-159 °C; **$^1$H NMR** (400 MHz, acetone-$d_6$) 7.63 (d, *J* = 7.8, 1 H), 7.53 (d, *J* = 8.5, 1 H), 7.24 (s, 4 H), 6.94 (t, *J* = 7.8, 1 H), 6.87 (t, *J* = 7.8, 1 H), 6.76 (d, *J* = 8.5, 1 H), 6.68 (d, *J* = 7.8, 1 H), 6.51 (s, 1 H), 5.10 (br s, 1 H), 3.25 (m, 1 H), 1.89 (dd, *J* = 13.5, 6.4, 1 H), 1.76 (dd, *J* = 13.5, 4.4, 1 H), 1.30 (s, 3 H ), 1.21 (s, 3 H), 0.83 (d, *J* = 7.3, 3 H); **$^{13}$C NMR** (100 MHz, $CDCl_3$) 150.6, 144.5, 138.6, 134.0, 130.9, 130.5, 128.4, 127.6, 124.9, 123.2, 122.2, 121.9, 120.2, 118.0, 115.8, 74.5, 50.0, 44.3, 31.6, 31.3, 27.5, 22.8. $[\alpha]^{20}_D$ = + 287 (MeOH).

**EXAMPLE 75**

(-)-(4*l*,5*l*)-5-(4-Chlorophenyl)-1,2,3,4-tetrahydro-2,2,4-trimethyl-*5H*-chromeno[3,4-*f*]quinoline (Compound **175**, structure **34** of **Scheme X**, where R=4-chlorophenyl)

This compound was prepared by resolution of (*R*/*S*-4*l*,5*l*)-5-(4-chlorophenyl)-1,2,3,4-tetrahydro-2,2,4-trimethyl-*5H*-chromeno[3,4-*f*]quinoline as described above (EXAMPLE 74) via HPLC using a chiral column, Chiracel OD-R, using a 9:1 mixture of methanol and water as mobile phase. The optical purity of Compound **175** was determined by HPLC to be > 95% *e.e.*; $[\alpha]^{20}_D$ = - 260 (MeOH).

**EXAMPLE 76**

(*R*/*S*-4*l*,5*u*)-5-(4-Chlorophenyl)-1,2,3,4-tetrahydro-2,2,4-trimethyl-*5H*-chromeno[3,4-*f*]quinoline (Compound **176**, structure **35** of **Scheme X**, where R=4-chlorophenyl)

This compound (1.1 mg, 7%) was obtained along with (*R*/*S*-4*l*,5*l*)-5-(4-chlorophenyl)-1,2,3,4-tetrahydro-2,2,4-trimethyl-*5H*-chromeno[3,4-*f*]quinoline as described above (EXAMPLE 75). Data for Compound **176: $^1$H NMR** (400 MHz, $CDCl_3$) 7.54 (d, *J* = 7.6, 1 H), 7.47 (d, *J* = 8.4, 1 H), 7.15 (d, *J* = 6.5,2 H), 7.10 (d, *J* = 6.5, 2 H), 7.01 (t, *J* = 7.6, 1 H), 6.89 (t, *J* = 7.6, 1 H), 6.83 (d, *J* = 7.6, 1 H), 6.59 (d, *J* = 8.4, 1 H), 6.47 (s, 1 H), 3.73 (br s, 1 H), 2.82 (m, 1

H), 1.76 (dd, *J* = 13.5, 7.0, 1 H), 1.73 (dd, *J* = 13.5, 4.5, 1 H), 1.46 (d, *J* = 7.1, 3 H), 1.36 (s, 3 H), 1.19 (s, 3 H); **$^{13}$C NMR** (100 MHz, $CDCl_3$) 150.5, 143.9, 138.4, 134.0, 130.3, 129.4, 128.6, 127.6, 124.2, 122.6, 122.1, 119.6, 118.0, 115.4, 74.4, 50.1, 42.9, 32.2,31.8,27.3,22.3.

**EXAMPLE 77**

(*R*/*S*)-5-(3-Chlorophenyl)-1,2-dihydro-2,2,4-trimethyl-5*H*-chromeno[3,4-*f*]quinoline (Compound **177**, structure **32** of **Scheme IX**, where R=3-chlorophenyl)

This compound was prepared by General Method 5 (EXAMPLE 60) from 3-bromochlorobenzene (195 mg, 1.0 mmol) and Compound **159** (20 mg, 0.07 mmol) to afford 14 mg (52%) of Compound **177** as a colorless oil, along with 2.3 mg (7%) of Compound **178** (EXAMPLE 78) as a colorless oil. Data for Compound **177: $^1$H NMR** (400 MHz, acetone-$d_6$) 7.61 (d, *J* = 7.8, 1 H), 7.57 (d, *J* = 8.4, 1 H), 7.28-7.18 (m, 4 H), 7.00 (t, *J* = 7.8, 1 H), 6.95 (s, 1 H), 6.89 (d, *J* = 7.8, 1 H), 6.84 (d, *J* = 8.4, 1 H), 6.82 (d, *J* = 8.1, 1 H), 5.58 (br s, 1 H), 5.49 (s, 1 H), 2.01 (s, 3 H), 1.27 (s, 3 H), 1.25 (s, 3 H).

**EXAMPLE 78**

(*R*/*S*)-5-(3-Chlorophenyl)-1,2,3,4-tetrahydro-2,2-dimethyl-4-methylidene-5*H*-chromeno[3,4-*f*]quinoline (Compound **178**, structure **33** of **Scheme IX**, where R=3-chlorophenyl)

This compound (2.3 mg, 7%) was obtained along with Compound **177** as described above (EXAMPLE 77). Data for Compound **178: $^1$H NMR** (400 MHz, acetone-$d_6$) 7.61 (d, *J* = 6.7, 1 H), 7.59 (d, *J* = 8.6, 1 H), 7.29-7.20 (m, 4 H), 6.98 (t, *J* = 6.7, 1 H), 6.88 (t, *J* = 6.7, 1 H), 6.79 (d, *J* = 6.7, 1 H), 6.77 (d, *J* = 8.6, 1 H), 6.62 (s, 1 H), 4.99 (s, 1 H), 4.59 (s, 1 H), 2.41 (d, *J* = 12.2, 1 H), 2.27 (d, *J* = 12.2, 1 H), 1.35 (s, 3 H), 1.13 (s, 3 H).

**EXAMPLE 79**

(*R*/*S*)-5-(4-Bromophenyl)-1,2-dihydro-2,2,4-trimethyl-*5H*-chromeno[3,4-*f*]quinoline (Compound **179**, structure **32** of **Scheme IX**, where R=4-bromophenyl)

This compound was prepared by General Method 5 (EXAMPLE 60) from 1,4-dibromobenzene (250 mg, 1.0 mmol) and Compound **159** (20 mg, 0.07 mmol) to afford 16 mg (54%) of Compound **179** as a colorless oil, along with 2.5 mg (8%) of Compound **180** (EXAMPLE 80) as a colorless oil. Data for Compound **179:** $^1$H NMR (400 MHz, acetone-$d_6$) 7.58 (d, *J* = 7.8, 1 H), 7.55 (d, *J* = 8.4, 1 H), 7.39 (d, *J* = 8.5, 2 H), 7.16 (d, *J* = 8.5, 2 H), 6.98 (t, *J* = 7.8, 1 H), 6.90 (s, 1 H), 6.86 (t, *J* = 7.8, 1 H), 6.83 (d, *J* = 8.4, 1 H), 6.77 (d, *J* = 7.8, 1 H), 5.54 (br s, 1 H), 5.47 (s, 1 H), 1.99 (s, 3 H), 1.26 (s, 3 H), 1.23 (s, 3 H).

**EXAMPLE 80**

(*R*/*S*)-5-(4-Bromophenyl)-1,2,3,4-tetrahydro-2,2-dimethyl-4-methylidene-*5H*-chromeno[3,4-*f*]quinoline (Compound **180**, structure **33** of **Scheme IX**, where R=4-bromophenyl)

This compound (2.5 mg, 8%) was obtained along with Compound **179** as described above (EXAMPLE 79). Data for Compound **180: $^1$H NMR** (400 MHz, acetone-$d_6$) 7.61 (d, *J* = 6.3, 1 H), 7.59 (d, *J* = 8.7, 1 H), 7.41 (d, *J* = 8.5, 2 H), 7.19 (d, *J* = 8.5, 2 H), 6.95 (t, *J* = 6.3, 1 H), 6.86 (t, *J* = 6.3, 1 H), 6.75 (d, *J* = 8.7, 1 H), 6.57 (s, 1 H), 4.97 (s, 1 H), 4.80 (s, 1 H), 2.40 (d, *J* = 12.2, 1 H), 2.26 (d, *J* = 12.2, 1 H), 1.34 (s, 3 H), 1.11 (s, 3 H).

**EXAMPLE 81**

(*R*/*S*)-5-(3-Bromophenyl)-1,2-dihydro-2,2,4-trimethyl-*5H*-chromeno[3,4-*f*]quinoline (Compound **181**, structure **32** of **Scheme IX**, where R=3-bromophenyl)

This compound was prepared by General Method 5 (EXAMPLE 60) from 1,3-dibromobenzene (250 mg, 1.0 mmol) and Compound **159** (15 mg, 0.05 mmol) to afford 13 mg (60%) of Compound **181** as a colorless oil, along with 2.0 mg (9%) of Compound **182** (EXAMPLE 82) as a colorless oil. Data for Compound **181: IR** (neat) 3364, 2962, 1699, 1591, 1469, 143 $cm^{-1}$; **$^1$H NMR** (400 MHz, acetone-$d_6$) 7.61 (d, *J* = 7.8, 1 H), 7.57 (d, *J* = 8.4, 1 H), 7.38 (s, 1 H), 7.36 (d, *J* = 8.5, 1 H), 7.26 (d, *J* = 6.6, 1 H), 7.19 (t, *J* = 7.8, 1 H), 7.00 (t, *J* = 8.3, 1 H), 6.98 (s, 1 H), 6.81-6.90 (m, 3 H),

5.60 (br s, 1 H), 5.50 (s, 1 H), 2.01 (s, 3 H), 1.27 (s, 3 H), 1.25 (s, 3 H).

## EXAMPLE 82

(*R*/*S*)-5-(3-Bromophenyl)-1,2,3,4-tetrahydro-2,2-dimethyl-4-methylidene-*5H*-chromeno[3,4-*f*]quinoline (Compound **182**, structure **33** of **Scheme IX**, where R=3-bromophenyl)

This compound (2.0 mg, 9%) was obtained along with Compound **181** as described above (EXAMPLE 81). Data for Compound **182:** $^1$**H NMR** (400 MHz, $CDCl_3$) 7.55 (d, *J* = 7.9, 1 H), 7.51 (d, *J* = 8.4, 1 H), 7.28 (d, *J* = 8.0,1 H), 7.12 (d, *J* = 7.9, 1 H), 7.05 (d, *J* = 7.8, 1 H), 7.01 (t, *J* = 7.8, 1 H), 6.92 (t, *J* = 7.4, 1 H), 6.82 (d, *J* = 8.0, 1 H), 6.60 (d, *J* = 8.5, 1 H), 6.59 (s, 1 H), 4.95 (s, 1 H), 4.58 (s, 1 H), 2.43 (d, *J* = 12.3, 1 H), 2.19 (d, *J* = 12.3, 1 H), 1.32 (s, 3 H), 1.14 (s, 3 H).

## EXAMPLE 83

(*R*/*S*)-5-(3,4-Dichlorophenyl)-1,2-dihydro-2,2,4-trimethyl-*5H*-chromeno[3,4-*f*]quinoline (Compound **183**, structure **32** of **Scheme IX**, where R=3,4-dichlorophenyl)

This compound was prepared by General Method 5 (EXAMPLE 60) from 1-bromo-3,4-dichlorobenzene (226 mg, 1.0 mmol) and Compound **159** (20 mg, 0.07 mmol) to afford 8.7 mg (30%) of Compound **183** as a colorless oil. Data for Compound **183:** $^1$**H NMR** (400 MHz, $CDCl_3$) 7.53 (d, *J* = 7.8, 1 H), 7.50 (d, *J* = 8.3, 1 H), 7.28-7.22 (m, 2 H), 7.20-7.12 (m, 2 H), 6.92 (t, *J* = 7.5, 1 H), 6.85 (d, *J* = 8.2, 1 H), 6.83 (s, 1 H), 6.71 (d, *J* = 8.4, 1 H), 5.48 (s, 1 H), 4.0 (br s, 1 H), 1.97 (s, 3 H), 1.30 (s, 3 H), 1.26 (s, 3 H).

## EXAMPLE 84

(*R*/*S*)-5-(3-Bromo-2-pyridyl)-1,2-dihydro-2,2,4-trimethyl-*5H*-chromeno[3,4-*f*]quinoline (Compound **184**, structure **32** of **Scheme IX**, where R=3-bromo-2-pyridyl)

This compound was prepared by General Method 5 (EXAMPLE 60) from 2,6-dibromopyridine (237 mg, 1.0 mmol) and Compound **159** (20 mg, 0.07 mmol) to afford 20 mg (67%) of Compound **184** as a colorless oil. Data for Compound **184:** $^1$**H NMR** (400 MHz, acetone-$d_6$) 7.63 (dd, *J* = 7.8, 1.5, 1 H), 7.54 (d, *J* = 8.5, 1 H), 7.52 (d, *J* = 7.8, 1 H), 7.39 (d, *J* = 7.9, 1 H), 7.13 (d, *J* = 7.6, 1 H), 7.03 (t, *J* = 7.6, 1 H), 6.92-6.80 (m, 4 H), 5.52 (s, 1 H), 5.48 (s, 1 H), 2.03 (s, 3 H), 1.25 (s, 3 H), 1.24 (s, 3 H).

## EXAMPLE 85

(*R*/*S*)-1,2-Dihydro-5-hydroxy-2,2,4-trimethyl-*5H*-chromeno[3,4-*f*]quinoline (Compound **185**, structure **46** of **Scheme XIV**, where $R^1$=$R^2$=H)

To a yellow solution of Compound **159** (20 mg, 0.07 mmol) in 1 mL toluene at -78 °C was added 0.10 mL of DIBALH (1.5 M in toluene, 0.075 mmol) and the resulting solution was stirred at -50 ± 10 °C for 20 min. The reaction was quenched with water (1 mL) and was extracted with ethyl acetate (2 x 5 mL). Removal of solvent and chromatography of the crude residue on a silica gel column using 20% ethyl acetate/hexane as eluents provided 6 mg (30%) of Compound **185** as a colorless oil. Data for Compound **185**: $^1$**H NMR** (400 MHz, $CDCl_3$) 7.71 (d, *J* = 7.5, 1 H), 7.53 (d, *J* = 8.4, 1 H), 7.19 (t, *J* = 7.5, 1 H), 7.08 (t, *J* = 7.5, 1 H), 7.07 (d, *J* = 8.4, 1 H), 6.85 (d, *J* = 5.8, 1 H), 6.70 (d, *J* = 7.5, 1 H), 5.52 (s, 1 H), 3.92 (br s, 1 H), 2.94 (d, *J* = 5.8, 1 H), 2.37 (s, 3 H), 1.32 (s, 3 H), 1.20 (s, 3 H).

## EXAMPLE 86

(*R*/*S*)-1,2-Dihydro-2,2,4-trimethyl-5-methoxy-*5H*-chromeno[3,4-*f*]quinoline (Compound **186**, structure **47** of **Scheme XIV**, where $R^1$=$R^2$=H, X=O, $R^3$=methyl)

To a solution of Compound **185** (25 mg, 0.085 mmol) in MeOH (7 mL) was added a catalytic amount of *p*-toluenesulphonic acid (~0.25 mg) and the solution was allowed to stir at rt for 5 min. The reaction mixture was quenched with a 10% NaOH solution (0.1 mL) then partitioned between EtOAc (10 mL) and water (3 mL). The organic layer was separated and washed with water (3 x 1 mL) and brine (3 x 1 mL) then dried ($Na_2SO_4$) and concentrated *in vacuo* . The crude product was purified on a 20 x 20 cm, 250 µm. TLC plate, eluting with 25% EtOAc: hexane to afford 8.2 mg

(32%) of Compound **186** as a colorless oil. Data for Compound **186:** $R_f$ = 0.28 (silica gel, 25% EtOAc: Hexane); **$^1$H NMR** (400 MHz, $CDCl_3$) 7.69 (d, *J* = 7.7, 1 H), 7.48 (d, *J* = 8.3, 1 H), 7.15 (t, *J* = 7.7, 1 H), 7.05 (m, 2 H), 6.65 (d, *J* = 8.3, 1 H), 6.35 (s, 1 H), 5.50 (s, 1 H), 3.90 (br s, 1 H), 3.49 (s, 3 H), 2.28 (s, 3 H), 1.33 (s, 3 H), 1.28 (s, 3 H).

**EXAMPLE 87**

(*R*/*S*)-1,2-Dihydro-2,2,4-trimethyl-5-propoxy-*5H*-chromeno[3,4-*f*]quinoline (Compound **187**, structure **47** of **Scheme XIV**, where $R^1$=$R^2$=H, X=O, $R^3$=*n*-propyl)

This compound was prepared in a manner similar to that of Compound **186** (EXAMPLE 86) from Compound **185** (12 mg) and *n*-propanol to afford 7.2 mg (57%) of Compound **187** as a colorless oil. Data for Compound **187:** $R_f$ = 0.43 (silica gel, 25% EtOAc: hexane); **$^1$H NMR** (400 MHz, $CDCl_3$) 7.68 (d, *J* = 7.7, 1 H), 7.49 (d, *J* = 8.3, I H), 7.17 (t , *J* = 7.6, 1 H), 7.05 (m, 2 H), 6.65 (d, *J* = 8.4, 1 H), 6.42 (s, 1 H), 5.50 (s, 1 H), 3.90 (br s, 1 H), 3.84 (dt, *J* = 9.2, 6.7, 1 H), 3.54 (dt, *J* = 9.3, 6.8, 1 H), 2.28 (s, 3 H), 1.49 (m, 2 H), 1.33 (s, 3 H), 1.18 (s, 3 H), 0.77 (t, *J* = 7.4, 3 H).

**EXAMPLE 88**

(*R*/*S*)-5-Allyl-1,2-dihydro-2,2,4-trimethyl-*5H*-chromeno[3,4-*f*]quinoline (Compound **188**, structure **48** of **Scheme XIV**, where $R^1$=$R^2$=$R^4$=$R^5$=$R^6$=H)

To a solution of Compound **186** (12 mg, 0.04 mmol) in dichloromethane (1.5 mL) at 0° C was added allyltrimethylsilane (0.005 mL, 0.062 mmol) and TMSOTf (0.01 mL, 0.057 mmol) under nitrogen. The reaction was stirred 5 h at rt. The reaction mixture was concentrated *in vacuo* and purified on a 5x20 cm, 250 μM, TLC plate, eluting with 25 % EtOAc in hexane to afford 2.3 mg (18%) of Compound **188** as a colorless oil. Data for Compound **188:** $R_f$ = 0.50 (silica gel, 25% EtOAc: Hexane); **$^1$H NMR** (400 MHz, acetone-$d_6$) 7.67 (d, *J* = 7.4, 1 H), 7.49 (d, *J* = 8.3, 1 H), 7.12 (t, *J* = 7.4, 1 H), 6.98 (t, *J* = 7.4, 1 H), 6.87 (d, *J* = 7.4, 1 H), 6.70 (d, *J* = 8.3, 1 H), 5.96-5.85 (m, 2 H), 5.52 (s, 1 H), 5.04 (s, 1 H), 5.00 (d, *J* = 8.6, 1 H), 2.54 (m, 1 H), 2.25 (m, 4 H), 1.27 (s, 3 H), 1.18 (s, 3 H).

**EXAMPLE 89**

(*R*/*S*)-1,2-Dihydro-2,2,4-trimethyl-5-propyl-*5H*-chromeno[3,4-*f*]quinoline (Compound **189**, structure **32** of **Scheme IX**, where R=*n*-propyl)

This compound was prepared by General Method 5 (EXAMPLE 60) from a 2.0 M solution of allylmagnesium chloride (0.2 mL, 0.4 mmol) in THF and Compound **159** (25 mg, 0.086 mmol) to afford 5.0 mg (18%) of Compound **189** as a yellow oil. Data for Compound **189:** $R_f$ = 0.27 (silica gel, 25% EtOAc: Hexane); **$^1$H NMR** (400 MHz, $CDCl_3$) 7.59 (d, *J* = 7.7, 1 H), 7.43 (d, *J* = 8.4, 1 H), 7.13 (t, *J* = 7.7, 1 H), 6.98 (t, *J* = 7.7, 1 H), 6.91 (d, *J* = 7.7, 1 H), 6.57 (d, *J* = 8.4, 1 H), 5.89 (d, *J* = 10.4, 1 H), 5.49 (s, 1 H), 3.90 (br s, 1 H), 2.25 (s, 3 H), 1.84 (m, 2 H), 1.49-1.35 (m, 2 H), 1.29 (s, 3 H), 1.20 (s, 3 H), 0.89 (t, *J* = 7.4, 3 H).

**EXAMPLE 90**

(*R*/*S*)-1,2-Dihydro-2,2,4-trimethyl-5-(2-pyridyl)-*5H*-chromeno[3,4-*f*]quinoline (Compound **190**, structure **32** of **Scheme IX**, where R=2-pyridyl)

To a solution of Compound **184** (10 mg, 0.023 mmol) in 1 mL of THF at - 78 °C was added a 1.0 M hexane solution of *n*-BuLi (0.05 mL, 0.07 mmol), giving rise to a yellow then dark red solution. The mixture was allowed to stir for 15 min and was quenched with water (1 mL). The mixture was extracted with ethyl acetate (2 x 10 mL) and the combined extracts were concentrated. Chromatography of the crude mixture on a silica gel column using 10-30% ethyl acetate / hexane as eluents afforded 7 mg (86%) of Compound **190** as a colorless oil. Data for Compound **190:** **$^1$H NMR** (400 MHz, acetone-$d_6$) 8.48 (dd, *J* = 5.4, 1.8, 1 H), 7.61 (dd, *J* = 7.8, 1.6, 1 H), 7.57 (td, *J* = 7.8, 1.8, 1 H), 7.54 (d, *J* = 8.3, 1 H), 7.16-7.13 (m, 2 H), 6.99 (td, *J* = 7.8, 1.6, 1 H), 6.93 (s, 1 H), 6.88 (td, *J* = 7.9, 1.0, 1 H), 6.80 (d, *J* = 8.5, 1 H), 6.77 (dd, *J* = 7.9, 1.1, 1 H), 5.48 (bs, 1 H), 5.44 (s, 1 H), 1.98 (s, 3 H), 1.23 (s, 3 H), 1.22 (s, 3 H).

**EXAMPLE 91**

(*R*/*S*)-5-(3-Fluorophenyl)-1,2-dihydro-2,2,4-trimethyl-*5H*-chromeno[3,4-*f*]quinoline (Compound **191**, structure **32** of **Scheme IX**, where R=3-fluorophenyl)

This compound was prepared by General Method 5 (EXAMPLE 60) from 1-bromo-3-fluorobenzene (175 mg, 1.0 mmol) and Compound **159** (20 mg, 0.07 mmol) to afford 12 mg (47%) of Compound **191** as a colorless oil, along with 1.5 mg (6%) of Compound **192** (EXAMPLE 92) as a colorless oil. Data for Compound **191:** $^1$**H NMR** (400 MHz, acetone-$d_6$) 7.60 (d, *J* = 7.9, 1 H), 7.57 (d, *J* = 8.4, 1 H), 7.26 (td, *J* = 7.9, 5.9, 1 H), 7.06 (d, *J* = 7.1, 1 H), 7.01-6.81 (m, 8 H), 5.58 (br s, 1 H), 5.49 (s, 1 H), 2.02 (s, 3 H), 1.27 (s, 3 H), 1.25 (s, 3 H).

**EXAMPLE 92**

(*R*/*S*)-5-(3-Fluorophenyl)-1,2,3,4-tetrahydro-2,2-dimethyl-4-methylidene-5H-chromeno[3,4-*f*]quinoline (Compound **192**, structure **33** of **Scheme IX**, where R=3-fluorophenyl)

This compound (1.5 mg, 6%) was obtained along with Compound **191** as described above (EXAMPLE 91). Data for Compound **192:** $^1$**H NMR** (400 MHz, $CDCl_3$) 7.54 (d, *J* = 8.0, 1 H), 7.51 (d, *J* = 8.5, 1 H), 7.15 (td, *J* = 7.9, 5.9, 1 H), 7.06-6.81 (m, 6 H), 6.61 (s, 1 H), 6.59 (d, *J* = 8.0, 1 H), 4.94 (s, 1 H), 4.61 (s, 1 H), 2.43 (d, *J* = 12.3, 1 H), 2.19 (d, *J* = 12.3, 1 H), 1.34 (s, 3 H), 1.14 (s, 3 H).

**EXAMPLE 93**

(*R*/*S*)-1,2-Dihydro-2,2,4-trimethyl-5-propylthio-*5H*-chromeno[3,4-*f*]quinoline (Compound **193**, structure **47** of **Scheme XIV**, where $R^1$=$R^2$=H, X=S, $R^3$=*n*-propyl)

To a solution of Compound **185** (12 mg, 0.04 mmol) in a 1:1 mixture of 1-propanethiol and methylene chloride (2 mL) was added 2 mg of *p*-TsOH at rt. The reaction was complete after 1 hour by TLC and was quenched with saturated aqueous $NaHCO_3$. The reaction mixture was extracted with EtOAc (2 x 10 mL) and the combined organic layers were washed with water and brine then dried over $Na_2SO_4$. Removal of solvent *in vacuo* followed by purification on a 5x20 cm, 250 µm, TLC plate, eluting with 25% EtOAc:hexane, afforded 14 mg (99%) of Compound **193** as a yellow oil. Data for Compound **193:** $R_f$= 0.43 (silica gel, 25% EtOAc: Hexane); $^1$**H NMR** (400 MHz, acetone-$d_6$) 7.69 (d, *J* = 7.6, 1 H), 7.49 (d, *J* = 8.4, 1 H), 7.16 (t , *J* = 7.6, 1 H), 7.05 (t, *J* = 7.6, 1 H), 6.93 (d, *J* = 7.6, 1 H), 6.72 (d, *J* = 8.4, 1 H), 5.51 (s, 1 H), 2.79-2.73 (m, 1 H), 2.62-2.57 (m, 1 H), 2.47 (s, 3 H), 1.70 (m, 2 H), 1.25 (s, 3 H), 1.20 (s, 3 H), 0.99 (t, *J* = 7.3, 3 H).

**EXAMPLE 94**

(*R*/*S*)-1,2-Dihydro-5-(3-methoxyphenyl)-2,2,4-trimethyl-*5H*-chromeno[3,4-*f*]quinoline (Compound **194**, structure **32** of **Scheme IX**, where R=3-methoxyphenyl)

This compound was prepared by General Method 5 (EXAMPLE 60) from 3-bromoanisole (187 mg, 1.0 mmol) and Compound **159** (20 mg, 0.07 mmol) to afford 2.6 mg (10%) of Compound **194** as a colorless oil. Data for Compound **194:** $^1$**H NMR** (400 MHz, acetone-$d_6$) 7.59 (d, *J* = 7.8, 1 H), 7.55 (d, *J* = 8.4, 1 H), 7.12 (t, *J* = 7.9, 1 H), 6.98 (t, *J* = 7.2, 1 H), 6.91 (s, 1 H), 6.88-6.71 (m, 6 H), 5.52 (br s, 1 H), 5.47 (s, 1 H), 3.67 (s, 3 H), 2.03 (s, 3 H), 1.26 (s, 3 H), 1.25 (s, 3 H).

**EXAMPLE 95**

(*R*/*S*) 1,2-Dihydro-2,2,4-trimethyl-5-[3-(trifluoromethyl)phenyl]-*5H*-chromeno[3,4-*f*]quinoline (Compound **195**, structure **32** of **Scheme IX**, where R=3-(trifluoromethyl)phenyl)

This compound was prepared by General Method 5 (EXAMPLE 60) from 3-bromobenzotrifluoride (225 mg, 1.0 mmol) and Compound **159** (20 mg, 0.07 mmol) to afford 10 mg (34%) of Compound **195** as a colorless oil. Data for Compound **195:** $^1$**H NMR** (400 MHz, acetone-$d_6$) 7.61 (d, *J* = 7.6, 1 H), 7.60 (d, *J* = 9.0, 1 H), 7.56-7.45 (m, 4 H), 7.04 (s, 1 H), 6.98 (t, *J* = 7.6, 1 H), 6.89-6.83 (m, 3 H), 5.60 (s, 1 H), 5.55 (s, 1 H), 2.02 (s, 3 H), 1.27 (s, 6 H).

**EXAMPLE 96**

(*R*/*S*)-5-(3-Fluoro-4-methylphenyl)-1,2-dihydro-2,2,4-trimethyl-*5H*-chromeno[3,4-*f*]quinoline (Compound **196**, structure **32** of **Scheme IX**, where R=3-fluoro-4-methylphenyl)

This compound was prepared by General Method 5 (EXAMPLE 60) from 4-bromo-2-fluorotoluene (189 mg, 1.0 mmol) and Compound **159** (20 mg, 0.07 mmol) to afford 15 mg (56%) of Compound **196** as a colorless oil. Data for Compound **196**: **$^1$H NMR** (400 MHz, acetone-$d_6$) 7.60 (d, *J* = 7.8, 1 H), 7.56 (d, *J* = 8.4, 1 H), 7.08 (t, *J* = 7.9, 1 H), 6.98 (t, *J* = 7.9, 1 H), 6.94 (d, *J* = 8.0, 1 H), 6.91 (s, 1 H), 6.90-6.80 (m, 4 H), 5.55 (br s, 1 H), 5.48 (s, 1 H), 2.12 (s, 3 H), 2.01 (s, 3 H), 1.26 (s, 3 H), 1.24 (s, 3 H).

**EXAMPLE 97**

(*R*/*S*)-5-(4-Bromo-3-pyridyl)-1,2-dihydro-2,2,4-trimethyl-*5H*-chromeno[3,4-*f*]quinoline (Compound **197**, structure **32** of **Scheme IX**, where R=4-bromo-3-pyridyl)

This compound was prepared by General Method 5 (EXAMPLE 60) from 2,5-dibromopyridine (237 mg, 1.0 mmol) and Compound **159** (20 mg, 0.07 mmol) to afford 7 mg (23%) of Compound **197** as a colorless oil. Data for Compound **197: $^1$H NMR** (400 MHz, acetone-$d_6$) 8.24 (d, *J* = 5.2, 1 H), 7.62 (dd, *J* = 8.0, 1.3, 1 H), 7.57 (d, *J* = 8.4, 1 H), 7.34 (s, 1 H), 7.27 (d, *J* = 6.5, 1 H), 7.06 (td, *J* = 7.4, 1.3, 1 H), 6.97 (s, 1 H), 6.94-6.88 (m, 1 H), 6.86 (d, *J* = 8.4, 1 H), 5.68 (br s, 1 H), 5.55 (s; 1 H), 2.06 (s, 3 H), 1.29 (s, 3 H), 1.28 (s, 3 H).

**EXAMPLE 98**

(*R*/*S*)-1,2-Dihydro-2,2,4-trimethyl-5-(3-pyridyl)-*5H*-chromeno[3,4-*f*]quinoline (Compound **198**, structure **32** of **Scheme IX**, where R=3-pyridyl)

This compound was prepared in a manner similar to that of Compound **190** (EXAMPLE 90), from Compound **197** (5 mg, 0.06 mmol) to afford 4 mg (quant) of Compound **198** as a colorless oil. Data for Compound **198: $^1$H NMR** (400 MHz, acetone-$d_6$) 8.42 (m, 2 H), 7.58 (dd, *J* = 7.7, 1.3, 1 H), 7.56 (d, *J* = 8.4, 1 H), 7.18 (d, *J* = 5.9, 2 H), 7.01 (t, *J* = 7.8, 1 H), 6.95 (s, 1 H), 6.89-6.83 (m, 3 H), 5.61 (br s, 1 H), 5.52 (s, 1 H), 2.03 (s, 3 H), 1.28 (s, 3 H), 1.26 (s, 3 H).

**EXAMPLE 99**

(*R*/*S*)-5-(4-Chloro-3-fluorophenyl)-1,2-dihydro-2,2,4-trimethyl-*5H*-chromeno[3,4-*f*]quinoline (Compound **199**, structure **32** of **Scheme IX**, where R=4-chloro-3-fluorophenyl)

This compound was prepared by General Method 5 (EXAMPLE 60) from 2-chloro-5-bromofluorobenzene (209 mg, 1.0 mmol) and Compound **159** (15 mg, 0.05 mmol) to afford 13 mg (64%) of Compound **199** as a colorless oil. Data for Compound **199: $^1$H NMR** (400 MHz, acetone-$d_6$) 7.61 (dd, *J* = 7.7, 1.4, 1 H), 7.57 (d, *J* = 8.3, 1 H), 7.38 (t, *J* = 7.9, 1 H), 7.13 (dd, *J* = 10.3, 1.8, 1 H), 7.05 (t, *J* = 7.8, 1 H), 7.00 (dd, *J* = 7.7, 1.3, 1 H), 6.93 (s, 1 H), 6.91-6.81 (m, 3 H), 5.62 (br s, 1 H), 5.50 (s, 1 H), 2.02 (s, 3 H), 1.27 (s, 3 H), 1.25 (s, 3 H).

**EXAMPLE 100**

(*R*/*S*)-1,2-Dihydro-2,2,4,5-tetramethyl-5*H*-chromeno[3,4-*f*]quinoline (Compound **200**, structure **32** of **Scheme IX**, where R=methyl)

This compound was prepared by General Method 5 (EXAMPLE 60) from Compound **159** (8 mg) to afford 4.8 mg (63%) of Compound **200** as a yellow oil. Data for Compound **200**: $R_f$= 0.44 (silica gel, 25% EtOAc: hexane); **$^1$H NMR** (400 MHz, $CDCl_3$) 7.60 (d, *J* = 7.8, 1 H), 7.43 (d, *J* = 8.4, 1 H), 7.15 (t, *J* = 8.0, 1 H), 7.00 (t, *J* = 8.0, 1 H), 6.91 (d, *J* = 8.1, H), 6.57 (d, *J* = 8.1, 1 H), 6.60 (d, *J* = 6.1, 1 H), 5.49 (s, 1 H), 3.85 (br s, 1 H), 2.26 (s, 3 H), 1.38 (d, *J* = 6.6, 3 H), 1.27 (s, 3 H), 1.22 (s, 3 H).

**EXAMPLE 101**

(*R*/*S*)-1,2-Dihydro-5-hexyl-2,2,4-trimethyl-5*H*-chromeno[3,4-*f*]quinoline (Compound **201**, structure **32** of **Scheme IX**, where R=*n*-hexyl)

This compound was prepared by General Method 5 (EXAMPLE 60) from Compound **159** (8 mg) and 1-iodohexane to afford 4.8 mg (63%) of Compound **201** as a yellow oil. Data for Compound **201:** $R_f$ = 0.33 (silica gel, 25% EtOAc: Hexane); **$^1$H NMR** (400 MHz, $CDCl_3$) 7.59 (d, *J* = 7.8, 1 H), 7.43 (d, *J* = 8.3, 1 H), 7.12 (t, *J* = 7.6, 1 H), 6.98 (t, *J* = 7.4, 1 H), 6.91 (d, *J* = 7.7, 1 H), 6.56 (d, *J* = 8.1, 1 H), 5.86 (d, *J* = 7.4, 1 H), 5.49 (s, 1 H), 2.25 (s, 3 H), 1.83 (m, 2 H), 1.41 (m, 3 H), 1.28 (s, 3 H), 1.20 (s, 3 H), 0.84 (t, *J* = 6.7, 3 H).

**EXAMPLE 102**

1,2-Dihydro-2,2,4-trimethyl-5*H*-chromeno[3,4-*f*]quinoline (Compound **202**, structure **32** of **Scheme IX**, where R=H)

To a solution of Compound **185** (EXAMPLE 85) (9.5 mg, 0.03 mmol) in methylene chloride (5 mL) maintained at -78 °C was added trifluoroacetic acid (10 mL) and triethylsilane (25 mL). The reaction mixture was allowed to warm to rt, quenched with 1 N NaOH (3 mL), and partitioned between EtOAc (10 mL) and water (5 mL). The organic layer was washed with brine (3 x 3 mL), dried ($Na_2SO_4$), filtered, and concentrated. Purification by PTLC (250 µm, 10/1 hexane/EtOAc) afforded 4.6 mg (52%) of Compound **202.** Data for Compound **202:** $R_f$ = 0.36 (silica gel, 25% EtOAc: Hexane); **$^1$H NMR** (400 MHz, $CDCl_3$) 7.58 (d, *J* = 8.0, 1 H), 7.38 (d, *J* = 8.3, 1 H), 7.15 (t, *J* = 8.0, 1 H), 7.02 (t, *J* = 8.0, 1 H), 6.94 (d, *J* = 8.0, 1 H), 6.58 (d, *J* = 8.3, 1 H), 5.47 (s, 1 H), 5.32 (s, 2 H), 3.90 (br s, 1 H), 2.10 (s, 3 H), 1.27 (s, 6 H).

**EXAMPLE 103**

(*R*/*S*)-1,2-Dihydro-5-(3-methylbutyl)-2,2,4-trimethyl-*5H*-chromeno[3,4-*f*]quinoline (Compound **203**, structure **32** of **Scheme IX**, where R=3-methylbutyl)

This compound was prepared by General Method 5 (EXAMPLE 60) from Compound **159** (13 mg) and 1-bromo-3-methylbutane to afford 1 mg (16%) of Compound **203** as a yellow oil. Data for Compound **203: TLC**: $R_f$ = 0.29 (silica gel, 25% EtOAc: hexane); **$^1$H NMR** (400 MHz, $CDCl_3$) 7.58 (d, *J* = 8.0, 1 H), 7.43 (d, *J* = 8.3, 1 H), 7.17 (t, *J* = 8.1, 1 H), 6.98 (t, *J* = 8.1, 1 H), 6.91 (d, *J* = 7.9, 1 H), 6.58 ( d, *J* = 8.0, 1 H), 5.81 (d, *J* = 8.9, 1 H), 5.49 (s, 1 H), 3.90 (br s, 1 H), 2.24 (s, 3 H), 1.80 (m, 1 H), 1.44 (m, 2 H), 1.28 (m, 5 H), 1.21 (s, 3 H), 0.79 (d, *J* = 6.2, 3 H), 0.70 (d, *J* = 6.2, 3 H).

**EXAMPLE 104**

(*R*/*S*)-5-(4-Chlorobutyl)-1,2-dihydro-2,2,4-trimethyl-5*H*-chromeno[3,4-*f*]quinoline (Compound **204**, structure **32** of **Scheme IX**, where R=4-chlorobutyl)

This compound was prepared by General Method 5 (EXAMPLE 60) from Compound **159** (8.3 mg) and 1-bromo-4-chlorobutane to afford 2.2 mg (27%) of Compound **204** as a yellow oil. Data for Compound **204:** $R_f$= 0.38 (silica gel, 25% EtOAc: Hexane); **$^1$H NMR** (400 MHz, $CDCl_3$) 7.59 (d, *J* = 8.0, 1 H), 7.43 (d, *J* = 8.3, 1 H), 7.13 (t, *J* = 7.7, 1 H), 7.00 (t, *J* = 8.4, 1 H), 6.91 (d, *J* = 7.8, 1 H), 6.57 (d, *J* = 8.3, 1 H), 5.86 (d, *J* = 10.4, 1 H), 5.49 (s, 1 H), 3.90 (br s, 1 H), 2.25 (s, 3 H), 1.83 (m, 2 H), 1.41 (m, 4 H), 1.29 (s, 3 H), 1.20 (s, 3 H), 0.84 (t, J = 7.3, 2 H).

**EXAMPLE 105**

(*R*/*S*)-5-Benzyl-1,2-dihydro-2,2,4-trimethyl-5*H*-chromeno[3,4-*f*]quinoline (Compound **205**, structure **32** of **Scheme IX**, where R=benzyl)

This compound was prepared by General Method 5 (EXAMPLE 60) from Compound **159** (16.8 mg) and benzylmagnesium chloride to afford 2.6 mg (16%) of Compound **205** as a yellow oil. Data for Compound **205: TLC**: $R_f$ = 0.20 (silica gel, 25% EtOAc: Hexane); **$^1$H NMR** (400 MHz, $CDCl_3$) 7.66 (d, *J* = 7.8, 1 H), 7.48 (d, *J* = 8.4, 1 H), 7.30-7.15 (m, 6 H), 7.10 (t, *J* = 7.8, 1 H), 6.89 (d, *J* = 8.4, 1 H), 6.61 (d, *J* = 8.4, 1 H), 6.13 (dd, *J* = 10.2, 3.4, 1 H), 5.49 (s, 1 H), 3.92 (br s, 1 H), 3.11 (dd, *J* = 14.6, 10.2, 1 H), 2.73 (dd, *J* = 14.6, 3.4, 1 H), 2.31 (s, 3 H), 1.54 (s, 3 H), 1.29 (s, 3 H).

**EXAMPLE 106**

(*R*/*S*)-5-(4-Bromobutyl)-1,2-dihydro-2,2,4-trimethyl-5*H*-chromeno[3,4-*f*]quinoline (Compound **206**, structure **32** of **Scheme IX**, where R=4-bromobutyl)

This compound was prepared by General Method 5 (EXAMPLE 60) from Compound **159** (13.7 mg) and 1,4-dibromobutane to afford 6.0 mg (45%) of Compound **206** as a yellow oil. Data for Compound **206**: $R_f$ = 0.22 (silica gel, 1:1 $CH_2Cl_2$/hexane); **$^1$H NMR** (400 MHz, $CDCl_3$) 7.59 (d, *J* = 8.0, 1 H), 7.44 (d, *J* = 8.3, 1 H), 7.12 (t, *J* = 7.7, 1 H), 6.98 (t, *J* = 8.0, 1 H), 6.93 (d, *J* = 8.0, 1 H), 6.57 (d, *J* = 8.3, 1 H), 5.85 (d, *J* = 10.4, 1 H), 5.49 (s, 1 H), 3.90 (s, 1 H), 2.25 (s, 3 H), 1.83 (m, 2 H), 1.41 (m, 4 H), 1.29 (s, 3 H), 1.20 (s, 3 H), 0.84 (t, J = 7.3, 2 H).

**EXAMPLE 107**

9-Fluoro-1,2-dihydro-2,2,4-trimethyl-5-coumarino[3,4-*f*]quinoline (Compound **207**, structure **41** of **Scheme XI**, where $R^1$=H, $R^2$=F)

5-Fluoro-2-methoxyphenylboronic acid (structure **37** of **Scheme XI**, where $R^1$=H, $R^2$=F)

In a 200-mL flask, a solution of 2-bromo-4-fluoroanisole (Aldrich: 4.00 mL, 30.8 mmol) in THF (50 mL) was cooled to -78°C ($CO_2$/IPA). To this solution *n*-BuLi (Aldrich: 2.5 M in hexanes; 12.4 mL, 31 mmol, 1.0 equivuiv) was added dropwise over a 30 min period. The reaction mixture was stirred at -78°C for 60 min and treated with trimethyl borate (Aldrich: 10.5 mL, 92.4 mmol, 3.0 equivuiv). The reaction mixture was allowed to slowly warm to rt, stirred overnight (12 h), and cooled to 0 °C (ice/$H_2O$). The solution was treated with 5% HCl until the pH reached 6. The reaction mixture was poured into sat'd $NH_4Cl$ (80 mL) and extracted with $CH_2Cl_2$ (3 x 100 mL). The extracts were washed with sat'd $NH_4Cl$ (1 x 80 mL), combined, dried ($MgSO_4$), filtered through a pad of Celite™, and concentrated to afford 4.90 g (94%) of a white semi-solid. Data for 5-fluoro-2-methoxyphenylboronic acid: **$^1$H NMR** (400 MHz, acetone-$d_6$): 7.47 (dd, *J* = 8.8, 3.3, 1 H); 7.17 (m, 1 H); 7.05 (dd, *J* = 9.0, 3.9, 1 H); 3.93 (s, 3 H).

Methyl (5'-fluoro-2'-methoxy-4-nitro-2-biphenyl)carboxylate (structure **39** of **Scheme XI**, where $R^1$=H, $R^2$=F)

In a 250-mL flask, a solution of methyl (2-bromo-5-nitro)benzoate (Compound **38**, **Scheme XI**) (Aldrich: 5.00 g, 19.2 mmol) in DME (60 mL) was treated with tetrakis(triphenylphosphine)palladium (Aldrich: 0.67 g, 0.58 mmol, 3.0 mol%). The reaction mixture was stirred at rt for 10 min. A solution of 5-fluoro-2-methoxyphenylboronic acid (4.90 g, 29 mmol, 1.5 equivuiv) in EtOH (8 mL) was added, followed by 2.0 M $Na_2CO_3$ (29 mL, 58 mmol, 3 equivuiv). The reaction mixture was heated to 80 °C for 6 h, cooled to rt, poured into 2.0 M $Na_2CO_3$ (100 mL), and extracted with EtOAc (3 x 100 mL). The extracts were washed with brine (1 x 100 mL), combined, dried ($MgSO_4$), filtered, and concentrated to an orange oil. Purification by SGC (hexane/EtOAc, 10/1) afforded 4.25 g (72%) of methyl (5'-fluoro-2'-methoxy-4-nitro-2-biphenyl)carboxylate as a yellow-orange solid. Data for methyl (5'-fluoro-2'-methoxy-4-nitro-2-biphenyl)carboxylate: **$^1$H NMR** (400 MHz, $CDCl_3$) 8.73 (d, *J* = 2.4, 1 H); 8.39 (dd, *J* = 8.3, 2.4, 1 H); 7.49 (d, *J* = 8.3, 1 H); 7.09 (td, *J* = 8.5, 3.1, 1 H); 7.00 (dd, *J* = 8.5, 3.1, 1 H); 6.85 (dd, *J* = 8.9, 3.2, 1 H); 3.76 (s, 3 H); 3.70 (s, 3 H).

5'-Fluoro-2'-methoxy-4-nitro-2-biphenylcarboxylic acid

In a 200-mL flask, a solution of methyl (5'-fluoro-2'-methoxy-4-nitro-2-biphenyl)carboxylate (4.24 g, 13.9 mmol) in THF (50 mL) was cooled to 0 °C (ice/$H_2O$) and treated with EtOH (10 mL) and 20% KOH (10 mL). The reaction mixture was allowed to warm to rt and stirred overnight, acidified to pH10 (pH paper) with 10% HCl, and extracted with EtOAc (3 x 75 mL). The extracts were washed with brine (1 x 80 mL), combined, dried ($MgSO_4$), filtered, and concentrated to afford 3.68 g (91 %) of 5'-fluoro-2'-methoxy-4-nitro-2-biphenylcarboxylic acid as a yellow solid. Data for 5'-fluoro-2'-methoxy-4-nitro-2-biphenylcarboxylic acid: **$^1$H NMR** (400 MHz, acetone-$d_6$): 8.68 (d, *J* = 2.6, 1 H); 8.46 (dd, *J* = 8.5, 2.6, 1 H); 7.68 (d, *J* = 8.5, 1 H); 7.16 (m, 2 H); 7.05 (dd, *J* = 8.8, 4.4, 1 H); 3.73 (s, 3 H).

6-Fluoro-2-nitro-3,4-benzocoumarin

In a 250-mL flask, a suspension of 5'-fluoro-2'-methoxy-4-nitro-2-biphenylcarboxylic acid (3.60 g, 12.3 mmol) in dichloroethane (30 mL) was treated with $SOCl_2$ (0.92 mL, 12.6 mmol, 1.0 equivuiv) and heated to a gentle reflux for 90 min. The reaction vessel was cooled to 0 °C (ice/$H_2O$) and $AlCl_3$ (0.91 g, 6.8 mmol, 0.55 equivuiv) was added portion-wise. The reaction mixture was allowed to slowly warm to rt, stirred 5 h, and quenched with 5% HCl (100 mL). The crude product was extracted with EtOAc (4 x 150 mL). The extracts were washed with sat'd $NH_4Cl$ (1 x 100 mL),

combined, dried ($MgSO_4$), filtered, and concentrated to afford 3.19 g (quant) of 6-fluoro-2-nitro-3,4-benzocoumarin as a yellow solid. Data for 6-fluoro-2-nitro-3,4-benzocoumarin: **$^1$H NMR** (400 MHz, DMSO-$d_6$): 8.84 (d, *J* = 2.3, 1 H); 8.67 (m, 2 H); 8.40 (d, *J* = 9.2, 1 H); 7.55 (m, 2 H).

2-Amino-6-fluoro-3,4-benzocoumarin (structure **40** of **Scheme XI**, where $R^1$=H, $R^2$=F)

In a 500-mL flask, a suspension of 6-fluoro-2-nitro-3,4-benzocoumarin (3.18 g, 12.2 mmol) in EtOAc (300 mL) was treated with 10% Pd/C (2.0 g) and AcOH (0.2 mL), and stirred under an atmosphere of $H_2$ for 1 h. The reaction mixture was filtered and the solids rinsed with acetone (200 mL). Concentration of the filtrate afforded 2.19 g (78%) of 2-amino-6-fluoro-3,4-benzocoumarin as a yellow solid. Data for 2-amino-6-fluoro-3,4-benzocoumarin: **$^1$H NMR** (400 MHz, acetone-$d_6$): 8.09 (d, *J* = 8.6, 1 H); 7.86 (dd, *J* = 9.8, 3.0, 1 H); 7.55 (d, *J* = 2.6, 1 H); 7.33 (dd, *J* = 9.2, 4.9, 1 H); 7.28 (dd, *J* = 9.2, 2.6, 1 H); 7.17 (dt, *J* = 3.0, 9.0, 1 H).

9-Fluoro-1,2-dihydro-2,2,4-trimethyl-5-isocoumarino[3,4-*f*]quinoline (Compound **207**, structure **41** of **Scheme XI**, where $R^1$=H, $R^2$=F)

In a 200-mL resealable pressure tube, a suspension of 2-amino-6-fluoro-3,4-benzocoumarin (1.10 g) in acetone (100 mL) was treated with iodine (Aldrich: 0.50 g) and heated to 110 °C for 32 h. The reaction mixture was cooled to rt, concentrated to remove the bulk of the acetone, and dissolved in $CH_2Cl_2$ (200 mL). The organic layer was washed with 0.5 N $Na_2S_2O_3$ (2 x 200 mL) and sat'd $NaHCO_3$ (1 x 100 mL). The aqueous layers were extracted with $CH_2Cl_2$ (2 x 100 mL). The combined organic layers were dried ($K_2CO_3$), filtered, and concentrated to afforded an orange solid. Purification by SGC (hexane/EtOAc, 5/1) afforded 0.51 g (34%) of Compound **207** as a bright yellow solid. Data for Compound **207: $^1$H NMR** (400 MHz, acetone-$d_6$) 7.96 (d, *J* = 8.6, 1 H); 7.83 (dd, *J* = 10.0, 2.9, 1 H); 7.30 (dd, *J* = 9.0, 4.9, 1 H); 7.22 (d, *J* = 8.6, 1 H); 7.17 (m, 1 H); 6.25 (br s, 1 H); 5.54 (s, 1 H); 1.30 (s, 6 H). The acetone multiplet obscures the C(4) methyl group.

**EXAMPLE 108**

8-Fluoro-1,2-dihydro-2,2,4-trimethyl-5-coumarino[3,4-*f*]quinoline (Compound **208**, structure **41** of **Scheme XI**, where $R^1$ =F, $R^2$=H)

2-Bromo-5-fluoroanisole (structure **36** of **Scheme XI**, where $R^1$=F, $R^2$=H)

In a 250 mL r.b. flask, a solution of 2-bromo-5-fluorophenol (Lancaster: 7.0 mL, 64 mmol, 1.0 equivuiv) in acetone (140 mL) was treated with iodomethane (Aldrich: 4.8 mL, 77 mmol, 1.2 equivuiv), potassium carbonate (8 g), and water (1 mL). The reaction mixture was heated at reflux for 6 h, cooled to rt, clarified with $H_2O$ (40 mL), and the bulk of the volatiles was removed under reduced pressure. The reaction mixture was extracted with EtOAc (3 x 120 mL); the extracts were washed with brine (1 x 80 mL), combined, dried ($K_2CO_3$), filtered, and concentrated to a clear oil. Bulb-to-bulb distillation (60-65 °C, 0.7 Torr) afforded 13.22 g (quant) of 2-bromo-5-fluoroanisole as a colorless liquid. Data for 2-bromo-5-fluoroanisole: **$^1$H NMR** (400 MHz, $CDCl_3$): 7.46 (dd, *J* = 10.6, 8.7, 1 H); 6.64 (dd, *J* = 10.4, 2.8, 1 H); 5.58 (dt, *J* = 10.4, 2.4, 1 H); 3.88 (s, 3 H).

4-Fluoro-2-methoxyphenylboronic acid (structure **37** of **Scheme XI**, where $R^1$=F, $R^2$=H)

In a 100 mL r.b. flask, a solution of 2-bromo-5-fluoroanisole (5.50 g, 26.8 mmol, 1.0 equivuiv) in THF (30 mL) was cooled to -78°C ($CO_2$/IPA) and n-BuLi (2.5 M in hexanes; 10.7 mL, 27 mmol, 1.0 equivuiv) was added via syringe over a 15 min period. The reaction mixture was stirred at -78°C for 45 min. Trimethyl borate (Aldrich: 9.1 mL, 80 mmol, 3.0 equivuiv) was added slowly via syringe. The reaction mixture was allowed to warm to rt, stirred an additional 10 h, and cooled to 0 °C. The reaction mixture was brought to pH6 with 5% HCl, poured into sat'd $NH_4Cl$ (60 mL), and extracted with methylene chloride (3 x 80 mL). The extracts were washed with sat'd $NH_4Cl$ (1 x 50 mL), combined, dried ($MgSO_4$), filtered, and concentrated to afford 4.22 g (93%) of crude 4-fluoro-2-methoxyphenylboronic acid as a white solid, which was used without further purification.

7-Fluoro-2-nitro-3,4-benzocoumarin

In a 200 mL r.b. flask, a solution of 2-bromo-5-nitrobenzoic acid (Compound **43, Scheme XII**) (Aldrich: 4.10 g, 16.7 mmol, 1.0 equivuiv) in DME (65 mL) was treated with tetrakis(triphenylphosphine) palladium (Aldrich: 0.58 g, 0.50 mmol, 3.0 mol%). The reaction mixture was stirred at rt for 10 min. A solution of 4-fluoro-2-methoxyphenylboronic

acid (4.20 g, 25 mmol, 1.5 equivuiv) in EtOH (10 mL) was added, followed by 2.0 M $Na_2CO_3$ (30 mL). The reaction mixture was heated to 80°C for 6 h, cooled to rt, poured into 5% HCl (100 mL), and extracted with EtoAc (3 x 100 mL). The extracts were washed with sat'd $NH_4Cl$ (1 x 100 mL) and brine (1 x 100 mL), combined, dried ($MgSO_4$), filtered, and concentrated to an orange solid. This crude material, consisting of impure 4'-fluoro-2'-methoxy-4-nitro-2-biphenylcarboxylic acid (structure **44** of **Scheme XII**, where $R^1$=F, $R^2$=H), was suspended in 1,2-dichloroethane (80 mL), treated with thionyl chloride (1.2 mL), and heated at reflux for 90 min. The reaction mixture was cooled to rt, treated with aluminum trichloride (0.4 g), and allowed to react overnight (11 h). The reaction mixture was poured into 20% KOH (80 mL) and extracted with methylene chloride (3 x 80 mL). The extracts were combined, dried ($MgSO_4$), filtered, and concentrated to an orange oil. The crude material was dissolved in methylene chloride (50 mL), adsorbed onto Celite™ (1 g), and concentrated to a fluffy orange powder. This powder was applied to a pad of silica gel in a 250 mL Buchner funnel (50 x 50 mm). The pad was rinsed with 100 mL of 2:1 hexane:EtOAc, which was discarded, and then 400 mL of 1:1 hexane:EtOAc. The filtrate was concentrated to afford 2.08 g (48%) of 7-fluoro-2-nitro-3,4-benzocoumarin as an orange solid. Data for 7-fluoro-2-nitro-3,4-benzocoumarin: **$^1$H NMR** (400 MHz, acetone-$d_6$) 9.02 (d, *J* = 2.4, 1 H); 8.71 (dd, *J* = 8.8, 2.4, 1 H); 8.65 (d, *J* = 8.8, 1 H); 8.53 (dd, *J* = 9.6, 6.1, 1 H); 7.34 (m, 2 H).

8-Fluoro-1,2-dihydro-2,2,4-trimethyl-5-coumarino[3,4-*f*]quinoline (Compound **208**, structure **39** of **Scheme XI**, where $R^1$=F, $R^2$=H)

In a 200-mL resealable pressure tube, a suspension of 2-amino-7-fluoro-3,4-benzocoumarin (1.61 g) in acetone (100 mL) was treated with iodine (Aldrich: 0.50 g) and heated to 110°C for 32 h. The reaction mixture was cooled to rt, concentrated to remove the bulk of the acetone, and dissolved in $CH_2Cl_2$ (200 mL). The organic layer was washed with 0.5 N $Na_2S_2O_3$ (2 x 200 mL) and sat'd $NaHCO_3$ (1 x 100 mL). The aqueous layers were extracted with $CH_2Cl_2$ (2 x 100 mL). The combined organic layers were dried ($K_2CO_3$), filtered, and concentrated to afford an orange solid. Purification by SGC (hexane/EtOAc, 5/1) afforded 0.46 g (21%) of Compound **208** as a bright yellow solid. Data for Compound **208: $^1$H NMR** (400 MHz, acetone-$d_6$) 8.12 (dd, *J* = 9.6, 5.9, 1 H); 7.92 (d, *J* = 9.6, 1 H); 7.22 (d, *J* = 8.6, 1 H); 7.11 (m, 2 H); 6.1 (br s, 1 H); 5.53 (d, *J* = 1.2, 1 H); 1.29 (s, 6 H). The acetone multiplet obscures the C(4) methyl group.

**EXAMPLE 109**

9-Chloro-1,2-dihydro-2,2,4-trimethyl-5-coumarino[3,4-*f*]quinoline (Compound **209**, structure **41** of **Scheme XI**, where $R^1$=H, $R^2$=Cl)

2-Bromo-4-chloroanisole (structure **36** of **Scheme XI**, where $R^1$=H, $R^2$=Cl)

In a 250 mL r.b. flask, a solution of 2-bromo-4-chlorophenol (Lancaster: 16.94 g, 81.6 mmol, 1.0 equivuiv) in acetone (160 mL) was treated sequivuentially with iodomethane (6.10 mL, 98 mmol, 1.2 equivuiv), potassium carbonate (12 g), and water (4 mL). The reaction mixture was heated at reflux for 3 h, cooled to rt, and the bulk of the volatiles was removed under reduced pressure. The residue was poured into water (140 mL) and extracted with EtOAc (3 x 150 mL). The extracts were washed with brine (1 x 100 mL), combined, dried ($K_2CO_3$), filtered through a pad of Celite™, and concentrated to a clear oil. Short-path distillation (80-85 °C, 1 Torr) afforded 17.74 g (98%) of 2-bromo-4-chloroanisole as a clear liquid. Data for 2-bromo-4-chloroanisole: **$^1$H NMR** (400 MHz, acetone-$d_6$) 7.53 (d, *J* = 2.5, 1 H); 7.24 (dd, *J* = 9.7, 2.5, 1 H); 6.81 (d, *J* = 9.7, 1 H); 3.88 (s, 3 H).

5-Chloro-2-methoxyphenylboronic acid (structure **37** of **Scheme XI**, where $R^1$=H, $R^2$=Cl)

This compound was prepared in a manner similar to that of 5-fluoro-2-methoxyphenylboronic acid (EXAMPLE 107) from 2-bromo-4-chloroanisole (2.00 g, 9.0 mmol, 1.0 equivuiv), *n*-BuLi (2.5 M in hexanes; 3.62 mL, 9.0 mmol, 1.0 equivuiv), and trimethylborate (3.0 mL, 26 mmol, 2.9 equivuiv) to afford 1.30 g (77%) of crude 5-chloro-2-methoxyphenylboronic acid as a white semi-solid. This compound was used in the next reaction with no further purification.

Methyl (5'-chloro-2'-methoxy-4-nitro-2-biphenyl)carboxylate (structure **39** of **Scheme XI**, where $R^1$=H, $R^2$=Cl)

This compound was prepared in a manner similar to that of methyl-(5'-fluoro-2'-methoxy-4-nitro-2-biphenyl) carboxylate (EXAMPLE 107) from methyl 2-bromo-5-nitrobenzoate (1.25 g, 4.8 mmol, 1.0 equivuiv), tetrakis(triphenylphosphine) palladium (Aldrich: 0.16 g, 0.14 mmol, 2.9 mol%), and 5-chloro-2-methoxyphenylboronic acid (1.30 g, 6.9 mmol, 1.5 equivuiv) to afford 0.85 g (55%) of methyl 5'-chloro-2'-methoxy-4-nitro-2-biphenylcarboxylate as a yellow-orange solid. Data for methyl 5'-chloro-2'-methoxy-4-nitro-2-biphenylcarboxylate: **$^1$H NMR** (400 MHz, $CDCl_3$): 8.73 (d, *J* = 2.4, 1 H); 8.38 (dd, *J* = 8.5, 2.5, 1 H); 7.49 (d, *J* = 8.5, 1 H); 7.36 (dd, *J* = 8.7, 2.5, 1 H); 7.23 (d, *J* = 2.5, 1 H); 6.85

(d, $J$ = 8.7, 1 H); 3.76 (s, 3 H); 3.70 (s, 3 H).

5'-Chloro-2'-methoxy-4-nitro-2-biphenylcarboxylic acid

This compound was prepared in a manner similar to that of 5'-fluoro-2'-methoxy-4-nitro-2-biphenylcarboxylic acid (EXAMPLE 107) from methyl 5'-chloro-2'-methoxy-4-nitro-2-biphenylcarboxylate (0.83 g, 2.6 mmol) to afford 0.75 g (95%) of 5'-chloro-2'-methoxy-4-nitro-2-biphenylcarboxylic acid as a yellow solid. Data for (5'-chloro-2'-methoxy-4-nitro-2-biphenyl)carboxylic acid: **$^1$H NMR** (400 MHz, acetone-$d_6$) 8.69 (d, $J$ = 2.5, 1 H); 8.46 (dd, $J$ = 8.3, 2.6, 1 H); 7.68 (d, $J$ = 8.5, 1 H); 7.41 (dd, $J$ = 8.9, 2.7, 1 H); 7.33 (d, $J$ = 2.8, 1 H); 7.08 (d, $J$ = 8.6, 1 H); 3.75 (s, 3 H).

6-Chloro-2-nitro-3,4-benzocoumarin

This compound was prepared in a manner similar to that of 6-fluoro-2-nitro-3,4-benzocoumarin (EXAMPLE 107) from 5'-chloro-2'-methoxy-4-nitro-2-biphenylcarboxylic acid (0.74 g, 2.3 mmol), $SOCl_2$ (0.17 mL, 2.3 mmol), and $AlCl_3$ (0.30 g, 2.5 mmol) to afford 0.64 g (quant) of 6-chloro-2-nitro-3,4-benzocoumarin as a yellow solid. Data for 6-chloro-2-nitro-3,4-benzocoumarin: **$^1$H NMR** (400 MHz, acetone-$d_6$) 9.04 (d, $J$ = 2.3, 1 H); 8.73 (m, 2 H); 8.51 (d, $J$ = 2.4, 1 H); 7.72 (dd, $J$ = 8.6, 2.4, 1 H); 7.50 (d, $J$ = 8.7, 1 H).

2-Amino-6-chloro-3,4-benzocoumarin (structure **40** of **Scheme XI**, where $R^1$=H, $R^2$=Cl)

This compound was prepared in manner similar to that of 2-amino-6-fluoro-3,4-benzocoumarin from 6-chloro-2-nitro-3,4-benzocoumarin (0.64 g, 2.3 mmol) to afford 0.50 g (88%) of 2-amino-6-chloro-3,4-benzocoumarin as a yellow solid. Data for 2-amino-6-chloro-3,4-benzocoumarin: **$^1$H NMR** (400 MHz, acetone-$d_6$) 8.11 (m, 2 H); 7.55 (d, $J$ = 2.5, 1 H); 7.39 (dd, $J$ = 8.6, 2.5, 1 H); 7.28 (m, 2 H).

9-Chloro-1,2-dihydro-2,2,4-trimethyl-5-coumarino[3,4-*f*]quinoline (Compound **209**, structure **41** of **Scheme XI**, where $R^1$=H, $R^2$=C1)

This compound was prepared in a manner similar to that of Compound **207** from 2-amino-6-chloro-3,4-benzocoumarin (0.50 g) to afford 0.14 g (21%) of Compound **209** as a bright yellow solid. Data for Compound **209: $^1$H NMR** (400 MHz, acetone-$d_6$): 8.10 (d, $J$ = 2.4, 1 H); 8.00 (d, $J$ = 8.7, 1 H); 7.39 (dd, $J$ = 8.7, 2.3, 1 H); 7.26 (d, $J$ = 8.8, 1 H); 7.23 (d, $J$ = 8.6, 1 H); 5.55 (s, 1 H); 1.30 (s, 6 H). The acetone multiplet obscures the C(4) methyl group.

**EXAMPLE 110**

(*R*/*S*)-5-Butyl-9-fluoro-1,2-dihydro-2,2,4-trimethyl-*5H*-chromeno[3,4-*f*]quinoline (Compound **210**, structure **42** of **Scheme XI**, where R=*n*-butyl, $R^1$=H, $R^2$=F)

This compound was prepared by General Method 5 (EXAMPLE 60) from Compound **207** (0.53 g, 1.7 mmol) and *n*-BuLi (2.5 M in hexanes, 2.7 mL, 6.8 mmol, 4.0 equivuiv) to afford 0.34 g (57%) of Compound **210** as a yellow foam. Data for Compound **210: $^1$H NMR** (400 MHz, acetone-$d_6$): 7.54 (d, $J$ = 8.5, 1 H); 7.49 (dd, $J$ = 10.2, 2.9, 1 H); 7.03 (dd, $J$ = 8.8, 4.9, 1 H); 6.88 (dt, $J$ = 2.9, 8.8, 1 H); 6.75 (d, $J$ = 8.5, 1 H); 5.80 (br s, 1 H); 5.49 (s, 1 H); 4.83 (t, $J$ = 7.6, 1 H); 2.36 (q, $J$ = 7.5, 2 H); 2.05 (s, 3 H); 1.46 (sextet, $J$ = 7.4, 2 H); 1.10 (br s, 8H); 0.93 (t, $J$ = 7.4, 3 H).

**EXAMPLE 111**

(*R*/*S*)-5-Butyl-8-fluoro-1,2-dihydro-2,2,4-trimethyl-*5H*-chromeno[3,4-*f*]quinoline (Compound **211**, structure **42** of **Scheme XI**, where R=*n*-butyl, $R^1$=F, $R^2$=H)

This compound was prepared by General Method 5 (EXAMPLE 60) from Compound **208** (29 mg, 0.09 mmol) and *n*-BuLi (2.5 M in hexanes, 0.16 mL, 0.40 mmol) to afford 6.5 mg (20%) of Compound **211** as a yellow foam. Data for Compound **211: $^1$H NMR** (400 MHz, acetone-$d_6$): 7.77 (dd, $J$ = 8.7, 6.3, 1 H); 7.51 (d, $J$ = 8.5, 1 H); 6.85 (m, 3 H); 5.80 (br s, 1 H); 5.49 (s, 1 H); 4.84 (t, $J$ = 7.5, 1 H); 2.37 (q, $J$ = 7.5, 2 H); 2.07 (s, 3 H); 1.47 (sextet, $J$ = 7.4, 2 H); 1.10 (br s, 8H); 0.93 (t, $J$ = 7.4, 3 H).

**EXAMPLE 112**

(*R*/*S*)-5-(3-Chlorophenyl)-9-fluoro-1,2-dihydro-2,2,4-trimethyl-*5H*-chromeno[3,4-*f*]quinoline (Compound **212**, structure **42** of **Scheme XI**, where R=3-chlorophenyl, $R^1$=H, $R^2$=F)

This compound was prepared by General Method 5 (EXAMPLE 60) from Compound **207** (50 mg, 0.16 mmol) and 3-bromochlorobenzene (120 mL) to afford 46 mg (70%) of Compound **212** as a colorless solid. Data for Compound **212:** $^1$**H NMR** (400 MHz, acetone-$d_6$): 7.56 (d, *J* = 8.4, 1 H); 7.36 (dd, *J* = 9.8, 2.9, 1 H); 7.25 (m, 4 H); 6.95 (s, 1 H); 6.85 (d, *J* = 8.5, 1 H); 6.81 (m, 1 H); 6.74 (td, *J* = 8.5, 2.9, 1 H); 5.51 (s, 1 H); 2.00 (d, *J* = 1.0, 3 H); 1.28 (s, 3 H); 1.26 (s, 3 H).

**EXAMPLE 113**

(*R*/*S*)-5-(4-Chloro-3-methylphenyl)-9-fluoro-1,2-dihydro-2,2,4-trimethyl-*5H*-chromeno[3,4-*f*]quinoline (Compound **213**, structure **42** of **Scheme XI**, where R=4-chloro-3-methylphenyl, $R^1$=H, $R^2$=F)

This compound was prepared by General Method 5 (EXAMPLE 60) from Compound **207** (50 mg, 0.16 mmol) and 5-bromo-2-chlorotoluene (0.21 g) to afford 42 mg (62%) of Compound **213** as a colorless solid. Data for Compound 213: $^1$**H NMR** (400 MHz, acetone-$d_6$): 7.55 (d, *J* = 8.4, 1 H); 7.34 (dd, *J* = 10.0, 2.8, 1 H); 7.22 (m, 2 H); 7.00 (br d, *J* = 10.3, 1 H); 6.89 (s, 1 H); 6.84 (d, *J* = 8.4, 1 H); 6.75 (m, 2 H); 5.49 (s, 1 H); 2.24 (s, 3 H); 1.99 (d, *J* = 1.2, 3 H); 1.27 (s, 3 H); 1.25 (s, 3 H).

**EXAMPLE 114**

(*R*/*S*)-5-(4-Chlorophenyl)-9-fluoro-1,2-dihydro-2,2,4-trimethyl-*5H*-chromeno[3,4-*f*]quinoline (Compound **214**, structure **42** of **Scheme XI,** where R=4-chlorophenyl, $R^1$=H, $R^2$=F)

This compound was prepared by General Method 5 (EXAMPLE 60) from Compound **207** (50 mg, 0.16 mmol) and 4-bromochlorobenzene (0.19 g) to afford 33 mg (50%) of Compound **214** as a pale yellow oil. Data for Compound **214:** $^1$**H NMR** (400 MHz, acetone-$d_6$) 7.55 (d, *J* = 8.4, 1 H); 7.34 (dd, *J* = 10.0, 2.8, 1 H); 7.27 (d, *J* = 8.6, 2 H); 7.22 (d, *J* = 8.6, 2 H); 6.92 (s, 1 H); 6.84 (d, *J* = 8.5, 1 H); 6.75 (m, 2 H); 5.60 (br s, 1 H); 5.48 (d, *J* = 1.3, 1 H); 1.99 (d, *J* = 1.3, 3 H); 1.27 (s, 3 H); 1.24 (s, 3 H).

**EXAMPLE 115**

(*R*/*S*)-9-Fluoro-1,2-dihydro-5-(4-methoxyphenyl)-2,2,4-trimethyl-*5H*-chromeno[3,4-*f*]quinoline (Compound **215**, structure **42** of **Scheme XI**, where R=4-methoxyphenyl, $R^1$=H, $R^2$=F)

This compound was prepared by General Method 5 (EXAMPLE 60) from Compound **207** (50 mg, 0.16 mmol) and 4-bromoanisole (0.13 mL) to afford 8 mg (12%) of Compound **215** as a pale yellow oil. Data for Compound **215:** $^1$**H NMR** (400 MHz, acetone-$d_6$) 7.53 (d, *J* = 8.4, 1 H); 7.34 (dd, *J* = 10.0, 2.8, 1 H); 7.11 (d, *J* = 8.8, 2 H); 6.86 (s, 1 H); 6.82 (d, *J* = 8.4, 1 H); 6.76 (d, *J* = 8.6, 2 H); 6.70 (m, 2 H); 5.6 (br s, 1 H); 5.46 (s, 1 H); 3.70 (s, 3 H); 1.99 (s, 3 H); 1.26 (s, 3 H); 1.23 (s, 3 H).

**EXAMPLE 116**

(*R*/*S*)-8-Fluoro-1,2-dihydro-5-methoxy-2,2,4-trimethyl-*5H*-chromeno[3,4-*f*]quinoline (Compound **216**, structure **47** of **Scheme XIV**, where $R^1$=F, $R^2$=H, $R^3$=methyl, X=0)

(*R*/*S*)-8-Fluoro-1,2-dihydro-5-hydroxy-2,2,4-trimethyl-*5H*-chromeno[3,4-*f*]quinoline (structure **46** of **Scheme XIV**, where $R^1$=F, $R^2$=H)

This compound was prepared in a manner similar to that of Compound **185** (EXAMPLE 85) from Compound **208** (170 mg) and DIBALH (1.0 M in hexane; 1.25 mL) to afford 27 mg (16%) of (*R*/*S*)-8-fluoro-1,2-dihydro-5-hydroxyl-2,2,4-trimethyl-*5H*-chromeno[3,4-*f*]quinoline as a white solid. Data for (*R*/*S*)-8-fluoro-1,2-dihydro-5-hydroxy-2,2,4-trimethyl-*5H*-chromeno[3,4-*f*]quinoline: $^1$**H NMR** (400 MHz, acetone-$d_6$): 7.74 (dd, *J* = 8.6, 6.3, 1 H); 7.50 (d, *J* = 8.4, 1 H); 6.85 (s, 1 H); 6.79 (m, 2 H); 6.72 (dd, *J* = 9.9, 2.7, 1 H); 5.51 (d, *J* = 1.2, 1 H); 2.82 (s, 3 H); 1.30 (s, 3 H); 1.17 (s, 3 H).

(*R*/*S*)-8-Fluoro-1,2-dihydro-5-methoxy-2,2,4-trimethyl-*5H*-chromeno[3,4-*f*]quinoline (Compound **216**, structure **47** of **Scheme XIV**, where $R^1$=F, $R^2$=H, $R^3$=methyl, X=0)

This compound was prepared in a manner similar to that of Compound **186** (EXAMPLE 86) from (*R*/*S*)-8-fluoro-1,2-dihydro-5-hydroxy-2,2,4-trimethyl-*5H*-chromeno[3,4-*f*]quinoline (24 mg) to afford 25 mg (quant) of Compound **216** as a white solid. Data for Compound **216:** $^1$**H NMR** (400 MHz, acetone-$d_6$) 7.74 (dd, *J* = 8.5, 6.2, 1 H); 7.50 (d, *J* = 8.4, 1 H); 6.85 (m, 2 H); 6.79 (d, *J* = 8.4, 1 H); 6.38 (s, 1 H); 5.52 (t, *J* = 1.0, 1 H); 3.46 (s, 3 H); 2.26 (d, *J* = 1.2, 3 H); 1.31 (s, 3 H); 1.15 (s, 3 H).

## EXAMPLE 117

(*R*/*S*)-5-(4-Chlorophenyl)-8-fluoro-1,2-dihydro-2,2,4-trimethyl-*5H*-chromeno[3,4-*f*]quinoline (Compound **217**, structure **42** of **Scheme XI**, where R=4-chlorophenyl, $R^1$=F, $R^2$=H)

This compound was prepared by General Method 5 (EXAMPLE 60) from Compound **208** (42 mg, 0.13 mmol) and 4-bromochlorobenzene (0.19 g) to afford 10 mg (18%) of Compound **217** as a pale yellow oil. Data for Compound **217:** $^1$**H NMR** (400 MHz, acetone-$d_6$) 7.62 (dd, *J* = 8.6, 6.3, 1 H); 7.53 (d, *J* = 8.4, 1 H); 7.27 (d, *J* = 8.7, 2 H); 7.23 (d, *J* = 8.7, 2 H); 6.96 (s, 1 H); 6.83 (d, *J* = 8.2, 1 H); 6.67 (m, 1 H); 6.58 (dd, *J*= 8.7, 2.5, 1 H); 5.48 (d, *J* = 1.3, 1 H); 1.99 (d, *J* = 1.2, 3 H); 1.26 (s, 3 H); 1.23 (s, 3 H).

## EXAMPLE 118

(*R*/*S*)-9-Chloro-5-(4-chlorophenyl)-1,2-dihydro-2,2,4-trimethyl-*5H*-chromeno[3,4-*f*]quinoline (Compound **218**, structure **42** of **Scheme XI**, where R=4-chlorophenyl, $R^1$=H, $R^2$=Cl)

This compound was prepared by General Method 5 (EXAMPLE 60) from Compound **209** (40 mg, 0.12 mmol) and 4-bromochlorobenzene (0.19 g) to afford 23 mg (44%) of Compound **218** as an off-white oil. Data for Compound **218:** $^1$**H NMR** (400 MHz, acetone-$d_6$) 7.59 (d, *J* = 2.5, 1 H); 7.58 (d, *J* = 8.4, 1 H); 7.27 (d, *J* = 8.6, 2 H); 7.22 (d, *J* = 8.6, 2 H); 6.96 (dd, *J* = 8.5, 2.4, 1 H); 6.94 (s, 1 H); 6.84 (d, *J* = 8.4, 1 H); 6.78 (d, *J* = 8.5, 1 H); 5.7 (br s, 1 H); 5.49 (d, *J* = 1.1, 1 H); 1.99 (d, *J* = 1.1, 3 H); 1.27 (s, 3 H); 1.24 (s, 3 H).

General Method 8. 1,2-Dihydro-2,2,4-trimethylquinolines (Compounds of structure **57** or **67**) from anilines (Compounds of structure **56** or **66**): pressure tube version

In a threaded resealable pressure tube, a solution of the aniline (a compound of structure **56** or **66)** in acetone (0.05-0.20M) was treated with iodine (5-20 mol%) and heated to 100-120 °C for 1-3 days. The reaction vessel was allowed to cool to rt and transferred to a r.b. flask. Addition of Celite™ followed by concentration afforded a fluffy orange powder which was purified by silica gel chromatography to afford the desired dihydroquinoline (Compound of structure **57** or **67).**

## EXAMPLE 213

9-Chloro-1,2-dihydro-2,2-dimethyl-5-coumarino[3,4-*f*]quinoline (Compound **313**, structure **88** of **Scheme XXIII**, where $R^{1-2}$=$R^{4-6}$=$R^9$=H, $R^7$=$R^8$=methyl, $R^3$=chloro)

2-Amino-6-chloro-3,4-benzocoumarin (structure 87 of **Scheme XXIII**, where $R^{1-2}$=$R^{4-6}$ =H, $R^3$=chloro, an intermediate from EXAMPLE 109) (100 mg, 0.407 mmol) and 1,1-dimethyl propargyl acetate (52 mg, 0.41 mmol) were dissolved in THF (5 mL) and treated with triethylamine (57 µL, 0.41 mmol). The resulting solution was treated with CuCl (20 mg, 0.20 mmol). The reaction mixture was heated at relux for 16 h. The reaction was quenched with 1% (v/v) HCl (2 mL) and diluted with EtOAc (20 mL). The mixture was poured into a separatory funnel and the aqueous layer was extracted with EtOAc (2 x 20 mL). The combined organics were washed with brine (1 x 20 mL), dried ($Na_2SO_4$), filtered, and concentrated onto Celite. The material was purified by flash chromatography on silica gel (50 g) using 15% EtOAc:hexanes as eluent to afford 50 mg of the dimethyl propargyl amine intermediate. This material was dissolved in THF and treated with CuCl (2 mg, 0.02 mmol) and heated at reflux for 16 h. The reaction was quenched with 1 % (v/v) HCl (2 mL) and diluted with EtOAc and water. The reaction mixture was poured into a separatory funnel and the aqueous was extracted with EtOAc (2 x 20 mL). The combined organics were washed with brine (15 mL), dried ($Na_2SO_4$), filtered, and concentrated onto Celite. The material was purified by flash chromatography on silica gel (20 g) using 15% EtOAc:hexanes to afford 30 mg (24%) of Compound **313** as a yellow solid. Data for Compound **313:** $^1$**H**

**NMR** (400 MHz, acetone-$d_6$) 8.07 (d, *J* = 2.4, 1H), 7.99 (d, *J* = 8.7, 1H), 7.91 (d, *J* = 10.4, 1H), 7.38 (dd, *J* = 8.6, 2.4, 1H), 7.26 (d, *J* = 8.7, 1H), 7.10 (d, *J* = 8.5, 1H), 6.04 (br s, 1H), 5.74 (dd, *J* = 10.4, 1.4, 1H), 1.36 (s, 6H).

**EXAMPLE 215**

9-Fluoro-1,2-dihydro-2,2,4,11-tetramethyl-5-coumarino[3,4-*f*]quinoline (Compound **315**, structure **88** of **Scheme XXIV**, where $R^{1-2}$=$R^4$=$R^6$=H, $R^3$=fluoro, $R^5$=$R^{7-9}$=methyl)

Methyl 2'-fluoro-5'-methoxy-6-methyl-4-nitro-2-biphenylcarboxylate (structure **92** of **Scheme XXIV**, where $R^{1-2}$=$R^4$=$R^6$=H, $R^3$=fluoro, $R^5$=methyl).

This compound was prepared in a manner similar to that of methyl 5'-fluoro-2'-methoxy-4-nitro-2-biphenylcarboxylate (EXAMPLE 107) from methyl 2-bromo-3-methyl-5-nitrobenzoate (1.73 g, 6.31 mmol), $(PPh_3)_4Pd$ (0.22 g, 0.19 mmol), and 5-fluoro-2-methoxyphenylboronic acid (EXAMPLE 107) (1.50 g, 8.8 mmol) to afford 0.77 g (38%) of methyl 2'-fluoro-5'-methoxy-6-methyl-4-nitro-2-biphenylcarboxylate. Data for 2'-fluoro-5'-methoxy-6-methyl-4-nitro-2-biphenylcarboxylate: $^1$H NMR (400 MHz, acctone-$d_6$) 8.61 (d, *J* = 2.3, 1H), 8.27 (d, *J* = 2.4, 1H), 7.09 (m, 1H), 6.91 (dd, *J* = 9.0, 4.3, 1H), 6.73 (dd, *J* = 8.2, 3.0, 1H), 3.70 (s, 3H), 3.69 (s, 3H), 2.19 (s, 3H).

2'-Fluoro-5'-methoxy-4-nitro-2-biphenylcarboxylic acid.

This compound was prepared in a manner similar to that of 5'-fluoro-2'-methoxy-4-nitro-2-biphenylcarboxylic acid (EXAMPLE 107) from methyl 2'-fluoro-5'-methoxy-6-methyl-4-nitro-2-biphenylcarboxylate (0.77 g) to afford 0.73 g (99%) of 2'-fluoro-5'-methoxy-4-nitro-2-biphenylcarboxylic acid as a white solid, which was used in the next step without further purfication.

6-Fluoro-4-methyl-2-nitro-3,4-benzocoumarin.

This compound was prepared in a manner similar to that of 6-fluoro-2-nitro-3,4-benzocoumarin (EXAMPLE 107) from 2'-fluoro-5'-methoxy-4-nitro-2-biphenylcarboxylic acid (0.73 g, 2.4 mmol), $SOCl_2$ (0.18 mL, 2.4 mmol), and $AlCl_3$ (0.32 g, 2.4 mmol) to afford 0.63 g (95%) of 6-fluoro-4-methyl-2-nitro-3,4-benzocoumarin as an orange powder. Data for 6-fluoro-4-methyl-2-nitro-3,4-benzocoumarin: **$^1$H NMR** (400 MHz, acetone-$d_6$) 8.99 (d, *J* = 2.5, 1H), 8.63 (d, *J* = 2.5, 1H), 8.29 (dd, *J* = 10.9, 2.4, 1H), 7.53 (m, 2H), 3.14 (s, 3H).

2-Amino-6-fluoro-4-methyl-3,4-benzocoumarin (structure **87** of **Scheme XXIV**, where $R^{1-2}$=$R^4$=$R^6$=H, R3=fluoro, R5=methyl).

This compound was prepared in a manner similar to that of 2-amino-6-fluoro-3,4-benzocoumarin (EXAMPLE 107) from 6-fluoro-4-methyl-2-nitro-3,4-benzocoumarin (0.61 g) to afford 0.54 g (99%) of 2-amino-6-fluoro-4-methyl-3,4-benzocoumarin as a white solid, which was used in the next step without further purification.

9-Fluoro-1,2-dihydro-2,2,4,11-tetramethyl-5-coumarino[3,4-*f*]quinoline (Compound **315**, structure **88** of **Scheme XXIV**, where $R^{1-2}$=$R^4$=$R^6$=H, $R^3$=fluoro, $R^5$=$R^{7-9}$=methyl).

This compound was prepared in a manner similar to that of Compound **207** from 2-amino-6-fluoro-4-methyl-3,4-benzocoumarin (0.54 g) to afford 0.29 g (40%) of Compound **315** as a yellow solid. Data for Compound **315: $^1$H NMR** (400 MHz, acetone-$d_6$) 7.87 (dd, *J* = 11.4, 2.9, 1H), 7.32 (dd, *J* = 9.0, 5.1, 1H), 7.28 (m, 1H), 7.02 (s, 1H), 5.52 (d, *J* = 1.2, 1H), 2.76 (s, 3H), 2.01 (s, 3H), 1.30 (s, 6H).

**EXAMPLE 217**

7-Chloro-1,2-dihydro-2,2,4-trimethyl-5-coumarino[3,4-*f*]quinoline (Compound **317**, structure **88** of **Scheme XXIV**, where $R^1$=chloro, $R^{2-6}$=H, $R^{7-9}$=methyl)

3-Chloro-2-methoxyphenylboronic acid (structure **90** of **Scheme XXIV**, where $R^1$=chloro, $R^{2-4}$=H).

This compound was prepared in a manner similar to that of 5-fluoro-2-methoxyphenylboronic acid (EXAMPLE 107) from 2-bromo-6-chloroanisole (0.71 g, 3.2 mmol), *n*-BuLi (2.5 M in hexanes; 1.28 mL, 3.2 mmol), and trimethylborate (1.09 mL, 9.6 mmol) to afford 0.55 g (91 %) of 3-chloro-2-methoxyphenylboronic acid which was used without

further purification.

Methyl (3'-chloro-2'-methoxy-4-nitro-2-biphenylcarboxylate).

This compound was prepared in a manner similar to that of methyl (5'-fluoro-2'-methoxy-4-nitro-2-biphenylcarboxylate) (EXAMPLE 107) from methyl 2-bromo-5-nitrobenzoate (0.58 g, 2.2 mmol), $(PPh_3)_4Pd$ (77 mg, 0.066 mmol), and 5-chloro-2-methoxyphenylboronic acid (0.54 g, 2.9 mmol) to afford 245 mg (35%) of methyl (3'-chloro-2'-methoxy-4-nitro-2-biphenylcarboxylate) as a clear oil. Data for methyl (3'-chloro-2'-methoxy-4-nitro-2-biphenylcarboxylate): **$^1$H NMR** (400 MHz, $CDCl_3$) 8.79 (d, *J* = 2.4, 1H), 8.40 (dd, *J* = 8.4, 2.4, 1H), 7.57 (d, *J* = 8.5, 1H), 7.45 (m, 1H), 7.15 (m, 2H), 3.75 (s, 3H), 3.47 (s, 3H).

3'-Chloro-2'-methoxy-4-nitro-2-biphenylcarboxylic acid.

This compound was prepared in a manner similar to that of 5'-fluoro-2'-methoxy-4-nitro-2-biphenylcarboxylic acid (EXAMPLE 107) from methyl (3'-chloro-2'-methoxy-4-nitro-2-biphenylcarboxylate) (230mg) to afford 0.21 g (99%) of 3'-chloro-2'-methoxy-4-nitro-2-biphenylcarboxylic acid as a white solid. Data for 3'-chloro-2'-methoxy-4-nitro-2-biphenylcarboxylic acid: **$^1$H NMR** (400 MHz, acetone-$d_6$) 8.76 (d, *J* = 2.5, 1H), 8.50 (dd, *J* = 8.4, 2.5, 1H), 7.74 (d, *J* = 8.5, 1H), 7.51 (dd, *J* = 7.9, 1.8, 1H), 7.31 (dd, *J* = 7.4, 1.8, 1H), 7.24 (t, *J* = 7.9), 3.47 (s, 3H).

8-Chloro-2-nitro-3,4-benzocoumarin.

This compound was prepared in a manner similar to that of 6-fluoro-2-nitro-3,4-benzocoumarin (EXAMPLE 107) from 3'-chloro-2'-methoxy-4-nitro-2-biphenylcarboxylic acid (0.20 g, 0.65 mmol), $SOCl_2$ (50 mL, 0.69 mmol), and $AlCl_3$ (85 mg, 0.65 mmol) to afford 0.18 g (99%) of 8-chloro-2-nitro-3,4-benzocoumarin as yellow crystals. Data for 8-chloro-2-nitro-3,4-benzocoumarin: **$^1$H NMR** (400 MHz, acetone-$d_6$) 9.06 (t, *J* = 1.4, 1 H), 8.74 (s, 2H), 8.45 (dd, *J* = 8.0, 1.4, 1H), 7.32 (dd, *J* = 8.0, 1.2, 1H), 7.51 (t, *J* = 8.0, 1H).

2-Amino-8-chloro-3,4-benzocoumarin (structure **87** of **Scheme XXIV**, where $R^1$=chloro, $R^{2-6}$=H).

This compound was prepared in a manner similar to that of 2-amino-6-fluoro-3,4-benzocoumarin (EXAMPLE 107) from 8-chloro-2-nitro-3,4-benzocoumarin (0.18 g, 0.65 mmol) to afford 0.10 g (62%) of 2-amino-8-chloro-3,4-benzocoumarin as a white solid, which was used in the next step without further purification.

7-Chloro-1,2-dihydro-2,2,4-trimethyl-5-coumarino[3,4-*f*]quinoline (Compound **317**, structure **88** of **Scheme XXIV**, where $R^1$=chloro, $R^{2-6}$=H, $R^{7-9}$=methyl).

This compound was prepared in a manner similar to that of Compound **207** from 2-amino-9-chloro-3,4-benzocoumarin (0.10 g) to afford 24 mg (18%) of Compound **317** as a yellow solid. Data for Compound **317: $^1$H NMR** (400 MHz, acetone-$d_6$) 8.04 (dd, *J* = 8.1, 1.1, 1H), 7.98 (d, *J* = 8.7, 1H), 7.48 (dd, *J* = 9.0, 1.1, 1H), 7.28 (t, *J* = 8.8, 1H), 7.23 (d, *J* = 8.6, 1H), 6.24 (br s, 1H), 5.55 (d, *J* = 1.2, 1H), 2.08 (s, 3H), 1.31 (s, 6H).

**EXAMPLE 218**

5-(3-Fluorobenzyl)-1,2-dihydro-2,2,4-trimethyl-5*H*-chromeno[3,4-*f*]quinoline (Compound **318**, structure **32** of **Scheme IX**, where R = 3-fluorobenzyl)

To a solution of Compound **225** (EXAMPLE 125) (10 mg, 0.03 mmol) in $CH_2Cl_2$ (5 ml) was added triethylsilane (0.05 ml, 0.3 mmol) and trifluoroacetic acid (0.024 ml, 0.3 mmol) at rt. The reaction was monitored by TLC and was found to be complete after 15 hours. The reaction mixture was quenched with an aqueous 10% NaOH solution (5mL) then extracted with EtOAc (10 mL). The organic layer was washed with brine (3 x 5 mL), dried ($Na_2SO_4$), then concentrated *in vacuo* to afford a yellow oil. The crude product was purified by prep TLC (5 x 20cm, 250mm, 1:1 $CH_2Cl_2$: hexane) to afford 1.0 mg (8%) of Compound **318** as a yellow oil. Data for Compound **318**: Rf=0.26 (silica gel, 25% EtOAc:hex); **$^1$H NMR** (400 MHz, $CDCl_3$) 7.66 (d, *J*= 8.5, 1 H), 7.48( d, *J*= 8.5 1 H), 7.23 (m, 2 H), 7.03 (m, 1 H), 6.89 (m, 3 H), 6.61 (d, *J*= 8.5, 1 H), 6.10 (m, 1 H), 5.49 (s, 1 H), 3.98 (brs, 1 H), 3.10 (m, 1 H), 2.73 (m, 1 H), 2.29 (s, 3 H), 1.29 (s, 3 H), 1.19 (s, 3 H).

**EXAMPLE 219**

(*R*/*S*)-9-Chloro-1,2-dihydro-5-methoxy-2,2,4-trimethyl-5*H*-chromeno[3,4-*f*]quinoline (Compound **319**, structure **47** of **Scheme XIV**, where $R^1$=H, $R^2$=chloro, $R^3$=methyl, X=O)

(*R*/*S*)-9-chloro-1,2-dihydro-5-hydroxy-2,2,4-trimethyl-5*H*-chromeno[3,4-*f*]quinoline (structure **46** of **Scheme XIV**, where $R^1$=H, $R^2$=chloro) and 6-(5-chloro-2-hydroxyphenyl)-1,2-dihydro-5-hydroxymethyl-2,2,4-trimethylquinoline (structure **94** of **Scheme XXV**, where $R^{1-2}$=$R^{4-6}$=H, $R^3$=chloro, $R^{7-9}$ =methyl).

Compound **209** (EXAMPLE 109) (100 mg, 0.307 mmol) was dissolved in THF, cooled to -40°C, and treated with DIBAL (614 µL, 0.614 mmol, 1 M in THF, Aldrich), warming to -20°C over 30 min. The reaction mixture was quenched with $NH_4Cl$ (sat) (2 mL) and allowed to warm to rt. The reaction mixture was poured into a separatory funnel containing EtOAc and water. The aqueous was extracted with EtOAc (2 x 20 mL). The combined organics were washed with NaCl (sat) (1 x 15 mL), dried ($Na_2SO_4$), filtered, and concentrated onto Celite. The material was purified by flash chromatography using 25% EtOAc:hexanes to afford 65 mg of (*R*/*S*)-9-chloro-1,2-dihydro-5-hydroxy-2,2,4-trimethyl-5*H*-chromeno[3,4-*f*]quinoline (structure **46** of **Scheme XIV,** where $R^1$=H, $R^2$=chloro) and 20 mg of 6-(5-chloro-2-hydroxyphenyl)-1,2-dihydro-5-hydroxymethyl-2,2,4-trimethylquinoline (structure 94 of **Scheme XXV,** where $R^{1-2}$=$R^{4-6}$=H, $R^3$=chloro, $R^{7-9}$ =methyl). Data for (*R*/*S*)-9-chloro-1,2-dihydro-5-hydroxy-2,2,4-trimethyl-5*H*-chromeno[3,4-*f*]quinoline: **$^1$H NMR** (400 MHz, acetone-$d_6$) 7.71 (d, *J* = 2.4, 1H), 7.55 (d, *J* = 8.4, 1H), 7.11 (dd, *J* = 8.5, 2.4, 1H), 6.94 (d, *J* = 8.4, 1H), 6.84 (d, *J* = 5.9, 1H), 6.78 (d, *J* = 8.2, 1H), 6.01 (d, *J* = 6.0, 1H), 5.56 (bs, 1H), 5.52 (s, 1H), 2.36 (s, 3H), 1.31 (s, 3H), 1.18 (s, 3H). Data for 6-(5-chloro-2-hydroxyphenyl)-1,2-dihydro-5-hydroxymethyl-2,2,4-trimethylquinoline: **$^1$H NMR** (400 MHz, acetone-$d_6$) 7.18 (dd, *J* = 8.5, 3.0, 1H), 7.10 (d, *J* = 2.5, 1H), 6.92 (d, *J* = 8.6, 1H), 6.75 (d, *J* = 8.0, 1H), 6.63 (d, *J* = 8.1, 1H), 5.46 (s, 1H), 5.25 (s, 1H), 4.55 (ABq, *J* = 11.4, 2H), 2.35 (s, 3H), 1.27 (s, 6H).

(*R*/*S*)-9-Chloro-1,2-dihydro-5-methoxy-2,2,4-trimethyl-5*H*-chromeno[3,4-*f*]quinoline (Compound **319**, structure **47** of **Scheme XIV**, where $R^1$=H, $R^2$=chloro, $R^3$=methyl, X=O).

(*R*/*S*)-9-chloro-1,2-dihydro-5-hydroxy-2,2,4-trimethyl-5*H*-chromeno[3,4-*f*]quinoline (30 mg, 0.092 mmol) was dissolved in methanol (3 mL) and treated with p-toluenesulfonic acid (10 mg). After 10 min the reaction was quenched with $NaHCO_3$ (2 mL). The resulting mixture was diluted with water (2 mL), poured into a separatory funnel, and extracted with EtOAc (3 x 20 mL). The combined organics were washed with NaCl(sat) (1 x 20 mL), dried ($Na_2SO_4$), filtered, and concentrated onto Celite. The material was purified by flash chromatography on silica (20 g) using 10 % EtOAc: hexanes as eluent to afford 20 mg (64%) of Compound **319** as an opaque oil. Data for Compound **319: $^1$H NMR** (400 MHz, acetone-$d_6$) 7.73 (d, *J* = 2.4, 1H), 7.56 (d, *J* = 8.3, 1H), 7.17 (dd, *J* = 8.2, 2.4, 1H), 7.08 (d, *J* = 8.3, 1H), 6.80 (d, *J* = 8.3, 1H), 6.37 (s, 1H), 5.62 (br s, 1H), 5.54 (s, 1H), 3.44 (s, 3H), 2.27 (2, 3H), 1.32 (s, 3H), 1.17 (s, 3H).

**EXAMPLE 220**

9-Chloro-1,2-dihydro-2,2,4-trimethyl-5*H*-chromeno[3,4-*f*]quinoline (Compound **320**, structure **93** of **Scheme XXV**, where $R^{1-2}$=$R^{4-6}$=H, $R^3$=chloro, $R^{7-9}$=methyl)

6-(5-Chloro-2-hydroxyphenyl)-1,2-dihydro-5-hydroxymethyl-2,2,4-trimethylquinoline (EXAMPLE 219; structure 94 of **Scheme XXV,** where $R^{1-2}$=$R^{4-6}$=H, $R^3$=chloro, $R^{7-9}$ =methyl)(20 mg, 0.061 mmol) was dissolved in $CH_2Cl_2$ and treated with thionyl chloride (5 µL, 0.067 mmol) and triethylamine (9 µL 0.067 mmol). After 2 h the reaction was quenched with water and poured into a separatory funnel containing $CH_2Cl_2$ (20 mL) and water (10mL). The aqueous was extracted with $CH_2Cl_2$ (2 x 20 mL). The combined organics were washed with NaCl (sat)(1 x 15 mL), dried ($Na_2SO_4$), filtered and concentrated. The resulting benzyl chloride intermediate was dissolved in 1,2-dichloroethane (1mL) and treated with triethylamine (100 mL), then heated to reflux. After 1 h the reaction was quenched with water and poured into a separatory funnel. The pH was adjusted to 6 (1%v/v HCl) and the aqueous was extracted with $CH_2Cl_2$ (2 x 20 mL) The combined organics were washed with NaCl(sat) (20 mL), dried ($Na_2SO_4$), filtered, and concentrated onto Celite. The material was purified by flash chromatography on silica gel (20 g) using 5% EtOAc:hexanes to afford 10 mg (53%) of Compound **320**. Data for Compound **320: $^1$H NMR** (400 MHz, acetone-$d_6$) 7.60 (d, *J* = 2.4, 1H), 7.43 (d, *J* = 8.4, 1H), 7.08 (dd, *J* = 8.5,2.4, 1h), 6.89 (d, *J* = 8.5, 1H), 6.70 (d, *J* = 8.4, 1H), 5.56 (br s, 1H), 5.49 (s, 1H), 5.32 (s, 2H), 2.11 (s, 3H), 1.25 (s, 6H).

**EXAMPLE 221**

(*R*/*S*)-9-Chloro-1,2-dihydro-2,2,4-trimethyl-5-propyloxy-5*H*-chromeno[3,4-*f*]quinoline (Compound **321**, structure **47** of **Scheme XIV**, where $R^1$=H, $R^2$=chloro, $R^3$=propyl, X=O)

(*R*/*S*)-9-Chloro-1,2-dihydro-5-hydroxy-2,2,4-trimethyl-5*H*-chromeno[3,4-*f*]quinoline (EXAMPLE 219; structure **46** of **Scheme XIV**, where $R^1$=H, $R^2$=chloro) (30 mg, 0.092 mmol) was dissolved in 1-propanol (3 mL) and treated with p-toluenesulfonic acid (10 mg). After 10 min the reaction was quenched with $NaHCO_3$ (2 mL). The resulting mixture was diluted with $H_2O$ (2 mL), poured into a separatory funnel, and extracted with EtOAc (3 x 20 mL). The combined organics were washed with NaCl(sat) (1 x 20 mL), dried ($Na_2SO_4$), filtered, and concentrated onto celite. The material was purified by flash chromatography on silica (20 g) using 10 % EtOAc:hexanes as eluent to afford 22 mg (61%) of Compound **321** as an opaque oil. Data for Compound **321:** $^1$**H NMR** (400 MHz; acetone-$d_6$) 7.73 (d, *J* = 2.4, 1H), 7.56 (d, *J* = 8.5, 1H), 7.14 (dd, *J* = 8.3, 2.5, 1H), 7.03 (d, *J* = 8.5, 1H), 6.80 (d, *J* = 8.5, 1H), 6.46 (s, 1H), 5.60 (br s, 1H), 5.53 (s, 1H), 3.81 (m, 1H), 3.59 (m, 1H), 2.29 (s, 3H), 1.46 (m, 2H), 1.32 (s, 3H), 1.17 (s, 3H), 0.75, (m, 3H).

**EXAMPLE 222**

(*R*/*S*)-9-Fluoro-1,2-dihydro-5-methoxy-2,2,4-trimethyl-5*H*-chromeno[3,4-*f*]quinoline (Compound **322**, structure **47** of **Scheme XIV**, where $R^1$=H, $R^2$=fluoro, $R^3$=methyl, X=O).

This compound was prepared in a manner similar to that described for Compound **319** (EXAMPLE 219) from Compound **207** (55 mg) to afford 34 mg (59%) of Compound **322** as a clear oil. Data for Compound **322:** $^1$**H NMR** (400 MHz, acetone-$d_6$) 7.53 (d, *J* = 8.5, 1H), 7.48 (dd, *J* = 9.9, 3.0, 1H), 7.05 (dd, *J* = 8.7, 4.9, 1H), 6.92 (m, 1H), 6.80 (d, *J* = 8.3, 1H), 6.34 (s, 1H), 5.54 (d, *J* = 1.4, 1H), 3.44 (s, 3H), 2.28 (d, *J* = 1.4, 3H), 1.32 (s, 3H), 1.16 (s, 3H).

**EXAMPLE 223**

(*R*/*S*)-9-Fluoro-1,2-dihydro-2,2,4-trimethyl-5-thiopropoxy-5*H*-chromeno[3,4-*f*]quinoline (Compound **323**, structure **47** of **Scheme XIV**, where $R^1$=H, $R^2$=fluoro, $R^3$=propyl, X=S).

(*R*/*S*)-9-Fluoro-1,2-dihydro-5-hydroxy-2,2,4-trimethyl-5*H*-chromeno[3,4-*f*]quinoline (structure **46** of **Scheme XIV**, where $R^1$=H, $R^2$=fluoro).

This compound was prepared in a manner similar to that of 9-chloro-1,2-dihydro-5-hydroxy-2,2,4-trimethyl-5*H*-chromeno[3,4-*f*]quinoline (EXAMPLE 219) from Compound **207** (0.16 g, 0.51 mmol) to afford 80 mg (50%) of 9-fluoro-1,2-dihydro-5-hydroxy-2,2,4-trimethyl-5*H*-chromeno[3,4-*f*]quinoline as a white solid. Data for 9-fluoro-1,2-dihydro-5-hydroxy-2,2,4-trimethyl-5*H*-chromeno[3,4-*f*]quinoline: $^1$**H NMR** (400 MHz, acetone-$d_6$) 7.52 (d, *J* = 8.5, 1H), 7.46 (dd, *J* = 9.9, 2.9, 1H), 6.93 (m, 1H), 6.86 (m, 2H), 6.78 (d, *J* = 8.5, 1H), 5.98 (d, *J* = 6.0, 1H), 5.56 (br s, 1H), 5.52 (d, *J* = 1.1, 1H), 2.37 (d, *J* = 1.2, 3H), 1.30 (s, 3H), 1.18 (s, 3H).

(*R*/*S*)-9-Fluoro-1,2-dihydro-2,2,4-trimethyl-5-thiopropoxy-5*H*-chromeno[3,4-*f*]quinoline (Compound **323**, structure **47** of **Scheme XIV**, where $R^1$=H, $R^2$=fluoro, $R^3$=propyl, X=S).

This compound was prepared in a manner similar to that of Compound **319** (EXAMPLE 219) from 9-fluoro-1,2-dihydro-5-hydroxy-2,2,4-trimethyl-5*H*-chromeno[3,4-*f*]quinoline (18 mg) to afford 21 mg (99%) of Compound **323** as a clear oil. Data for Compound **323:** $^1$**H NMR** (400 MHz, acetone-$d_6$) 7.48 (d, *J* = 8.5, 1H), 7.45 (dd, *J* = 10.0, 1.7, 1H), 7.14 (s, 1H), 6.95 (m, 2H), 6.73 (d, *J* = 8.5, 1H), 5.52 (d, *J* = 1.3 1H), 2.76 (m, 1H), 2.58 (dt, *J* = 12.9, 7.4, 1H), 2.47 (d, *J* = 1.2,3H), 1.66 (m, 2H), 1.25 (s, 3H), 1.22 (s, 3H), 0.95 (t, *J* = 7.3, 3H).

**EXAMPLE 224**

(*R*/*S*)-9-Fluoro-1,2-dihydro-2,2,4-trimethyl-5-propoxy-5*H*-chromeno[3,4-*f*]quinoline (Compound **324**, structure **47** of **Scheme XIV**, where $R^1$=H, $R^2$=fluoro, $R^3$=propyl, X=O).

This compound was prepared in a manner similar to that of Compound **319** (EXAMPLE 219) from 9-fluoro-1,2-dihydro-5-hydroxy-2,2,4-trimethyl-*5H*-chromeno[3,4-*f*]quinoline (EXAMPLE 223) (20 mg) to afford 21 mg (95%) of Compound **324** as a white solid. Data for Compound **324:** $^1$**H NMR** (400 MHz, acetone-$d_6$) 7.53 (d, *J* = 8.4, 1H), 7.47 (dd, *J* = 9.9, 2.9, 1H), 7.02 (dd, *J* = 8.8, 5.0, 1H), 6.95 (m, 1H), 6.80 (d, *J* = 8.5, 1H), 5.53 (d, *J* = 1.5, 1H), 3.81 (dt, *J*

= 9.2, 6.7, 1H), 3.58 (dt, *J* = 9.2, 6.7, 1H), 2.29 (d, *J* = 1.5, 3H), 1.46 (sext, *J* = 6.9, 2H), 1.32 (s, 3H), 1.16 (s, 3H), 0.75 (t, *J* = 7.4, 3H).

## EXAMPLE 225

(*R*/*S*)-5-Butyl-9-chloro-1,2-dihydro-2,2,4-trimethyl-5*H*-chromeno[3,4-*f*]quinoline (Compound **325**, structure **42** of **Scheme XI**, where $R^1$=H, $R^2$=chloro, R=butyl).

This compound was prepared by General Method 5 (EXAMPLE 60) from Compound **209** (38 mg, 0.12 mmol) and 2.5 M *n*-BuLi in hexanes (0.28 mL, 0.70 mmol) to afford 7 mg (16%) of Compound **325** as a clear oil. Data for Compound **325: $^1$H NMR** (400 MHz, acetone-$d_6$) 7.72 (d, *J* = 2.4, 1 H), 7.58 (d, *J* = 8.5, 1H), 7.12 (dd, *J* = 8.3, 2.5, 1H), 7.05 (d, *J* = 8.5, 1H), 6.75 (d, *J* = 8.5, 1H), 5.53 (s, 1H), 4.82 (t, *J* = 8.0, 1H), 2.40 (m, 2H), 2.09 (s, 3H), 1.5-1.4 (m, 6H), 1.25 (br s, 6H), 0.95 (t, *J* = 7.8, 3H).

## EXAMPLE 226

(*R*/*S*)-5-Butyl-1,2-dihydro-9-methoxy-2,2,4-trimethyl-5*H*-chromeno[3,4-*f*]quinoline (Compound **326**, structure **42** of **Scheme XI**, where $R^1$=H, $R^2$=methoxy, R=butyl).

This compound (12 mg, 33%) was obtained as a by-product in the formation of Compound **355** (EXAMPLE 255) as a colorless oil. Data for Compound **326: $^1$H NMR** (400 MHz, acetone-$d_6$) 7.47 (d, *J* = 8.4, 1 H), 7.20 (d, *J* = 2.8, 1H), 6.80 (d, *J* = 8.5, 1H), 6.69 (m, 2H), 5.79 (dd, *J* = 10.3, 3.2, 1H), 5.51 (d, *J* = 1.2, 1H), 3.80 (s, 3H), 2.24 (d, *J* = 1.0, 1 H), 1.74 (m, 1H), 1.5-1.3 (m, 5H), 1.27 (s, 3H), 1.18 (s, 3H), 0.84 (t, *J* = 7.5, 3H).

## EXAMPLE 227

(*R*/*S*)-9-Fluoro-1,2-dihydro-2,2,4,5-tetramethyl-5*H*-chromeno[3,4-*f*]quinoline (Compound **327**, structure **42** of **Scheme XI**, where $R^1$=H, $R^2$=fluoro, R=methyl).

This compound was prepared by General Method 5 (EXAMPLE 60) from Compound **207** (36 mg, 0.12 mmol) and 1.4 M MeLi in ether (0.45 mL, 0.63 mmol) to afford 6 mg (16%) of Compound **327** as a clear oil. Data for Compound **327: $^1$H NMR** (400 MHz, acetone-$d_6$) 7.46 (d, *J* = 8.4, 1H), 7.41 (dt, *J* = 10.0, 1.5, 1H), 6.84 (m, 2H), 6.80 (d, *J* = 8.5, 1H), 6.07 (q, *J* = 6.5, 1H), 5.53 (d, *J* = 1.4, 1H), 2.25 (d, *J* = 1.1, 3H), 1.32 (d, *J* = 6.5, 3H), 1.26 (s, 3H), 1.20 (s, 3H).

## EXAMPLE 228

(*R*/*S*)-9-Fluoro-1,2-dihydro-2,2,4-trimethyl-5*H*-chromeno[3,4-*f*]quinoline (Compound **328**, structure **42** of **Scheme XI**, where $R^1$=R=H, $R^2$=fluoro).

This compound was prepared in a manner similar to that described for Compound **202** (EXAMPLE 102) from 9-fluoro-1,2-dihydro-5-hydroxy-2,2,4-trimethyl-5*H*-chromeno[3,4-*f*]quinoline (EXAMPLE 223) (15 mg) to afford 14 mg (99%) of Compound **328** as a clear glass. Data for Compound **328: $^1$H NMR** (400 MHz, acetone-$d_6$) 7.41 (d, *J* = 8.4, 1H), 7.41 (dt, *J* = 10.0, 1.5, 1H), 6.84 (m, 2H), 6.70 (d, *J* = 8.4, 1H), 5.49 (d, *J* = 1.2, 1H), 5.29 (s, 2H), 2.11 (d, *J* = 1.6, 3H), 1.26 (s, 6H).

## EXAMPLE 229

(*R*/*S*)-1,2-Dihydro-9-methoxy-2,2,4-trimethyl-5*H*-chromeno[3,4-*f*]quinoline (Compound **329**, structure **42** of **Scheme XI**, where $R^1$=R=H, $R^2$=methoxy).

(*R*/*S*)-1,2-Dihydro-5-hydroxy-9-methoxy-2,2,4-trimethyl-5*H*-chromeno[3,4-*f*]quinoline (structure **46** of **Scheme XIV**, where $R^1$=H, $R^2$=methoxy).

This compound was prepared in a manner similar to that of 9-chloro-1,2-dihydro-5-hydroxy-2,2,4-trimethyl-5*H*-chromeno[3,4-*f*]quinoline (EXAMPLE 219) from Compound **314** (24 mg, 0.075 mmol) to afford 15 mg (62%) of 1,2-dihydro-5-hydroxy-9-methoxy-2,2,4-trimethyl-5*H*-chromeno[3,4-*f*]quinoline as a white solid, which was used directly in the next step.

(*R*/*S*)-1,2-Dihydro-9-methoxy-2,2,4-trimethyl-5*H*-chromeno[3,4-*f*]quinoline (Compound **329**, structure **42** of **Scheme XI**, where $R^1$=R=H, $R^2$=methoxy)

This compound was prepared in a manner similar to that described for Compound **202** (EXAMPLE 102) from 1,2-dihydro-5-hydroxy-9-methoxy-2,2,4-trimethyl-5*H*-chromeno[3,4-*f*]quinoline (15 mg) to afford 12 mg (98%) of Compound **329** as a clear glass. Data for Compound **329: $^1$H NMR** (400 MHz, acetone-$d_6$) 7.41 (d, *J* = 8.3, 1H), 7.16 (d, *J* = 3.0, 1H), 6.81 (d, *J* = 8.6, 1H), 6.68 (m, 1H), 5.48 (d, *J* = 1.2, 1H), 5.23 (s, 2H), 3.80 (s, 3H), 2.10 (d, *J* = 1.2, 3H), 1.24 (s, 6H).

**EXAMPLE 230**

(*R*/*S*)-1,2-Dihydro-2,2,4,9-tetramethyl-5*H*-chromeno[3,4-*f*]quinoline (Compound **330**, structure **42** of **Scheme XI**, where $R^1$=R=H, $R^2$=methyl)

This compound was prepared in a manner similar to Compound **229** (EXAMPLE 229) from Compound **316** (34 mg, 0.11 mmol) to afford 16 mg (50%) of Compound **330** as a clear oil. Data for Compound **330: $^1$H NMR** (400 MHz, acetone-$d_6$) 7.42 (m, 1H), 7.41 (d, *J* = 8.4, 1H), 6.90 (m, 1H), 6.76 (d, *J* = 8.0, 1H), 6.64 (d, *J* = 8.4, H), 5.48 (s, 1H), 5.41 (br s, 1H), 5.25 (s, 2H), 2.30 (s, 3H), 2.11 (d, *J* = 1.4, 3H), 1.24 (s, 6H).

**EXAMPLE 231**

(*R*/*S*)-7-Chloro-1,2-dihydro-2,2,4-trimethyl-5*H*-chromeno[3,4-*f*]quinoline (Compound **331**, structure **93** of **Scheme XXIV**, where $R^1$=chloro, $R^{2-6}$=H, $R^{7-9}$=methyl)

This compound was prepared in a manner similar to Compound **229** (EXAMPLE 229) from Compound **317** (20 mg, 0.061 mmol) to afford 11 mg (58%) of Compound **331** as a clear oil. Data for Compound **331: $^1$H NMR** (400 MHz, acetone-$d_6$) 7.57 (dd, *J* = 7.9, 1.2, 1H), 7.42 (d, *J* = 8.4, 1H), 7.18 (dd, *J* = 7.9, 1.2, 1H), 6.98 (t, *J* = 7.9, 1 H), 6.70 (d, *J* = 8.4, 1H), 5.56 (br s, 1H), 5.50 (d, *J* = 1.2, 1H), 5.40 (s, 2H), 2.14 (d, *J* = 1.3, 3H), 1.25 (s, 6H).

**EXAMPLE 232**

(*R*/*S*)-9-Chloro-1,2-dihydro-2,2,4,5-tetramethyl-5*H*-chromeno[3,4-*f*]quinoline (Compound **332**, structure **42** of **Scheme XI**, where $R^1$=H, $R^2$=chloro, R=methyl)

This compound was prepared by General Method 5 (EXAMPLE 60) from Compound **209** (40 mg, 0.123 mmol) and methyllithium (438 µl, 0.614 mmol, 1.4 M in ether, Aldrich) to afford 8 mg (20%) of Compound **332** as an opaque oil. Data for Compound **332: $^1$H NMR** (400 MHz, acetone-$d_6$) 7.65 (d, *J*= 2.5, 1h), 7.49 (d, *J*= 8.4, 1H), 7.08 (dd, *J*= 8.5, 2.4, 1H), 6.85 (d, *J*= 8.5, 1H), 6.70 (d, *J*= 8.5, 1H), 6.09 (s, 1H), 5.52 (s, 1H), 2.25 (s, 3H), 1.32 (d, *J* = 6.5, 3H), 1.26 (s, 3H), 1.20 (s, 3H).

**EXAMPLE 233**

(*R*/*S*)-5-(4-Bromophenyl)-9-chloro-1,2-dihydro-2,2,4-trimethyl-5*H*-chromeno[3,4-*f*]quinoline (Compound **333**, structure **42** of **Scheme XI**, where R=4-bromophenyl, $R^1$=H, $R^2$=Cl)

This compound was prepared by General Method 5 (EXAMPLE 60) from Compound **209** (40 mg, 0.123 mmol) and 1,4-dibromobenzene (203 mg, 0.859 mmol) to afford 1 mg (19%) of Compound **333** as a pale cream colored oil. Data for Compound **333: $^1$H NMR** (400 MHz, acetone-$d_6$) 7.59 (d, *J*= 2.4, 1H), 7.58 (d, *J*= 6.3, 1H), 7.42 (d, *J*= 8.5, 2H), 7.16 (d, *J*= 8.5, 2H), 6.94 (dd, *J* = 8.2, 4.2, 1H), 6.92 (s, 1H), 6.84 (d, *J*= 8.4, 1H), 6.77 (d, *J*= 8.4, 1H), 5.68 (br s, 1H), 5.48 (s, 1H), 1.98 (s, 3H), 1.27 (s, 3H), 1.24 (s, 3H).

**EXAMPLE 234**

(*R*/*S*)-9-Chloro-5-(3-chlorophenyl)-1,2-dihydro-2,2,4-trimethyl-5*H*-chromeno[3,4-*f*]quinoline (Compound **334**, structure **42** of **Scheme XI**, where R=3-chlorophenyl, $R^1$=H, $R^2$=Cl)

This compound was prepared by General Method 5 (EXAMPLE 60) from Compound **209** (40 mg, 0.123 mmol) and 3-bromochlorobenzene (164 mg, 0.856 mmol) to afford 9 mg (17%) of Compound **334** as a pale yellow oil. Data

for Compound **334:** $^{1}$**H NMR** (400 MHz, acetone-$d_6$) 7.61 (d, *J*= 2.3, 1H), 7.59 (d, *J*= 9.0, 1H), 7.25 (m, 4H), 6.95 (m, 2H), 6.85 (d, *J*= 8.3, 1H), 6.83 (d, *J*= 7.3, 1H), 5.72 (br s, 1H), 5.50 (s, 1H), 2.00 (s, 3H), 1.28 (s, 3H), 1.26 (s, 3H).

**EXAMPLE 235**

(*R*/*S*)-9-Chloro-1,2-dihydro-2,2,4-trimethyl-5-(3-methylphenyl)-5*H*-chromeno[3,4-*f*]quinoline (Compound **335**, structure **42** of **Scheme XI**, where R=3-methylphenyl, $R^1$=H, $R^2$=Cl)

This compound was prepared by General Method 5 (EXAMPLE 60) from Compound **209** (20 mg, 0.061 mmol) and 3-bromotoluene (147 mg, 0.859 mmol) to afford 10 mg (41%) of Compound **335** as a pale white oil. Data for Compound **335:** $^{1}$**H NMR** (400 MHz, acetone-$d_6$) 7.59 (d, *J*= 2.4, 1H), 7.58 (d, *J*= 9.1, 1H), 7.19 (m, 2H), 6.95 (m,3H), 6.83 (d, *J*= 8.5, 1H), 6.78 (d, *J*= 8.5, 1H), 5.64 (br s, 1H), 5.81 (s, 1H), 2.20 (s, 3H), 2.05 (s, 3H), 1.27 (s, 3H), 1.24 (s, 3H).

**EXAMPLE 236**

(*R*/*S*)-9-Chloro-5-(4-chloro-3-methylphenyl)-1,2-dihydro-2,2,4-trimethyl-5*H*-chromeno[3,4-*f*]quinoline (Compound **336**, structure **42** of **Scheme XI**, where R=4-chloro-3-methylphenyl, $R^1$=H, $R^2$=Cl)

This compound was prepared by General Method 5 (EXAMPLE 60) from Compound **209** (20 mg, 0.061 mmol) and 5-bromo-2-chlorotoluene (177 mg, 0.859 mmol) to afford 9 mg (34%) of Compound 336 as a cream colored oil. Data for Compound **336:** $^{1}$**H NMR** (400 MHz, acetone-$d_6$) 7.60 (d, *J*= 2.4, 1H), 7.57 (d,*J*= 8.5, 1H), 7.23 (m, 2H), 7.00 (m, 2H), 6.91 (s, 1H), 6.84 (d, *J*= 8.2, 1H), 6.79 (d, *J*= 8.5, 1H), 5.68 (br s, 1H), 5.48 (ds, 1H), 2.23 (s, 3H), 1.99 (s, 3H), 1.27 (s, 3H), 1.25 (s, 3H).

**EXAMPLE 237**

(*R*/*S*)-9-Chloro-1,2-dihydro-5-[3-(trifluoromethyl)phenyl]-2,2,4-trimethyl-5*H*-chromeno[3,4-*f*]quinoline (Compound **337**, structure **42** of **Scheme XI**, where R=3-(trifluoromethyl)phenyl, $R^1$=H, $R^2$=Cl)

This compound was prepared by General Method 5 (EXAMPLE 60) from Compound **209** (40 mg, 0.123 mmol) and 3-bromobenzotrifluoride (276 mg, 1.23 mmol) to afford 11 mg (20%) of Compound **337** as a cream colored oil. Data for Compound **337:** $^{1}$**H NMR** (400 MHz, acetone-$d_6$) 7.61 (d, *J*= 2.3, 1H), 7.52 (m, 4H), 7.07 (s, 1H), 6.99 (dd, *J*= 8.5, 2.4, 1H), 6.87 (d, *J*= 8.3, 1H), 6.84 (d, *J*= 8., 1H), 5.73 (br s, 1H), 5.51 (s, 1H), 2.01 (s, 3H), 1.27 (s, 6H).

**EXAMPLE 238**

(*R*/*S*)-9-Chloro-5-(3,5-dichlorophenyl)-1,2-dihydro-2,2,4-trimethyl-5*H*-chromeno[3,4-*f*]quinoline (Compound **338**, structure **42** of **Scheme XI**, where R=3,5-dichlorophenyl, $R^1$=H, $R^2$=Cl)

This compound was prepared by General Method 5 (EXAMPLE 60) from Compound **209** (40 mg, 0.123 mmol) and 1-bromo-3,5-dichlorobenzene (277 mg, 1.23 mmol) using diethyl ether for the formation of the aryl lithium in the first step. The final step afforded 11 mg (20%) of Compound **338** as a pale yellow oil. Data for Compound **338:** $^{1}$**H NMR** (400 MHz, acetone-$d_6$) 7.64 (d, *J*= 2.3, 1H), 7.61 (d, *J*= 8.5, 1H), 7.32 (s, 1H), 7.20 (s, 1H), 7.19 (s, 1H), 7.03 (dd, *J* = 8.9, 2.4, 1H), 6.91 (s, 1H), 6.89 (d, *J* = 6.7, 1H), 6.88 (d, *J* = 6.7, 1H), 5.78 (br s, 1H), 5.53 (s, 1H), 2.03 (s, 3H), 1.28 (s, 3H), 1.27 (s, 3H).

**EXAMPLE 239**

(*R*/*S*)-9-Chloro-1,2-dihydro-5-(4-methoxyphenyl)-2,2,4-trimethyl-5*H*-chromeno[3,4-*f*]quinoline (Compound **339**, structure **42** of **Scheme XI**, where R= 4-methoxyphenyl, $R^1$=H, $R^2$=Cl)

This compound was prepared by General Method 5 (EXAMPLE 60) from Compound **209** (40 mg, 0.123 mmol) and 4-bromoanisole (230 mg, 1.23 mmol) to afford 11 mg (21%) of Compound **339** as a pale yellow oil. Data for Compound **339:** $^{1}$**H NMR** (400 MHz, acetone-$d_6$) 7.59 (d, *J*= 2.5, 1H), 7.56 (d, *J*= 8.5, 1H), 7.11 (d, *J*= 8.7, 2H), 6.94 (dd, *J*= 8.5, 2.4, 1H), 6.89 (s, 1H), 6.82 (d, *J*= 8.5, 1H), 6.75 (m, 3H), 5.61 (br s, 1H), 5.45 (s, 1H), 3.69 (s, 3H), 1.99 (s, 3H), 1.26 (s, 3H), 1.23 (s, 3H).

## EXAMPLE 240

(*R*/*S*)-9-Chloro-5-(3-fluoro-4-methoxyphenyl)-1,2-dihydro-2,2,4-trimethyl-5*H*-chromeno[3,4-*f*]quinoline (Compound **340**, structure **42** of **Scheme XI**, where R=3-fluoro-4-methoxyphenyl, $R^1$=H, $R^2$=Cl)

This compound was prepared by General Method 5 (EXAMPLE 60) from Compound **209** (20 mg, 0.061 mmol) and 4-bromo-2-fluoroanisole (88 mg, 0.429 mmol) to afford 8 mg (29%) of Compound **340** as a pale yellow oil. Data for Compound **340:** $^1$**H NMR** (400 MHz, acetone-$d_6$) 7.60 (d, *J*= 2.4, 1H), 7.58 (d, *J*= 8.5, 1H), 7.02 (dd, *J*= 10.2, 2.4, 1H), 6.97 (dd, *J*= 8.5, 2.3, 1H), 6.94 (d, *J*= 8.5, 1H), 6.90 (s, 1H), 6.89 (m, 1H), 6.84 (d, *J*= 8.5, 1H), 6.79 (d, *J*= 8.5, 1H), 5.68 (br s, 1H), 5.48 (s, 1H), 3.79 (s, 3H), 2.00 (s, 3H), 1.27 (s, 3H), 1.24 (s, 3H).

## EXAMPLE 241

(*R*/*S*)-9-Chloro-5-(4-fluorophenyl)-1,2-dihydro-2,2,4-trimethyl-5*H*-chromeno[3,4-*f*]quinoline(Compound **341**, structure **42** of **Scheme XI**, where R=4-fluorophenyl, $R^1$=H, $R^2$=Cl)

This compound was prepared by General Method 5 (EXAMPLE 60) from Compound **209** (40 mg, 0.123 mmol) and 4-fluorophenyl magnesium bromide (1 ml, 1.03 mmol, 1 M, Aldrich) to afford 11 mg (22%) of Compound **341** as a pale yellow oil. Data for Compound **341:** $^1$**H NMR** (400 MHz, acetone-$d_6$) 7.60 (d, *J* = 2.4, 1H), 7.58 (d, *J* = 7.3, 1H), 7.24 (m, 2H), 6.96 (m, 4H), 6.84 (d, *J* = 8.3, 1H), 5.67 (br s, 1H), 5.48 (s, 1H), 1.98 (s, 3H), 1.26 (s, 3H), 1.24 (s, 3H).

## EXAMPLE 242

(*R*/*S*)-9-Chloro-5-(3-chloro-4-methoxy-5-methylphenyl)-1,2-dihydro-2,2,4-trimethyl-5*H*-chromeno[3,4-*f*]quinoline (Compound **342**, structure **42** of **Scheme XI**, where R=3-chloro-4-methoxy-5-methylphenyl, $R^1$=H, $R^2$=Cl)

This compound was prepared by General Method 5 (EXAMPLE 60) from Compound **209** (40 mg, 0.123 mmol) and 4-bromo-2-chloro-5-methylanisole (181 mg, 0.770 mmol) to afford 12 mg (21 %) of Compound **342** as a pale yellow oil. Data for Compound **342:** $^1$**H NMR** (400 MHz, acetone-$d_6$) 7.61 (d, *J* = 2.4, 1H), 7.58 (d, *J* = 8.4, 1 H), 7.03 (m, 1H), 6.99 (dd, *J* = 8.5, 2.4, 2H), 6.91 (s, 1H), 6.84 (dd, J = 8.3, 3.8, 2H), 5.69 (br s, 1H), 5.49 (s, 1H), 3.70 (s, 3H), 2.18 (s, 3H), 2.01 (s, 3H), 1.27 (s, 3H), 1.26 (s, 3H).

## EXAMPLE 243

(*R*/*S*)-9-Chloro-5-(4-fluoro-3-methylphenyl)-1,2-dihydro-2,2,4-trimethyl-5*H*-chromeno[3,4-*f*]quinoline (Compound **343**, structure **42** of **Scheme XI**, where R=4-fluoro-3-methylphenyl, $R^1$=H, $R^2$=Cl)

This compound was prepared by General Method 5 (EXAMPLE 60) from Compound **209** (40 mg, 0.123 mmol) and 4-fluoro-3-phenyl magnesium bromide (1 ml, 1.026 mmol, 1M, Aldrich) to afford 8 mg (16%) of Compound 343 as a pale yellow oil. Data for Compound **343:** $^1$**H NMR** (400 MHz, acetone-$d_6$) 7.59 (d, *J* = 2.6, 1H), 7.57 (d, *J* = 8.6, 1H), 7.12 (d, *J* = 8.1, 1H), 6.99 (m, 1H), 6.96 (dd, *J* = 8.2, 2.4, 1H), 6.90 (m, 1H), 6.84 (d, *J* = 8.3, 1H), 6.77 (d, *J* = 8.5, 1H), 5.68 (br s, 1H), 5.48 (s, 1H), 2.14 (s, 3H), 1.25 (s, 3H), 1.24 (s, 3H).

## EXAMPLE 244

(*R*/*S*)-9-Chloro-5-(3-fluorophenyl)-1,2-dihydro-2,2,4-trimethyl-5*H*-chromeno[3,4-*f*]quinoline (Compound **344**, structure **42** of **Scheme XI**, where R=3-fluorophenyl, $R^1$=H, $R^2$=Cl)

This compound was prepared by General Method 5 (EXAMPLE 60) from Compound **209** (40 mg, 0.123 mmol) and 1-bromo-3-fluorobenzene (150 mg, 0.860 mmol) to afford 11 mg (22%) of Compound **344** as a pale yellow oil. Data for Compound **344:** $^1$**H NMR** (400 MHz, acetone-$d_6$) 7.61 (d, *J* = 2.4, 1H),7.59 (d, *J* = 8.4, 1 H), 7.29 (m, 1H), 7.04 (d, *J* = 7.9, 1H), 6.97 (m, 4H), 6.85 (d, *J* = 8.5, 1H), 6.80 (d, *J* = 8.5, 1H), 5.7 (br s, 1H), 5.50 (s, 1H), 2.01 (s, 3H), 1.27 (s, 3H), 1.25 (s, 3H).

**EXAMPLE 245**

(*R*/*S*)-1,2-Dihydro-2,2,4-trimethyl-5-[(3,4-methylenedioxy)phenyl]-5*H*-chromeno[3,4]-*f*quinoline (Compound **345**, structure **32** of **Scheme IX**, where R=3,4-(methylenedioxy)phenyl

This compound was prepared by the General Method 5 (EXAMPLE 60) from 4-bromo-1,2-(methylenedioxy) benzene (201 mg, 1.0 mmol) and Compound **159** (15 mg, 0.05 mmol) to afford 1.5 mg (8%) of Compound **345** as a colorless oil. Data for Compound **345**: **$^1$H NMR** (400 MHz, acetone-$d_6$) 7.60 (d, *J* = 7.6, 1 H), 7.55 (d, *J* = 8.4, 1 H), 6.98 (t, *J* = 7.6, 1 H), 6.88-6.60 (m, 6 H), 5.98 (s, 1 H), 5.91 (s, 2 H), 5.51 (bs, 1 H), 5.46 (s, 1 H), 2.02 (s, 3 H), 1.25 (s, 3 H), 1.23 (s, 3 H).

**EXAMPLE 246**

(*R*/*S*)-5-(4-Chloro-3-methylphenyl)-1,2-dihydro-2,2,4-trimethyl-5*H*-chromeno[3,4]-*f*quinoline (Compound **346**, structure **32** of **Scheme IX**, where R=4-chloro-3-methylphenyl)

This compound was prepared by the General Method 5 (EXAMPLE 60) from 5-bromo-2-chlorotoluene (206 mg, 1.0 mmol) and Compound **159** (10 mg, 0.03 mmol) to afford 8.0 mg (67%) of Compound **346** as a colorless oil. Data for Compound **346: $^1$H NMR** (400 MHz, acetone-$d_6$) 7.60 (d, *J* = 7.6, 1 H), 7.55 (d, *J*= 8.4, 1 H), 7.23-7.19 (m, 2 H), 7.01 (d, *J*= 9.9, 1 H), 6.97 (d, *J*= 7.7, 1 H), 6.89 (s, 1 H), 6.88-6.81 (m, 2 H), 6.78 (d, *J* = 8.0, 1 H), 5.55 (bs, 1 H), 5.48 (s, 1 H), 2.22 (s, 3 H), 2.00 (s, 3 H), 1.26 (s, 3 H), 1.24 (s, 3 H).

**EXAMPLE 247**

(*R*/*S*)-5-(4-Bromo-3-pyridyl)-1,2,3,4-tetrahydro-2-2-dimethyl-4-methylidene-5*H*-chromeno[3,4-*f*]quinoline (Compound **347**, structure **33** of **Scheme IX**, where R=4-bromo-3-pyridyl).

This compound (1.8 mg, 3%) was obtained as a colorless oil along with Compound **197** as described above (EXAMPLE 97). Data for Compound **347: $^1$H NMR** (400 MHz, $CDCl_3$) 8.22 (d, *J* = 5.2, 1 H), 7.56-7.49 (m, 2 H), 7.34 (s, 1 H), 7.12 (d, *J* = 6.5, 1 H), 7.03 (td, *J* = 7.4, 1.3, 1 H), 6.92 (td, *J* = 7.4, 1.3, 1 H), 6.86 (d, *J* = 7.5, 1 H), 6.61 (d, *J* = 8.2, 1 H), 6.58 (s, 1 H), 4.98 (s, 1 H), 4.52 (s, 1 H), 2.43 (d, *J* = 14.5, 1 H), 2.22 (d, *J* = 14.5, 1 H), 1.34 (s, 3 H), 1.17 (s, 3 H).

**EXAMPLE 248**

(*R*/*S*)-5-(3,5-Difluorophenyl)-1,2-dihydro-2,2,4-trimethyl-5*H*-chromeno[3,4-*f*]quinoline (Compound **348**, structure **32** of **Scheme IX**, where R=3,5-difluorophenyl)

This compound was prepared by the General Method 5 (EXAMPLE 60) from 1-bromo-3,5-difluorobenzene (193 mg, 1.0 mmol) and Compound **159** (20 mg, 0.07 mmol) to afford 14 mg (53%) of Compound **348** as a colorless oil. Data for Compound **348: $^1$H NMR** (400 MHz, acetone-$d_6$) 7.63 (d, *J* = 7.6, 1 H), 7.58 (d, *J* = 8.4, 1 H), 7.03 (t, *J* = 7.7, 1 H), 6.95 (s, 1 H), 6.94-6.83 (m, 6 H), 5.62 (bs, 1 H), 5.11 (s, 1 H), 2.04 (s, 3 H), 1.27 (s, 3 H), 1.26 (s, 3 H).

**EXAMPLE 249**

(*R*/*S*)-5-(3,5-Dichlorophenyl)-1,2-dihydro-2,2,4-trimethyl-5*H*-chromeno[3,4-*f*]quinoline (Compound **349**, structure **32** of **Scheme IX**, where R=3,5-dichlorophenyl)

This compound was prepared by the General Method 5 (EXAMPLE 60) from 1-bromo-3,5-dichlorobenzene (226 mg, 1.0 mmol) and Compound **159** (15 mg, 0.05 mmol) to afford 20 mg (95%) of Compound **349** as a colorless oil. Data for Compound **349: IR** (neat) 3350, 2940, 1690, 1590, 1480, 1070; **$^1$H NMR** (400 MHz, acetone-$d_6$) 7.63 (d, *J* = 7.7, 1 H), 7.58 (d, *J* = 8.4, 1 H), 7.29 (t, *J* = 1.9, 1 H), 7.20 (d, *J* = 1.9, 2 H), 7.03 (t, *J* = 7.7, 1 H), 6.97 (s, 1 H), 6.93-6.85 (m, 3 H), 5.63 (bs, 1 H), 5.53 (s, 1 H), 2.04 (s, 3 H), 1.28 (s, 3 H), 1.27 (s, 3 H); **$^{13}$C NMR** (100 MHz, acetone-$d_6$) 151.0, 147.3, 145.7, 135.5, 135.1, 135.0, 129.0, 128.8, 128.6, 128.4, 127.8, 125.3, 124.6, 123.2, 123.0, 120.3, 119.7, 118.3, 116.4, 116.3, 74.9,51.2,24.0.

**EXAMPLE 250**

(*R*/*S*)-5-(3-Bromo-5-methylphenyl)-1,2-dihydro-2,2,4-trimethyl-5*H*-chromeno[3,4-*f*]quinoline (Compound **350**, structure **32** of **Scheme IX**, where R=3-bromo-5-methylphenyl)

This compound was prepared by the General Method 5 (EXAMPLE 60) from 3,5-dibromotoluene (250 mg, 1.0 mmol) and Compound **159** (10 mg, 0.03 mmol) to afford 6.1 mg (46%) of Compound **350** as a colorless oil. Data for Compound **350:** $^1$**H NMR** (400 MHz, acetone-$d_6$) 7.61 (d, *J* = 7.7, 1 H), 7.56 (d, *J* = 8.4, 1 H), 7.17 (s, 1 H), 7.14 (s, 1 H), 7.10 (s, 1 H), 7.01 (t, *J* = 7.7, 1 H), 6.91 (s, 1 H), 6.90-6.82 (m, 3 H), 5.58 (bs, 1 H), 5.50 (s, 1 H), 2.21 (s, 3 H), 2.02 (s, 3 H), 1.27 (s, 3 H), 1.26 (s, 3 H).

**EXAMPLE 251**

(*R*/*S*)-5-(3-Bromo-5-fluorophenyl)-1,2-dihydro-2,2,4-trimethyl-5*H*-chromeno[3,4-*f*]quinoline (Compound **351**, structure **32** of **Scheme IX**, where R=3-bromo-5-fluorophenyl)

This compound was prepared by the General Method 5 (EXAMPLE 60) from 1,3-dibromo-5-fluorobenzene (254 mg, 1.0 mmol) and Compound **159** (10 mg, 0.03 mmol) to afford 6.2 mg (46%) of Compound **351** as a white powder, along with 0.7 mg (5%) of Compound **352** (EXAMPLE 252). Data for Compound **351:** mp 82-84 °C; **IR** (neat) 3367, 1699, 1595, 1581, 1469, 1435, 1251; $^1$**H NMR** (400 MHz, acetone-$d_6$) 7.63 (d, *J* = 7.7, 1 H), 7.58 (d, *J* = 8.4, 1 H), 7.23 (d, *J* = 5.2, 1 H), 7.20 (s, 1 H), 7.08-7.02 (m, 2 H), 6.97 (s, 1 H), 6.94-6.85 (m, 3 H), 5.64 (bs, 1 H), 5.53 (s, 1 H), 2.04 (s, 3 H), 1.28 (s, 3 H), 1.27 (s, 3 H); $^{13}$**C NMR** (100 MHz, acetone-$d_6$) 163.4 (d, *J* = 250 Hz), 151.1, 147.3, 146.4 (d, *J* = 7.0 Hz), 135.0, 129.1, 128.8, 128.4, 128.3, 125.3, 124.6, 123.2, 123.0, 122.9, 120.4, 119.7, 119.2 (d, *J* = 24.8 Hz), 118.3, 116.4, 115.2 (d, *J* = 22.2 Hz), 74.9, 51.2, 29.4, 24.0.

**EXAMPLE 252**

(*R*/*S*)-5-(3-Bromo-5-fluorophenyl)-1,2,3,4-tetrahydro-2,2-dimethyl-4-methylidene-5*H*-chromeno[3,4-*f*]quinoline (Compound **352**, structure **33** of **Scheme IX**, where R=3-bromo-5-fluorophenyl)

The compound (0.7 mg, 5%) was obtained along with Compound **351** as described above (EXAMPLE 251) as a colorless oil. Data for Compound **352:** $^1$**H NMR** (400 MHz, $CDCl_3$) 7.54 (d, *J* = 7.7, 1 H), 7.51 (d, *J* = 8.4, 1 H), 7.24 (d, *J* = 5.5, 1 H), 7.06-6.84 (m, 5 H), 6.60 (d, *J* = 8.4, 1 H), 6.57 (s, 1 H), 4.96 (s, 1 H), 4.56 (s, 1 H), 4.01 (bs, 1 H), 2.42 (d, *J* = 12.3, 1 H), 2.21 (d, *J* = 12.3, 1 H), 1.34 (s, 3 H), 1.15 (s, 3 H).

**EXAMPLE 253**

(*R*/*S*)-5-[4-Fluoro-3-(trifluoromethyl)phenyl]-1,2-dihydro-2,2,4-trimethyl-5*H*-chromeno[3,4-*f*]quinoline (Compound **353**, structure **32** of **Scheme IX**, where R=4-fluoro-3-(trifluoromethyl)phenyl)

This compound was prepared by the General Method 5 (EXAMPLE 60) from 5-bromo-2-fluorobenzotrifluoride (243 mg, 1.0 mmol) and Compound **159** (10 mg, 0.03 mmol) to afford 3.5 mg (27%) of Compound **353** as a colorless oil. Data for Compound **353:** $^1$**H NMR** (400 MHz, acetone-$d_6$) 7.62 (d, *J* = 7.7, 1 H), 7.61-7.53 (m, 3 H), 7.27 (t, *J* = 7.7, 1 H), 7.04-6.82 (m, 5 H), 5.62 (bs, 1 H), 5.51 (s, 1 H), 2.02 (s, 3 H), 1.26 (s, 6 H).

**EXAMPLE 254**

(*R*/*S*)-9-Fluoro-1,2-dihydro-2,2,4-trimethyl-5-(3-methylphenyl)-5*H*-chromeno[3,4-*f*]quinoline (Compound **354**, structure **42** of **Scheme XI,** where R=3-methylphenyl, $R^1$=H, $R^2$=F)

This compound was prepared by General Method 5 (EXAMPLE 60) from Compound **207** (31 mg, 0.10 mmol) and 3-bromotoluene (90 mL, 0.74 mmol) to afford 18 mg (46%) of Compound **354** as a colorless glass. Data for Compound **354:** $^1$**H NMR** (400 MHz, acetone-$d_6$) 7.53 (d, *J* = 8.5, 1H), 7.33 (dd, *J* = 9.9, 2.9, 1H), 7.08 (m, 2H), 6.98 (d, *J* = 6.7, 2H), 6.89 (s, 1H), 6.83 (d, *J* = 8.5, 1H), 6.75 (m, 2H), 5.48 (s, 1H), 2.20 (s, 3H), 1.99 (s, 3H), 1.27 (s, 3H), 1.25 (s, 3H).

**EXAMPLE 255**

(*R*/*S*)-1,2-Dihydro-9-methoxy-2,2,4-trimethyl-5-(3-methylphenyl)-5*H*-chromeno[3,4-*f*]quinoline (Compound **355**, structure **42** of **Scheme XI**, where R=3-methylphenyl, $R^1$=H, $R^2$=methoxy)

This compound was prepared by General Method 5 (EXAMPLE 60) from Compound **314** (32 mg, 0.10 mmol) and 3-bromotoluene (90 mL, 0.74 mmol) to afford 10 mg (25%) of Compound **355** as a colorless glass. Data for Compound **355:** $^1$**H NMR** (400 MHz, acetone-$d_6$) 7.53 (d, *J* = 8.5, 1H), 7.13 (d, *J* = 2.8, 1H), 7.08 (m, 2H), 6.99 (m, 2H), 6.83 (d, *J* = 6.0, 1H), 6.80 (s, 1H), 6.70 (d, *J* = 8.7, 1H), 6.55 (dd, *J* = 8.7, 2.8, 1H), 5.46 (d, *J* = 1.2, 1H), 3.72 (s, 3H), 2.24 (s, 3H), 1.98 (s, 3H), 1.26 (s, 3H), 1.24 (s, 3H).

**EXAMPLE 256**

(*R*/*S*)-9-Fluoro-5-(3-fluoro-4-methoxyphenyl)-1,2-dihydro-2,2,4-trimethyl-5*H*-chromeno[3,4-*f*]quinoline (Compound **356**, structure **42** of **Scheme XI**, where R=3-fluoro-4-methoxyphenyl, $R^1$=H, $R^2$=F)

This compound was prepared by General Method 5 (EXAMPLE 60) from Compound **207** (41 mg, 0.12 mmol) and 4-bromo-3-fluoroanisole (0.13 mL, 1.0 mmol) to afford 11 mg (20%) of Compound **356** as a yellow oil. Data for Compound **356:** $^1$**H NMR** (400 MHz, acetone-$d_6$) 7.55 (d, *J* = 8.5, 1H), 7.35 (dd, *J* = 10.0, 2.8, 1H), 7.01 (dd, *J* = 12.5, 1.9, 1H), 6.95 (t, *J* = 6.9, 1H), 6.87 (m, 3H), 6.74 (m, 2H), 5.48 (d, *J* = 1.2, 1H), 3.79 (s, 3H), 1.97 (s, 3H), 1.27 (s, 3H), 1.24 (s, 3H).

**EXAMPLE 257**

(*R*/*S*)-9-Fluoro-1,2-dihydro-2,2,4-trimethyl-5-[3-(trifluoromethyl)phenyl]-5*H*-chromeno[3,4-*f*]quinoline (Compound **357**, structure **42** of **Scheme XI**, where R=3-(trifluoromethyl)phenyl, $R^1$=H, $R^2$=F)

This compound was prepared by General Method 5 (EXAMPLE 60) from Compound **207** (40 mg, 0.12 mmol) and 3-bromobenzotrifluoride (0.14 mL, 1.0 mmol) to afford 11 mg (20%) of Compound **357** as a yellow oil. Data for Compound **357**: $^1$**H NMR** (400 MHz, acetone-$d_6$) 7.54 (d, *J* = 8.5, 1H), 7.35 (dd, *J* = 9.9, 2.9, 1H), 7.10 (m, 2H), 6.98 (d, *J* = 6.7, 2H), 6.89 (s, 1 H), 6.85 (d, *J* = 8.5, 1H), 6.75 (m, 2H), 5.48 (s, 1H), 1.99 (s, 3H), 1.27 (s, 3H), 1.25 (s, 3H).

**EXAMPLE 258**

(*R*/*S*)-9-Fluoro-5-(4-fluoro-3-methylphenyl)-1,2-dihydro-2,2,4-trimethyl-5*H*-chromeno[3,4-*f*]quinoline (Compound **358**, structure **42** of **Scheme XI**, where R=4-fluoro-3-methylphenyl, $R^1$=H, $R^2$=F)

This compound was prepared by General Method 5 (EXAMPLE 60) from Compound **207** (38 mg, 0.12 mmol) and 1.0 M 4-fluoro-3-methylphenyl magnesium chloride in THF (Aldrich) to afford 25 mg (51 %) of Compound **358** as a yellow oil. Data for Compound **358:** $^1$**H NMR** (400 MHz, acetone-$d_6$) 7.54 (d, *J* = 8.4, 1H), 7.34 (dd, *J* = 10.0, 2.8, 1H), 7.14 (m, 1H), 7.00 (m, 1H), 6.91 (d, *J* = 9.6, 1H), 6.88 (s, 1H), 6.83 (d, *J* = 8.4, 1H), 6.79-6.68 (m, 2H), 5.48 (s, 1H), 2.13 (s, 3H), 1.99 (s, 3H), 1.27 (s, 3H), 1.24 (s, 3H).

**EXAMPLE 268**

(Z)-5-Benzylidene-9-chloro-1,2-dihydro-2,2-dimethyl-5*H*-chromeno[3,4-*f*]quinoline (Compound **368**, structure **95** of **Scheme XXVI**, where $R^{1-2}$=$R^{4-6}$=$R^9$=H, $R^7$=$R^8$=methyl, $R^{10}$=phenyl)

This compound was prepared by General Method 6 (EXAMPLE 119) from Compound **313** (25 mg, 0.080 mmol) and benzyl magnesium chloride (0.802 mL, 0.802 mmol, 1 M solution in ether, Aldrich) to afford 5 mg (16%) of Compound **368** as a yellow oil. Data for Compound **368:** $^1$**H NMR** (400 MHz, acetone-$d_6$) 7.86 (d, *J* = 7.1, 1H), 7.79 (d, *J* = 2.2, 1H), 7.63 (d, *J* = 8.5, 1H), 7.40 (m, 2H), 7.20 (m, 4H), 6.89 (d, *J* = 8.6, 1H), 6.78 (d, *J* = 8.4, 1H), 5.99 (s, 1H), 5.70 (d, *J* = 8.3, 1H), 1.37 (s, 6H).

**EXAMPLE 277**

(Z)-5-Benzylidene-9-fluoro-1,2-dihydro-2,2,4,11-tetramethyl-5*H*-chromeno[3,4-*f*]quinoline (Compound **377**, structure **95** of **Scheme XXVI**, where $R^{1-2}$=$R^4$=$R^6$=H, $R^3$=F, $R^5$=$R^{7-9}$ =methyl, $R^{10}$=phenyl)

This compound was prepared by General Method 6 (EXAMPLE 119) from Compound **315** (28 mg, 0.087 mmol) and 1.0 M benzylmagnesium chloride in $Et_2O$ (Aldrich) to afford 19 mg (56%) of Compound **377** as a yellow foam. Data for Compound **377: $^1$H NMR** (400 MHz, acetone-$d_6$) 7.79 (d, *J* = 7.6, 2H), 6.63 (dd, *J* = 11.4, 2.9, 1H), 7.39 (app t, *J* = 7.8, 2H), 7.25 (m, 2H), 6.97 (m, 1H), 6.68 (s, 1H), 5.74 (s, 1H), 5.52 (d, *J* = 1.2, 1H), 2.61 (s, 3H), 1.97 (s, 3H), 1.33 (br s, 6H).

**EXAMPLE 278**

(*R*/*S*)-5-(4-Chlorophenyl)-1,2,3,4-tetrahydro-2,2-dimethyl-5*H*-chromeno[3,4-*f*]-4-quinolinone (Compound **378**, structure **97** of **Scheme XXVII**, where R=4-chlorophenyl)

To a solution of Compound **164** (EXAMPLE 64) (220 mg, 0.6 mmol) in 6 mL of THF at -78 °C was added 1.6 M *n*-BuLi hexane solution (1 mL, 1.6 mmol), followed by di-*t*-butyl dicarbonate (0.7 g, 3.2 mmol) in 2 mL of THF. The reaction mixture was warmed to rt and stirred for 15 h, then was quenched with 2% NaOH aqueous solution. The mixture was extracted with EtOAc (2 x 30 mL) and was concentrated. Chromatography of the crude residue on a silica gel column using 10 % EtOAc/hexane as solvent afforded the N-Boc Compound **164** (87 mg) in 30 % yield in addition to 60 % of the starting material (132 mg). The N-Boc material (40 mg, 0.082 mmol) in methanol (20 mL) at -78 °C was treated with $O_3$ for 3 min and then with methyl sulfide (0.5 mL) for 30 min. Removal of solvent and chromatography of the crude mixture afforded a colorless oil, which was treated with excess TFA (0.5 mL) in 1 mL of $CH_2Cl_2$ for 60 min. The reaction was quenched with 2% NaOH (5 mL) and was extracted with EtOAc (2 x 30 mL). Removal of the solvent and chromatography again provided 15 mg (47%) of Compound **378** as a yellow oil. Data for Compound **378: $^1$H NMR** (400 MHz, acetone-$d_6$) 7.86 (d, *J* = 8.8, 1 H), 7.61 (d, *J* = 7.7, 1 H), 7.40 (s, 1 H), 7.04 (t, *J* = 7.7, 1 H), 6.99 (d, *J* = 8.8, 1 H), 6.90 (t, *J* = 7.7, 1 H), 6.82 (d, *J* = 7.7, 1 H), 6.38 (bs, 1 H), 2.65 (d, *J* = 15.2, 1 H), 2.44 (d, *J* = 15.2, 1 H), 1.97 (s, 3 H), 1.37 (s, 3 H), 1.27 (s, 3 H).

EXAMPLE 279

(*R*/*S*)-5-(4-Chlorophenyl)-1,2,3,4-tetrahydro-2,2,3,3-tetramethyl-5*H*-chromeno[3,4-*f*]-4-quinolinone (Compound **379**, structure **98** of **Scheme XXVIII**, where R=4-chlorophenyl, $R^1$=methyl)

To a suspension of 40% NaH in mineral oil (10 mg, 0.25 mmol) in THF (1 mL) was added a solution of (*R*/*S*)-1-(*t*-butoxycarbonyl)-5-(4-chlorophenyl)-1,2,3,4-tetrahydro-2,2-dimethyl-5*H*-chromeno[3,4-*f*]-4-quinolinone (structure 96 of **Scheme XXVIII**, where R=4-chlorophenyl, $R^1$=methyl) (10 mg, 0.02 mmol) and excess MeI (0.1 mL). The reaction was stirred at rt for 2 h and was quenched with water (1 mL), and extracted with EtOAc (2 x 5 mL). Removal of solvent provided the crude mixture, which was treated with TFA (0.2 mL) in dichloromethane (1 mL) for 60 min. Chromatography of the crude mixture on a silica gel column using 15 % EtOAc/Hexane as solvent afforded 6.5 mg (78%) of Compound **379** as a colorless oil. Data for Compound **379: $^1$H NMR** (400 MHz, $CDCl_3$) 7.73 (d, *J* = 8.7, 1 H), 7.49 (d, *J* = 7.7, 1 H), 7.30 (s, 1 H), 7.13 (s, 4 H), 7.04 (t, *J* = 7.7, 1 H), 6.91 (t, *J* = 7.7, 1 H), 6.83 (d, *J* = 7.7, 1 H), 6.71 (d, *J* = 8.7, 1 H), 4.28 (s, I H), 1.29 (s, 3 H), 1.20 (s, 3 H), 1.13 (s, 3 H), 1.03 (s, 3 H).

**EXAMPLE 280**

(*R*/*S*)-5-(4-Chlorophenyl)-1,2-dihydro-2,2-dimethyl-5*H*-chromeno[3,4-f]quinoline (Compound **380**, structure **1A** of **Scheme XXIX**, where R=4-chlorophenyl)

To a solution of Compound **379** (EXAMPLE 279) (10 mg, 0.025 mmol) in toluene (1 mL) at -78 °C was added 0.5 M DIBAL toluene solution (0.1 mL, 0.05 mmol) and the resulting mixture was warmed up to rt. The reaction mixture was quenched with water (1 mL) and was extracted with EtOAc (2 x 5 mL). Removal of solvent and chromatography of the mixture on a silica gel column afforded 6.8 mg (70%) of 5-(4-chlorophenyl)-1,2,3,4-tetrahydro-4-hydroxy-2; 2-dimethyl-5*H*-chromeno[3,4-*f*]quinoline as a colorless oil, which was treated in dichloromethane (1 mL) with a catalytic amount of TsOH for 3 h to provide 4.1 mg (63%) of Compound **380** as a colorless oil. Data for Compound **380**: **$^1$H NMR** (400 MHz, acetone-$d_6$) 7.60 (d, *J* = 7.7, 1 H), 7.52 (d, *J* = 8.5, 1 H), 7.27 (d, *J* = 8.6, 2 H), 7.25 (d, *J* = 8.6, 2 H), 7.01 (t, *J* = 7.7, 1 H), 6.89 (t, *J* = 7.7 Hz, 1 H), 6.81 (d, *J* = 7.7, 1 H), 6.67 (d, *J* = 8.5, 1 H), 6.57 (s, 1 H), 6.33 (d, *J* = 10.0,

1 H), 5.57 (d, *J* = 10.0, 1 H), 5.55 (bs, 1 H), 1.32 (s, 3 H), 1.30 (s, 3 H).

**EXAMPLE 283**

(*R*/*S*)-5-(4-Chlorophenyl)-1,2,3,4-tetrahydro-2,2-dimethyl-5*H*-chromeno[3,4-*f*]-3-quinolinone (Compound **383**, structure **4A** of **Scheme XXIX**, where R=4-chlorophenyl)

This compound (0.63 mg, 3%) was prepared in a manner similar to that described for Compound **234** (EXAMPLE 134) from Compound **380** (EXAMPLE 280) as a colorless oil. The major product (41%) was Compound **378** (EXAMPLE 278). Data for Compound **383**: **$^1$H NMR** (400 MHz, $CDCl_3$) 7.62 (d, *J* = 8.3, 1 H), 7.60 (d, *J* = 7.7, 1 H), 7.17 (d, *J* = 8.6, 2 H), 7.09 (d, *J* = 8.6, 2 H), 7.06 (t, *J* = 7.7, 1 H), 6.94 (t, *J* = 7.7, 1 H), 6.83-6.80 (m, 2 H), 6.26 (s, 1 H), 3.88 (bs, 1 H), 3.55 (d, *J* = 20.0, 1 H), 3.11 (d, *J* = 10.0, 1 H), 1.33 (s, 3 H), 1.32 (s, 3 H).

**EXAMPLE 285**

(*R*/*S*)-3,5-Dibutyl-1,2,3,4-tetrahydro-3-hydroxy-2,2,4-trimethyl-5*H*-chromeno[3,4-*f*]quinoline (Compound **385**, structure **9A** of **Scheme XXXI**, where $R^1$=$R^2$=H, $R^3$=*n*-butyl)

To a solution of (*R*/*S*)-1,2,3,4-tetrahydro-2,2,4-trimethylcoumarino[3,4-*f*]-3-quinolinone (EXAMPLE 284) (4 mg, 0.01 mmol) in ether (3 mL) at rt was added 1.6 M *n*-BuLi hexane solution (0.05 mL, 0.08 mmol) and the resulting mixture was stirred at rt for 2 h, then was quenched with water (5 mL). The mixture was extracted with EtOAc (2 x 5 mL) and was concentrated and purified by silica gel chromatography to afford the intermediate, which was treated with $Et_3SiH$ (0.05 mL) and TFA (0.05 mL) in dichloromethane (1 mL) for 60 min. The reaction was quenched with 2% NaOH (5 mL) and was extracted with EtOAc (2 x 5 mL). Removal of solvent and chromatography of the mixture afforded 0.8 mg (20%) of Compound **385** as a colorless oil. The relative stereochemistry of this compound was not determined. Data for Compound **385: $^1$H NMR** (400 MHz, $CDCl_3$) 7.61 (d, *J* = 7.8, 1 H), 7.44 (d, *J* = 8.3Hz, 1 H), 7.14 (t, *J* = 7.8, 1 H), 6.98 (t, *J* = 7.8, 1 H), 6.94 (d, *J* = 7.8, 1 H), 6.53 (d, *J* = 8.3, 1 H), 5.53 (dd, *J* = 10.3, 3.5, 1 H), 3.42 (bs, 1 H), 2.94 (q, *J* = 7.0, 1 H), 2.65 (s, 1 H), 1.88-1.63 (m, 2 H), 1.53-1.22 (m, 10 H), 1.44 (d, *J* = 7.0, 3 H), 1.33 (s, 3 H), 1.08 (s, 3 H), 0.94 (t, J = 7.2, 3 H), 0.87 (t, J = 7.2, 3 H).

**EXAMPLE 297**

(*R*/*S-3l,4u,5u*)-5-(4-Chlorophenyl)-1,2,3,4-tetrahydro-3-methoxy-2,2,4-trimethyl-5*H*-chromeno[3,4-*f*]quinoline (Compound **397**, structure **26A** of **Scheme XXXVI**, where $R^1$=$R^2$=H, $R^3$=4-chlorophenyl, $R^4$=methyl)

To a solution of (*R*/*S-3l,4u,5u*)-5-(4-chlorophenyl)-1,2,3,4-tetrahydro-3-hydroxy-2,2,4-trimethyl-5*H*-chromeno [3,4-*f*]quinoline (structure 14A of Scheme **XXXIII**, where $R^1$=$R^2$=H, $R^3$=4-chlorophenyl, an intermediate from EXAMPLE 135) (8 mg, 0.016 mmol) in DMF (0.5 mL) and excess MeI (0.5 mL) was added 60% NaH in mineral oil (10 mg, 0.25 mmol). The resulting white slurry was stirred at rt for 2 h and was quenched with water (5 mL). The mixture was extracted with EtOAc (2 x 10 mL) and was concentrated to give the crude product, which was treated with TFA (0.2 mL) in $CH_2Cl_2$ (1 mL) for 60 min and was quenched with 5 % NaOH (5 mL). The mixture was extracted with EtOAc (2 x 10 mL), concentrated and was purified by silica gel chromatography to afford 5.0 mg (75%) of Compound **397** as a colorless oil. Data for Compound **397: $^1$H NMR** (400 MHz, $CDCl_3$) 7.53 (d, *J* = 7.7, 1 H), 7.48 (d, *J* = 8.4, 1 H), 7.12 (s, 4 H), 7.04 (t, *J* = 7.7, 1 H), 6.89 (t, *J* = 7.7, 1 H), 6.87 (d, *J* = 7.7, 1 H), 6.63 (d, *J* = 8.4, 1 H), 6.48 (s, 1 H), 3.74 (bs, 1 H), 3.26 (s, 3 H), 3.08 (d, *J* = 3.8, 1 H), 2.83 (qd, *J* = 7.3, 3.8, 1 H), 1.52 (d, *J* = 7.3,3 H), 1.35 (s, 3 H), 1.50 (s, 3 H).

**EXAMPLE 298**

(*R*/*S-3l*,*4u*,*5l*)-5-(4-Chlorophenyl)-1,2,3,4-tetrahydro-3-methoxy-2,2,4-trimethyl-5*H*-chromeno[3,4-*f*]quinoline (Compound **398**, structure **25A** of **Scheme XXXVI**, where $R^1$=$R^2$=H, $R^3$=4-chlorophenyl, $R^4$=methyl)

To a solution of (*R*/*S-3l*,*4u*,*5l*)-5-(4-chlorophenyl)-1,2,3,4-tetrahydro-3-hydroxy-2,2,4-trimethyl-5*H*-chromeno [3,4-*f*]quinoline (structure 13A of **Scheme XXXIII,** where $R^1$=$R^2$=H, $R^3$=4-chlorophenyl, an intermediate from EXAMPLE 135) (8 mg, 0.016 mmol) in DMF (0.5 mL) and excess MeI (0.5 mL) was added 60% NaH in mineral oil (10 mg, 0.25 mmol). The resulting white slurry was stirred at rt for 2 h and was quenched with water (5 mL). The mixture was extracted with EtOAc (2 x 10 mL) and was concentrated to give the crude product, which was treated with TFA (0.2 mL) in $CH_2Cl_2$ (1 mL) for 60 min and was quenched with 5 % NaOH (5 mL). The mixture was extracted with EtOAc (2 x 10 mL), concentrated and was purified by silica gel chromatography to afford 4.7.mg (70%) of Compound **398** as a

colorless oil. Data for Compound **398**: **$^1$H NMR** (400 MHz, $CDCl_3$) 7.55 (d, *J* = 7.7, 1 H), 7.47 (d, *J* = 8.4, 1 H), 7.16 (d, *J* = 8.7, 2 H), 7.12 (d, *J* = 8.7, 2 H), 6.97 (t, *J* = 7.7, 1 H), 6.89 (t, *J* = 7.7, 1 H), 6.73 (d, *J* = 7.7, 1 H), 6.68 (d, *J* = 8.4, 1 H), 6.34 (s, 1 H), 3.65 (bs, 1 H), 3.53 (s, 3 H), 3.03 (s, 1 H), 3.02 (qd, *J* = 7.0, 0.8, 1 H), 1.30 (s, 3 H), 1.11 (s, 3 H), 0.88 (d, J = 7.0, 3 H).

## EXAMPLE 299

(*R*/*S*-*3l*,*4u*,*5l*)-5-(4-Chlorophenyl)-1,2,3,4-tetrahydro-3-propyloxy-2,2,4-trimethyl-5*H*-chromeno[3,4-*f*]quinoline (Compound **399**, structure **25A** of **Scheme XXXVI**, where $R^1$=$R^2$=H, $R^3$=4-chlorophenyl, $R^4$=propyl)

To a solution of (*R*/*S*-*3l*,*4u*,*5l*)-5-(4-chlorophenyl)-1,2,3,4-tetrahydro-3-hydroxy-2,2,4-trimethyl-5*H*-chromeno [3,4-*f*]quinoline (structure 13A of **Scheme XXXIII**, where $R^1$=$R^2$=H, $R^3$=4-chlorophenyl, an intermediate from EXAMPLE 135) (7 mg, 0.014 mmol) in DMF (0.5 mL) and excess PrI (0.5 mL) was added 60% NaH in mineral oil (10 mg, 0.25 mmol). The resulting white slurry was stirred at rt for 2 h and was quenched with water (5 mL). The mixture was extracted with EtOAc (2 x 10 mL) and was concentrated to give the crude product, which was treated with TFA (0.2 mL) in $CH_2Cl_2$ (1 mL) for 60 min and was quenched with 5 % NaOH (5 mL). The mixture was extracted with EtOAc (2 x 10 mL), concentrated and was purified by silica gel chromatography to afford 2.5 mg (40%) of Compound **399** as a colorless oil. Data for Compound **399: $^1$H NMR** (400 MHz, $CDCl_3$) 7.57 (d, *J* = 7.7, 1 H), 7.48 (d, *J* = 8.4, 1 H), 7.16 (d, *J* = 8.6, 2 H), 7.12 (d, *J* = 8.6, 2 H), 6.99 (t, *J* = 7.7, 1 H), 6.89 (t, *J* = 7.7, 1 H), 6.73 (d, *J* = 7.7, 1 H), 6.68 (d, *J* = 8.4, 1 H), 6.33 (s, 1 H), 3.65 (bs, 1 H), 3.58 (m, 2 H), 3.11 (d, *J* = 5.8, 1 H), 3.00 (qd, *J* = 7.0, 5.8, 1 H), 1.65-1.50 (m, 2 H), 1.30 (s, 3 H), 1.10 (s, 3 H), 0.93 (t, *J* = 7.4, 3 H), 0.88 (d, *J* = 7.0, 3 H).

## EXAMPLE 300

(*R*/*S*-*3l,4u,5u*)-5-(4-Chlorophenyl)-1,2,3,4-tetrahydro-3-propyloxy-2,2,4-trimethyl-5*H*-chromeno[3,4-*f*]quinoline (Compound **400**, structure **26A** of **Scheme XXXVI**, where $R^1$=$R^2$=H, $R^3$=4-chlorophenyl, $R^4$=propyl)

To a solution of (*R*/*S*-*3l,4u,5u*)-5-(4-chlorophenyl)-1,2,3,4-tetrahydro-3-hydroxy-2,2,4-trimethyl-5*H*-chromeno [3,4-*f*]quinoline (structure 14A of Scheme **XXXIII**, where $R^1$=$R^2$=H, $R^3$=4-chlorophenyl, an intermediate from EXAMPLE 135) (8 mg, 0.016 mmol) in DMF (0.5 mL) and excess PrI (0.5 mL) was added 60% NaH in mineral oil (10 mg, 0.25 mmol). The resulting white slurry was stirred at rt for 2 h and was quenched with water (5 mL). The mixture was extracted with EtOAc (2 x 10 mL) and was concentrated to give the crude product, which was treated with TFA (0.2 mL) in $CH_2Cl_2$ (1 mL) for 60 min and was quenched with 5 % NaOH (5 mL). The mixture was extracted with EtOAc (2 x 10 mL), concentrated and was purified by silica gel chromatography to afford 2.5 mg (40%) of Compound **400** as a colorless oil. Data for Compound **400: $^1$H NMR** (400 MHz, $CDCl_3$) 7.54 (d, *J* = 7.7, 1 H), 7.48 (d, *J* = 8.4, 1 H), 7.12 (d, *J* = 8.6, 2 H), 7.09 (d, *J* = 8.6, 2 H), 7.02 (t, *J* = 7.7, 1 H), 6.89 (t, *J* = 7.7, 1 H), 6.87 (d, *J* = 7.7, 1 H), 6.62 (d, *J* = 8.4, 1 H), 6.48 (s, 1 H), 3.72 (bs, 1 H), 3.28 (m, 2 H), 3.16 (d, *J* = 4.0, 1 H), 2.78 (qd, *J* = 7.2, 4.0, 1 H), 1.51 (d, *J* = 7.2, 3 H), 1.51-1.36 (m, 2 H), 1.34 (s, 3 H), 1.14 (s, 3 H), 0.80 (t, *J* = 7.4, 3 H).

## EXAMPLE 304

(*R*/*S*)-1,2,3,4-Tetrahydro-1,2,2,4-tetramethyl-6-trifluoromethyl-8-pyranono[5,6-*g*]quinoline (Compound **404**, structure **28A** of **Scheme XXXVIII**, where $R^1$=$R^2$=$R^5$=H, $R^3$=trifluoromethyl, Z=O)

In a flame dried r.b. flask was dissolved Compound **250** (EXAMPLE 150) (50 mg, 161 μmol) in glacial acetic acid (10 mL). To the stirred solution was added *para*-formadehyde (51 mg, 1.61 mmol, 10 equiv). The cloudy yellow solution stirred for 10 min, then $NaCNBH_3$ (50 mg, 805 μmol, 5 equiv) was added at once. Upon addition the solution emitted gas for approx. 5 min then turned a brilliant fluorescent yellow/green. After stirring at rt under a blanket of $N_2$ for 20 h, the solution was slowly poured over ice and quenched with NaOH (20%), extracted with EtOAc (2 x 50 mL), washed with brine (2 x 25 mL), dried over $Na_2SO_4$ and concentrated *in vacuo* to give 51.3 mg (99 %) of Compound **404** as a yellow-green solid. Data for Compound **404:** $R_f$ = 0.39 (hexanes/EtOAc; 3:1). **$^1$H NMR** (400 MHz, $CDCl_3$) 7.31 (dd, *J* = 1.5, 1.5, 1 H), 6.44 (s, 1 H), 6.35 (s, 1 H), 2.90 (s, 3 H, N-C$\underline{H}_3$), 2.83 (m, partially obscured by Me, 1 H, C4-H), 1.84 (dd, *J* = 4.2, 13.3, 1 H, C3-H), 1.53 (dd, *J* = 13.0, 13.0, 1 H), 1.36 (d, *J* = 6.6, 3 H, C4-C$\underline{H}_3$), 1.35 (s, 3 H, C2-C$\underline{H}_3$), 1.29 (s, 3 H, C2-C$\underline{H}_3$).

**EXAMPLE 356**

(*R*/*S*)-9-Chloro-1,2-dihydro-2,2,4-trimethyl-5-phenyl-5*H*-chromeno[3,4-*f*]quinoline (Compound **456**, structure **42** of **Scheme XI**, where R=phenyl $R^1$=H, $R^2$=Cl)

This compound was prepared by General Method 5 (Example 60) from Compound **209** (75 mg, 0.230 mmol) and phenyl magnesium bromide (1.84 mL, 1.84 mmol) to afford 61 mg (68 %) of Compound **456** as a clear film. Data for Compound **456: $^1$H NMR** (400 MHz, acetone-$d_6$) 7.58 (d, *J* = 2.3, 1 H), 7.56 (s, 1 H), 7.22 (m, 4 H), 7.19 (m, 1 H), 6.94 (dd, *J* = 8.5, 2.5, 2 H), 6.83 (d, *J* = 8.5, 1 H), 6.76 (d, *J* = 8.5, 1 H), 5.63 (br s, 1 H), 5.46 (d, *J* = 8.5, 1 H), 1.98 (s, 3 H), 1.26 (s, 3 H), 1.24 (s, 3 H).

**EXAMPLE 357**

(*R*/*S*)-5-Butyl-1,2-dihydro-2,2,4,9-tetramethyl-5*H*-chromeno[3,4-*f*]quinoline (Compound **457**, structure **42** of **Scheme XI**, where R=*n*-butyl, $R^1$=H, $R^2$=methyl).

This compound was prepared by General Method 5 (EXAMPLE 60) from Compound **316** (EXAMPLE 216) (44 mg, 0.14 mmol) and *n*-BuLi (2.5 M in hexanes, 0.30 mL, 0.75 mmol, 5.2 equivuiv) to afford 12 mg (24%) of Compound **457** as a pale yellow glass. Data for Compound **457: $^1$H NMR** (400 MHz, acetone-$d_6$): 7.48 (s, 1 H), 7.45 (d, *J* = 8.2, 1 H), 6.91 (d, *J* = 6.6, 1 H), 6.76 (d, *J* = 8.0, 1 H), 6.67 (d, *J* = 8.2, 1H), 5.80 (dd, *J* = 7.9, 3.3, 1 H), 5.51 (s, 1 H), 5.36 (br s, 1 H), 2.81 (s, 3 H), 2.78 (s, 3 H), 1. 75 (m, 1 H), 1.55-1.35 (m, 3 H), 1.30-1.20 (m, 2 H), 1.27 (s, 3 H), 1.18 (s, 3 H), 0.84 (t, J = 7.3, 3 H).

**Steroid Receptor Activity**

Utilizing the "cis-trans" or "co-transfection" assay described by Evans et al., Science, 240:889-95 (May 13, 1988), the disclosure of which is herein incorporated by reference, the compounds of the present invention were tested and found to have strong, specific activity as both agonists, partial agonists and antagonists of PR, AR, ER, GR and MR. This assay is described in further detail in U.S. Patent Nos. 4,981,784 and 5,071,773, the disclosures of which are incorporated herein by reference.

The co-transfection assay provides a method for identifying functional agonists and partial agonists which mimic, or antagonists which inhibit, the effect of native hormones, and quantifying their activity for responsive IR proteins. In this regard, the co-transfection assay mimics an *in vivo* system in the laboratory. Importantly, activity in the co-transfection assay correlates very well with known *in vivo* activity, such that the co-transfection assay functions as a qualitative and quantitative predictor of a tested compounds *in vivo* pharmacology. See, e.g., T. Berger et al. 41 *J.* Steroid Biochem. Molec. Biol. 773 (1992), the disclosure of which is herein incorporated by reference.

In the co-transfection assay, a cloned cDNA for an IR (e.g., human PR, AR or GR) under the control of a constitutive promoter (e.g., the SV 40 promoter) is introduced by transfection (a procedure to induce cells to take up foreign genes) into a background cell substantially devoid of endogenous IRs. This introduced gene directs the recipient cells to make the IR protein of interest. A second gene is also introduced (co-transfected) into the same cells in conjunction with the IR gene. This second gene, comprising the cDNA for a reporter protein, such as firefly luciferase (LUC), controlled by an appropriate hormone responsive promoter containing a hormone response element (HRE). This reporter plasmid functions as a reporter for the transcription-modulating activity of the target IR. Thus, the reporter acts as a surrogate for the products (mRNA then protein) normally expressed by a gene under control of the target receptor and its native hormone.

The co-transfection assay can detect small molecule agonists or antagonists of target IRs. Exposing the transfected cells to an agonist ligand compound increases reporter activity in the transfected cells. This activity can be conveniently measured, e.g., by increasing luciferase production, which reflects compound-dependent, IR-mediated increases in reporter transcription. To detect antagonists, the co-transfection assay is carried out in the presence of a constant concentration of an agonist to the target IR (e.g., progesterone for PR) known to induce a defined reporter signal. Increasing concentrations of a suspected antagonist will decrease the reporter signal (e.g., luciferase production). The co-transfection assay is therefore useful to detect both agonists and antagonists of specific IRs. Furthermore, it determines not only whether a compound interacts with a particular IR, but whether this interaction mimics (agonizes) or blocks (antagonizes) the effects of the native regulatory molecules on target gene expression, as well as the specificity and strength of this interaction.

The activity of selected steroid receptor modulator compounds of the present invention were evaluated utilizing the co-transfection assay, and in standard IR binding assays, according to the following illustrative Examples.

**EXAMPLE 358**

**Co-transfection assay**

CV-1 cells (African green monkey kidney fribroblasts) were cultured in the presence of Dulbecco's Modified Eagle Medium (DMEM) supplemented with 10% charcoal resin-stripped fetal bovine serum then transferred to 96-well microtiter plates one day prior to transfection.

To determine PR agonist and antagonist activity of the compounds of the present invention, the CV-1 cells were transiently transfected by calcium phosphate coprecipitation according to the procedure of Berger et al., 41 *J. Steroid Biochem. Mol. Biol.*, 733 (1992) with the following plasmids: pSVhPR-B (5 ng/well), MTV-LUC reporter (100 ng/well), pRS-β-Gal (50 ng/well) and filler DNA (pGEM; 45 ng/well). The receptor plasmid, pSVhPR-B, contains the human PR-B under constitutive control of the SV-40 promoter, and is more fully described in E. Vegeto et al., "The mechanism of RU 486 antagonism is dependent on the conformation of the carboxy-terminal tail of the human progesterone receptor", 69 *Cell,* 703 (1992), the disclosure of which is herein incorporated by reference. Similarly, the AR, ER, GR and MR agonist and antagonist activity of the compounds of the present invention were determined according to the same procedure described herein, except that the plasmids pRShAR, pRShER, pRShGR and pRShMR were substituted for the plasmid pSVhPR-B described above. Each of these plasmids are more fully described in *J*.A. Simental et al., "Transcriptional activation and nuclear targeting signals of the human androgen receptor", 266 *J. Biol. Chem*., 510 (1991) (pRShAR), M.T. Tzukerman et al., "Human estrogen receptor transactivational capacity is determined by both cellular and promoter context and mediated by two functionally distinct intramolecular regions", 8 *Mol. Endocrinol*., 21 (1994) (pRShER), V. Giguere et al., "Functional domains of the human glucocorticoid receptor", 46 *Cell,* 645 (1986) (pRShGR), and *J*.L. Arriza et al., "Cloning of human mineralocorticoid receptor complementary DNA: structural and functional kinship with glucocorticoid receptor", 237 *Science,* 268 (1987) (pRShMR), the disclosures of which are herein incorporated by reference.

The reporter plasmid, MTV-LUC, contains the cDNA for firefly luciferase (LUC) under control of the mouse mammary tumor virus (MTV) long terminal repeat, a conditional promoter containing a progesterone response element. This plasmid is more fully described in Berger et al. supra. In addition, for ER agonist and antagonist determinations, the reporter plasmid MTV-ERE5-LUC, which contains LUC under control of the mouse mammary tumor virus (MTV) long terminal repeat in which the glucocorticoid response elements have been deleted and replaced with five copies of a 33-base pair ERE as described in Tzukerman et al., supra, was substituted for the MTV-LUC plasmid described herein. pRS-β-Gal, coding for constitutive expression of E. coli β-galactosidase (β-Gal), was included as an internal control for evaluation of transfection efficiency and compound toxicity.

Six hours after transfection, media was removed and the cells were washed with phosphate-buffered saline (PBS). Media containing reference compounds (i.e. progesterone as a PR agonist, mifepristone ((11beta,17beta)-11-[4-(dimethylamino)phenyl]-17-hydroxy-17-(1-propynyl)estra-4,9-dien-3-one: RU486; Roussel Uclaf) as a PR antagonist; dihydrotestosterone (DHT; Sigma Chemical) as an AR agonist and 2-OH-flutamide (the active metabolite of 2-methyl-N-[4-nitro-3-(trifluoromethyl)phenyl]pronanamide; Schering-Plough) as an AR antagonist; estradiol (Sigma) as an ER agonist and ICI 164,384 (N-butyl-3,17-dihydroxy-N-methyl-(7-alpha,17-beta)-estra-1,3,5(10)-triene-7-undecanamide; ICI Americas) as an ER antagonist; dexamethasone (Sigma) as a GR agonist and RU486 as a GR antagonist; and aldosterone (Sigma) as a MR agonist and spirolactone ((7-alpha-[acetylthio]-17-alpha-hydroxy-3-oxopregn-4-ene-21-carboxylic acid gamma-lactone; Sigma) as an MR antagonist) and/or the modulator compounds of the present invention in concentrations ranging from $10^{-12}$ to $10^{-5}$ M were added to the cells. Three to four replicates were used for each sample. Transfections and subsequivuent procedures were performed on a Biomek 1000 automated laboratory work station.

After 40 hours, the cells were washed with PBS, lysed with a Triton X-100-based buffer and assayed for LUC and β-Gal activities using a luminometer or spectrophotometer, respectively. For each replicate, the normalized response (NR) was calculated as:

LUC response/β-Gal rate where β-Gal rate = β-Gal•1x$10^{-5}$/β-Gal incubation time.

The mean and standard error of the mean (SEM) of the NR were calculated. Data was plotted as the response of the compound compared to the reference compounds over the range of the dose-response curve. For agonist experiments, the effective concentration that produced 50% of the maximum response ($EC_{50}$) was quantified. Agonist efficacy was a function (%) of LUC expression relative to the maximum LUC production by the reference agonist for PR, AR, ER, GR or MR. Antagonist activity was determined by testing the amount of LUC expression in the presence of a fixed amount of progesterone as a PR agonist, DHT as an AR agonist, estradiol as an ER agonist, dexamethasone as a GR agonist, or aldosterone as an MR agonist at the $EC_{50}$ concentration. The concentration of test compound that inhibited 50% of LUC expression induced by the reference agonist was quantified ($IC_{50}$). In addition, the efficacy of antagonists was determined as a function (%) of maximal inhibition.

## IR Binding assay

### PR and GR Binding:

In addition, the binding of the compounds of the present invention to the steroid receptors was also investigated according to the following methodology for PR and GR. PR and GR proteins were prepared from Baculovirus extracts by incorporating the appropriate cDNAs for human progesterone receptor A form (PR-A; P. Kastner et al., 9 *EMBO*, 1603 (1990), the disclosure of which is herein incorporated by reference) and human glucocorticoid receptor alpha (GRα) into appropriate baculovirus the expression plasmids as described in E.A. Allegretto et al., 268 *J. Biol. Chem.*, 26625 (1993); G. Srinivasan and B. Thompson, 4 *Mol. Endo.*, 209 (1990); and D.R. O'Reilly et al., In, "Baculovirus Expression Vectors", D.R. O'Reilly et al., eds., W.H. Freeman, New York, NY, pp. 139-179 (1992), the disclosures of which are herein incorporated by reference. Assay buffers consisted of the following: PR, 10% glycerol, 10 mM Tris, 1 mM EDTA, 12 mM monothioglycerol (MTG) and 1mM PMSF, pH = 7.5 @ 4°C; GR, 10% glycerol, 25 mM sodium phosphate, 10 mM KF, 2mM DTT, 0.25 mM CHAPS, and 20 mM sodium molybdate, pH = 7.5.

The PR and GR steroid receptor binding assays were performed in the same manner. The final assay volume was 500 µL for PR and 250 µL for GR, and contained ~5 µg of extract protein for PR and ~50 mg for GR, as well as 2-4 nM of the appropriate [$^3$H] steroid (e.g, [$^3$H] progesterone and [$^3$H] dexamethasone, respectively) and varying concentrations of competing ligand at concentrations that ranged from 0 - $10^{-5}$ M. Incubations were carried out at 4°C for 16 hours.

Non-specific binding was defined as that binding remaining in the presence of 500 nM of the appropriate unlabelled steroid. At the end of the incubation period, bound from free ligand were separated by either charcoal (PR) or hydroxylapatite (GR). The amount of bound tritiated hormone was determined by liquid scintillation counting of an aliquot (700 mL) of the supernatant fluid or the hydroxylapatite pellet.

For the saturation analyses, the difference between the total binding and the nonspecific binding, normalized by the β-Gal rate, was defined as specific binding. The specific binding was evaluated by Scatchard analysis to determine the $K_d$ for $^3$H-DHT. <u>See e.g.</u>, D. Rodbard, "Mathematics and statistics of ligand assays: an illustrated guide" <u>In:</u> *J.* Langon and *J.J.* Clapp, eds., *Ligand Assay*, Masson Publishing U.S.A., Inc., New York, pp. 45-99, (1981), the disclosure of which is herein incorporated by reference. For the competition studies, the data was plotted as the amount of $^3$H-DHT (% of control in the absence of test compound) remaining over the range of the dose-response curve for a given compound. The concentration of test compound that inhibited 50% of the amount of $^3$H-DHT bound in the absence of competing ligand was quantified ($IC_{50}$) after log-logit transformation. The $K_i$ values were determined by application of the Cheng-Prusoff equivuation to the $IC_{50}$ values, where:

$$K_i = \frac{IC_{50}}{(1+[^3\text{H-DHT}])/K_d \text{ for } ^3\text{H-DHT}}$$

To date, binding assays have not been performed utilizing ER or MR proteins.

After correcting for non-specific binding, $IC_{50}$ values were determined. The $IC_{50}$ value is defined as the concentration of competing ligand needed to reduce specific binding by 50%. The $IC_{50}$ value was determined graphically from a log-logit plot of the data. The $K_i$ values were determined by application of the Cheng-Prusoff equivuation to the $IC_{50}$ values, the labeled ligand concentration and the $K_d$ of the labeled ligand.

The agonist, antagonist and binding activity assay results of selected steroid receptor modulator compounds of present invention and the standard reference compounds on PR, ER, GR and MR, as well as the cross-reactivity of selected compounds on all of these receptors, are shown in Tables 1-5 below. Efficacy is reported as the percent maximal response observed for each compound relative to the reference agonist and antagonist compounds indicated above. Also reported in Tables 1-5 for each compound is its antagonist potency or $IC_{50}$ (which is the concentration (nM), requivuired to reduce the maximal response by 50%), its agonist potency or $EC_{50}$ (nM). PR, AR and GR protein binding activity ($K_i$ in nM) is shown in Tables 1-2 and 4.

Table 1:

Agonist, antagonist and binding activity of selected steroid receptor modulator compounds of present invention and the reference agonist compound, Progesterone (**Prog**), and reference antagonist compound, mifepristone (**RU486**), on PR.

| **Cmpd** | **PR Agonist CV-1 Cells** | | **PR Antagonist CV-1 Cells** | | **PR Binding** |
|---|---|---|---|---|---|
| **No.** | **Efficacy (%)** | **Potency (nM)** | **Efficacy (%)** | **Potency (nM)** | **$K_i$ (nM)** |
| **161** | 47 | 203 | 75 | 209 | 3 |
| **163** | 77 | 15 | 45 | 3,617 | 1 |
| **191** | 26 | 9 | 74 | 150 | 1 |
| **195** | 89 | 13 | na | na | 3 |
| **210** | 72 | 16 | na | na | 3 |
| **328** | 86 | 2200 | 65 | 26 | 7 |
| **331** | na | na | 88 | 210 | 273 |
| **332** | 138 | 3 | na | na | 0.4 |
| **379** | na | na | 87 | 350 | 25 |
| **Prog** | 100 | 4 | na | na | 3 |
| **RU486** | na | na | 96 | 0.1 | 0.8 |

**na = not active (i.e. efficacy of <20 and potency of >10,000)**
**nt = not tested**

Table 3 :

Agonist, antagonist and binding activity of selected steroid receptor modulator compounds of present invention and the reference agonist compound, Estrogen (**Estr**), and reference antagonist compound, ICI 164,384 **(ICI 164),** on ER.

| **Cmpd** | **ER Agonist CV-1 Cells** | | **ER Antagonist CV-1 Cells** | |
|---|---|---|---|---|
| **No.** | **Efficacy (%)** | **Potency (nM)** | **Efficacy (%)** | **Potency (nM)** |
| **161** | nt | nt | 86 | 505 |
| **170** | nt | nt | 78 | 580 |
| **191** | nt | nt | 93 | 330 |
| **192** | na | na | 80 | 195 |
| **194** | nt | nt | 94 | 390 |
| **195** | 90 | 1900 | 68 | 4406 |
| **Estr** | 100 | 7 | na | na |
| **ICI 164** | na | na | 99 | 43 |

**na = not active (i.e. efficacy of <20 and potency of >10,000)**
**nt = not tested**

Table 4:

| Antagonist and binding activity of selected steroid receptor modulator compounds of present invention and the reference antagonist compounds, RU486 and Spironolactone (**Spir**), on GR and MR, respectively. | | | | | |
|---|---|---|---|---|---|
| **Cmpd** | **GR Antagonist CV-1 Cells** | | **MR Antagonist CV-1 Cells** | | **GR Binding** |
| **No.** | **Efficacy (%)** | **Potency (nM)** | **Efficacy (%)** | **Potency (nM)** | **$K_i$ (nM)** |
| **161** | 97 | 600 | 58 | 1000 | 137 |
| **167** | 96 | 855 | 61 | 2000 | 21 |
| **170** | 94 | 1550 | 84 | 410 | 47 |
| **192** | 81 | 280 | 70 | 320 | 214 |
| **195** | 96 | 590 | 47 | 1900 | 26 |
| **RU486** | 100 | 1 | 77 | 1100 | 0.4 |
| **Spir** | 80 | 2000 | 96 | 25 | nt |
| **nt - not tested** | | | | | |

Table 5:

| Overall agonist and antagonist potency of selected steroid receptor modulator compounds of present invention and the reference agonist and antagonist compounds shown in Tables 1-4 on PR, AR, ER, GR and MR. | | | | | | | | |
|---|---|---|---|---|---|---|---|---|
| **Cmpd** | **PR Potency** | | **AR Potency** | | **ER Potency** | | **GR Potency** | **MR Potency** |
| **No.** | **Agon (nM)** | **Antag (nM)** | **Agon (nM)** | **Antag (nM)** | **Agon (nM)** | **Antag (nM)** | **Antag (nM)** | **Antag (nM)** |
| **163** | 15 | 3617 | nt | 1550 | na | 2150 | 1330 | 1450 |
| **170** | 73 | 145 | nt | 290 | nt | 580 | 1550 | 410 |
| **191** | 9 | 150 | nt | 520 | nt | 330 | nt | nt |
| **192** | na | 89 | nt | 79 | nt | 195 | 280 | 320 |
| **195** | 13 | na | nt | 470 | 1900 | 4406 | 590 | 1900 |
| **Prog** | 4 | na | 1300 | na | na | na | na | nt |
| **RU486** | na | 0.1 | na | 12 | na | 1500 | 0.7 | 1100 |
| **DHT** | na | 1800 | 6 | na | 1700 | na | na | nt |
| **Flut** | na | 1900 | na | 26 | na | na | na | na |
| **Estr** | nt | nt | na | na | 7 | na | na | nt |
| **ICI 164** | na | na | na | na | na | 160 | na | na |
| **Spir** | nt | 268 | nt | nt | na | na | 2000 | 25 |
| **na=not active (i.e., efficacy of <20 and potency of >10,000)**<br>**nt=not tested** | | | | | | | | |

As can be seen in the Tables, Compounds 163, 191 and 332 are highly selective PR agonists, while Compound 328 is a highly selective PR antagonist. Importantly, these PR antagonist Compounds show very little or no cross reactivity on GR, or any of the other tested steroid receptors. In contrast, the known PR antagonist, RU486, shows strong cross reactivity on both GR and AR, showing essentially equivual potency as both a PR and GR antagonist. Thus RU486 may not be generally useful for long-term, chronic administration due to this undesirable GR cross reactivity.

**EXAMPLE 359**

The effectiveness of selected compounds of the present invention as PR agonists was investigated in the well recognized uterine wet weight assay, as described in G.J. Marcus, "Mitosis in the rat uterus during the estrous cycle, early pregnancy and early pseudopregnancy", 10 *Biol. Reprod.,* 447 (1974), S. Sakamoto et al., "Effects of estrogen and progesterone on thymidine kinase activity in the immature rat uterus", 145 *Am. J. Obstet. Gynecol.,* 711 (1983), and C.W. Emmens and R.I. Dorfman, "Estrogens" (Ch. 2) and "Antiestrogens" (Ch. 3). in *Methods in Hormone Research,* ed. R.I. Dorfman, Academic Press, New York, New York, pp101-130 (1969), the disclosures of which are herein incorporated by reference. Four to five week old, ovariectomized, Sprague-Dawely rats (Harlan-Sprague-Dawely, Indianapolis, IN) were obtained 1 week after surgery and allowed to acclimate for an additional week after shipment. Compound **163**, Compound **210,** medroxyprogesterone acetate (MPA) (Sigma, St. Louis, MO) a synthetic progesterone agonist, and estrone (E1) (Sigma, St. Louis, MO) a synthetic estrogen agonist, were fully dissolved in purified sesame oil (Croda, Parsippany,NJ). Animals were randomized into treatment groups (4 rats/group) and administered Compound **163**, Compound **210**, or MPA (0.3, 1.0 or 3.0 mg/rat, 0.5 mL volumes, oral, once a day for three days in the presence of estrone (10 µg/day, subcutaneous). Additional control groups of rats were administered estrone or vehicle (i.e. sesame oil) alone. Animals were sacrificed on the fourth day of the experiment. Upon necropsy, uterine wet weights were obtained, and are reported in Table 6 below.

Table 6:

| Mean uterine wet weights in presence of estrone (10 µg), MPA, a recognized PR agonist, and Compounds 163 and 210 of the present invention. | | | | | |
|---|---|---|---|---|---|
| **Group** | **E1 (µg)** | **MPA (mg)** | **Cmpd 163 (mg)** | **Cmpd 210 (mg)** | **Mean Uterine Wet Weight (mg)** |
| **Control** | none | none | none | none | 45 |
| **1** | 10 | none | none | none | 205 |
| **2** | 10 | 0.3 | none | none | 140 |
| **3** | 10 | 1.0 | none | none | 130 |
| **4** | 10 | 3.0 | none | none | 130 |
| **5** | 10 | none | 3.0 | none | 125 |
| **6** | 10 | none | none | 0.3 | 110 |
| **7** | 10 | none | none | 1.0 | 100 |
| **8** | 10 | none | none | 3.0 | 100 |

As can be seen in Table 6, estrone alone increased uterine wet weight 4-fold over control treated animals. MPA co-administered with estrone significantly decreased the uterine wet weight at doses of 0.3 mg, 1.0 mg, and 3.0 mg/rat. Compound **163** at a dosage of 3 mg/rat, decreased by approximately half, the mean uterine wet weight, as did Compound **210** at doses of 0.3 mg, 1.0 mg, and 3.0 mg/rat.

**Pharmacological and Other Applications**

As will be discernible to those skilled in the art, the non-steroid modulator compounds of the present invention can be readily utilized in pharmacological applications where PR, ER, GR and/or MR antagonist or agonist activity is desired, and where it is desired to minimize cross reactivities with other steroid receptor related IRs. In vivo applications of the invention include administration of the disclosed compounds to mammalian subjects, and in particular to humans.

The following Example provides illustrative pharmaceutical composition formulations:

**EXAMPLE 362**

Hard gelatin capsules are prepared using the following ingredients:

| | **Quantity (mg/capsule)** |
|---|---|
| COMPOUND 191 | 140 |

(continued)

| | Quantity (mg/capsule) |
|---|---|
| Starch, dried | 100 |
| Magnesium stearate | 10 |
| Total | 250 mg |

The above ingredients are mixed and filled into hard gelatin capsules in 250 mg quantities.

A tablet is prepared using the ingredients below:

| | Quantity (mg/ tablet) |
|---|---|
| COMPOUND 191 | 140 |
| Cellulose, microcrystalline | 200 |
| Silicon dioxide, fumed | 10 |
| Stearic acid | 10 |
| Total | 360 mg |

The components are blended and compressed to form tablets each weighing 665 mg.

Tablets, each containing 60 mg of active ingredient, are made as follows:

| | Quantity (mg/ tablet) |
|---|---|
| COMPOUND 191 | 60 |
| Starch | 45 |
| Cellulose, microcrystalline | 35 |
| Polyvinylpyrrolidone (PVP) (as 10% solution in water) | 4 |
| Sodium carboxymethyl starch (SCMS) | 4.5 |
| Magnesium stearate | 0.5 |
| Talc | 1.0 |
| Total | 150 mg |

The active ingredient, starch, and cellulose are passed through a No. 45 mesh U.S. sieve and mixed thoroughly. The solution of PVP is mixed with the resultant powders, which are then passed through a No. 14 mesh U.S. sieve. The granules so produced are dried at 50° C and passed through a No. 18 mesh U.S. sieve. The SCMS, magnesium stearate, and talc, previously passed through a No. 60 mesh U.S. sieve, and then added to the granules which, after mixing, are compressed on a tablet machine to yield tablets each weighing 150 mg.

Suppositories, each containing 225 mg of active ingredient, may be made as follows:

| | |
|---|---|
| COMPOUND 191 | 225 mg |
| Saturated fatty acid glycerides | 2,000 mg |
| Total | 2,225 mg |

The active ingredient is passed through a No. 60 mesh U.S. sieve and suspended in the saturated fatty acid glycerides previously melted using the minimum heat necessary. The mixture is then poured into a suppository mold of normal 2g capacity and allowed to cool.

An intravenous formulation may be prepared as follows:

| | |
|---|---|
| COMPOUND 191 | 100 mg |
| Isotonic saline | 1,000 mL |
| Glycerol | 100 mL |

The compound is dissolved in the glycerol and then the solution is slowly diluted with isotonic saline. The

solution of the above ingredients is then administered intravenously at a rate of 1 mL per minute to a patient.

While in accordance with the patent statutes, description of the preferred embodiments and processing conditions have been provided, the scope of the invention is not to be limited thereto or thereby. Various modifications and alterations of the present invention will be apparent to those skilled in the art without departing from the scope and spirit of the present invention.

Consequently, for an understanding of the scope of the present invention, reference is made to the following claims.

## Claims

1. A compound having the formulae:

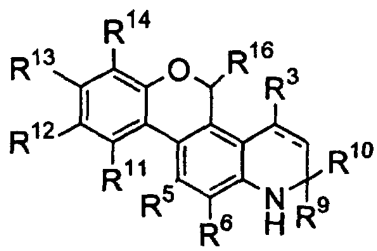

(V)

OR

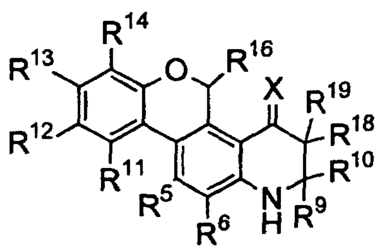

(VI)

wherein:

$R^3$ is hydrogen or methyl;
$R^5$ and $R^6$ are each hydrogen;
$R^9$ and $R^{10}$ are each methyl;
$R^{11}$ through $R^{14}$ each independently are hydrogen, F, Cl, Br, I, $COR^2$, $C_1$-$C_4$ alkyl, $CF_3$ or $OR^2$ where $R^2$ is hydrogen, $C_1$-$C_4$ alkyl or $CF_3$;
X is $CH_2$ or O;
$R^{16}$ is hydrogen, OH, $OR^{17}$, $SR^{17}$, $C_1$ - $C_6$ alkyl, $C_2$-$C_6$ alkenyl, $C_2$-$C_6$ alkynyl, phenylmethyl, phenyl optionally substituted with $C_1$-$C_4$ alkyl, F, Cl, Br, I, $CF_3$, $C_1$-$C_4$ alkoxyl, or dimethyl amino; pyridyl optionally substituted with methyl, F, Cl, Br, $CF_3$, or $C_1$-$C_4$ alkoxy; furanyl optionally substituted with $C_1$-$C_4$ alkyl or $C_1$-$C_4$ alkoxy; thienyl optionally substituted with $C_1$-$C_4$ alkyl or $C_1$-$C_4$ alkoxy; pyrrolyl optionally substituted with $C_1$-$C_4$ alkyl; 1-triazolyl; 2-benzofuranyl; or 2-benzothienyl; where $R^{17}$ is a $C_1$-$C_4$ alkyl or haloalkyl group;
$R^{18}$ and R19 are both hydrogen or both methyl; and
the dotted lines in the structures depict optional double bonds.

2. A progesterone receptor compound according to claim 1 selected from the group consisting of
(R/S)-5-Butyl-1,2-dihydro-2,2,4-trimethyl-5H-chromeno[3,4-f]quinoline (Compound 160);
(R/S)-1,2-Dihydro-2,2,4-trimethyl-5-phenyl-5H-chromeno[3,4-f]quinoline (Compound 161);
(R/S)-1,2,3,4-Tetrahydro-2,2-dimethyl-4-methylidene-5-phenyl-5H-chromeno[3,4-f]quinoline (Compound 162);
(R/S)-5-(4-Chlorophenyl)-1,2-dihydro-2,2,4-trimethyl-5H-chromeno[3,4-f]quinoline (Compound 163);

(R/S)-5-(4-Chlorophenyl)-1,2,3,4-tetrahydro-2,2-dimethyl-4-methylidene-5H-chromeno[3,4-f]quinoline (Compound 164);
(R/S)-5-(4-Fluorophenyl)-1,2-dihydro-2,2,4-trimethyl-5H-chromeno[3,4-f]quinoline (Compound 165);
(R/S)-5-(4-Acetylphenyl)-1,2-dihydro-2,2,4-trimethyl-5H-chromeno[3,4-f]quinoline (Compound 166);
(R/S)-1,2-Dihydro-2,2,4-trimethyl-5-(4-methylphenyl)-5H-chromeno[3,4-f]quinoline (Compound 167);
(R/S)-1,2-Dihydro-5-(4-methoxyphenyl)-2,2,4-trimethyl-5H-chromeno[3,4-f]quinoline (Compound 168);
(R/S)-1,2-Dihydro-2,2,4-trimethyl-5-[4-(trifluoromethyl)phenyl]-5H-chromeno[3,4-f]quinoline (Compound 169);
(R/S)-1,2-Dihydro-2,2,4-trimethyl-5-(thiophen-3-yl)-5H-chromeno[3,4-f]quinoline(Compound 170);
(-)-1,2-Dihydro-2,2,4-trimethyl-5-(4-methylphenyl)-5H-chromeno[3,4-f]quinoline (Compound 171);
(-)-5-(4-Chlorophenyl)-1,2-dihydro-2,2,4-trimethyl-5H-chromeno[3,4-f]quinoline (Compound 172);
(R/S)-1,2-Dihydro-2,2,4-trimethyl-5-(3-methylphenyl)-5H-chromeno[3,4-f]quinoline (Compound 173);
(+)-(4l,5l)-5-(4-Chlorophenyl)-1,2,3,4-tetrahydro-2,2,4-trimethyl-5H-chromeno[3,4-f]quinoline (Compound 174);
(-)-(4l,5l)-5-(4-Chlorophenyl)-1,2,3,4-tetrahydro-2,2,4-trimethyl-5H-chromeno[3,4-f]quinoline (Compound 175);
(R/S-4l,5u)-5-(4-Chlorophenyl)-1,2,3,4-tetrahydro-2,2,4-trimethyl-5H-chromeno[3,4-f]quinoline (Compound 176);
(R/S)-5-(3-Chlorophenyl)-1,2-dihydro-2,2,4-trimethyl-5H-chromeno[3,4-f]quinoline (Compound 177);
(R/S)-5-(3-Chlorophenyl)-1,2,3,4-tetrahydro-2,2-dimethyl-4-methylidene-5H-chromeno[3,4-f]quinoline (Compound 178);
(R/S)-5-(4-Bromophenyl)-1,2-dihydro-2,2,4-trimethyl-5H-chromeno[3,4-f]quinoline (Compound 179);
(R/S)-5-(4-Bromophenyl)-1,2,3,4-tetrahydro-2,2-dimethyl-4-methylidene-5H-chromeno[3,4-f]quinoline (Compound 180);
(R/S)-5-(3-Bromophenyl)-1,2-dihydro-2,2,4-trimethyl-5H-chromeno[3,4-f]quinoline (Compound 181);
(R/S)-5-(3-Bromophenyl)-1,2,3,4-tetrahydro-2,2-dimethyl-4-methylidene-5H-chromeno[3,4-f]quinoline (Compound 182);
(R/S)-5-(3,4-Dichlorophenyl)-1,2-dihydro-2,2,4-trimethyl-5H-chromeno[3,4-f]quinoline (Compound 183);
(R/S)-5-(3-Bromo-2-pyridyl)-1,2-dihydro-2,2,4-trimethyl-5H-chromeno[3,4-f]quinoline (Compound 184);
(R/S)-1,2-Dihydro-5-hydroxy-2,2,4-trimethyl-5H-chromeno[3,4-f]quinoline (Compound 185);
(R/S)-1,2-Dihydro-2,2,4-trimethyl-5-methoxy-5H-chromeno[3,4-f]quinoline (Compound 186);
(R/S)-1,2-Dihydro-2,2,4-trimethyl-5-propoxy-5H-chromeno[3,4-f]quinoline (Compound 187);
(R/S)-5-Allyl-1,2-dihydro-2,2,4-5H-chromeno[3,4-f]quinoline (Compound 188);
(R/S)-1,2-Dihydro-2,2,4-trimethyl-5-propyl-5H-chromeno[3,4-f]quinoline (Compound 189);
(R/S)-1,2-Dihydro-2,2,4-trimethyl-5-(2-pyridyl)-5H-chromeno[3,4-f]quinoline (Compound 190);
(R/S)-5-(3-Fluorophenyl)-1,2-dihydro-2,2,4-trimethyl-5H-chromeno[3,4-f]quinoline (Compound 191);
(R/S)-5-(3-Fluorophenyl)-1,2,3,4-tetrahydro-2,2-dimethyl-4-methylidene-5H-chromeno[3,4-f]quinoline (Compound 192);
(R/S)-1,2-Dihydro-2,2,4-trimethyl-5-propylthio-5H-chromeno[3,4-f]quinoline (Compound 193);
(R/S)-1,2-Dihydro-5-(3-methoxyphenyl)-2,2,4-trimethyl-5H-chromeno[3,4-f]quinoline (Compound 194);
(R/S)-1,2-Dihydro-2,2,4-trimethyl-5-[3-(trifluoromethyl)phenyl]-5H-chromeno[3,4-f]quinoline (Compound 195);
(R/S)-5-(3-Fluoro-4-methylphenyl)-1,2-dihydro-2,2,4-trimethyl-5H-chromeno[3,4-f]quinoline (Compound 196);
(R/S)-5-(4-Bromo-3-pyridyl)-1,2-dihydro-2,2,4-trimethyl-5H-chromeno[3,4-f]quinoline (Compound 197);
(R/S)-1,2-Dihydro-2,2,4-trimethyl-5-(3-pyridyl)-5H-chromeno[3,4-f]quinoline (Compound 198);
(R/S)-5-(4-Chloro-3-fluorophenyl)-1,2-dihydro-2,2,4-trimethyl-5H-chromeno[3,4-f]quinoline (Compound 199);
(R/S)-1,2-Dihydro-2,2,4,5-tetramethyl-5H-chromeno[3,4-f]quinoline (Compound 200);
(R/S)-1,2-Dihydro-5-hexyl-2,2,4-trimethyl-5H-chromeno[3,4-f]quinoline (Compound 201);
1,2-Dihydro-2,2,4-trimethyl-5H-chromeno[3,4-f]quinoline (Compound 202) ;
(R/S)-1,2-Dihydro-5-(3-methylbutyl)-2,2,4-trimethyl-5H-chromeno[3,4-f]quinoline (Compound 203);
(R/S)-5-(4-Chlorobutyl)-1,2-dihydro-2,2,4-trimethyl-5H-chromeno[3,4-f]quinoline (Compound 204);
(R/S)-5-Benzyl-1,2-dihydro-2,2,4-trimethyl-5H-chromeno[3,4-f]quinoline (Compound 205);
(R/S)-5-(4-Bromobutyl)-1,2-dihydro-2,2,4-trimethyl-5H-chromeno[3,4-f]quinoline (Compound 206);
(R/S)-5-Butyl-9-fluoro-1,2-dihydro-2,2,4-trimethyl-5H-chromeno[3,4-f]quinoline (Compound 210);
(R/S)-5-Butyl-8-fluoro-1,2-dihydro-2,2,4-trimethyl-5H-chromeno[3,4-f]quinoline (Compound 211);
(R/S)-5-(3-Chlorophenyl)-9-fluoro-1,2-dihydro-2,2,4-trimethyl-5H-chromeno[3,4-f]quinoline (Compound 212);
(R/S)-5-(4-Chloro-3-methylphenyl)-9-fluoro-1,2-dihydro-2,2,4-trimethyl-5H-chromeno[3,4-f]quinoline (Compound 213);
(R/S)-5-(4-Chlorophenyl)-9-fluoro-1,2-dihydro-2,2,4-trimethyl-5H-chromeno[3,4-f]quinoline (Compound 214);
(R/S)-9-Fluoro-1,2-dihydro-5-(4-methoxyphenyl)-2,2,4-trimethyl-5H-chromeno[3,4-f]quinoline (Compound 215);
(R/S)-8-Fluoro-1,2-dihydro-5-methoxyl-2,2,4-trimethyl-5H-chromeno[3,4-f]quinoline (Compound 216);
(R/S)-5-(4-Chlorophenyl)-8-fluoro-1,2-dihydro-2,2,4-trimethyl-5H-chromeno[3,4-f]quinoline (Compound 217);
(R/S)-9-Chloro-5-(4-chlorophenyl)-1,2-dihydro-2,2,4-trimethyl-5H-chromeno[3,4-f]quinoline (Compound 218);

(R/S)-5-(3-Fluorobenzyl)-1,2-dihydro-2,2,4-trimethyl-5H-chromeno[3,4-f]quinoline (Compound 318);
(R/S)-9-Chloro-1,2-dihydro-5-methoxy-2,2,4-trimethyl-5H-chromeno[3,4-f]quinoline (Compound 319);
9-Chloro-1,2-dihydro-2,2,4-trimethyl-5H-chromeno[3,4-f]quinoline (Compound 320);
(R/S)-9-Chloro-1,2-dihydro-2,2,4-trimethyl-5-propyloxy-5H-chromeno[3,4-f]quinoline (Compound 321);
(R/S)-9-Fluoro-1,2-dihydro-5-methoxy-2,2,4-trimethyl-5H-chromeno[3,4-f]quinoline (Compound 322);
(R/S)-9-Fluoro-1,2-dihydro-2,2,4-trimethyl-5-thiopropoxy-5H-chromeno[3,4-f]quinoline (Compound 323);
(R/S)-9-Fluoro-1,2-dihydro-2,2,4-trimethyl-5-propoxy-5H-chromeno[3,4-f]quinoline (Compound 324);
(R/S)-5-Butyl-9-chloro-1,2-dihydro-2,2,4-trimethyl-5H-chromeno[3,4-f]quinoline (Compound 325);
(R/S)-5-Butyl-1,2-dihydro-9-methoxy-2,2,4-trimethyl-5H-chromeno[3,4-f]quinoline (Compound 326);
(R/S)-9-Fluoro-1,2-dihydro-2,2,4,5-tetramethyl-5H-chromeno[3,4-f]quinoline (Compound 327);
9-Fluoro-1,2-dihydro-2,2,4-trimethyl-5H-chromeno[3,4-f]quinoline (Compound 328);
1,2-Dihydro-9-methoxy-2,2,4-trimethyl-5H-chromeno[3,4-f]quinoline (Compound 329);
1,2-Dihydro-2,2,4,9-tetramethyl-5H-chromeno[3,4-f]quinoline (Compound 330);
7-Chloro-1,2-dihydro-2,2,4-trimethyl-5H-chromeno[3,4-f]quinoline (Compound 331);
(R/S)-9-Chloro-1,2-dihydro-2,2,4,5-tetramethyl-5H-chromeno[3,4-f]quinoline (Compound 332);
(R/S)-5-(4-Bromophenyl)-9-chloro-1,2-dihydro-2,2,4-trimethyl-5H-chromeno[3,4-f]quinoline (Compound 333);
(R/S)-9-Chloro-5-(3-chlorophenyl)-1,2-dihydro-2,2,4-trimethyl-5H-chromeno[3,4-f]quinoline (Compound 334);
(R/S)-9-Chloro-1,2-dihydro-2,2,4-trimethyl-5-(3-methylphenyl)-5H-chromeno[3,4-f]quinoline (Compound 335);
(R/S)-9-Chloro-5-(4-chloro-3-methylphenyl)-1,2-dihydro-2,2,4-trimethyl-5H-chromeno[3,4-f]quinoline (Compound 336);
(R/S)-9-Chloro-1,2-dihydro-5-[3-(trifluaromethyl)phenyl]-2,2,4-trimethyl-5H-chromeno[3,4-f]quinoline (Compound 337);
(R/S)-9-Chloro-5-(3,5-dichlorophenyl)-1,2-dihydro-2,2,4-trimethyl-5H-chromeno[3,4-f]quinoline (Compound 338);
(R/S)-9-Chloro-1,2-dihydro-5-(4-methoxyphenyl)-2,2,4-trimethyl-5H-chromeno[3,4-f]quinoline (Compound 339);
(R/S)-9-Chloro-5-(3-fluoro-4-methoxyphenyl)-1,2-dihydro-2,2,4-trimethyl-5H-chromeno[3,4-f]quinoline (Compound 340);
(R/S)-9-Chloro-5-(4-fluorophenyl)-1,2-dihydro-2,2,4-trimethyl-5H-chromeno[3,4-f]quinoline (Compound 341);
(R/S)-9-Chloro-5-(3-Chloro-4-methoxy-5-methylphenyl)-1,2-dihydro-2,2,4-trimethyl-5H-chromeno[3,4-f]quinoline (Compound 342);
(R/S)-9-Chloro-5-(4-fluoro-3-methylphenyl)-1,2-dihydro-2,2,4-trimethyl-5H-chromeno[3,4-f]quinoline (Compound 343);
(R/S)-9-Chloro-5-(3-fluorophenyl)-1,2-dihydro-2,2,4-trimethyl-5H-chromeno[3,4-f]quinoline (Compound 344);
(R/S)-1,2-Dihydro-2,2,4-trimethyl-5-[3,4-methylenedioxy)phenyl]-5H-chromeno[3,4-f]quinoline (Compound 345);
(R/S)-5-(4-Chloro-3- methylphenyl)-1,2-dihydro-2,2,4-trimethyl-5H-chromeno[3,4-f]quinoline (Compound 346);
(R/S)-5-(4-Bromo-3-pyridyl)-1,2,3,4-tetrahydro-2,2-dimethyl-4-methylidene-5H-chromeno[3,4-f]quinoline (Compound 347);
(R/S)-5-(3,5-Difluorophenyl)-1,2-dihydro-2,2,4-trimethyl-5H-chromeno[3,4-f]quinoline (Compound 348);
(R/S)-5-(3,5-Dichlorophenyl)-1,2-dihydro-2,2,4-trimethyl-5H-chromeno[3,4-f]quinoline (Compound 349);
(R/S)-5-(3-Bromo-5- methylphenyl)-1,2-dihydro-2,2,4-trimethyl-5H-chromeno[3,4-f]quinoline (Compound 350);
(R/S)-5-(3-Bromo-5- fluorophenyl)-1,2-dihydro-2,2,4-trimethyl-5H-chromeno[3,4-f]quinoline (Compound 351);
(R/S)-5-(3-Bromo-5-fluorophenyl)-1,2,3,4-tetrahydro-2,2-dimethyl-4-methylidene-5H-chromeno[3,4-f]quinoline (Compound 352);
(R/S)-5-[4-Fluoro-3-(trifluoromethyl)phenyl)-1,2-dihydro-2,2,4-trimethyl-5H-chromeno[3,4-f]quinoline (Compound 353);
(R/S)-9-Fluoro-1,2-dihydro-2,2,4-trimethyl-5-(3-methylphenyl)-5H-chromeno[3,4-f]quinoline (Compound 354);
(R/S)-1,2-Dihydro-9-methoxy-2,2,4-trimethyl-5-(3-methylphenyl)-5H-chromeno[3,4-f]quinoline (Compound 355);
(R/S)-9-Fluoro-5(3-fluoro-4-methoxyphenyl)-1,2-dihydro-2,2,4-trimethyl-5H-chromeno[3,4-f]quinoline (Compound 356);
(R/S)-9-Fluoro-1,2-dihydro-2,2,4-trimethyl-5-[3-(trifluoromethyl)phenyl]-5H-chromeno[3,4-f]quinoline (Compound 357);
(R/S)-9-Fluoro-5-(4-fluoro-3-methylphenyl)-1,2-dihydro-2,2,4-trimethyl-5H-chromeno[3,4-f]quinoline (Compound 358);
(R/S)-5-(4-Chlorophenyl)-1,2,3,4-tetrahydro-2,2-dimethyl-5H-chromeno[3,4-f]-4-quinolinone (Compound 378);
(R/S)-5-(4-Chlorophenyl)-1,2,3,4-tetrahydro-2,2,3,3-tetramethyl-5H-chromeno[3,4-f]-4-quinolinone (Compound 379);
(R/S)-5-(4-Chloraphenyl)-1,2-dihydro-2,2-dimethyl-5H-chromeno[3,4-f]-4-quinoline (Compound 380); and
(R/S)-9-Chloro-1,2-dihydro-2,2,4-trimethyl-5-phenyl-5H-chromeno[3,4-f]quinoline (Compound 456).

**3.** A pharmaceutical composition comprising a compound of claim 1 and a pharmaceutically acceptable carrier.

**4.** A pharmaceutical composition according to claim 3, wherein the composition is formulated for oral, topical, intravenous, suppository or parental administration.

**5.** A pharmaceutical composition according to claim 3, wherein the compound is administered to a patient as a dosage unit at from about 1µg/kg of body weight to 500 mg/kg of body weight.

**6.** A pharmaceutical composition according to claim 3, wherein the compound is administered to a patient as a dosage unit at from about 10µg/kg of body weight to 250 mg/kg of body weight.

**7.** A pharmaceutical composition according to claim 3, wherein the compound is administered to a patient as a dosage unit at from about 20µg/kg of body weight to 100 mg/kg of body weight.

**8.** Use of a compound according to claim 1 or 2 for the preparation of a medicament which is effective in treating and/or modulating human fertility, female hormone replacement, dysfunctional uterine bleeding, endometriosis, leiomyomas, acne, male-pattern baldness, osteoporosis, prostatic hyperplasia, cancer of the breast, cancer of the ovaries, endometrial cancer, prostate cancer, carbohydrate, protein and lipid metabolism, electrolyte and water balance, and functioning of the cardiovascular, kidney, central nervous, immune and skeletal muscle systems.

**9.** Use according to claim 8 wherein the medicament comprises a pharmaceutically acceptable carrier.

**10.** The use of a compound in accordance with claim 1 in a method for determining the presence of one or more steroid receptors in a sample comprising combining a compound according to claim 1 with the sample containing one or more unknown steroid receptors and determining whether said compound binds to a receptor in the sample.

**11.** The use of a compound in accordance with claim 1 in a method of purifying steroid receptors comprising combining a compound according to claim 1 with a sample containing steroid receptors, allowing said compound to bind said steroid receptors, and separating out the bound combination of said compound and said steroid receptors.

## Patentansprüche

**1.** Verbindung mit der Formel:

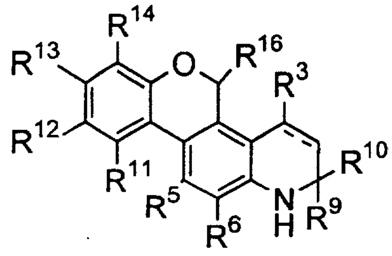

(V)

oder

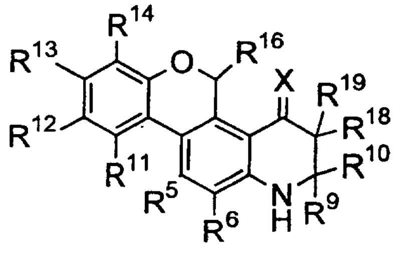

(VI)

wobei:

$R^3$ gleich Wasserstoff oder Methyl ist;
$R^5$ und $R^6$ jeweils Wasserstoff sind;
$R^9$ und $R^{10}$ jeweils Methyl sind;
$R^{11}$ bis $R^{14}$ jeweils unabhängig Wasserstoff, F, Cl, Br, I, $COR^2$, $C_1$-$C_4$-Alkyl, $CF_3$ oder $OR^2$ sind, wobei $R^2$ gleich Wasserstoff, $C_1$-$C_4$-Alkyl oder $CF_3$ ist;
X gleich $CH_2$ oder O ist;
$R^{16}$ gleich Wasserstoff, OH, $OR^{17}$, $SR^{17}$, $C_1$-$C_6$-Alkyl, $C_2$-$C_6$-Alkenyl, $C_2$-$C_6$-Alkinyl, Phenylmethyl, Phenyl, das gegebenenfalls substituiert ist mit $C_1$-$C_4$-Alkyl, F, Cl, Br, I, $CF_3$, $C_1$-$C_4$-Alkoxyl oder Dimethylamino; Pyridyl, das gegebenenfalls substituiert ist mit Methyl, F, Cl, Br, $CF_3$ oder $C_1$-$C_4$-Alkoxy; Furanyl, das gegebenenfalls substituiert ist mit $C_1$-$C_4$-Alkyl oder $C_1$-$C_4$-Alkoxy; Thienyl, das gegebenenfalls substituiert ist mit $C_1$-$C_4$-Alkyl oder $C_1$-$C_4$-Alkoxy; Pyrrolyl, das gegebenenfalls substituiert ist mit $C_1$-$C_4$-Alkyl; 1-Triazolyl; 2-Benzofuranyl; oder 2-Benzothienyl ist; wobei $R^{17}$ eine $C_1$-$C_4$-Alkyl- oder Halogenalkylgruppe ist;
$R^{18}$ und $R^{19}$ beide Wasserstoff oder beide Methyl sind; und
die gepunkteten Linien in den Strukturen optionale Doppelbindungen darstellen.

**2.** Progesteronrezeptorverbindung nach Anspruch 1, die aus der Gruppe ausgewählt ist, bestehend aus:

(R/S)-5-Butyl-1,2-dihydro-2,2,4-trimethyl-5H-chromeno[3,4-f]chinolin (Verbindung 160);

(R/S)-1,2-Dihydro-2,2,4-trimethyl-5-phenyl-5H-chromeno[3,4-f]chinolin (Verbindung 161);

(R/S)-1,2,3,4-Tetrahydro-2,2-dimethyl-4-methyliden-5-phenyl-5H-chromeno[3,4-f]chinolin (Verbindung 162);

(R/S)-5-(4-Chlorphenyl)-1,2-dihydro-2,2,4-trimethyl-5H-chromeno[3,4-f]chinolin (Verbindung 163);

(R/S)-5-(4-Chlorphenyl)-1,2,3,4-tetrahydro-2,2-dimethyl-4-methyliden-5H-chromeno[3,4-f]chinolin (Verbindung 164);

(R/S)-5-(4-Fluorphenyl)-1,2-dihydro-2,2,4-trimethyl-5H-chromeno[3,4-f]chinolin (Verbindung 165);

(R/S)-5-(4-Acetylphenyl)-1,2-dihydro-2,2,4-trimethyl-5H-chromeno[3,4-f]chinolin (Verbindung 166);

(R/S)-1,2-Dihydro-2,2,4-trimethyl-5-(4-methylphenyl)-5H-chromeno[3,4-f]chinolin (Verbindung 167);

(R/S)-1,2-Dihydro-5-(4-methoxyphenyl)-2,2,4-trimethyl-5H-chromeno[3,4-f]chinolin (Verbindung 168);

(R/S)-1,2-Dihydro-2,2,4-trimethyl-5-[4-(trifluormethyl)phenyl]-5H-chromeno[3,4-f]chinolin (Verbindung 169) ;

(R/S)-1,2-Dihydro-2,2,4-trimethyl-5-(thiophen-3-yl)-5H-chromeno[3,4-f]chinolin(Verbindung 170);

(-)-1,2-Dihydro-2,2,4-trimethyl-5-(4-methylphenyl)-5H-chromeno[3,4-f]chinolin (Verbindung 171);

(-)-5-(4-Chlorphenyl)-1,2-dihydro-2,2,4-trimethyl-5H-chromeno[3,4-f]chinolin (Verbindung 172);

(R/S)-1,2-Dihydro-2,2,4-trimethyl-5-(3-methylphenyl)-5H-chromeno[3,4-f]chinolin (Verbindung 173);

(+)-(4l,5l)-5-(4-Chlorphenyl)-1,2,3,4-tetrahydro-2,2,4-trimethyl-5H-chromeno[3,4-f]chinolin (Verbindung 174);

(-)-(4l,5l)-5-(4-Chlorphenyl)-1,2,3,4-tetrahydro-2,2,4-trimethyl-5H-chromeno[3,4-f]chinolin (Verbindung 175);

(R/S-4l,5u)-5-(4-Chlorphenyl)-1,2,3,4-tetrahydro-2,2,4-trimethyl-5H-chromeno[3,4-f]chinolin (Verbindung 176);

(R/S)-5-(3-Chlorphenyl)-1,2-dihydro-2,2,4-trimethyl-5H-chromeno[3,4-f]chinolin (Verbindung 177);

(R/S)-5-(3-Chlorphenyl)-1,2,3,4-tetrahydro-2,2-dimethyl-4-methyliden-5H-chromeno[3,4-f]chinolin (Verbindung 178);

(R/S)-5-(4-Bromphenyl)-1,2-dihydro-2,2,4-trimethyl-5H-chromeno[3,4-f]chinolin (Verbindung 179);

(R/S)-5-(4-Bromphenyl)-1,2,3,4-tetrahydro-2,2-dimethyl-4-methyliden-5H-chromeno[3,4-f]chinolin (Verbindung 180);

(R/S)-5-(3-Bromphenyl)-1,2-dihydro-2,2,4-trimethyl-5H-chromeno[3,4-f]chinolin (Verbindung 181);

(R/S)-5-(3-Bromphenyl)-1,2,3,4-tetrahydro-2,2-dimethyl-4-methyliden-5H-chromeno[3,4-f]chinolin (Verbindung 182);

(R/S)-5-(3,4-Dichlorphenyl)-1,2-dihydro-2,2,4-trimethyl-5H-chromeno[3,4-f]chinolin (Verbindung 183);

(R/S)-5-(3-Brom-2-pyridyl)-1,2-dihydro-2,2,4-trimethyl-5H-chromeno[3,4-f]chinolin (Verbindung 184);

(R/S)-1,2-Dihydro-5-hydroxy-2,2,4-trimethyl-5H-chromeno[3,4-f]chinolin (Verbindung 185);

(R/S)-1,2-Dihydro-2,2,4-trimethyl-5-methoxy-5H-chromeno[3,4-f]chinolin (Verbindung 186);

(R/S)-1,2-Dihydro-2,2,4-trimethyl-5-propoxy-5H-chromeno[3,4-f]chinolin (Verbindung 187);

(R/S)-5-Allyl-1,2-dihydro-2,2,4-5H-chromeno[3,4-f]-chinolin (Verbindung 188);

(R/S)-1,2-Dihydro-2,2,4-trimethyl-5-propyl-5H-chromeno[3,4-f]chinolin (Verbindung 189);

(R/S)-1,2-Dihydro-2,2,4-trimethyl-5-(2-pyridyl)-5H-chromeno[3,4-f]chinolin (Verbindung 190);

(R/S)-5-(3-Fluorphenyl)-1,2-dihydro-2,2,4-trimethyl-5H-chromeno[3,4-f]chinolin (Verbindung 191);

(R/S)-5-(3-Fluorphenyl)-1,2,3,4-tetrahydro-2,2-dimethyl-4-methyliden-5H-chromeno[3,4-f]chinolin (Verbindung 192);

(R/S)-1,2-Dihydro-2,2,4-trimethyl-5-propylthio-5H-chromeno[3,4-f]chinolin (Verbindung 193);

(R/S)-1,2-Dihydro-5-(3-methoxyphenyl)-2,2,4-trimethyl-5H-chromeno[3,4-f]chinolin (Verbindung 194);

(R/S)-1,2-Dihydro-2,2,4-trimethyl-5-[3-(trifluormethyl)phenyl]-5H-chromeno[3,4-f]chinolin (Verbindung 195) ;

(R/S)-5-(3-Fluor-4-methylphenyl)-1,2-dihydro-2,2,4-trimethyl-5H-chromeno[3,4-f]chinolin (Verbindung 196);

(R/S)-5-(4-Brom-3-pyridyl)-1,2-dihydro-2,2,4-trimethyl-5H-chromeno[3,4-f]chinolin (Verbindung 197);

(R/S)-1,2-Dihydro-2,2,4-trimethyl-5-(3-pyridyl)-5H-chromeno[3,4-f]chinolin (Verbindung 198);

(R/S)-5-(4-Chlor-3-fluorphenyl)-1,2-dihydro-2,2,4-trimethyl-5H-chromeno[3,4-f]chinolin (Verbindung 199);

(R/S)-1,2-Dihydro-2,2,4,5-tetramethyl-5H-chromeno[3,4-f]chinolin (Verbindung 200);

(R/S)-1,2-Dihydro-5-hexyl-2,2,4-trimethyl-5H-chromeno[3,4-f]chinolin (Verbindung 201);

1,2-Dihydro-2,2,4-trimethyl-5H-chromeno[3,4-f]-chinolin (Verbindung 202);

(R/S)-1,2-Dihydro-5-(3-methylbutyl)-2,2,4-trimethyl-5H-chromeno[3,4-f]chinolin (Verbindung 203);

(R/S)-5-(4-Chlorbutyl)-1,2-dihydro-2,2,4-trimethyl-5H-chromeno[3,4-f]chinolin (Verbindung 204);

(R/S)-5-Benzyl-1,2-dihydro-2,2,4-trimethyl-5H-chromeno[3,4-f]chinolin (Verbindung 205);

(R/S)-5-(4-Brombutyl)-1,2-dihydro-2,2,4-trimethyl-5H-chromeno[3,4-f]chinolin (Verbindung 206);

(R/S)-5-Butyl-9-fluor-1,2-dihydro-2,2,4-trimethyl-5H-chromeno[3,4-f]chinolin (Verbindung 210);

(R/S)-5-Butyl-8-fluor-1,2-dihydro-2,2,4-trimethyl-5H-chromeno[3,4-f]chinolin (Verbindung 211);

(R/S)-5-(3-Chlorphenyl)-9-fluor-1,2-dihydro-2,2,4-trimethyl-5H-chromeno[3,4-f]chinolin (Verbindung 212) ;

(R/S)-5-(4-Chlor-3-methylphenyl)-9-fluor-1,2-dihydro-2,2,4-trimethyl-5H-chromeno[3,4-f]chinolin (Verbindung 213);

(R/S)-5-(4-Chlorphenyl)-9-fluor-1,2-dihydro-2,2,4-trimethyl-5H-chromeno[3,4-f]chinolin (Verbindung 214) ;

(R/S)-9-Fluor-1,2-dihydro-5-(4-methoxyphenyl)-2,2,4-trimethyl-5H-chromeno[3,4-f]chinolin (Verbindung 215);

(R/S)-8-Fluor-1,2-dihydro-5-methoxyl-2,2,4-trimethyl-5H-chromeno[3,4-f]chinolin (Verbindung 216);

(R/S)-5-(4-Chlorphenyl)-8-fluor-1,2-dihydro-2,2,4-trimethyl-5H-chromeno[3,4-f]chinolin (Verbindung 217);

(R/S)-9-Chlor-5-(4-chlorphenyl)-1,2-dihydro-2,2,4-trimethyl-5H-chromeno[3,4-f]chinolin (Verbindung 218);

(R/S)-5-(3-Fluorbenzyl)-1,2-dihydro-2,2,4-trimethyl-5H-chromeno[3,4-f]chinolin (Verbindung 318);

(R/S)-9-Chlor-1,2-dihydro-5-methoxy-2,2,4-trimethyl-5H-chromeno[3,4-f]chinolin (Verbindung 319);

9-Chlor-1,2-dihydro-2,2,4-trimethyl-5H-chromeno[3,4-f]chinolin (Verbindung 320);

(R/S)-9-Chlor-1,2-dihydro-2,2,4-trimethyl-5-propyloxy-5H-chromeno[3,4-f]chinolin (Verbindung 321);

(R/S)-9-Fluor-1,2-dihydro-5-methoxy-2,2,4-trimethyl-5H-chromeno[3,4-f]chinolin (Verbindung 322);

(R/S)-9-Fluor-1,2-dihydro-2,2,4-trimethyl-5-thiopropoxy-5H-chromeno[3,4-f]chinolin (Verbindung 323) ;

(R/S)-9-Fluor-1,2-dihydro-2,2,4-trimethyl-5-propoxy-5H-chromeno[3,4-f]chinolin (Verbindung 324);

(R/S)-5-Butyl-9-chlor-1,2-dihydro-2,2,4-trimethyl-5H-chromeno[3,4-f]chinolin (Verbindung 325);

(R/S)-5-Butyl-1,2-dihydro-9-methoxy-2,2,4-trimethyl-5H-chromeno[3,4-f]chinolin (Verbindung 326);

(R/S)-9-Fluor-1,2-dihydro-2,2,4,5-tetramethyl-5H-chromeno[3,4-f]chinolin (Verbindung 327);

9-Fluor-1,2-dihydro-2,2,4-trimethyl-5H-chromeno[3,4-f]chinolin (Verbindung 328);

1,2-Dihydro-9-methoxy-2,2,4-trimethyl-5H-chromeno[3,4-f]chinolin (Verbindung 329);

1,2-Dihydro-2,2,4,9-tetramethyl-5H-chromeno[3,4-f]-chinolin (Verbindung 330);

7-Chlor-1,2-dihydro-2,2,4-trimethyl-5H-chromeno-[3,4-f]chinolin (Verbindung 331);

(R/S)-9-Chlor-1,2-dihydro-2,2,4,5-tetramethyl-5H-chromeno[3,4-f]chinolin (Verbindung 332);

(R/S)-5-(4-Bromphenyl)-9-chlor-1,2-dihydro-2,2,4-trimethyl-5H-chromeno[3,4-f]chinolin (Verbindung 333);

(R/S)-9-Chlor-5-(3-chlorphenyl)-1,2-dihydro-2,2,4-trimethyl-5H-chromeno[3,4-f]chinolin (Verbindung 334);

(R/S)-9-Chlor-1,2-dihydro-2,2,4-trimethyl-5-(3-methylphenyl)-5H-chromeno[3,4-f]chinolin (Verbindung 335) ;

(R/S)-9-Chlor-5-(4-chlor-3-methylphenyl)-1,2-dihydro-2,2,4-trimethyl-5H-chromeno[3,4-f]chinolin (Verbindung 336);

(R/S)-9-Chlor-1,2-dihydro-5-[3-(trifluormethyl)-phenyl]-2,2,4-trimethyl-5H-chromeno[3,4-f]chinolin (Verbindung 337);

(R/S)-9-Chlor-5-(3,5-dichlorphenyl)-1,2-dihydro-2,2,4-trimethyl-5H-chromeno[3,4-f]chinolin (Verbindung 338);

(R/S)-9-Chlor-1,2-dihydro-5-(4-methoxyphenyl)-2,2,4-trimethyl-5H-chromeno[3,4-f]chinolin (Verbindung 339) ;

(R/S)-9-Chlor-5-(3-fluor-4-methoxyphenyl)-1,2-dihydro-2,2,4-trimethyl-5H-chromeno[3,4-f]chinolin (Verbindung 340);

(R/S)-9-Chlor-5-(4-fluorphenyl)-1,2-dihydro-2,2,4-trimethyl-5H-chromeno[3,4-f]chinolin (Verbindung 341);

(R/S)-9-Chlor-5-(3-chlor-4-methoxy-5-methylphenyl)-1,2-dihydro-2,2,4-trimethyl-5H-chromeno[3,4-f]-chinolin (Verbindung 342);

(R/S)-9-Chlor-5-(4-fluor-3-methylphenyl)-1,2-dihydro-2,2,4-trimethyl-5H-chromeno[3,4-f]chinolin (Verbindung 343);

(R/S)-9-Chlor-5-(3-fluorphenyl)-1,2-dihydro-2,2,4-trimethyl-5H-chromeno[3,4-f]chinolin (Verbindung 344);

(R/S)-1,2-Dihydro-2,2,4-trimethyl-5-[3,4-methylendioxy)phenyl]-5H-chromeno[3,4-f]chinolin (Verbindung 345);

(R/S)-5-(4-Chlor-3-methylphenyl)-1,2-dihydro-2,2,4-trimethyl-5H-chromeno[3,4-f]chinolin (Verbindung 346) ;

(R/S)-5-(4-Brom-3-pyridyl)-1,2,3,4-tetrahydro-2,2-dimethyl-4-methyliden-5H-chromeno[3,4-f]chinolin (Verbindung 347);

(R/S)-5-(3,5-Difluorphenyl)-1,2-dihydro-2,2,4-trimethyl-5H-chromeno[3,4-f]chinolin (Verbindung 348);

(R/S)-5-(3,5-Dichlorphenyl)-1,2-dihydro-2,2,4-trimethyl-5H-chromeno[3,4-f]chinolin (Verbindung 349);

(R/S)-5-(3-Brom-5-methylphenyl)-1,2-dihydro-2,2,4-trimethyl-5H-chromeno[3,4-f]chinolin (Verbindung 350) ;

(R/S)-5-(3-Brom-5-fluorphenyl)-1,2-dihydro-2,2,4-trimethyl-5H-chromeno[3,4-f]chinolin (Verbindung 351);

(R/S)-5-(3-Brom-5-fluorphenyl)-1,2,3,4-tetrahydro-2,2-dimethyl-4-methyliden-5H-chromeno[3,4-f]chinolin (Verbindung 352);

(R/S)-5-[4-Fluor-3-(trifluormethyl)phenyl]-1,2-dihydro-2,2,4-trimethyl-5H-chromeno[3,4-f]chinolin (Verbindung 353);

(R/S)-9-Fluor-1,2-dihydro-2,2,4-trimethyl-5-(3-methylphenyl)-5H-chromeno[3,4-f]chinolin (Verbindung 354);

(R/S)-1,2-Dihydro-9-methoxy-2,2,4-trimethyl-5-(3-methylphenyl)-5H-chromeno[3,4-f]chinolin (Verbindung 355) ;

(R/S)-9-Fluor-5(3-fluor-4-methoxyphenyl)-1,2-dihydro-2,2,4-trimethyl-5H-chromeno[3,4-f]chinolin (Verbindung 356);

(R/S)-9-Fluor-1,2-dihydro-2,2,4-trimethyl-5-[3-(trifluormethyl)phenyl]-5H-chromeno[3,4-f]chinolin (Verbindung 357);

(R/S)-9-Fluor-5-(4-fluor-3-methylphenyl)-1,2-dihydro-2,2,4-trimethyl-5H-chromeno[3,4-f]chinolin (Verbindung 358);

(R/S)-5-(4-Chlorphenyl)-1,2,3,4-tetrahydro-2,2-dimethyl-5H-chromeno[3,4-f]-4-chinolinon (Verbindung 378) ;

(R/S)-5-(4-Chlorphenyl)-1,2,3,4-tetrahydro-2,2,3,3-tetramethyl-5H-chromeno[3,4-f]-4-chinolinon (Verbindung 379);

(R/S)-5-(4-Chlorphenyl)-1,2-dihydro-2,2-dimethyl-5H-chromeno[3,4-f]-4-chinolin (Verbindung 380); und

(R/S)-9-Chlor-1,2-dihydro-2,2,4-trimethyl-5-phenyl-5H-chromeno[3,4-f]chinolin (Verbindung 456).

**3.** Pharmazeutische Zusammensetzung, die eine Verbindung gemäß Anspruch 1 und einen pharmazeutisch akzeptablen Träger umfaßt.

**4.** Pharmazeutische Zusammensetzung nach Anspruch 3, wobei die Zusammensetzung für eine orale, topische, intravenöse, suppositorische oder parenterale Verabreichung formuliert ist.

**5.** Pharmazeutische Zusammensetzung nach Anspruch 3, wobei die Verbindung einem Patienten als eine Dosiseinheit von ungefähr 1 µg/kg an Körpergewicht bis 500 mg/kg an Körpergewicht verabreicht wird.

**6.** Pharmazeutische Zusammensetzung nach Anspruch 3, wobei die Verbindung einem Patienten als eine Dosiseinheit von ungefähr 10 µg/kg an Körpergewicht bis 250 mg/kg an Körpergewicht verabreicht wird.

**7.** Pharmazeutische Zusammensetzung nach Anspruch 3, wobei die Verbindung einem Patienten als eine Dosiseinheit von ungefähr 20 µg/kg an Körpergewicht bis 100 mg/kg an Körpergewicht verabreicht wird.

**8.** Verwendung einer Verbindung gemäß Anspruch 1 oder 2 zur Herstellung eines Medikaments, das wirksam ist bei der Behandlung und/oder der Modulation der Fertilität beim Menschen, dem Ersatz weiblicher Hormone, einer disfunktionalen Uterusblutung, einer Endometriosie, eines Leiomyoms, von Akne, des Haarausfalls beim Mann, einer Osteoporose, einer Prostatahyperplasie, von Brustkrebs, von Krebs der Eierstöcke, eines Korpuskarzinoms, von Prostatakrebs, des Kohlenhydrat-, Protein- und Lipidmetabolismus, des Elektrolyt- und Wassergleichgewichts und der Funktion des Herzkreislauf-, Nieren-, Zentralnerven-, Immun- und Skelettmuskelsystems.

**9.** Verwendung nach Anspruch 8 wobei das Medikament einen pharmazeutisch akzeptablen Träger umfaßt.

**10.** Verwendung einer Verbindung gemäß Anspruch 1 in einem Verfahrn zur Bestimmung des Vorhandenseins von einem oder mehreren Steroidrezeptoren in einer Probe, umfassend das Kombinieren einer Verbindung gemäß Anspruch 1 mit der Probe, die einen oder mehrere unbekannte Steroidrezeptoren enthält, und Bestimmen, ob die Verbindung an einen Rezeptor in der Probe bindet.

**11.** Verwendung einer Verbindung gemäß Anspruch 1 in einem Verfahren zur Reinigung von Steroidrezeptoren, umfasend das Kombinieren einer Verbindung gemäß Anspruch 1 mit einer Probe, die Steroidrezeptoren enthält, Binden-Lassen der Verbindung an die Steroidrezeptoren und Abtrennen der verbundenen Kombination der Verbindung und der Steroidrezeptoren.

## Revendications

**1.** Composé ayant les formules :

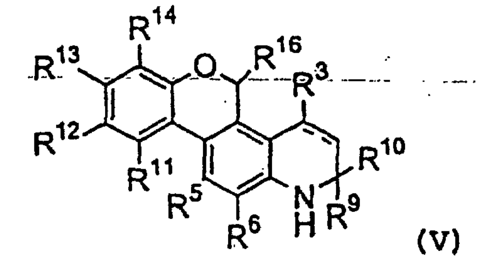

(V)

OU

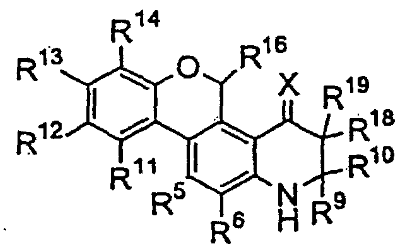

(VI)

dans lesquelles :

$R^3$ est un hydrogène ou un méthyle ;
$R^5$ et $R^6$ sont chacun un hydrogène ;
$R^9$ et $R^{10}$ sont chacun un méthyle;
$R^{11}$ à $R^{14}$ sont chacun indépendamment un hydrogène, F, Cl, Br, I, $COR^2$, un $C_1$-$C_4$ alkyle, $CF^3$ ou $OR^2$ où $R^2$ est un hydrogène, un $C_1$-$C_4$ alkyle ou $CF^3$ ;
X est $CH_2$ ou O ;
$R^{16}$ est un hydrogène, OH, $OR^{17}$, $SR^{17}$, un $C_1$-$C_6$ alkyle, un $C_2$-$C_6$ alcényle, un $C_2$-$C_6$ alcynyle, un phénylméthyle, un phényle facultativement substitué par un $C_1$-$C_4$ alkyle, F, Cl, Br, I, $CF_3$, un $C_1$-$C_4$ alcoxyle ou un diméthyl amino ; un pyridyle facultativement substitué par un méthyle, F, Cl, Br, $CF_3$ ou un $C_1$-$C_4$ alcoxy ; un furanyle facultativement substitué par un $C_1$-$C_4$ alkyle ou un $C_1$-$C_4$ alcoxy ; un thiényle facultativement substitué par un $C_1$-$C_4$ alkyle ou un $C_1$-$C_4$ alcoxy ; un pyrrolyle facultativement substitué par un $C_1$-$C_4$ alkyle ; un 1-triazolyle; un 2-benzofuranyle ; ou un 2-benzothiényle ; où $R^{17}$ est un groupe $C_1$-$C_4$ alkyle ou haloalkyle ;
$R^{18}$ et $R^{19}$ sont tous deux un hydrogène ou tous deux un méthyle; et
les lignes pointillées dans les structures représentent des doubles liaisons facultatives.

**2.** Un composé récepteur de la progestérone selon la revendication 1, sélectionné dans le groupe constitué par
(R/S)-5-Butyl-1,2-dihydro-2,2,4-triméthyl-5H-chroméno[3,4-f]quinoléine (Composé 160) ;
(R/S)-1,2-Dihydro-2,2,4-triméthyl-5-phényl-5H-chroméno[3,4-f]quinoléine (Composé 161);
(R/S)-1,2,3,4-Tétrahydro-2,2-diméthyl-4-méthylidène-5-phényl-5H-chroméno[3,4-f]quinoléine (Composé 162) ;
(R/S)-5-(4-Chlorophényl)-1,2-dihydro-2,2,4-triméthyl-5H-chroméno[3,4f]quinoléine (Composé 163) ;
(R/S)-5-(4-Chlorophényl)-1,2,3,4-tétrahydro-2,2-diméthyl-4-méthylidène-5H-chroméno[3,4-f]quinoléine (Composé 164);
(R/S)-5-(4-Fluorophényl)-1,2-dihydro-2,2,4-triméthyl-5H-chroméno[3,4-f]quinoléine (Composé 165) ;
(R/S)-5-(4-Acétylphényl)-1,2-dihydro-2,2,4-triméthyl-5H-chroméno[3,4-f]quinoléine (Composé 166) ;
(R/S)-1,2-Dihydro-2,2,4-triméthyl-5-(4-méthylphényl)-5H-chroméno[3,4-f]quinoléine (Composé 167) ;
(R/S)-1,2-Dihydro-5-(4-méthoxyphényl)-2,2,4-triméthyl-5H-chroméno[3,4-f]quinoléine (Composé 168) ;
(R/S)-1,2-Dihydro-2,2,4-triméthyl-5-[4-(trifluorométhyl)phényl]-5H-chroméno[3,4-f]quinoléine (Composé 169) ;
(R/S)-1,2-Dihydro-2,2,4-triméthyl-5-(thiophèn-3-yl)-5H-chroméno[3,4-f]quinoléine(Composé 170) ;
(-)-1,2-Dihydro-2,2,4-triméthyl-5-(4-méthylphényl)-5H-chroméno[3,4-f]quinoléine (Composé 171) ;
(-)-5-(4-Chlorophényl)-1,2-dihydro-2,2,4-triméthyl-5H-chroméno[3,4-f]quinoléine (Composé 172) ;
(R/S)-1,2-Dihydro-2,2,4-triméthyl-5-(3-méthylphényl)-5H-chroméno[3,4-f]quinoléine (Composé 173) ;
(+)-(41,51)-5-(4-Chlorophényl)-1,2,3,4-tétrahydro-2,2,4-triméthyl-5H-chroméno[3,4-f]quinoléine (Composé 174) ;

(-)-(41,51)-5-(4-Chlorophényl)-1,2,3,4-tétrahydro-2,2,4-triméthyl-5H-chroméno[3,4-f]quinoléine (Composé 175) ;
(R/S-4l,5u)-5-(4-Chlorophényl)-1,2,3,4-tétrahydro-2,2,4-triméthyl-5H-chroméno[3,4-f]quinoléine (Composé 176) ;
(R/S)-5-(3-Chlorophényl)-1,2-dihydro-2,2,4-triméthyl-5H-chroméno[3,4-f]quinoléine (Composé 177) ;
(R/S)-5-(3-Chlorophényl)-1,2,3,4-tétrahydro-2,2-diméthyl-4-méthylidène-5H-chroméno[3,4-f]quinoléine (Composé 178) ;
(R/S)-5-(4-Bromophényle)-1,2-dihydro-2,2,4-triméthyl-5H-chroméno[3,4-f]quinoléine (Composé 179) ;
(R/S)-5-(4-Bromophényl)-1,2,3,4-tétrahydro-2,2-diméthyl-4-méthylidène-5H-chroméno[3,4-f]quinoléine (Composé 180) ;
(R/S)-5-(3-Bromophényl)-1,2-dihydro-2,2,4-triméthyl-5H-chroméno[3,4-f]quinoléine (Composé 181) ;
(R/S)-5-(3-Bromophényl)-1,2,3,4-tétrahydro-2,2-diméthyl-4-méthylidène-5H-chroméno[3,4-f]quinoléine (Composé 182) ;
(R/S)-5-(3,4-Dichlorophényl)-1,2-dihydro-2,2,4-triméthyl-5H-chroméno[3,4-f]quinoléine (Composé 183) ;
(R/S)-5-(3-Bromo-2-pyridyl)-1,2-dihydro-2,2,4-triméthyl-5H-chroméno[3,4-f]quinoléine (Composé 184) ;
(R/S)-1,2-Dihydro-5-hydroxy-2,2,4-triméthyl-5H-chroméno[3,4-f]quinoléine (Composé 185) ;
(R/S)-1,2-Dihydro-2,2,4-triméthyl-5-méthoxy-5H-chroméno[3,4-f]quinoléine (Composé 186);
(R/S)-1,2-Dihydro-2,2,4-triméthyl-5-propoxy-5H-chroméno[3,4-f]quinoléine (Composé 187) ;
(R/S)-5-Allyl-1,2-dihydro-2,2,4-5H-chroméno[3,4-f]quinoléine (Composé 188) ;
(R/S)-1,2-Dihydro-2,2,4-triméthyl-5-propyl-5H-chroméno[3,4-f]quinoléine (Composé 189);
(R/S)-1,2-Dihydro-2,2,4-triméthyl-5-(2-pyridyl)-5H-chroméno[3,4-f]quinoléine (Composé 190) ;
(R/S)-5-(3-Fluorophényl)-1,2-dihydro-2,2,4-triméthyl-5H-chroméno[3,4-f]quinoléine (Composé 191);
(R/S)-5-(3-Fluorophényl)-1,2,3,4-tétrahydro-2,2-diméthyl-4-méthylidène-5H-chroméno[3,4-f]quinoléine (Composé 192) ;
(R/S)-1,2-Dihydro-2,2,4-triméthyl-5-propylthio-5H-chroméno[3,4-f]quinoléine (Composé 193) ;
(R/S)-1,2-Dihydro-5-(3-méthoxyphényl)-2,2,4-triméthyl-5H-chroméno[3,4-f]quinoléine (Composé 194) ;
(R/S)-1,2-Dihydro-2,2,4-triméthyl-5-[3-(trifluorométhyl)phényl]-5H-chroméno[3,4-f]quinoléine (Composé 195) ;
(R/S)-5-(3-Fluoro-4-méthylphényl)-1,2-dihydro-2,2,4-triméthyl-5H-chroméno[3,4f]quinoléine (Composé 196) ;
(R/S)-5-(4-Bromo-3-pyridyl)-1,2-dihydro-2,2,4-triméthyl-5H-chroméno[3,4-f]quinoléine (Composé 197) ;
(R/S)-1,2-Dihydro-2,2,4-triméthyl-5-(3-pyridyl)-5H-chroméno[3,4-f]quinoléine (Composé 198) ;
(R/S)-5-(4-Chloro-3-fluorophényl)-1,2-dihydro-2,2,4-triméthyl-5H-chroméno[3,4-f]quinoléine (Composé 199) ;
(R/S)-1,2-Dihydro-2,2,4,5-tétraméthyl-5H-chroméno[3,4-f]quinoléine (Composé 200) ;
(R/S)-1,2-Dihydro-5-héxyl-2,2,4-triméthyl-5H-chroméno[3,4f]quinoléine (Composé 201) ;
1,2-Dihydro-2,2,4-triméthyl-5H-chroméno[3,4-f]quinoléine (Composé 202) ;
(R/S)-1,2-Dihydro-5-(3-méthylbutyl)-2,2,4-triméthyl-5H-chroméno[3,4-f]quinoléine (Composé 203) ;
(R/S)-5-(4-Chlorobutyl)-1,2-dihydro-2,2,4-triméthyl-5H-chroméno[3,4-f]quinoléine (Composé 204) ;
(R/S)-5-Benzyl-1,2-dihydro-2,2,4-triméthyl-5H-chroméno[3,4-f]quinoléine (Composé 205) ;
(R/S)-5-(4-)Bromobutyl)-1,2-dihydro-2,2,4-triméthyl-5H-chroméno[3,4-f]quinoléine (Composé 206) ;
(R/S)-5-Butyl-9-fluoro-1,2-dihydro-2,2,4-triméthyl-5H-chroméno[3,4-f]quinoléine (Composé 210) ;
(R/S)-5-Butyl-8-fluoro-1,2-dihydro-2,2,4-triméthyl-5H-chroméno[3,4-f]quinoléine (Composé 211);
(R/S)-5-(3-Chlorophényl)-9-fluoro-1,2-dihydro-2,2,4-triméthyl-5H-chroméno[3,4-f]quinoléine (Composé 212) ;
(R/S)-5-(4-Chloro-3-méthylphényl)-9-fluoro-1,2-dihydro-2,2,4-triméthyl-5H-chroméno[3,4-f]quinoléine (Composé 213) ;
(R/S)-5-(4-Chlorophényl)-9-fluoro-1,2-dihydro-2,2,4-triméthyl-5H-chroméno[3,4-f]quinoléine (Composé 214) ;
(R/S)-9-Fluoro-1,2-dihydro-5-(4-méthoxyphényl)-2,2,4-triméthyl-5H-chroméno[3,4-f]quinoléine (Composé 215) ;
(R/S)-8-Fluoro-1,2-dihydro-5-méthoxyl-2,2,4-triméthyl-5H-chroméno[3,4-f]quinoléine (Composé 216) ;
(R/S)-5-(4-Chlorophényl)-8-fluoro-1,2-dihydro-2,2,4-triméthyl-5H-chroméno[3,4-f]quinoléine (Composé 217) ;
(R/S)-9-Chloro-5-(4-Chlorophényl)-1,2-dihydro-2,2,4-triméthyl-5H-chroméno[3,4-f]quinoléine (Composé 218) ;
(R/S)-5-(3-Fluorobenzyl)-1,2-dihydro-2,2,4-triméthyl-5H-chroméno[3,4-f]quinoléine (Composé 318) ;
(R/S)-9-Chloro-1,2-dihydro-5-méthoxy-2,2,4-triméthyl-5H-chroméno[3,4-f]quinoléine (Composé 319) ;
9-Chloro-1,2-dihydro-2,2,4-triméthyl-5H-chroméno[3,4-f]quinoléine (Composé 320) ;
(R/S)-9-Chloro-1,2-dihydro-2,2,4-triméthyl-5-propyloxy-5H-chroméno[3,4-f]quinoléine (Composé 321) ;
(R/S)-9-Fluoro-1,2-dihydro-5-méthoxy-2,2,4-triméthyl-5H-chroméno[3,4-f]quinoléine (Composé 322) ;
(R/S)-9-Fluoro-1,2-dihydro-2,2,4-triméthyl-5-thiopropoxy-5H-chroméno[3,4-f]quinoléine (Composé 323) ;
(R/S)-9-Fluoro-1,2-dihydro-2,2,4-triméthyl-5-propoxy-5H-chroméno[3,4-f]quinoléine (Composé 324) ;
(R/S)-5-Butyl-9-chloro-1,2-dihydro-2,2,4-triméthyl-5H-chroméno[3,4-f]quinoléine (Composé 325) ;
(R/S)-5-Butyl-1,2-dihydro-9-méthoxy-2,2,4-triméthyl-5H-chroméno[3,4-f]quinoléine (Composé 326) ;
(R/S)-9-Fluoro-1,2-dihydro-2,2,4,5-tétraméthyl-5H-chroméno[3,4-f]quinoléine (Composé 327) ;
9-Fluoro-1,2-dihydro-2,2,4-triméthyl-5H-chroméno[3,4-f]quinoléine (Composé 328) ;
1,2-Dihydro-9-méthoxy-2,2,4-triméthyl-5H-chroméno[3,4-f]quinoléine (Composé 329) ;

1,2-Dihydro-2,2,4,9-tétraméthyl-5H-chroméno[3,4-f]quinoléine (Composé 330) ;
7-Chloro-1,2-dihydro-2,2,4-triméthyl-5H-chroméno[3,4-f]quinoléine (Composé 331) ;
(R/S)-9-Chloro-1,2-dihydro-2,2,4,5-tétraméthyl-5H-chroméno[3,4-f]quinoléine (Composé 332) ;
(R/S)-5-(4-Bromophényl)-9-chloro-1,2-dihydro-2,2,4-triméthyl-5H-chroméno[3,4-f]quinoléine (Composé 333) ;
(R/S)-9-Chloro-5-(3-Chlorophényl)-1,2-dihydro-2,2,4-triméthyl-5H-chroméno[3,4-f]quinoléine (Composé 334) ;
(R/S)-9-Chloro-1,2-dihydro-2,2,4-triméthyl-5-(3-méthylphényl)-5H-chroméno[3,4-f]quinoléine (Composé 335) ;
(R/S)-9-Chloro-5-(4-chloro-3-méthylphényl)-1,2-dihydro-2,2,4-triméthyl-5H-chroméno[3,4-f]quinoléine (Composé 336) ;
(R/S)-9-Chloro-1,2-dihydro-5-[3-(trifluorométhyl)phényl]-2,2,4-triméthyl-5H-chroméno[3,4-f]quinoléine (Composé 337) ;
(R/S)-9-Chloro-5-(3,5-dichlorophényl)-1,2-dihydro-2,2,4-triméthyl-5H-chroméno[3,4-f]quinoléine (Composé 338) ;
(R/S)-9-Chloro-1,2-dihydro-5-(4-méthoxyphényl)-2,2,4-triméthyl-5H-chroméno[3,4-f]quinoléine (Composé 339);
(R/S)-9-Chloro-5-(3-fluoro-4-méthoxyphényl)-1,2-dihydro-2,2,4-triméthyl-5H-chroméno[3,4-f]quinoléine (Composé 340);
(R/S)-9-Chloro-5-(4-fluorophenyl)-1,2-dihydro-2,2,4-triméthyl-5H-chroméno[3,4-f]quinoléine (Composé 341) ;
(R/S)-9-Chloro-5-(3-Chloro-4-méthoxy-5-méthylphényl)-1,2-dihydro-2,2,4-triméthyl-5H-chroméno[3,4-f]quinoléine (Composé 342) ;
(R/S)-9-Chloro-5-(4-fluoro-3-méthylphényl)-1,2-dihydro-2,2,4-triméthyl-5H-chroméno[3,4-f]quinoléine (Composé 343) ;
(R/S)-9-Chloro-5-(3-fluorophényl)-1,2-dihydro-2,2,4-triméthyl-5H-chroméno[3,4-f]quinoléine (Composé 344) ;
(R/S)-1,2-Dihydro-2,2,4-triméthyl-5-[3,4-méthylènedioxy)phényl)-5H-chroméno[3,4-f]quinoléine (Composé 345) ;
(R/S)-5-(4-Chloro-3-méthylphényl)-1,2-dihydro-2,2,4-triméthyl-5H-chroméno[3,4-f]quinoléine (Composé 346) ;
(R/S)-5-(4-Bromo-3-pyridyl)-1,2,3,4-tétrahydro-2,2-diméthyl-4-méthylidène-5H-chroméno[3,4-f]quinoléine (Composé 347);
(R/S)-5-(3,5-Difluorophényl)-1,2-dihydro-2,2,4-triméthyl-5H-chroméno[3,4-f]quinoléine (Composé 348) ;
(R/S)-5-(3,5-Dichlorophényl)-1,2-dihydro-2,2,4-triméthyl-5H-chroméno[3,4-f]quinoléine (Composé 349) ;
(R/S)-5-(3-Bromo-5-méthylphényl)-1,2-dihydro-2,2,4-triméthyl-5H-chroméno[3,4-f]quinoléine (Composé 350) ;
(R/5)-5-(3-Bromo-5-fluorophényl)-1,2-dihydro-2,2,4-triméthyl-5H-chroméno[3,4-f]quinoléine (Composé 351) ;
(R/S)-5-(3-Bromo-5-fluorophényl)-1,2,3,4-tétrahydro-2,2-diméthyl-4-méthylidène-5H-chroméno[3,4-f]quinoléine (Composé 352) ;
(R/S)-5-[4-Fluoro-3-(trifluorométhyl)phényl]-1,2-dihydro-2,2,4-triméthyl-5H-chroméno[3,4-f]quinoléine (Composé 353) ;
(R/S)-9-Fluoro-1,2-dihydro-2,2,4-triméthyl-5-(3-méthylphényl)-5H-chroméno[3,4-f]quinoléine (Composé 354) ;
(R/5)-1,2-Dihydro-9-méthoxy-2,2,4-triméthyl-5-(3-méthylphényl)-5H-chroméno[3,4-f]quinoléine (Composé 355) ;
(R/S)-9-Fluoro-5(3-fluoro-4-méthoxyphényl)-1,2-dihydro-2,2,4-triméthyl-5H-chroméno[3,4-f]quinoléine (Composé 356) ;
(R/S)-9-Fluoro-1,2-dihydro-2,2,4-triméthyl-5-[3-(trifluorométhyl)phényl]-5H-chroméno[3,4-f]quinoléine (Composé 357) ;
(R/S)-9-Fluora-5-(4-fluoro-3-méthylphényl)-1,2-dihydro-2,2,4-triméthyl-5H-chroméno[3,4-f]quinoléine (Composé 358) ;
(R/S)-5-(4-Chlorophényl)-1,2,3,4-tétrahydro-2,2-diméthyl-5H-chroméno[3,4-f]-4-quinolinone (Composé 378) ;
(R/S)-5-(4-Chlorophényl)-1,2,3,4-tétrahydro-2,2,3,3-tétraméthyl-5H-chroméno[3,4-f]-4-quinolinone (Composé 379) ;
(R/S)-5-(4-Chlorophényl)-1,2-dihydro-2,2-diméthyl-5H-chroméno[3,4-f]-4-quinoléine (Composé 380) ; et
(R/S)-9-Chloro-1,2-dihydro-2,2,4-triméthyl-5-phényl-5H-chroméno[3,4-f]quinoléine (Composé 456).

**3.** Composition pharmaceutique comprenant un composé selon la revendication 1 et un excipient pharmaceutiquement acceptable.

**4.** Composition pharmaceutique selon la revendication 3, dans laquelle la composition est formulée pour une administration orale, topique, intraveineuse, parentérale ou par suppositoire.

**5.** Composition pharmaceutique selon la revendication 3, dans laquelle le composé est administré à un patient sous la forme d'une unité posologique d'environ 1 µg/kg de poids corporel à 500 mg/kg de poids corporel.

**6.** Composition pharmaceutique selon la revendication 3, dans laquelle le composé est administré à un patient sous la forme d'une unité posologique d'environ 10 µg/kg de poids corporel et 250 mg/kg de poids corporel.

7. Composition pharmaceutique selon la revendication 3, dans laquelle le composé est administré à un patient sous la forme d'une unité posologique d'environ 20 µg/kg de poids corporel et 100 mg/kg de poids corporel.

8. Utilisation d'un composé selon la revendication 1 ou 2 pour la préparation d'un médicament qui est efficace pour le traitement et/ou la modulation de la fertilité humaine, de l'hormonothérapie substitutive féminine, de la ménométrorragie fonctionnelle, de l'endométriose, du léiomyome, de l'acné, de la calvitie hippocratique, de l'ostéoporose, de l'hyperplasie prostatique, du cancer du sein, du cancer de l'ovaire, du cancer de l'endomètre, du cancer de la prostate, du métabolisme des glucides, des protéines et des lipides, de l'équilibre des électrolytes et de l'eau et du fonctionnement des systèmes cardio-vasculaire, rénal, nerveux central, immunitaire et des muscles squelettiques.

9. Utilisation selon la revendication 8, dans laquelle le médicament comprend un excipient pharmaceutiquement acceptable.

10. Utilisation d'un composé selon la revendication 1 dans un procédé destiné à déterminer la présence d'un ou de plusieurs récepteurs des stéroïdes dans un échantillon, comprenant l'action de combiner un composé selon la revendication 1 avec l'échantillon contenant un ou plusieurs récepteurs des stéroïdes inconnus et l'action de déterminer si ledit composé se lie à un récepteur dans l'échantillon.

11. Utilisation d'un composé selon la revendication 1 dans un procédé de purification de récepteurs des stéroïdes, comprenant l'action de combiner un composé selon la revendication 1 avec un échantillon contenant des récepteurs des stéroïdes, l'action de laisser ledit composé se lier aux dits récepteurs des stéroïdes et l'action de séparer la combinaison liée dudit composé et desdits récepteurs de stéroïdes.